(19) Europäisches Patentamt
European Patent Office
Office européen des brevets

(11) **EP 4 103 189 B1**

(12) **EUROPEAN PATENT SPECIFICATION**

(45) Date of publication and mention of the grant of the patent:
**14.05.2025 Bulletin 2025/20**

(21) Application number: **21706379.1**

(22) Date of filing: **12.02.2021**

(51) International Patent Classification (IPC):
***A61K 31/522*** *(2006.01)* ***A61K 31/513*** *(2006.01)*
***A61K 31/53*** *(2006.01)* ***A61K 31/4025*** *(2006.01)*
***A61K 31/436*** *(2006.01)* ***A61K 31/4365*** *(2006.01)*
***A61K 31/4375*** *(2006.01)* ***A61K 31/497*** *(2006.01)*
***A61K 31/498*** *(2006.01)* ***A61K 39/395*** *(2006.01)*
***A61K 45/06*** *(2006.01)* ***A61P 35/00*** *(2006.01)*
***A61P 35/04*** *(2006.01)*

(52) Cooperative Patent Classification (CPC):
(C-Sets available)
**A61K 31/522; A61K 31/4025; A61K 31/436; A61K 31/4365; A61K 31/4375; A61K 31/497; A61K 31/498; A61K 31/513; A61K 31/53; A61K 31/553; A61K 45/06; A61P 35/00; A61P 35/04;** A61K 39/395; C07K 16/2818; (Cont.)

(86) International application number:
**PCT/GB2021/050360**

(87) International publication number:
**WO 2021/161047 (19.08.2021 Gazette 2021/33)**

# (54) USP7 INHIBITOR FOR USE IN THE TREATMENT OF CANCER

USP7-INHIBITOREN ZUR VERWENDUNG IN DER BEHANDLUNG VON KREBS

INHIBITEUR D’USP7 DESTINÉ À ÊTRE UTILISÉ DANS LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2020 GB 202001980**

(43) Date of publication of application:
**21.12.2022 Bulletin 2022/51**

(73) Proprietor: **Almac Discovery Limited**
**Craigavon BT63 5QD (GB)**

(72) Inventors:
- **JACQ, Xavier**
  **Craigavon BT63 5QD (GB)**
- **LOBB, Ian Thomas**
  **Craigavon BT63 5QD (GB)**
- **WAPPETT, Mark**
  **Craigavon BT63 5QD (GB)**
- **JURISIC, Anamarija**
  **Craigavon BT63 5QD (GB)**
- **ROUNTREE, James Samuel Shane**
  **Craigavon BT63 5QD (GB)**
- **HELM, Matthew Duncan**
  **Craigavon BT63 5QD (GB)**

(74) Representative: **Mewburn Ellis LLP**
**City Tower**
**40 Basinghall Street**
**London EC2V 5DE (GB)**

(56) References cited:
**WO-A1-2018/073602 US-B1- 9 717 717**

- **KUMAR SURESH ET AL: "Abstract 1691: USP7 inhibitors impair Foxp3+ T regulatory cell function and promote antitumor immunity against solid tumors | Cancer Research", CANCER RESEARCH, 1 July 2017 (2017-07-01), XP055801763, Retrieved from the Internet <URL:https://cancerres.aacrjournals.org/content/77/13_Supplement/1691> [retrieved on 20210505]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WANG LIQING ET AL: "Ubiquitin-specific Protease-7 Inhibition Impairs Tip60-dependent Foxp3 + T-regulatory Cell Function and Promotes Antitumor Immunity", EBIOMEDICINE, vol. 13, 1 November 2016 (2016-11-01), NL, pages 99 - 112, XP055801744, ISSN: 2352-3964, Retrieved from the Internet <URL:https://www.ncbi.nlm.nih.gov/pmc/articles/PMC5264272/pdf/main.pdf> DOI: 10.1016/j.ebiom.2016.10.018**
- **JACQ XAVIER ET AL: "Abstract 6057: Discovery of a novel function for USP7 inhibitors: Reprogramming the tumor microenvironment | Cancer Research", CANCER RESEARCH, 1 August 2020 (2020-08-01), XP055801714, Retrieved from the Internet <URL:https://cancerres.aacrjournals.org/content/80/16_Supplement/6057> [retrieved on 20210505]**


(52) Cooperative Patent Classification (CPC): (Cont.)
C07K 16/2827

C-Sets
**A61K 31/4025, A61K 2300/00;**
**A61K 31/436, A61K 2300/00;**
**A61K 31/4365, A61K 2300/00;**
**A61K 31/4375, A61K 2300/00;**
**A61K 31/497, A61K 2300/00;**
**A61K 31/498, A61K 2300/00;**
**A61K 31/513, A61K 2300/00;**
**A61K 31/522, A61K 2300/00;**
**A61K 31/53, A61K 2300/00;**
A61K 39/395, A61K 2300/00

## Description

### FIELD OF INVENTION

The present invention relates to the treatment of cancer by inhibition of USP7 activity in fibroblasts. Treatment of cancer in accordance with the invention can be through, for example, inhibition of extra-cellular matrix remodelling in the tumour microenvironment, inhibition of VEGF, inhibition of angiogenesis or metastasis, modulation of the immune system, or a combination thereof.

### BACKGROUND

During the transition from healthy tissue to cancer and then on to metastasis, the tumour microenvironment (TME) initiates crucial changes that licenses the formation and growth of primary tumours, and the progression from primary tumours to aggressive and metastatic disease.
Influencing the specific factors and molecular mechanisms underpinning these transitions in the TME provides new routes for improving the potency and scope of cancer therapy strategies.
USP7 has emerged as a potential therapeutic target due to its key role in regulation of oncogenic pathways and thus the potential to target USP7 in order to inhibit the cancer cell proliferation.
No effect has previously been described of USP7 inhibition on fibroblasts or on the tumour microenvironment.

### SUMMARY OF INVENTION

The invention is set out in the appended claims.
Any references in the description to methods of treatment by therapy or surgery or in vivo diagnosis methods refer to the compounds, pharmaceutical compositions and medicaments of the present invention for use in those methods.
It is demonstrated herein for the first time that USP7 plays a critical role in affecting the tumour microenvironment. The effects of USP7 inhibition demonstrated herein include promoting remodelling of the extracellular matrix (ECM), thereby promoting tumour invasion and metastasis. USP7 also affects angiogenesis and VEGF levels both systemically and in the tumour microenvironment (TME). Notably, it is demonstrated herein that USP7 inhibition, particularly in the fibroblast compartment of the TME, leads to a significant decrease in both cell invasion and angiogenesis. USP7 inhibition also results in modulation of the tumour immune environment (e.g. by promoting infiltration of CD8 T cells). Significantly, *in vivo* USP7 inhibition inhibits tumour growth in cell models that are not affected by direct inhibition by USP7 inhibitors *in vitro.* Thus, provided herein is a newly discovered means of treating cancer by inhibiting USP7 in fibroblasts that is independent from the previously-identified role of USP7 in driving tumorigenesis directly in the cancer cells.
Accordingly, in one aspect is provided a method of treating cancer by inhibiting USP7 activity in fibroblasts, the method comprising administering to a subject in need thereof a composition comprising a USP7 inhibitor.
In certain embodiments, administration of the composition comprising the USP7 inhibitor treats the cancer by inhibiting USP7 activity in cancer-associated fibroblasts (CAFs).
It is demonstrated herein that inhibition of USP7 in fibroblasts reduces the tumorigenic effects of fibroblasts, for example by one or more of reducing cell invasion; reducing MMP secretion; reducing basement membrane degradation; and reducing systemic and TME VEGF levels.
Thus, in certain embodiments, administration of the composition comprising the USP7 inhibitor treats the cancer by reducing the level of VEGF in the serum of the subject. In certain embodiments administration of the composition comprising the USP7 inhibitor treats the cancer by reducing the level of VEGF in the tumour microenvironment.
In certain embodiments, administration of the composition comprising the USP7 inhibitor treats the cancer by inhibiting production of VEGF by cancer-associated fibroblasts (CAFs).
In a further aspect is provided a method of treating cancer by modulating the tumour immune environment, the method comprising administering to a subject in need thereof a composition comprising a USP7 inhibitor, wherein administration of the USP7 inhibitor modulates the tumour immune environment.
In a further aspect is provided a method of treating cancer by increasing tumour infiltrating lymphocytes (TILs), the method comprising administering to a subject in need thereof a composition comprising a USP7 inhibitor, wherein administration of the USP7 inhibitor increases the number of TILs, preferably CD8+ TILs.
In a further aspect is provided a method of treating cancer by decreasing the proportion of Treg cells relative to CD8+ T cells in the TME, the method comprising administering to a subject in need thereof a composition comprising a USP7 inhibitor, wherein administration of the USP7 inhibitor decreases the proportion of Treg cells relative to CD8+ T cells in the TME.
In a further aspect is provided a method of treating cancer by decreasing the number of macrophages in the TME, the method comprising administering to a subject in need thereof a composition comprising a USP7 inhibitor, wherein

administration of the USP7 inhibitor decreases the number of macrophages in the TME.

In a further aspect is provided a method of treating cancer by administering to a subject in need thereof a combination therapy, the combination therapy comprising a composition comprising a USP7 inhibitor and composition comprising an immune checkpoint inhibitor.

In a further aspect is provided a USP7 inhibitor for use in a method of treating cancer, the method comprising administering to a subject in need thereof a combination therapy, the combination therapy comprising a composition comprising the USP7 inhibitor and a composition comprising an immune checkpoint inhibitor.

In a further aspect is provided an immune checkpoint inhibitor for use in a method of treating cancer, the method comprising administering to a subject in need thereof a combination therapy, the combination therapy comprising a composition comprising a USP7 inhibitor and a composition comprising the immune checkpoint inhibitor.

In a further aspect is provided a combination therapy for use in a method of treating cancer, the method comprising administering to a subject in need thereof the combination therapy, the combination therapy comprising a composition comprising a USP7 inhibitor and a composition comprising an immune checkpoint inhibitor.

In certain embodiments, administration of the composition comprising the USP7 inhibitor treats the cancer by inhibiting extra-cellular matrix (ECM) remodelling by cancer-associated fibroblasts. In such embodiments, the ECM is the ECM of the tumour microenvironment.

In certain embodiments, administration of the composition comprising the USP7 inhibitor treats the cancer by inhibiting degradation of the basement membrane, optionally degradation of the tubular basement membrane.

In certain embodiments, administration of the composition comprising the USP7 inhibitor treats the cancer by inhibiting angiogenesis, optionally neo-angiogenesis. In such embodiments, angiogenesis is inhibited in the tumour microenvironment.

In certain embodiments, administration of the composition comprising the USP7 inhibitor treats the cancer by modulation of the tumour immune environment (e.g. by promoting infiltration of CD8 T cells).

In certain embodiments, the composition is administered at a dose that achieves an inhibition of tumour growth.

In certain embodiments, the composition is administered at a dose that achieves an inhibition of tumour invasion.

In certain embodiments, the composition is administered at a dose that achieves an inhibition of tumour metastasis.

In certain embodiments, the composition is administered at a dose that achieves modulation of the tumour immune environment.

In certain embodiments, the cancer treated by the method is formed of cancer cells, and the cancer cells are resistant to the USP7 inhibitor. In certain embodiments, the cancer treated by the method is formed of cancer cells, and the cancer cells are resistant to the USP7 inhibitor *in vitro.*

In a further aspect is provided a USP7 inhibitor for use in a method of treating cancer according to the invention.

Each aspect or embodiment provided herein may be combined with any other aspect(s) or embodiment(s) unless indicated to the contrary. In particular, any feature indicated as being preferred or advantageous may be combined with any other feature or features, most-favourably those indicated as being preferred or advantageous.

## FIGURES

**Figure 1:** USP7 inhibition decreases sVEGF levels in fibroblasts: co-culture of primary dermal fibroblasts and PBMCs. Co-cultures of primary human dermal fibroblasts with activated PBMCs were treated with vehicle (DMSO) and various concentrations of AD-04 for 48 hours. Biomarker levels were measured using ELISA and presented as log-transformed ratios.

**Figure 2:** USP7 inhibition decreases sVEGF levels in fibroblasts: co-culture of primary dermal fibroblasts, PBMCs and cancer cells. Co-cultures of primary human fibroblasts with Non-Small Lung Cancer or colorectal cancer cells were treated with vehicle (DMSO) and various concentrations of AD-04. Biomarker levels were measured using ELISA and presented as log-transformed ratios.

**Figure 3:** USP7 inhibition decreases secreted VEGF levels in fibroblasts: co-culture of cancer cells, activated PBMCs and primary dermal or lung fibroblasts. HT29 cells co-cultured with primary human lung (WI-38 and IMR-90) or dermal (HDF) fibroblasts and PBMCs stimulated with anti-CD3/CD28 antibodies were treated with AD-04 and *ent*-AD-04 at indicated concentrations for 48h. VEGF levels released to cell culture media was measured by ELISA.

**Figure 4:** USP7 inhibitor does not modulate secreted VEGF levels in cancer cells: the modulation of VEGF secretion by USP7 inhibitors observed in primary fibroblasts does not occur in tumour cells. HT29, H1299, LNCaP and MCF-7 cells were treated with AD-04 and ent-AD-04 at indicated concentrations for 48h. Levels of sVEGF released to cell culture media was measured by ELISA.

**Figure 5:** USP7 inhibition decreased secreted VEGF levels in activated fibroblasts. The USP7-mediated decrease in VEGF secretion observed in the co-cultures can be solely attributed to modulation of secreted VEGF in primary fibroblasts. WI-38, IMR-90 and HDF were activated with CD3/CD28 stimulated PBMCs, FGF-2, or TGF-β and treated with AD-04 and *ent*-AD-04 at indicated concentrations for 48h. VEGF levels released to cell culture media was measured by ELISA.

**Figure 6:** USP7 inhibition decreased secreted VEGF levels in cancer-associated fibroblasts. Cancer-associated fibroblasts secrete VEGF and thus further activation is not required to observe a USP7-mediated decrease in VEGF secretion. Primary colorectal adenocarcinoma-associated fibroblasts were treated with AD-04 and ent-AD-04 at indicated concentrations for 48h. VEGF levels released to cell culture media was measured by ELISA.

**Figure 7:** USP7 inhibition-mediated decrease in VEGF secretion is dependent on a functional p53 pathway. Only in cells with a functional and wild-type p53 pathway VEGF secretion can be down-regulated by USP7 inhibition. SV40 immortalized fibroblasts WI-38-VA13 with destabilized p53 gene were treated with vehicle (DMSO), AD-04 and *ent*-AD-04 at indicated concentrations for 48h followed by VEGF detection by ELISA.

**Figure 8:** In addition to the modulation of secreted VEGF, USP7 inhibition also decreases intracellular VEGF levels in primary fibroblast lysates but not in cancer cells or immortalized fibroblasts. Cells were treated with vehicle (DMSO) and AD-04 for 48h, lysed and intracellular VEGF was measured by ELISA.

**Figure 9:** USP7 inhibition decreases VEGF levels in fibroblasts under hypoxia. The necessary activation of fibroblast required for observing USP7-mediated decrease in VEGF observed upon incubation with growth factors (FGF or TGFβ) or activated PBMCs can also be recapitulated by hypoxia. Fibroblasts were treated with indicated concentrations of AD-04 and *ent*-AD-04 for 48h in a hypoxic chamber. Supernatants were collected and assayed for VEGF by ELISA.

**Figure 10:** USP7 inhibition reduces HIF-1α half-life in primary human fibroblasts. Secreted VEGF is known to be under the control of the HIF-1α transcription factor: USP7 inhibitor-dependent decrease in VEGF secretion is mediated *via* the modulation of HIF-1α stability. Hypoxia-cultured primary fibroblasts were pretreated with cycloheximide (100 μg/mL) and subsequently exposed to AD-04 (1μM) or vehicle (DMSO). Cells were lysed at the indicated time points (from 0 to 60 min) and samples analysed by immuno-blotting probing for HIF-1α. HIF-1α half-life was quantified by densitometry analysis. β-Actin was included as a normalization control. These data are representative data from at least two independent experiments.

**Figure 11:** USP7 inhibition by AD-04 increases K48-linked HIF-1α poly-ubiquitination in fibroblasts. Primary human fibroblasts were treated with vehicle (DMSO), AD-04 (1μM) or MG132 (10μM) under hypoxia. K63/K48 (top panel) or K63 (bottom panel) polyubiquitinated proteins were pulled down using TUBE™ agarose (TUBE1 is K63/K48-specific while TUBE2 is K63-specific) followed by immuno-blot analysis using anti-HIF-1α antibody. These data are representative data from at least two independent experiments.

**Figure 12:** HIF-1α Network Pathway Ranking. As expected, the HIF1A mRNA expression network is the most significantly enriched pathway (NCI-Nature database) in samples coming from hypoxia activated fibroblasts compared to non-activated (normoxia) fibroblast samples.

**Figure 13:** VEGFA mRNA expression is induced in hypoxia and down-regulated at the gene level upon treatment with USP7 inhibitor AD-04. The effect of USP7 inhibitors on the expression levels of VEGFA was evaluated by RNAseq in primary human fibroblasts. VEGFA levels were monitored by RNAseq analysis from human primary fibroblasts cultures in normoxia or hypoxia in presence of DMSO vehicle or the USP7 inhibitor AD-04.

**Figure 14:** The HIF1A mRNA expression network (KEGG definition) is changing in hypoxia activated fibroblasts

treated with AD-04. USP7 inhibitor treatment of primary human fibroblasts results in modulation of known genes in the HIF1A expression network at 6 (black bars) and 24 hours (grey bars).

**Figure 15:** USP7 inhibitor induces differential VEGFA alternative splicing in hypoxia activated fibroblasts. In addition to modulating VEGFA secretion, USP7 inhibitor-treated primary human fibroblasts display alternative patterns of VEGFA alternative splicing. At 24 hours the mRNA expression of the short (191aa - left panel) anti-angiogenic VEGF-165 is upregulated- and the long (371aa - right panel) pro-angiogenic isoform of VEGF-165 is strongly down-regulated. (Black (left boxplot) - DMSO; Grey (right boxplot) - AD-04).

**Figure 16:** MDM-2 antagonists do not affect sVEGF levels in activated fibroblasts, cancer cells or immortalized fibroblasts in normoxia. The USP7 inhibitor modulation of VEGF secretion in primary human fibroblasts is not mediated *via* MDM2 protein ubiquitylation at doses of MDM-2 antagonists that do not affect proliferation in normoxia. Cells were treated with vehicle (DMSO), AD-04, its inactive enantiomer (ent-AD-04), Nutlin-3a, RG7112 or SAR405838 at indicated concentrations for 48 hours in normoxia. Cell culture supernatants were collected and sVEGF was measured by ELISA.

**Figure 17:** MDM-2 antagonists do not affect sVEGF levels in activated fibroblasts, cancer cells or immortalized fibroblasts in hypoxia. The USP7 inhibitor modulation of VEGF secretion in primary human fibroblasts is not mediated *via* MDM2 protein ubiquitylation at doses of MDM-2 antagonists that do not affect proliferation in hypoxia. Cells were treated with vehicle (DMSO), AD-04, its inactive enantiomer (*ent*-AD-04), Nutlin-3a, RG7112 or SAR405838 at indicated concentrations for 48 hours in hypoxia. Cell culture supernatants were collected and sVEGF was measured by ELISA.

**Figure 18:** USP7 inhibitor AD-04 does not affect proliferation of primary fibroblasts, immortalized fibroblasts or cancer cells compared to MDM2 antagonists. The functional impact of USP7-mediated decrease in VEGF secretion is not on fibroblast or cancer cell proliferation. Cancer cells, primary and immortalized fibroblasts were treated with vehicle (DMSO) and increasing concentrations of AD-04, its inactive enantiomer (*ent*-AD-04), Nutlin-3a, RG7112 or SAR405838 in normoxia (Figure 18A and 18A) or hypoxia (Figure 18C). After 72 hours cell viability was measured by Cell-Titer-Glo. The results were normalized to the control non-treated group (mean ± SD, n=3).

**Figure 19:** AD-04 shows potent USP7 target engagement in primary human fibroblasts. To ensure that the effect of USP7 inhibitors is mediated *via* direct USP7 modulation in human primary fibroblasts, we monitored USP7 cellular target engagement using activity-based probe competition assays. HDF and IMR-90 were treated with vehicle (DMSO) or increasing concentrations of AD-04 inhibitor for 2 hours. Following incubation, cells were lysed and incubated with the ubiquitin-propargylamine (Ub-PA) activity probe. Samples were then analysed by immuno-blotting using the anti-USP7 antibody. $EC_{50}$ values were determined upon densitometry analysis. Band intensities were quantified using ImageJ software where the upper bands (USP7-Ub) and lower bands (USP7) were calculated as a percentage of the corresponding DMSO controls (-/+ Ub-PA) and values were then normalised to the sum of the lower and upper bands for each concentration.

**Figure 20:** AD-04 shows potent USP7 target engagement in cancer cells. To ensure that the effect of USP7 inhibitors is mediated *via* direct USP7 modulation in cancer cells, we monitored USP7 cellular target engagement using activity-based probe competition assays. HT-29 and CT-26 cells were treated with vehicle (DMSO) or increasing concentrations of AD-04 inhibitor for 2 hours. Following incubation, cells were lysed and incubated with the ubiquitin-propargylamine (Ub-PA) probe. Samples were then analysed by immuno-blotting using the anti-USP7 antibody. $EC_{50}$ values were determined upon densitometry analysis. Band intensities were quantified using ImageJ software where the upper bands (USP7-Ub) and lower bands (USP7) were calculated as a percentage of the corresponding DMSO controls (-/+ Ub-PA) and values were then normalised to the sum of the lower and upper bands for each concentration.

**Figure 21:** USP7 CRISPR/Cas9 knock-out in primary dermal fibroblasts results in sVEGF decrease. The specificity of the effect of the selective USP7 inhibitor AD-04 on VEGF secretion in primary human fibroblasts was confirmed by selective USP7 CRISPR knock-out. The efficiency of USP7 knock-out was confirmed by immuno-blot analysis using anti-USP7 antibody and β-actin as loading control (left). USP7 knock-out HDF were incubated in hypoxia for 48h and sVEGF levels were measured in cell culture supernatants

by ELISA.

**Figure 22:** USP7 inhibition does not modulate proliferation in cancer cells, immortalized fibroblasts, activated fibroblasts and endothelial cells. Cells were treated with vehicle (DMSO), AD-04 or ent-AD-04 for 96 hours. During a proliferation assay, pictures were taken every 2 hours using a time-lapse microscope. Charts were used to display cell confluency over time.

**Figure 23:** USP7 inhibition does not modulate migration in cancer cells, immortalised fibroblasts, activated fibroblasts and endothelial cells. In a scratch-wound healing assay, cells were seeded on Matrigel®-coated wells, wounded and treated with vehicle (DMSO), AD-04 or ent-AD-04. Pictures of wound closure were taken every 2 hours during a 4-day assay using a time-lapse microscope. Charts are displaying the percentage of wound closure over time.

**Figure 24:** USP7 inhibition (AD-04) inhibits cell invasion of human primary fibroblasts but not endothelial or cancer cells. The scratch wound cell invasion assay was used to measure the ability of indicated cells to invade through Matrigel® basement membrane. Pictures were taken every 2 hours over 4 days using a time-lapse microscope and wound closure over time is presented in charts.

**Figure 25:** USP7 inhibition (AD-04) decreases MMP-7 secretion in invading primary human fibroblasts. The scratch wound cell invasion assay was used to measure the ability of primary human fibroblasts to invade through Matrigel® basement membrane. Cell culture supernatants from invasion assay were collected after 96 hours and MMP-7 levels were measured using Luminex®.

**Figure 26:** USP7 inhibition (AD-04) inhibits tube formation from co-culture of fibroblast and endothelial cells but does not inhibit pre-formed tubes. HDF and HUVEC co-cultures were treated with vehicle (DMSO) and indicated concentrations of AD-04, *ent*-AD-04 and Avastin prior (top) or after (bottom) tube formation followed by CD31 staining. Representative pictures taken at day 14 are shown. Tube formation was quantified by measuring tube length (μm) and is shown.

**Figure 27:** USP7 inhibition (AD-04) decreases MMP-2 secreted levels in HDF and HUVEC co-culture from newly formed tubes. HDF and HUVEC co-cultures were treated with vehicle (DMSO) and indicated concentrations of AD-04, *ent*-AD-04 and Avastin prior (left) or after (right) tube formation. Cell culture supernatants from tube formation were collected and MMP-2 levels were measured using Luminex®.

**Figure 28:** USP7 inhibition has no direct effect on HUVEC tube formation *in vitro.* HUVEC were seeded on growth factor-reduced Matrigel®-coated wells followed by treatment with vehicle (DMSO) and indicated concentrations of AD-04, *ent*-AD-04 or Avastin. The tube formation images were taken 6h after tube initiation.

**Figure 29:** USP7 does not inhibit CT-26 cancer cell line proliferation *in vitro.* CT-26 syngeneic models are *in vivo* models to monitor the effect of compounds on the TME. Cells were treated with vehicle (DMSO) or increasing concentrations of AD-04, its inactive enantiomer (*ent*-AD-04)*,* Nutlin-3a or Sunitinib. After 72 hours cell viability was measured by CellTiter-Glo®.

**Figure 30:** Effect of USP7 on the tumour microenvironment *in vivo.* The USP7 inhibitor AD-04 inhibits the growth of the CT-26 cell line in syngeneic xenografts *in vivo.* Tumour volume of CT-26 xenograft implanted immuno-competent syngeneic mice treated with vehicle control, AD-04 or Sorafenib was measured twice weekly until day 15 post tumour implantation. AD-04 produced a tumour growth inhibition of 53%, very similar to the level achieved by the positive control Sorafenib, 58%, (left panel) and was well tolerated during the study (right panel). $n$ = 13; error bars indicate s.e.m.; *, $P$< 0.05; ** $P$< 0.01 (two-way ANOVA). No significant body weight changes of tumour-bearing mice from each group (right).

**Figure 31:** USP7 inhibition modulates secreted VEGF levels *in vivo.* Dosing CT-26-bearing syngeneic mice with USP7 inhibitor AD-04 leads to decreased circulating VEGF serum levels. Serum samples from CT-26 tumour-bearing mice treated with vehicle, AD-04 or Sorafenib were collected at day 15 post tumour grafting and circulating serum VEGF levels were measured by ELISA. All statistical differences displayed are compared with control treatment; ****p<0.0001, ***p<0.001; **p<0.01; *p<0.05; ns: not significant (one-way ANOVA/Dunnet post-test).

**Figure 32:** USP7 inhibition modulates the tumour microenvironment immune compartment. USP7 inhibition increases recruitment of cytotoxic lymphocytes and decreases number of macrophages in CT-26 tumours. Flow cytometry analysis of tumour infiltrating immune cells in the CT-26 syngeneic model dosed with the USP7 inhibitor for 10 days.

**Figure 33:** ADC-159, a novel oral USP7 inhibitor decreases VEGF levels in fibroblasts under hypoxia. The novel oral USP7 inhibitor ADC-159 decreases VEGF secretion in primary human fibroblasts activated by low oxygen levels for 48 hours in a hypoxic chamber. Supernatants were collected and assayed for VEGF ELISA.

**Figure 34:** ADC-160, ADC-198, ADC-199 and ADC-556 USP7 inhibitors decrease VEGF levels in fibroblasts under hypoxia. Fibroblasts were treated with indicated concentrations of USP7 inhibitors for 48h in a hypoxic chamber. Supernatants were collected and assayed for VEGF by ELISA.

**Figure 35:** ADC-159 shows an inhibitory effect on newly formed tubes from co-culture of fibroblast and endothelial cells. HDF and HUVEC co-cultures were treated with vehicle (DMSO) or indicated concentrations of ADC-159 and Avastin prior (top) or after (bottom) tube formation followed by anti-CD31 staining. Representative pictures taken at day 14 after tube initiation are shown. Tube formation was quantified by measuring tube length ($\mu$m).

**Figure 36:** ADC-159 prevents tumour vessel maturation in CT-26 syngeneic mouse model *in vivo.* FFPE tumour blocks were prepared after 15 days of treatment with vehicle and ADC-159 and co-stained with endothelial cell marker CD31 (green), pericyte marker NG2 (red) and DAPI (blue; n=5 blocks/group). Mature blood vessels have fine branching positive for CD31 and NG2 staining (A, left image) which is lost when treated with USP7 inhibitor (B, right image).

**Figure 37:** ADC-159 decreases tumour area and increases necrosis area in the CT-26 syngeneic mouse model *in vivo.* Tumour volume of CT-26 xenograft implanted in immuno-competent syngeneic mice treated with vehicle control and ADC-159 at 100 mg/kg dose was measured twice weekly until day 15 post tumour implantation. FFPE tumour blocks were prepared followed by quantification of tumour and necrosis using HALO software.

**Figure 38:** Effect of USP7 inhibition in combination with immune checkpoint inhibitors (ICI) on the tumour microenvironment *in vivo.* The USP7 inhibitor ADC-159 inhibits the growth of the CT-26 cell line in syngeneic xenografts *in vivo* and synergizes with ICI (A). Tumour volume of CT-26 xenograft implanted in immuno-competent syngeneic mice treated with vehicle control, ADC-159, anti-PD-L1, anti-CTLA-4 or in combination was measured twice weekly until day 13 post tumour implantation. ADC-159 produced a tumour growth inhibition (TGI) of 23%, very similar to the level achieved by anti-PDL-1 (22%). The combination of ADC-159 and anti-PDL- further reduced tumour growth (TGI=43%). Treatment with anti-CTLA-4 resulted in 43% of TGI which was similar to TGI produced by ADC-159 and anti-CTLA-4 combo (top panel). The treatment was well tolerated, and no significant body weight changes were detected (B). n = 15; error bars indicate s.e.m.; ** $P < 0.01$. *** $P < 0.001$, (two-way ANOVA).

**Figure 39:** USP7 inhibition leads to increased survival when combined with anti-PD-L1 and anti-CTLA-4 immune checkpoint inhibitors. (A) Kaplan-Meier survival curve in the mouse allograft study using CT-26 murine colorectal cancer cells shows prolonged survival in animals treated with ADC-159 and ICIs. (B) Individual growth curves show improved anti-tumour response when USP7 inhibitor ADC-159 is combined with ICIs. n=10 animals/group.

**Figure 40:** USP7 inhibitor ADC-159 modulates the tumour microenvironment immune compartment *in vivo.* USP7 inhibition increases recruitment of cytotoxic lymphocytes as shown by flow cytometry analysis of tumour infiltrating immune cells in the CT-26 syngenic model dosed with the USP7 inhibitor ADC-159 alone or in combination with ICIs for 13 days.

## DETAILED DESCRIPTION

It is demonstrated for the first time herein that USP7 plays a critical role in affecting the tumour microenvironment that is not mediated by the cells of the cancer itself. For example, USP7 is demonstrated to be mediating the effects of

fibroblasts on the tumour microenvironment (TME). It is demonstrated herein that USP7 affects the fibroblast-mediated remodelling of the TME extracellular matrix (ECM), as well as contributing to fibroblast expression of VEGF. Inhibiting USP7 in fibroblasts, especially cancer-associated fibroblasts (CAFs), is thus able to limit tumour growth and invasion via a number of different effects.

Indeed, it is demonstrated herein that USP7 inhibition in the fibroblast compartment of the TME leads to a significant decrease in both cell invasion and angiogenesis. USP7 inhibition in fibroblasts in the TME also results in modulation of the tumour immune environment so as to promote infiltration of CD8 T cells.

The efficacy of USP7 inhibition in fibroblasts as an effective cancer therapy is striking and advantageous because treatment is effective even when the cancer cells are resistant to direct inhibition by a USP7 inhibitor.

Thus, provided herein is a newly-discovered means of treating cancer by inhibiting USP7 in non-cancerous cells that is independent from the previously-identified role of USP7 role in directly driving tumorigenesis in the cancer cells. Surprisingly, inhibiting USP7 in fibroblasts is shown to confer potent anti-tumour effects independent from any effect of USP7 inhibitors on the cancer cells themselves.

Accordingly, in one aspect is provided a method of treating cancer by inhibiting USP7 activity in fibroblasts, the method comprising administering to a subject in need thereof a composition comprising a USP7 inhibitor.

Cancer-associated fibroblasts (or tumour-associated fibroblasts) are fibroblasts present in the tumour-microenvironment. Typically, CAFs are derived from normal fibroblasts in the normal surrounding tissue, but can also be derived from pericytes, smooth muscle cells, fibrocytes, or mesenchymal stem cells (MSCs). Markers of CAFs include smooth muscle actin ($\alpha$SMA), vimentin, platelet-derived growth factor receptor $\alpha$ (PDGFR-$\alpha$), platelet-derived growth factor receptor $\beta$ (PDGFR-$\beta$), fibroblast specific protein 1 (FSP-1) and fibroblast activation protein (FAP).

Typically, CAFs exhibit an activated fibroblast phenotype, for example exhibiting elevated levels of fibroblast activation protein (FAP) compared to normal fibroblasts. CAFs secrete growth factors such as VEGF and FGF which promote growth of the tumour, for example by promoting angiogenesis. CAFs also promote tumour growth, invasion and metastasis by remodelling of the extracellular matrix (ECM) in the tumour microenvironment (TME). For example, CAFs are able to remodel the ECM to include more survival signals such as IGF-1 and IGF-2, thus promoting survival of the surrounding cancer cells. CAFs can also produce enzymes (e.g. matrix metalloproteinases MMPs) that degrade ECM components such as the basement membrane. Tumour cells can invade tissues by migrating through the degraded membrane, in some instances by attaching to migrating CAFs. Migration of tumour cells through the ECM can lead to metastases.

In certain embodiments, administration of the composition comprising the USP7 inhibitor treats the cancer by inhibiting USP7 activity in cancer-associated fibroblasts (CAFs).

It is demonstrated herein that inhibition of USP7 in fibroblasts reduces the tumorigenic effects of fibroblasts, for example by reducing cell invasion, reducing MMP secretion, reducing basement membrane degradation, and reducing systemic and TME VEGF levels. Administration of a USP7 inhibitor can thus treat cancer by inhibiting one or more, for example two or three or all, of these tumorigenic effects.

Thus, in certain embodiments of the methods provided herein, administration of the composition comprising the USP7 inhibitor treats the cancer by inhibiting extra-cellular matrix (ECM) remodelling by cancer-associated fibroblasts. In such embodiments, the ECM is the ECM of the tumour microenvironment.

In certain embodiments, administration of the composition comprising the USP7 inhibitor treats the cancer by inhibiting degradation of a basement membrane.

In certain embodiments, administration of the composition comprising the USP7 inhibitor inhibits degradation of one or more ECM components selected from elastin, collagen (e.g. collagen type IV) and laminin. In certain embodiments, administration of the composition comprising the USP7 inhibitor inhibits degradation of collagen IV.

In certain preferred embodiments, administration of the USP7 inhibitor inhibits fibroblast (optionally CAF) secretion of MMP2 and/or MMP7. In certain preferred embodiments, administration of the USP7 inhibitor inhibits fibroblast (optionally CAF) secretion of MMP7. In certain preferred embodiments, administration of the USP7 inhibitor inhibits fibroblast (optionally CAF) secretion of MMP2. In certain preferred embodiments, administration of the USP7 inhibitor inhibits fibroblast (optionally CAF) secretion of MMP2 and MMP7.

It is demonstrated in the Examples that inhibition of USP7 in activated fibroblasts inhibits invasion of the fibroblasts through the basement membrane. This may be due to the reduced expression of matrix metalloproteinase by the fibroblasts following USP7 inhibition. Inhibiting fibroblast invasion (e.g. CAF invasion) is particularly advantageous since tumour cells can attach themselves to invasive fibroblasts and thus reducing fibroblast invasion can reduce cancer cell invasion.

In certain embodiments, administration of the composition comprising the USP7 inhibitor inhibits fibroblast invasion through the basement membrane. In certain embodiments, administration of the composition comprising the USP7 inhibitor inhibits CAF invasion through the basement membrane.

By inhibiting fibroblast secretion of MMP2, MMP7, or both, inhibition of USP7 in fibroblasts is particularly advantageous because as well as degrading ECM components such as the basement membrane, MMP2 and MMP7 are

known to contribute to epithelial to mesenchymal transition (EMT), for example by triggering TGF-β activation.

EMT - the process by which epithelial cells lose adhesion and become mesenchymal stem cells - is a known contributor to tumour metastasis, particularly in epithelial cell-derived cancers. Thus, by decreasing MMP2 and MMP7 production by fibroblasts, inhibition of USP7 is able to regulate EMT, and this further. reduces tumour invasion and metastasis.

In certain embodiments, therefore, administration of the composition comprising the USP7 inhibitor treats the cancer by modulating EMT (e.g. by reducing EMT).

In addition to their role in modelling of the extracellular matrix, fibroblasts have the capacity to alter the extracellular microenvironment and therefore regulate vascularization processes. Fibroblast-derived proteins, including growth factors and matrix proteins, have been shown to induce, support and modulate endothelial cell sprouting and the expansion of capillary-like networks (tubes). Formation of tubes contributes to the vascularisation of a tumour as well as providing further means for metastasis of tumour cells. Reducing the formation of fibroblast-mediated epithelial tube formation through USP7 inhibition thus offers a further route to treating the tumour and reducing metastases.

Accordingly, in certain embodiments, administration of the USP7 inhibitor treats the cancer by inhibiting fibroblast-mediated epithelial tube formation. In certain preferred embodiments, administration of the USP7 inhibitor treats the cancer by inhibiting *de novo* fibroblast-mediated epithelial tube formation.

Fibroblasts, especially CAFs, also contribute to tumour-related angiogenesis through production of growth factors such as VEGF. It is demonstrated herein for the first time that USP7 inhibition reduces VEGF production by fibroblasts, thereby reducing VEGF levels systemically as well as in the tumour microenvironment. VEGF is a well-validated target for cancer therapy, with anti-VEGF bevacizumab (Avastin™) used to treat at least colon cancer, lung cancer, glioblastoma and renal cancer. Inhibiting USP7 to reduce VEGF therefore provides a further means for treating cancer.

Thus, in certain preferred embodiments, administration of the composition comprising the USP7 inhibitor treats the cancer by reducing the level of VEGF in the serum of the subject.

In certain preferred embodiments administration of the composition comprising the USP7 inhibitor treats the cancer by reducing the level of VEGF in the tumour microenvironment.

In certain preferred embodiments, administration of the composition comprising the USP7 inhibitor treats the cancer by inhibiting production of VEGF by cancer-associated fibroblasts (CAFs). In certain embodiments inhibition of USP7 inhibits secretion of VEGF by CAFs. In certain embodiments inhibition of USP7 reduces VEGF mRNA levels in CAFs.

As used herein, VEGF refers to VEGFA, encoded by the *VEGFA* gene.

Inhibition of fibroblast (e.g. CAF) production of VEGF by inhibition of USP7 is mediated through destabilisation of hypoxia inducible factor alpha (HIFα), the transcription factor for VEGF. By inhibiting USP7, a USP7 inhibitor reduces the half-life of HIFα in fibroblasts, thereby reducing the driver of VEGF expression.

Accordingly, in certain embodiments administration of the USP7 inhibitor destabilizes hypoxia-inducible transcription factor (HIF1α), thereby inhibiting VEGF production by cancer-associated fibroblasts. In certain embodiments administration of the USP7 inhibitor destabilizes hypoxia-inducible transcription factor (HIF1α) in cancer-associated fibroblasts, thereby inhibiting tumour-associated angiogenesis.

It is demonstrated herein for the first time that inhibition of USP7 in fibroblasts can inhibit a number of pro-tumorigenic factors and effects, and can thus reduce tumour growth and/or invasion. A number of these factors, for example VEGF production by fibroblasts and fibroblast-mediated epithelial tube formation can contribute alone or in combination to angiogenesis. Accordingly, inhibition of USP7 in fibroblasts provides a new means for inhibiting angiogenesis, thereby reducing tumour survival.

Thus, in certain embodiments, administration of the composition comprising the USP7 inhibitor treats the cancer by inhibiting angiogenesis. In certain embodiments, administration of the composition comprising the USP7 inhibitor treats the cancer by inhibiting neo-angiogenesis. In such embodiments, angiogenesis is inhibited in the tumour microenvironment.

In certain embodiments, administration of the composition comprising the USP7 inhibitor up-regulates expression of short (191aa) VEGF-165 mRNA. In certain embodiments, administration of the composition comprising the USP7 inhibitor down-regulates expression of long (371aa) VEGF-165 mRNA.

The importance of having tumour infiltrating lymphocytes (TILs) in the tumour microenvironment for an effective immune response to the tumour is well-established. Notably therefore, it is further demonstrated herein that inhibition of USP7 results in modulation of the tumour immune environment so as to increase TIL infiltration.

In particular, inhibition of USP7 results in increased levels of tumour infiltrating lymphocytes (TILs), specifically cytotoxic CD8+ TILs. Without wishing to be bound by theory, the increase in cytotoxic CD8+ TILs following USP7 inhibition may be due to the effects reported herein of USP7 inhibition on TME remodelling, for example by cancer associated fibroblasts, with the reduced CAF-mediated remodelling permitting greater TIL infiltration.

It is further notable that USP7 inhibition also reduces the proportion of Treg cells in the TME relative to CD8+ TILs.

The dampening effect of Treg cells on the local immune response in the TME is a known mechanism by which tumour cells evade immune control. By modulating the tumour immune environment, in particular by promoting TIL infiltration and also reducing the relative number of Treg cells in the TME, USP7 inhibition provides a further means for promoting effective cancer treatment.

Thus, in a further aspect of the invention is provided a method of treating cancer by inhibiting USP7 activity, the method comprising administering to a subject in need thereof a composition comprising a USP7 inhibitor, wherein administration of the USP7 inhibitor modulates the tumour immune environment. Modulation of the tumour immune environment can be characterised by a change in the number and/or type of immune cells present in the TME. For example, modulation of the tumour immune environment may be characterised by an increase in TILs in the TME, a decrease in Treg cells in the TME, and/or a decrease in macrophages in the TME.

In a further aspect is provided a method of treating cancer by inhibiting USP7 activity, the method comprising administering to a subject in need thereof a composition comprising a USP7 inhibitor, wherein administration of the composition comprising the USP7 inhibitor increases the number of TILs in the TME, preferably CD8+ TILs.

In a further aspect is provided a method of treating cancer by inhibiting USP7 activity, the method comprising administering to a subject in need thereof a composition comprising a USP7 inhibitor, wherein administration of the composition comprising the USP7 inhibitor decreases the proportion of Treg cells relative to CD8+ T cells in the TME.

In a further aspect is provided a method of treating cancer by inhibiting USP7 activity, the method comprising administering to a subject in need thereof a composition comprising a USP7 inhibitor, wherein administration of the composition comprising the USP7 inhibitor decreases the number of macrophages in the TME.

In certain embodiments, the modulation of the tumour immune environment (e.g. increase in TILs, decrease in Treg cells and/or macrophages) is mediated through inhibition of USP7 in cells other than the tumour cells. In certain embodiments, the modulation is mediated through inhibition of USP7 in fibroblasts, for example CAFs.

As demonstrated in the accompanying Examples, administration of a USP7 inhibitor in combination with an immune checkpoint inhibitor (ICI) combine synergistically to prolong survival in a tumour model. Without being bound by theory, it is hypothesised that the modulation of the TME mediated by the USP7 inhibitor results in greater recruitment and infiltration of TILs to the tumour site, thereby providing a larger population of potential effector cells on which the ICI can act.

Accordingly, in a further aspect is provided a method of treating cancer by administering to a subject in need thereof a combination therapy, the combination therapy comprising a composition comprising a USP7 inhibitor and composition comprising an immune checkpoint inhibitor.

In a further aspect is provided a USP7 inhibitor for use in a method of treating cancer, the method comprising administering to a subject in need thereof a combination therapy, the combination therapy comprising a composition comprising the USP7 inhibitor and a composition comprising an immune checkpoint inhibitor.

In a further aspect is provided an immune checkpoint inhibitor for use in a method of treating cancer, the method comprising administering to a subject in need thereof a combination therapy, the combination therapy comprising a composition comprising a USP7 inhibitor and a composition comprising the immune checkpoint inhibitor.

In a further aspect is provided a combination therapy for use in a method of treating cancer, the method comprising administering to a subject in need thereof the combination therapy, the combination therapy comprising a composition comprising a USP7 inhibitor and a composition comprising an immune checkpoint inhibitor.

In preferred embodiments the immune checkpoint inhibitor is selected from an inhibitor of PD1, PD-L1, CTLA4, TIGIT, 41BB, OX40, GITR. In certain embodiments the checkpoint inhibitor is selected from an anti-PD1 antibody, an anti-PD-L1 antibody, an anti-CTLA4 antibody, an anti-41BB antibody, an anti-OX40 antibody, an anti-GITR antibody, and an anti-ICOS antibody. In certain embodiments the checkpoint inhibitor is selected from an anti-PD1 antibody, an anti-PD-L1 antibody, and an anti-CTLA4 antibody. In certain embodiments the checkpoint inhibitor is selected from an anti-PD1 antibody and an anti-PD-L1 antibody. In certain embodiments the checkpoint inhibitor is an anti-CTLA4 antibody. In certain embodiments the checkpoint inhibitor is selected from: pembrolizumab (Keytruda™), nivolumab (Opdivo™), cemiplimab (Libtayo™), Atezolizumab (Tecentriq™), Avelumab (Bavencio™), Durvalumab (Imfinzi™), and Ipilimumab (Yervoy™).

In a preferred embodiment of all aspects of the invention, the composition is administered at a dose that achieves an inhibition of tumour growth.

Preferably, the composition is administered at a dose that achieves an inhibition of tumour invasion.

Preferably, the composition is administered at a dose that achieves an inhibition of tumour metastasis.

Preferably, the composition is administered at a dose that achieves modulation of the tumour immune environment.

Preferably, the composition is administered at a dose that achieves an inhibition of angiogenesis in the tumour microenvironment.

A particular advantage of the newly-identified effects of USP7 inhibitors on fibroblasts is that the effects are independent of any action (or lack of action) of the USP7 inhibitor directly on the cancer cells. Even when a cancer is not responsive to direct inhibition of USP7 in the cancer cells, an anti-tumour effect is achieved through inhibition of USP7 in fibroblasts, for example those fibroblasts associated with the cancer.

Thus, in certain embodiments, the cancer treated by the method is formed of cancer cells, and the cancer cells are resistant to the USP7 inhibitor.

Cancer cells are resistant to the USP7 inhibitor when survival and proliferation of the cancer cells is not affected by direct exposure of the cancer cells to the USP7 inhibitor. Whether cancer cells are resistant to the USP7 can be determined by exposing the cancer cells to the USP7 inhibitor *in vitro* and monitoring their growth and survival.

USP7 inhibitors have been used to target cancer proliferation through modulating the ubiquitination of oncoprotein MDM2. MDM2 has been hypothesised as having a role in HIFα expression; however, it is demonstrated herein that the effects of USP7 inhibition in fibroblasts are not mediated through MDM2. Thus, treatment of cancer by inhibition of USP7 in fibroblasts in accordance with the invention is effective when treating cancers that are resistant to inhibitors of the MDM2 pathway.

Thus, in certain embodiments the cancer is formed of cancer cells and the cancer cells are resistant to an inhibitor of the MDM2 pathway. In certain embodiments the cancer is formed of cancer cells and the cancer cells are resistant to an MDM2 inhibitor, e.g. an inhibitor of MDM2's regulation of p53.

In certain embodiments the cancer is formed of cancer cells and the cancer cells are resistant to an MDM2 inhibitor selected from: RG7112, RG7388 (Idasanutlin), SAR405838, MK-8242, AMG232, CGM097, HDM201, CGM097, and ALRN-6924.

As already described, inhibiting USP7 in fibroblasts is effective in reducing the pro-tumour effects of fibroblasts (e.g. angiogenesis, ECM remodelling, VEGF production). Because the methods provided herein treat cancer through action on fibroblasts, in particular cancer-associated fibroblasts, and do not require any effect on the cancer cells themselves, the methods are suitable for treating a broad range of cancers.

Thus, in certain embodiments, the cancer treated in accordance with the invention is selected from: renal cancer (e.g., renal cell carcinoma), breast cancer, brain tumours, lymphomas (e.g., Hodgkin's and non-Hodgkin's lymphoma, lymphocytic lymphoma, primary CNS lymphoma, B-cell lymphoma (e.g. CLL), T-cell lymphoma (e.g. Sezary Syndrome)), nasopharyngeal carcinomas, melanoma (e.g., metastatic malignant melanoma), prostate cancer, colon cancer, lung cancer, bone cancer, pancreatic cancer, skin cancer, cancer of the head or neck (e.g. head and neck squamous cell carcinoma (HNSCC)), cutaneous carcinoma, cutaneous or intraocular malignant melanoma, uterine cancer, ovarian cancer, rectal cancer, cancer of the anal region, stomach cancer, testicular cancer, carcinoma of the fallopian tubes, carcinoma of the endometrium, carcinoma of the cervix, carcinoma of the vagina, carcinoma of the vulva, cancer of the oesophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, cancer of the bladder, neoplasm of the central nervous system (CNS), spinal axis tumour, brain stem glioma, pituitary adenoma, Kaposi's sarcoma, epidermoid cancer, squamous cell cancer, mesothelioma.

In preferred embodiments the cancer is characterised by the presence of cancer-associated fibroblasts in the tumour microenvironment.

In certain embodiments the cancer is a carcinoma or breast cancer. In certain embodiments, the cancer is adenocarcinoma. In certain embodiments, the cancer is colorectal carcinoma. In certain embodiments, the cancer is prostate carcinoma. In certain embodiments, the cancer is colon cancer. In certain embodiments, the cancer is lung cancer (e.g. non-small cell lung cancer). In certain embodiments, the cancer is glioblastoma. In certain embodiments, the cancer is renal cancer.

In certain preferred embodiments, the cancer is a solid tumour.

As already described, the methods of the invention will be particularly effective at treating metastatic cancer due to the ability to reduce remodelling of the ECM, thereby reducing migration of cancer cells through the basement membrane, a known factor in cancer metastasis.

Accordingly, in certain preferred embodiments, the method is a method of treating metastatic cancer. Treating metastatic cancer in this context includes preventing or reducing metastasis, slowing the progression to metastasis and/or reducing the risk of metastasis. In certain embodiments the method comprises treatment of a cancer that has already metastasised. In certain such embodiments, the treatment of the cancer is of the secondary tumour.

In certain embodiments the cancer treated is a primary tumour.

By targeting fibroblasts, for example CAFs, methods of the invention will be effective at treating cancers that have not been responsive to therapies intended to target the cancer cells themselves. Accordingly, the methods of the invention are advantageous for treating patients who have not responded to an alternative cancer therapy, for example first line cancer therapy. The methods will also be effective at treating patients who have relapsed, for example after successful first line therapy.

Accordingly, in certain embodiments, the subject has previously been administered an initial therapeutic agent and did not exhibit a response. In certain embodiments, the subject has previously been administered an initial therapeutic agent and has relapsed.

In certain such embodiments the initial therapeutic agent is not a USP7 inhibitor. In certain alternative embodiments, the initial therapeutic agent is an inhibitor of the p53/MDM2 pathway. In certain embodiments the initial therapeutic

agent is an inhibitor of the p53/MDM2 interaction.

As a consequence of their TME reprograming activities, USP7 inhibitors have the potential to deliver combination efficacy with other agents known to modulate the TME such as immune checkpoint inhibitors (e.g. inhibitors of PD-1, PD-L1, CTLA4), anti-angiogenic agents (e.g. VEGF inhibitors, VEGFR inhibitors), or extra-cellular matrix reprogramming agents. Methods of the invention will be particularly effective in combination with checkpoint inhibitors as inhibition of USP7 in fibroblasts in accordance with the invention is demonstrated herein to lead to a greater infiltration of CD8 T cells to the tumour site and greater than additive improvements in survival and tumour volume.

Accordingly, in certain embodiments, the method further comprises administration of an additional therapeutic agent.

In certain embodiments, the additional therapeutic agent is selected from a checkpoint inhibitor (also referred to as immune checkpoint inhibitor) and an anti-angiogenic agent.

In certain embodiments, the checkpoint inhibitor is selected from an inhibitor of PD1, PD-L1, CTLA4, TIGIT, 41BB, OX40, GITR. In certain embodiments the checkpoint inhibitor is selected from an anti-PD1 antibody, an anti-PD-L1 antibody, an anti-CTLA4 antibody, an anti-41BB antibody, an anti-OX40 antibody, an anti-GITR antibody, and an anti-ICOS antibody. In certain embodiments the checkpoint inhibitor is selected from an anti-PD1 antibody, an anti-PD-L1 antibody, and an anti-CTLA4 antibody. In certain embodiments the checkpoint inhibitor is selected from an anti-PD1 antibody and an anti-PD-L1 antibody. In certain embodiments the checkpoint inhibitor is an anti-CTLA4 antibody. In certain embodiments the checkpoint inhibitor is selected from: pembrolizumab (Keytruda™), nivolumab (Opdivo™), cemiplimab (Libtayo™), Atezolizumab (Tecentriq™), Avelumab (Bavencio™), Durvalumab (Imfinzi™), and Ipilimumab (Yervoy™).

In certain embodiments the anti-angiogenic agent is a VEGF inhibitor or a VEGFR inhibitor. In certain embodiments the anti-angiogenic agent is selected from: Axitinib (Inlyta®), Bevacizumab (Avastin®), Cabozantinib (Cometriq®), Everolimus (Afinitor®), Lenalidomide (Revlimid®), Lenvatinib mesylate (Lenvima®), Pazopanib (Votrient®), Ramucirumab (Cyramza®), Regorafenib (Stivarga®), Sorafenib (Nexavar®), Sunitinib (Sutent®), Thalidomide (Synovir, Thalomid®), Vandetanib (Caprelsa®), and Ziv-aflibercept (Zaltrap®).

In certain embodiments, the additional therapeutic agent is administered in combination with the composition comprising the USP7 inhibitor, e.g. as part of a combination therapy. In certain embodiments, the USP7 inhibitor and the additional therapeutic agent are administered simultaneously. In certain alternative embodiments, the USP7 inhibitor and the additional therapeutic agent are not administered simultaneously.

In certain embodiments, the USP7 inhibitor and the additional therapeutic agent are co-formulated. In certain embodiments, the USP7 inhibitor and the additional therapeutic agent are separately formulated.

In certain embodiments, the composition comprising the USP7 inhibitor further comprises a pharmaceutically acceptable excipient.

Pharmaceutical compositions may be formulated according to their particular use and purpose by mixing, for example, excipient, binding agent, lubricant, disintegrating agent, coating material, emulsifier, suspending agent, solvent, stabilizer, absorption enhancer and / or ointment base. The composition may be suitable for oral, injectable, rectal or topical administration.

Suitable pharmaceutically acceptable excipients would be known by the person skilled in the art, for example: fats, water, physiological saline, alcohol (e.g. ethanol), glycerol, polyols, aqueous glucose solution, extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant, saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose; and other conventionally used additives such as gelatin, talc, plant oil and gum arabic.

For example, the pharmaceutical composition may be administered orally, such as in the form of tablets, coated tablets, hard or soft gelatine capsules, solutions, emulsions, or suspensions. Administration can also be carried out rectally, for example using suppositories, locally or percutaneously, for example using ointments, creams, gels or solution, or parenterally, for example using injectable solutions.

For the preparation of tablets, coated tablets or hard gelatine capsules, the compounds of the present invention may be admixed with pharmaceutically inert, inorganic or organic excipients. Examples of suitable excipients include lactose, maize starch or derivatives thereof, talc or stearic acid or salts thereof. Suitable excipients for use with soft gelatine capsules include, for example, vegetable oils, waxes, fats and semi-solid or liquid polyols.

For the preparation of solutions and syrups, excipients include, for example, water, polyols, saccharose, invert sugar and glucose.

For injectable solutions, excipients include, for example, water, alcohols, polyols, glycerine and vegetable oil.

For suppositories and for local and percutaneous application, excipients include, for example, natural or

hardened oils, waxes, fats and semi-solid or liquid polyols.

The pharmaceutical compositions may also contain preserving agents, solublizing agents, stabilizing agents, wetting agents, emulsifiers, sweeteners, colorants, odorants, buffers, coating agents and / or antioxidants.

For combination therapies, the second drug may be provided in pharmaceutical composition with the USP7 inhibitor or may be provided separately.

In certain preferred embodiments, the composition comprising the USP7 inhibitor is administered orally. In certain preferred embodiments, the composition comprising the USP7 inhibitor is administered by injection, for example subcutaneously or intramuscularly.

In a preferred embodiment of all aspects of the invention, the subject to be treated is a human subject.

In a further aspect provided in accordance with the invention is a USP7 inhibitor for use in a method of treating cancer as provided herein.

USP7 inhibitors

USP7 inhibitors are known in the art and can be used in the methods of the invention. Preferably the USP7 inhibitor is a small molecule inhibitor. Preferably the USP7 inhibitor is an organic compound having a molecular weight of 900 daltons or less.

Examples of suitable USP7 inhibitors are described in WO2018/073602, US 2008/0103149 A1, WO 2010/114881 A1, WO 2010/081783 A1, WO 2011/086178 A1, WO 2013/030218 A1, EP 2565186 A1, EP 1749822 A1, WO 2016/109515 A1, WO 2016/109480 A1, WO 2016/126929 A1, WO 2016/126926 A1, WO 2016/126935 A1, WO 2016/150800 A1, WO2017/158381, WO2017/158388, WO2017/212010, WO2017/212012 and US20190142834.

Suitable USP7 inhibitors include those provided in WO2018/073602.

In certain embodiments, the USP7 inhibitor is selected from a compound of formula (I):

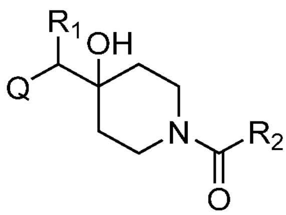

**(I)**

including a pharmaceutically acceptable salt, tautomer, stereoisomer or N-oxide derivative thereof, wherein:

$R_1$ is H, OH or an optionally substituted alkyl group, preferably $R_1$ is H;

$R_2$ is an optionally substituted C1-C6 alkyl, optionally substituted C2-C6 alkenyl, optionally substituted C2-C6 alkynyl, optionally substituted C3-C6 cycloalkyl, optionally substituted C4-C6 alkylcycloalkyl, optionally substituted C4-C6 aryl, optionally substituted C3-C6 heteroaryl, optionally substituted C4-C8 aryloxy, optionally substituted C7-C10 arylalkyl or optionally substituted C5-C10 heteroarylalkyl group; and

Q is an optionally substituted nitrogen containing heterocyclyl group. In certain embodiments, Q is selected from:

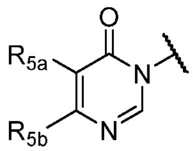

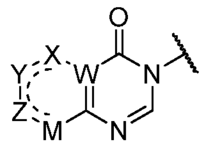

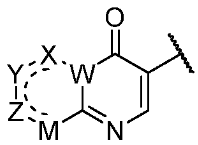

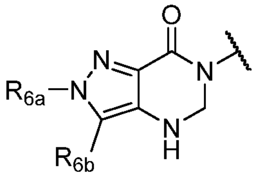

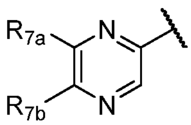

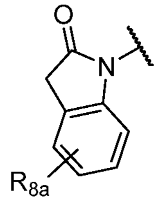

wherein:

W is N or C

X is S, O, N, or CH

Y is $CR_{6a}$, $CR_{9a}$, N, or $NR_{6a}$,

Z is $CR_{6b}$, N, $NR_{6b}$, $NR_{9b}$, or O

M is absent or $CR_{8a}$
wherein if X is S, Z is N and M is absent; and wherein if M is $CR_{8a}$ Y is not N;

$R_{5a}$ is H, halo, optionally substituted C1-C6 alkyl, or optionally substituted amino;

$R_{5b}$ is H, halo, optionally substituted C1-C6 alkyl, optionally substituted C2-C6 alkynyl, benzyl, optionally monosubstituted C3-C6 heteroaryl, optionally substituted C3-C6 heterocycloalkyl, optionally substituted C1-C6 alkoxy, NR'R", or $R^aNR'R"$,

wherein $R^a$ is C1-C6 alkyl or C2-C6 alkenyl; and
wherein R' and R" are each independently selected from H, oxo-substituted C1-C6 alkyl, hydroxy-substituted C1-C6 alkyl, optionally substituted C1-C6 alkoxy, optionally substituted C3-C6 cycloalkyl, optionally substituted C1-C7 alkylamine, optionally substituted C2-C7 alkenylamine, optionally substituted C3-C10 heterocycloalkyl, optionally substituted C4-C10 aryl, optionally substituted C3-C10 heteroaryl, optionally substituted C5-C10 alkylaryl, optionally substituted C4-C10 alkylheterocycloalkyl, and C4-C6 alkylheteroaryl, or wherein R' and R" together form an optionally substituted C3-C8 heterocycloalkyl including the N to which they are attached;

$R_{6a}$ is H, optionally substituted C1-C6 alkyl, optionally substituted amino, optionally substituted C4-C6 aryl, optionally substituted C1-C6 sulfide, optionally substituted C1-C6 sulfonyl, or optionally substituted amino;

$R_{6b}$ is H, cyano, halo, optionally substituted C1-C6 alkyl, optionally substituted C2-C6 alkenyl, optionally substituted C2-C6 alkynyl, optionally substituted C3-C6 cycloalkyl, optionally substituted C4-C6 cycloalkenyl, optionally substituted C2-C6 ynol, optionally substituted C4-C6 aryl, optionally substituted C3-C6 heteroaryl, optionally substituted amino;

$R_{7a}$ is H;

$R_{7b}$ is H or optionally substituted C4-C6 aryl
or wherein $R_{7a}$ and $R_{7b}$ together form an optionally substituted C1-C6 aryl group together with the carbons to which they are attached;

$R_{8a}$ is H or is optionally substituted C4-C6 aryl;

$R_{9a}$ is Cl, F, Br, I, or cyano;

$R_{9b}$ is H, optionally substituted C1-C6 alkyl, optionally substituted C4-C6 aryl, optionally substituted C3-C8 heteroaryl, C1-C6 alkoxy.

In preferred embodiments, the USP7 inhibitor is a compound according to formula (I), or a pharmaceutically acceptable salt thereof, wherein Q is selected from:

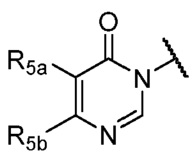

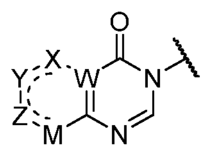

wherein:

W is N or C

X is S, O, N, or CH

Y is $CR_{6a}$, $CR_{9a}$, N, or $NR_{6a}$,

Z is $CR_{6b}$, N, $NR_{6b}$, $NR_{9b}$, or O

M is absent or $CR_{8a}$
wherein if X is S, Z is N and M is absent; and wherein if M is $CR_{8a}$ Y is not N;

$R_{5a}$ is H, halo, optionally substituted C1-C6 alkyl, or optionally substituted amino;

$R_{5b}$ is H, halo, optionally substituted C1-C6 alkyl, optionally substituted C2-C6 alkynyl, benzyl, optionally monosubstituted C3-C6 heteroaryl, optionally substituted C3-C6 heterocycloalkyl, optionally substituted C1-C6 alkoxy, NR'R", or $R^a$NR'R",

wherein $R^a$ is C1-C6 alkyl or C2-C6 alkenyl; and
wherein R' and R" are each independently selected from H, oxo-substituted C1-C6 alkyl, hydroxy-substituted C1-C6 alkyl, optionally substituted C1-C6 alkoxy, optionally substituted C3-C6 cycloalkyl, optionally substituted C1-C7 alkylamine, optionally substituted C2-C7 alkenylamine, optionally substituted C3-C10 heterocycloalkyl, optionally substituted C4-C10 aryl, optionally substituted C3-C10 heteroaryl, optionally substituted C5-C10 alkylaryl, optionally substituted C4-C10 alkylheterocycloalkyl, and C4-C6 alkylheteroaryl, or wherein R' and R" together form an optionally substituted C3-C8 heterocycloalkyl including the N to which they are attached;

$R_{6a}$ is H, optionally substituted C1-C6 alkyl, optionally substituted amino, optionally substituted C4-C6 aryl, optionally substituted C1-C6 sulfide, optionally substituted C1-C6 sulfonyl, or optionally substituted amino;

$R_{6b}$ is H, cyano, halo, optionally substituted C1-C6 alkyl, optionally substituted C2-C6 alkenyl, optionally substituted C2-C6 alkynyl, optionally substituted C3-C6 cycloalkyl, optionally substituted C4-C6 cycloalkenyl, optionally substituted C2-C6 ynol, optionally substituted C4-C6 aryl, optionally substituted C3-C6 heteroaryl, optionally substituted amino;

$R_{8a}$ is H or is optionally substituted C4-C6 aryl;

$R_{9a}$ is Cl, F, Br, I, or cyano;

$R_{9b}$ is H, optionally substituted C1-C6 alkyl, optionally substituted C4-C6 aryl, optionally substituted C3-C8 heteroaryl, C1-C6 alkoxy.

In preferred embodiments, the USP7 inhibitor is a compound according to formula (I), or a pharmaceutically acceptable salt thereof, wherein Q is selected from:

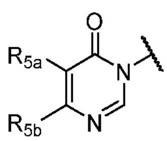

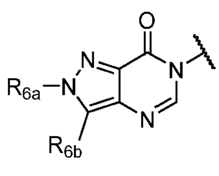

and

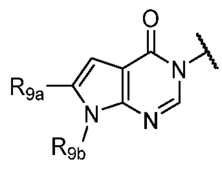

Wherein $R_{5a}$, $R_{5b}$, $R_{6a}$, $R_{6b}$, $R_{9a}$, and $R_{9b}$ are as defined above.

In certain embodiments, for any of the functional groups for which one or more substituents are optional, the optional substituents are independently selected from OH, F, Cl, Br, I, CN, C1-C6 alkyl, $CF_3$, $CHF_2$, $CH_2F$, $CH_2OH$, COOH, $C(O)CH_3$, CH2NHC(O)$OCH_2CH_3$, C2-C6 alkenyl, C2-C6 alkynyl, C3-C6 cycloalkyl, C1-C6 alkoxy, amino, C1-C6 alkylamine, C5-C6 aryl, C3-C6 heteroaryl, benzyl, oxo and amide, or two adjacent substituents may together constitute a ring.

In preferred embodiments, the USP7 inhibitor is a compound according to formula (I), or a pharmaceutically acceptable salt thereof, wherein Q is:

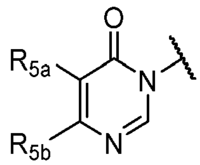

and:

$R_{5a}$ is H,

$R_{5b}$ is selected from optionally methyl- or ethylamine-substituted pyrazole, and NR'R", wherein R' and R" are each independently selected from H, methyl, cyclohexylamine, optionally methyl-, fluoro-, or fluorophenyl-substituted C2-C7 ethylamine, optionally substituted phenyl or wherein R' and R" together form an optionally substituted C3-C8 heterocycloalkyl including the N to which they are attached.

In preferred embodiments, R' is H and R" is ethylpyrollidine optionally substituted with methyl, fluoro, or fluorophenyl.

In preferred embodiments, the USP7 inhibitor is a compound according to formula (I), or a pharmaceutically acceptable salt thereof, wherein Q is:

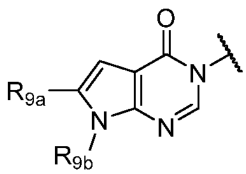

and

$R_{9a}$ Cl, F, Br, I, or cyano;

$R_{9b}$ is H, optionally substituted C1-C6 alkyl, or optionally substituted C4-C6 aryl;

wherein the optional substituents are selected from F, Cl, Br, methoxy, OH, CH2OH, C1-C6 alkylamine, cyclopropane, tetrahydrofuran, dioxolane, furan, methylpyrazole optionally substituted with fluoro, and morpholine.

In preferred embodiments, $R_{9a}$ is Cl, Br, I, or cyano and $R_{9b}$ is phenyl optionally substituted with F, Cl, Br, methoxy, OH, C1-C6 alkylamine, cyclopropane, tetrahydrofuran, dioxolane, furan, methylpyrazole.

In certain preferred embodiments, $R_{9a}$ is Cl.

In certain preferred such embodiments, $R_{9b}$ is selected from:

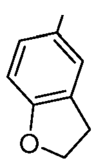 ; 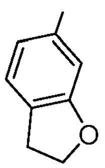 ; 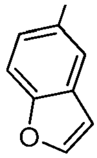 ; and 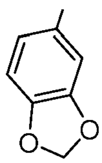

In preferred embodiments, $R_{9b}$ is:

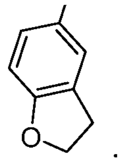

In preferred embodiments, the USP7 inhibitor is a compound according to formula (I), or a pharmaceutically acceptable salt thereof, wherein Q is:

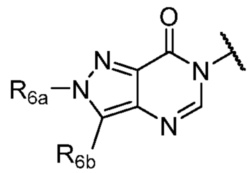

and

$R_{6a}$ is H or C1-C6 alkyl;

$R_{6b}$ is H, halo, optionally substituted C2-C6 alkenyl, optionally substituted C2-C6 alkynyl, optionally substituted C3-C6 cycloalkyl, optionally substituted C4-C6 aryl, optionally substituted C3-C6 heteroaryl;

wherein the optional substituents are independently selected from F, CN, OH, CH2OH, amide, NH2, C1-C6 alkylamine, C3-C6 cycloalkylamine, CF3, COOH, methylmorpholine, CH(CF3)NH2, CH(CHF2)NH2, CH2NHC(O) OCH2CH3.

In preferred embodiments, $R_{6a}$ is H, methyl or ethyl; $R_{6b}$ is H, Br, optionally substituted propenyl, ethynyl, optionally substituted propynyl, optionally substituted pentynyl, optionally substituted cyclohexane, optionally substituted phenyl, pyrazole, pyridine;
wherein the optional substituents are independently selected from F, CN, OH, CH2OH, amide, NH2, C1-C6 alkylamine, C3-C6 cycloalkylamine, CF3, CH(CF3)NH2, CH(CHF2)NH2.

In preferred embodiments, $R_{6a}$ is methyl and $R_{6b}$ is phenyl optionally substituted with one or more of F, CN, OH, CH2OH, NH2, CH2NH2, CH2CH2NH2, CH(CH3)NH2, amide, cyclopropylamine, and cyclobutylamine.

In preferred embodiments, $R_2$ is optionally substituted oxazole or optionally substituted C3-C6 cycloalkyl. In certain preferred embodiments, $R_2$ is optionally substituted oxazole or optionally substituted cyclopropyl.

In certain such embodiments, each one or more optional substituent is independently selected from C1-C6 alkyl and C3-C6 cycloalkyl. Preferably the optional substituent is methyl or cyclopropyl.

In certain preferred embodiments, $R_2$ is oxazole substituted with cyclopropane. In certain preferred embodiments, $R_2$ is cyclopropyl substituted with methyl.

In certain preferred embodiments, $R_2$ is selected from:

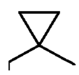 and 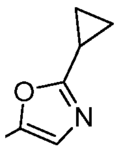 .

In preferred embodiments where the USP7 inhibitor is a compound exhibiting stereoisomerism, the compound is the R-enantiomer. In preferred embodiments where the USP7 inhibitor is a compound exhibiting stereoisomerism, the compound is the S-enantiomer.

In certain embodiments the USP7 inhibitor is a compound according to formula (I), or a pharmaceutically acceptable salt thereof, selected from the compounds exemplified in WO2018/073602.

In certain embodiments, the USP7 inhibitor is a compound, or a pharmaceutically acceptable salt thereof, selected from the following, where Example number is given in reference to WO2018/073602:

Example 1:(*R*)-6-Chloro-3-((4-hydroxy-1-(3-phenylbutanoyl)piperidin-4-yl)methyl)pyrimidin-4(3*H*)-one

Example 2: (*R*)-3-((4-Hydroxy-1-(3-phenylbutanoyl)piperidin-4-yl)methyl)-6-(pyridin-4-yl)pyrimidin-4(3*H*)-one

Example 3: (*R*)-3-((4-Hydroxy-1-(3-phenylbutanoyl)piperidin-4-yl)methyl)-6-(1*H*-pyrazol-5-yl)pyrimidin-4(3*H*)-one

Example 4: (*R*)-3-((4-Hydroxy-1-(3-phenylbutanoyl)piperidin-4-yl)methyl)-6-(phenylamino)pyrimidin-4(3*H*)-one

Example 5: (*R*)-6-Amino-3-((4-hydroxy-1-(3-phenylbutanoyl)piperidin-4-yl)methyl)pyrimidin-4(3*H*)-one

Example 6: (*R*)-6-((2-(Dimethylamino)ethyl)amino)-3-((4-hydroxy-1-(3-phenylbutanoyl)piperidin-4-yl)methyl)pyrimidin-4(3*H*)-one

Example 7: (*R*)-6-((2-(Dimethylamino)ethyl)(methyl)amino)-3-((4-hydroxy-1-(3-phenylbutanoyl)piperidin-4-yl)methyl)pyrimidin-4(3*H*)-one

Example 8: (*R*)-3-((4-Hydroxy-1-(3-phenylbutanoyl)piperidin-4-yl)methyl)-6-(4-methylpiperazin-1-yl)pyrimidin-4(3*H*)-one

Example 9: (*R*)-3-((4-Hydroxy-1-(3-phenylbutanoyl)piperidin-4-yl)methyl)-6-methoxypyrimidin-4(3*H*)-one

Example 10: (*R*)-6-(2-(Dimethylamino)ethoxy)-3-((4-hydroxy-1-(3-phenylbutanoyl)piperidin-4-yl)methyl)pyrimidin-4(3*H*)-one

Example 11: (*R*)-3-((4-Hydroxy-1-(3-phenylbutanoyl)piperidin-4-yl)methyl)-6-((2-(pyrrolidin-1-yl)ethyl)amino)pyrimidin-4(3*H*)-one

Example 12: 6-((*S*)-3-Aminopyrrolidin-1-yl)-3-((4-hydroxy-1-((*R*)-3-phenylbutanoyl)piperidin-4-yl)methyl)pyrimidin-4(3*H*)-one

Example 13: (*R*)-6-(3-Aminoazetidin-1-yl)-3-((4-hydroxy-1-(3-phenylbutanoyl)piperidin-4-yl)methyl)pyrimidin-4(3*H*)-one

Example 14: (*R*)-5-Amino-6-chloro-3-((4-hydroxy-1-(3-phenylbutanoyl)piperidin-4-yl)methyl)pyrimidin-4(3*H*)-one

Example 20: (*R*)-6-((4-Hydroxy-1-(3-phenylbutanoyl)piperidin-4-yl)methyl)-2-methyl-2*H*-pyrazolo[4,3-*d*]pyrimidin-7(6*H*)-one

Example 21: (*R*)-3-Bromo-6-((4-hydroxy-1-(3-phenylbutanoyl)piperidin-4-yl)methyl)-2-methyl-2H-pyrazolo[4,3-*d*]pyrimidin-7(6*H*)-one (Intermediate B)

Example 22: (*R*)-3-Ethynyl-6-((4-hydroxy-1-(3-phenylbutanoyl)piperidin-4-yl)methyl)-2-methyl-2*H*-pyrazolo[4,3-*d*]pyrimidin-7(6*H*)-one

Example 23: (*R*)-6-((4-Hydroxy-1-(3-phenylbutanoyl)piperidin-4-yl)methyl)-2-methyl-3-(trifluoromethyl)-2*H*-pyrazolo[4,3-*d*]pyrimidin-7(6*H*)-one

Example 24: (*R*)-6-((4-Hydroxy-1-(3-phenylbutanoyl)piperidin-4-yl)methyl)-3-(3-hydroxy-3-methylbut-1-yn-1-yl)-2-methyl-2*H*-pyrazolo[4,3-*d*]pyrimidin-7(6*H*)-one

Example 25: (*R*)-6-((4-Hydroxy-1-(3-phenylbutanoyl)piperidin-4-yl)methyl)-2-methyl-3-(1*H*-pyrazol-5-yl)-2*H*-pyrazolo[4,3-*d*]pyrimidin-7(6*H*)-one

Example 26: (*R*)-6-((4-Hydroxy-1-(3-phenylbutanoyl)piperidin-4-yl)methyl)-2-methyl-3-(pyridin-4-yl)-2*H*-pyrazolo[4,3-*d*]pyrimidin-7(6*H*)-one

Example 27: (*R*)-6-((4-Hydroxy-1-(3-phenylbutanoyl)piperidin-4-yl)methyl)-2-methyl-3-phenyl-2*H*-pyrazolo[4,3-*d*]pyrimidin-7(6*H*)-one

Example 28: (*R*)-3-(6-((4-Hydroxy-1-(3-phenylbutanoyl)piperidin-4-yl)methyl)-2-methyl-7-oxo-6,7-dihydro-2*H*-pyrazolo[4,3-*d*]pyrimidin-3-yl)benzamide

Example 29: (*R*)-3-(3-Aminophenyl)-6-((4-hydroxy-1-(3-phenylbutanoyl)piperidin-4-yl)methyl)-2-methyl-2*H*-pyrazolo[4,3-*d*]pyrimidin-7(6*H*)-one

Example 30: (*R*)-3-(4-(Aminomethyl)phenyl)-6-((4-hydroxy-1-(3-phenylbutanoyl)piperidin-4-yl)methyl)-2-methyl-2*H*-pyrazolo[4,3-*d*]pyrimidin-7(6*H*)-one

Example 31: (*R*)-6-((4-Hydroxy-1-(3-phenylbutanoyl)piperidin-4-yl)methyl)-3-(3-hydroxyprop-1-yn-1-yl)-2-methyl-2*H*-pyrazolo[4,3-*d*]pyrimidin-7(6*H*)-one

Example 32: (*R*)-6-((4-Hydroxy-1-(3-phenylbutanoyl)piperidin-4-yl)methyl)-2-methyl-3-(prop-1-en-2-yl)-2*H*-pyrazolo[4,3-*d*]pyrimidin-7(6*H*)-one

Example 33: (*R*)-6-((4-Hydroxy-1-(3-phenylbutanoyl)piperidin-4-yl)methyl)-3-isopropyl-2-methyl-2*H*-pyrazolo [4,3-*d*]pyrimidin-7(6*H*)-one

Example 34: (*R*)-6-((4-Hydroxy-1-(3-phenylbutanoyl)piperidin-4-yl)methyl)-3-isopropyl-2-methyl-5,6-dihydro-2*H*-pyrazolo[4,3-*d*]pyrimidin-7(4*H*)-one

Example 35: (*R*)-6-((1-(3,4-Dimethylpent-4-enoyl)-4-hydroxypiperidin-4-yl)methyl)-2-methyl-3-phenyl-2*H*-pyrazolo [4,3-*d*]pyrimidin-7(6*H*)-one

Example 36: (*R*)-6-((4-Hydroxy-1-(4-methyl-3-(trifluoromethyl)pent-4-enoyl)piperidin-4-yl)methyl)-2-methyl-3-phenyl-2*H*-pyrazolo[4,3-*d*]pyrimidin-7(6*H*)-one

Example 37: (*R*)-6-((4-Hydroxy-1-(4,4,4-trifluoro-3-phenylbutanoyl)piperidin-4-yl)methyl)-2-methyl-3-phenyl-2*H*-pyrazolo[4,3-*d*]pyrimidin-7(6*H*)-one

Example 38: 6-((4-Hydroxy-1-(3-phenylpropanoyl)piperidin-4-yl)methyl)-2-methyl-2*H*-pyrazolo[4,3-*d*]pyrimidin-7(6*H*)-one

Example 39: 6-((4-Hydroxy-1-(3-phenylpropanoyl)piperidin-4-yl)methyl)-2-methyl-3-(trifluoromethyl)-2*H*-pyrazolo [4,3-*d*]pyrimidin-7(6*H*)-one

Example 40: (*R*)-3-((4-Hydroxy-1-(3-phenylbutanoyl)piperidin-4-yl)methyl)-6-(1-methyl-1*H*-pyrazol-4-yl)pyrimidin-4(3*H*)-one

Example 41: (*R*)-3-((4-Hydroxy-1-(3-phenylbutanoyl)piperidin-4-yl)methyl)-6-(1-isobutyl-1*H*-pyrazol-4-yl)pyrimidin-4(3*H*)-one

Example 42: 3-((4-Hydroxy-1-((*R*)-3-phenylbutanoyl)piperidin-4-yl)methyl)-6-((1,2,3,4-tetrahydro-1,4-epiminonaphthalen-6-yl)amino)pyrimidin-4(3*H*)-one hydrochloride

Example 43: (*R*)-6-((4-Hydroxy-1-(3-phenylbutanoyl)piperidin-4-yl)methyl)-2-methyl-3-(phenylamino)-2*H*-pyrazolo [4,3-*d*]pyrimidin-7(6*H*)-one

Example 44: (*R*)-6-Chloro-3-((4-hydroxy-1-(3-phenylbutanoyl)piperidin-4-yl)methyl)-5-methylpyrimidin-4(3*H*)-one

Example 45: (*R*)-5-Bromo-3-((4-hydroxy-1-(3-phenylbutanoyl)piperidin-4-yl)methyl)-6-((2-(pyrrolidin-1-yl)ethyl)amino)pyrimidin-4(3*H*)-one

Example 46: (*R*)-3-(4-(Aminomethyl)phenyl)-6-((4-hydroxy-1-(4,4,4-trifluoro-3-phenylbutanoyl)piperidin-4-yl) methyl)-2-methyl-2*H*-pyrazolo[4,3-*d*]pyrimidin-7(6*H*)-one

Example 47: 6-((*S*)-3-Aminopiperidin-1-yl)-3-((4-hydroxy-1-((*R*)-3-phenylbutanoyl)piperidin-4-yl)methyl)pyrimidin-4(3*H*)-one hydrochloride

Example 48: (*R*)-6-(4-Aminopiperidin-1-yl)-3-((4-hydroxy-1-(3-phenylbutanoyl)piperidin-4-yl)methyl)pyrimidin-4(3*H*)-one hydrochloride

Example 49: (R)-3-((4-Hydroxy-1-(3-phenylbutanoyl)piperidin-4-yl)methyl)-6-(2,8-diazaspiro[4.5]decan-8-yl)pyrimidin-4(3*H*)--one hydrochloride

Example 50: 6-((*S*)-3-(Dimethylamino)piperidin-1-yl)-3-((4-hydroxy-1-((*R*)-3-phenylbutanoyl)piperidin-4-yl)methyl) pyrimidin-4(3*H*)-one

Example 51: (*R*,*E*)-3-((4-Hydroxy-1-(3-phenylbutanoyl)piperidin-4-yl)methyl)-6-(3-(pyrrolidin-1-yl)prop-1-en-1-yl) pyrimidin-4(3*H*)-one

Example 52: (*R*)-3-((4-Hydroxy-1-(3-phenylbutanoyl)piperidin-4-yl)methyl)-6-((2-(piperidin-1-yl)ethyl)amino)pyrimidin-4(3*H*)-one

Example 53: (*R,S*)-3-(4-(Aminomethyl)phenyl)-6-((4-hydroxy-1-(4-methoxy-3-phenylbutanoyl)piperidin-4-yl) methyl)-2-methyl-2*H*-pyrazolo[4,3-*d*]pyrimidin-7(6*H*)-one

Example 54: (*R*)-6-(1-(2-(Dimethylamino)ethyl)-1*H*-pyrazol-4-yl)-3-((4-hydroxy-1-(3-phenylbutanoyl)piperidin-4-yl) methyl)pyrimidin-4(3*H*)-one, formic acid

Example 55: 6-((*E*)-3-((*R*)-3-Aminopyrrolidin-1-yl)prop-1-en-1-yl)-3-((4-hydroxy-1-((*R*)-3-phenylbutanoyl)piperidin-4-yl)methyl)pyrimidin-4(3*H*)-one, formic acid

Example 56: 6-((*E*)-3-((*S*)-3-Aminopyrrolidin-1-yl)prop-1-en-1-yl)-3-((4-hydroxy-1-((*R*)-3-phenylbutanoyl)piperidin-4-yl)methyl)pyrimidin-4(3*H*)-one, formic acid

Example 57: 3-((4-Hydroxy-1-((*R*)-3-phenylbutanoyl)piperidin-4-yl)methyl)-6-(((*S*)-1-phenyl-3-(pyrrolidin-1-yl)propan-2-yl)amino)pyrimidin-4(3*H*)-one, formic acid

Example 58: 3-((4-Hydroxy-1-((*R*)-3-phenylbutanoyl)piperidin-4-yl)methyl)-6-(((*R*)-2-(pyrrolidin-1-yl)propyl)amino) pyrimidin-4(3*H*)-one

Example 59: 3-((4-Hydroxy-1-((*R*)-3-phenylbutanoyl)piperidin-4-yl)methyl)-6-(((*S*)-2-(pyrrolidin-1-yl)propyl)amino) pyrimidin-4(3*H*)-one, formic acid

Example 60: 3-((4-Hydroxy-1-((*R*)-3-phenylbutanoyl)piperidin-4-yl)methyl)-6-(((*R*)-1-(pyrrolidin-1-yl)propan-2-yl) amino)pyrimidin-4(3*H*)-one, formic acid

Example 61: 3-((4-Hydroxy-1-((R)-3-phenylbutanoyl)piperidin-4-yl)methyl)-6-(((*S*)-1-(pyrrolidin-1-yl)propan-2-yl) amino)pyrimidin-4(3*H*)-one, formic acid

Example 62: 6-((*S*)-3-(Dimethylamino)pyrrolidin-1-yl)-3-((4-hydroxy-1-((*R*)-3-phenylbutanoyl)piperidin-4-yl)methyl) pyrimidin-4(3*H*)-one

Example 63: *rac*-6-(((±-*trans*-1 ,2)-2-Aminocyclohexyl)amino)-3-((4-hydroxy-1-((*R*)-3-phenylbutanoyl)piperidin-4-yl)methyl)pyrimidin-4(3*H*)-one, formic acid

Example 64: 6-(((±-*cis*-1,2)-2-Aminocyclohexyl)amino)-3-((4-hydroxy-1-((*R*)-3-phenylbutanoyl)piperidin-4-yl) methyl)pyrimidin-4(3*H*)-one, formic acid

Example 65: 3-((4-Hydroxy-1-((*R*)-3-phenylbutanoyl)piperidin-4-yl)methyl)-6-((((*R*)-1-methylpyrrolidin-2-yl)methyl) amino)pyrimidin-4(3*H*)-one, formic acid

Example 66: 3-((4-Hydroxy-1-((*R*)-3-phenylbutanoyl)piperidin-4-yl)methyl)-6-((((*S*)-1-methylpyrrolidin-2-yl)methyl) amino)pyrimidin-4(3*H*)-one, formic acid

Example 67: (*R*)-3-((4-Hydroxy-1-(3-phenylbutanoyl)piperidin-4-yl)methyl)pyrimidin-4(3*H*)-one

Example 69: 3-((4-Hydroxy-1-((*R*)-3-phenylbutanoyl)piperidin-4-yl)methyl)-6-((2-((*S*)-2-methylpyrrolidin-1-yl)ethyl)amino)pyrimidin-4(3*H*)-one, formic acid

Example 70: 3-((4-Hydroxy-1-((*R*)-3-phenylbutanoyl)piperidin-4-yl)methyl)-6-((2-((*R*)-2-(methoxymethyl)pyrrolidin-1-yl)ethyl)amino)pyrimidin-4(3*H*)-one

Example 71: 3-((4-Hydroxy-1-((*R*)-3-phenylbutanoyl)piperidin-4-yl)methyl)-6-((2-((*S*)-3-methylpyrrolidin-1-yl)ethyl)amino)pyrimidin-4(3*H*)-one, formic acid

Example 73: 6-((2-((*R*)-3-Fluoropyrrolidin-1-yl)ethyl)amino)-3-((4-hydroxy-1-((*R*)-3-phenylbutanoyl)piperidin-4-yl)methyl)pyrimidin-4(3*H*)-one, formic acid

Example 74: 6-((2-((*S*)-3-Fluoropyrrolidin-1-yl)ethyl)amino)-3-((4-hydroxy-1-((*R*)-3-phenylbutanoyl)piperidin-4-yl)methyl)pyrimidin-4(3*H*)-one, formic acid

Example 75: 3-(4-((*R*)-1-Aminoethyl)phenyl)-6-((4-hydroxy-1-((*R*)-4,4,4-trifluoro-3-phenylbutanoyl)piperidin-4-yl)methyl)-2-methyl-2*H*-pyrazolo[4,3-*d*]pyrimidin-7(6*H*)-one

Example 76: 3-(4-((*S*)-1-Aminoethyl)phenyl)-6-((4-hydroxy-1-((*R*)-4,4,4-trifluoro-3-phenylbutanoyl)piperidin-4-yl)methyl)-2-methyl-2*H*-pyrazolo[4,3-*d*]pyrimidin-7(6*H*)-one

Example 79: (*R*)-3-(4-((Dimethylamino)methyl)phenyl)-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 80: (*R*)-3-(4-(Aminomethyl)phenyl)-6-((1-(4,4-difluoro-3-phenylbutanoyl)-4-hydroxypiperidin-4-yl)methyl)-2-methyl-2*H*-pyrazolo[4,3-*d*]pyrimidin-7(6*H*)-one

Example 81: (*R*)-3-((4-Hydroxy-1-(3-phenylbutanoyl)piperidin-4-yl)methyl)-6-isopropylpyrimidin-4(3*H*)-one

Example 82: (*R*)-3-(4-(Aminomethyl)-3-fluorophenyl)-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 83: (*R*)-6-((4-Hydroxy-1-(4,4,4-trifluoro-3-phenylbutanoyl)piperidin-4-yl)methyl)-2-methyl-3-(4-((methylamino)methyl)phenyl)-2*H*-pyrazolo[4,3-*d*]pyrimidin-7(6*H*)-one

Example 84: (*R*)-3-(4-(Aminomethyl)phenyl)-6-((1-(3-(3,5-difluorophenyl)-4,4,4-trifluorobutanoyl)-4-hydroxypiperidin-4-yl)methyl)-2-methyl-2*H*-pyrazolo[4,3-*d*]pyrimidin-7(6*H*)-one

Example 85: (*R*)-3-(4-(Aminomethyl)phenyl)-6-((4-hydroxy-1-(4,4,4-trifluoro-3-(4-fluorophenyl)butanoyl)piperidin-4-yl)methyl)-2-methyl-2*H*-pyrazolo[4,3-*d*]pyrimidin-7(6*H*)-one

Example 86: (*R*)-6-((2-(4-Fluoroisoindolin-2-yl)ethyl)amino)-3-((4-hydroxy-1-(3-phenylbutanoyl)piperidin-4-yl)methyl)pyrimidin-4(3*H*)-one, formic acid

Example 87: (*R*)-3-(4-(2-Aminoethyl)phenyl)-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 88: (*R*)-3-(4-(1-Aminocyclobutyl)phenyl)-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 89: (*R*)-3-((1-(4,4-Difluoro-3-phenylbutanoyl)-4-hydroxypiperidin-4-yl)methyl)-6-((2-(pyrrolidin-1-yl)ethyl)amino)pyrimidin-4(3*H*)-one, formic acid

Example 95: 6-((4-Hydroxy-1-(3-phenylpropanoyl)piperidin-4-yl)methyl)-2-methyl-3-(trifluoromethyl)-2*H*-pyrazolo[4,3-*d*]pyrimidin-7(6*H*)-one

Example 97: 3-(2-Fluorophenyl)-6-((4-hydroxy-1-((*R*)-3-phenylbutanoyl)piperidin-4-yl)methyl)-2-methyl-2*H*-pyrazolo[4,3-*d*]pyrimidin-7(6*H*)-one

Example 98: (*R*)-3-(6-((4-Hydroxy-1-(3-phenylbutanoyl)piperidin-4-yl)methyl)-2-methyl-7-oxo-6,7-dihydro-2*H*-pyrazolo[4,3-*d*]pyrimidin-3-yl)benzonitrile

Example 99: 3-(2-Aminophenyl)-6-((4-hydroxy-1-((*R*)-3-phenylbutanoyl)piperidin-4-yl)methyl)-2-methyl-2*H*-pyrazolo[4,3-*d*]pyrimidin-7(6*H*)-one

Example 100: (*R*)-3-((4-Hydroxy-1-(3-phenylbutanoyl)piperidin-4-yl)methyl)-6-morpholinopyrimidin-4(3*H*)-one

Example 102: (*R*)-1-((4-Hydroxy-1-(3-phenylbutanoyl)piperidin-4-yl)methyl)-[4,5'-bipyrimidin]-6(1*H*)-one

Example 103: (*R*)-3-((4-Hydroxy-1-(3-phenylbutanoyl)piperidin-4-yl)methyl)-6-((2-hydroxyethyl)amino)pyrimidin-4(3*H*)-one

Example 104: (*R*)-6-((4-Hydroxy-1-(3-phenylbutanoyl)piperidin-4-yl)methyl)-3-(3-hydroxyphenyl)-2-methyl-2*H*-pyrazolo[4,3-*d*]pyrimidin-7(6*H*)-one

Example 106: (*R*)-4-(6-((4-Hydroxy-1-(3-phenylbutanoyl)piperidin-4-yl)methyl)-2-methyl-7-oxo-6,7-dihydro-2*H*-pyrazolo[4,3-*d*]pyrimidin-3-yl)benzamide

Example 107: (*R*)-6-((4-Hydroxy-1-(3-phenylbutanoyl)piperidin-4-yl)methyl)-3-(4-(hydroxymethyl)phenyl)-2-methyl-2*H*-pyrazolo[4,3-*d*]pyrimidin-7(6*H*)-one

Example 108: (*R*)-6-((4-Hydroxy-1-(3-phenylbutanoyl)piperidin-4-yl)methyl)-2-methyl-3-(3-(morpholinomethyl)phenyl)-2*H*-pyrazolo[4,3-*d*]pyrimidin-7(6*H*)-one

Example 109: (*R*)-3-((4-Hydroxy-1-(3-phenylbutanoyl)piperidin-4-yl)methyl)-6-((2-methoxyethyl)amino)pyrimidin-4(3*H*)-one

Example 110: 3-((4-Hydroxy-1-((*R*)-3-phenylbutanoyl)piperidin-4-yl)methyl)-6-(((*R*)-pyrrolidin-3-yl)amino)pyrimidin-4(3*H*)-one

Example 111: (*R*)-3-((4-Hydroxy-1-(3-phenylbutanoyl)piperidin-4-yl)methyl)-5-methyl-6-((2-(pyrrolidin-1-yl)ethyl)amino)pyrimidin-4(3*H*)-one

Example 112: Benzyl 4-((3-(4-(aminomethyl)phenyl)-2-methyl-7-oxo-2,7-dihydro-6*H*-pyrazolo[4,3-*d*]pyrimidin-6-yl)methyl)-4-hydroxypiperidine-1-carboxylate

Example 113: (*R*)-N-(1-((4-Hydroxy-1-(3-phenylbutanoyl)piperidin-4-yl)methyl)-6-oxo-1,6-dihydropyrimidin-4-yl)-*N*-(2-(pyrrolidin-1-yl)ethyl)acetamide, formic acid

Example 114: 3-((4-Hydroxy-1-((*R*)-3-phenylbutanoyl)piperidin-4-yl)methyl)-6-((2-((*R*)-2-methylpyrrolidin-1-yl)ethyl)amino)pyrimidin-4(3*H*)-one, formic acid

Example 116: (*R*)-3-((4-Hydroxy-1-(3-phenylbutanoyl)piperidin-4-yl)methyl)-6-(phenylethynyl)pyrimidin-4(3*H*)-one

Example 117: (*R*)-6-Benzyl-3-((4-hydroxy-1-(3-phenylbutanoyl)piperidin-4-yl)methyl)pyrimidin-4(3*H*)-one

Example 118: 3-(4-((*R*)-1-Amino-2,2,2-trifluoroethyl)phenyl)-6-((4-hydroxy-1-((*R*)-4,4,4-trifluoro-3-phenylbutanoyl)piperidin-4-yl)methyl)-2-methyl-2*H*-pyrazolo[4,3-*d*]pyrimidin-7(6*H*)-one

Example 119: 3-(4-((*S*)-1-Amino-2,2,2-trifluoroethyl)phenyl)-6-((4-hydroxy-1-((*R*)-4,4,4-trifluoro-3-phenylbutanoyl)piperidin-4-yl)methyl)-2-methyl-2*H*-pyrazolo[4,3-*d*]pyrimidin-7(6*H*)-one

Example 120: (*R*)-3-(4-(Aminomethyl)phenyl)-2-ethyl-6-((4-hydroxy-1-(3-phenylbutanoyl)piperidin-4-yl)methyl)-2*H*-pyrazolo[4,3-*d*]pyrimidin-7(6*H*)-one

Example 121: (*R*)-3-((4-Hydroxy-1-(3-phenylbutanoyl)piperidin-4-yl)methyl)-6-(phenyl(2-(pyrrolidin-1-yl)ethyl)amino)pyrimidin-4(3*H*)-one, formic acid

Example 122: 3-(4-((*S*)-1-Amino-2,2-difluoroethyl)phenyl)-6-((4-hydroxy-1-((*R*)-4,4,4-trifluoro-3-phenylbutanoyl)piperidin-4-yl)methyl)-2-methyl-2*H*-pyrazolo[4,3-*d*]pyrimidin-7(6*H*)-one

Example 123: (*R*)-3-(4-(Aminomethyl)-3-(trifluoromethyl)phenyl)-6-((4-hydroxy-1-(4,4,4-trifluoro-3-phenylbutanoyl) piperidin-4-yl)methyl)-2-methyl-2*H*-pyrazolo[4,3-*d*]pyrimidin-7(6*H*)-one

Example 124: (*R*)-3-(4-(1-Aminocyclopropyl)phenyl)-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 125: (*S*)-3-(4-(Aminomethyl)phenyl)-6-((4-hydroxy-1-(4,4,4-trifluoro-3-(5-methylthiophen-2-yl)butanoyl)piperidin-4-yl)methyl)-2-methyl-2*H*-pyrazolo[4,3-*d*]pyrimidin-7(6*H*)-one

Example 127: 3-(4-(Aminomethyl)phenyl)-6-((1-(3,3-dicyclopropylpropanoyl)-4-hydroxypiperidin-4-yl)methyl)-2-methyl-2*H*-pyrazolo[4,3-*d*]pyrimidin-7(6*H*)-one

Example 130: (*R*)-6-((4-Hydroxy-1-(4,4,4-trifluoro-3-phenylbutanoyl)piperidin-4-yl)methyl)-2-methyl-3-(1-methyl-12,3,6-tetrahydropyridin-4-yl)-2*H*-pyrazolo[4,3-*d*]pyrimidin-7(6*H*)-one

Example 131: 6-((1-Acetyl-4-hydroxypiperidin-4-yl)methyl)-2-methyl-3-phenyl-2*H*-pyrazolo[4,3-*d*]pyrimidin-7(6*H*)-one

Example 132: 3-((1-Acetyl-4-hydroxypiperidin-4-yl)methyl)-6-((2-(pyrrolidin-1-yl)ethyl)amino)pyrimidin-4(3*H*)-one

Example 133: 3-((1-(3,3-Dicyclopropylpropanoyl)-4-hydroxypiperidin-4-yl)methyl)-6-((2-(pyrrolidin-1-yl)ethyl)amino)pyrimidin-4(3*H*)-one

Example 134: (*R*)-3-(Cyclohex-1-en-1-yl)-6-((4-hydroxy-1-(4,4,4-trifluoro-3-phenylbutanoyl)piperidin-4-yl) methyl)-2-methyl-2*H*-pyrazolo[4,3-*d*]pyrimidin-7(6*H*)-one

Example 135: (*R*)-3-(3-(Dimethylamino)prop-1-yn-1-yl)-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 136: (*R*)-3-Cyclohexyl-6-((4-hydroxy-1-(4,4,4-trifluoro-3-phenylbutanoyl)piperidin-4-yl)methyl)-2-methyl-2*H*-pyrazolo[4,3-*d*]pyrimidin-7(6*H*)-one

Example 137: (*R*)-6-((4-Hydroxy-1-(4,4,4-trifluoro-3-phenylbutanoyl)piperidin-4-yl)methyl)-2-methyl-3-(4-(morpholinomethyl)phenyl)-2*H*-pyrazolo[4,3-*d*]pyrimidin-7(6*H*)-one

Example 138: 3-(4-(Aminomethyl)phenyl)-6-((1-(3-cyclobutylpropanoyl)-4-hydroxypiperidin-4-yl)methyl)-2-methyl-2*H*-pyrazolo[4,3-*d*]pyrimidin-7(6*H*)-one

Example 140: 3-((4-hydroxy-1-((*R*)-3-phenylbutanoyl)piperidin-4-yl)methyl)-6-((*R*)-2-(hydroxymethyl)pyrrolidin-1-yl)pyrimidin-4(3*H*)-one

Example 141: (*R*)-3-((4-hydroxy-1-(3-phenylbutanoyl)piperidin-4-yl)methyl)-6-(3-(methoxymethyl)azetidin-1-yl)pyrimidin-4(3*H*)-one

Example 142: (*R*)-3-((4-hydroxy-1-(3-phenylbutanoyl)piperidin-4-yl)methyl)-6-((2-(isopropylamino)ethyl)amino)pyrimidin-4(3*H*)-one

Example 143: (*R*)-*N*-(2-((1-((4-hydroxy-1-(3-phenylbutanoyl)piperidin-4-yl)methyl)-6-oxo-1,6-dihydropyrimidin-4-yl) amino)ethyl)acetamide

Example 144: (*R*)-3-((4-hydroxy-1-(3-phenylbutanoyl)piperidin-4-yl)methyl)-6-(4-hydroxypiperidin-1-yl)pyrimidin-4(3*H*)-one

Example 145: (*R*)-3-((4-hydroxy-1-(3-phenylbutanoyl)piperidin-4-yl)methyl)-6-((2-((4-(trifluoromethyl)pyrimidin-2-yl)amino)ethyl)amino)pyrimidin-4(3*H*)-one

Example 146: (*R*)-3-((4-hydroxy-1-(3-phenylbutanoyl)piperidin-4-yl)methyl)-6-((2-(phenylamino)ethyl)amino)pyrimidin-4(3*H*)-one

Example 147: *tert*-butyl ((1-(1-((4-hydroxy-1-((*R*)-3-phenylbutanoyl)piperidin-4-yl)methyl)-6-oxo-1,6-dihydropyrimidin-4-yl)pyrrolidin-2-yl)methyl)carbamate (mixture of diastereomers)

Example 148: 4-(1-((4-hydroxy-1-((*R*)-3-phenylbutanoyl)piperidin-4-yl)methyl)-6-oxo-1,6-dihydropyrimidin-4-yl)-*N*,*N*,2-trimethylmorpholine-2-carboxamide (mixture of diastereomers)

Example 149: 3-((4-hydroxy-1-((*R*)-3-phenylbutanoyl)piperidin-4-yl)methyl)-6-(3-morpholinopyrrolidin-1-yl)pyrimidin-4(3*H*)-one (mixture of diastereomers)

Example 150: 3-((4-hydroxy-1-((*R*)-3-phenylbutanoyl)piperidin-4-yl)methyl)-6-(3-hydroxy-3-methylpyrrolidin-1-yl)pyrimidin-4(3*H*)-one (mixture of diastereomers)

Example 151 : 3-((4-hydroxy-1-((*R*)-3-phenylbutanoyl)piperidin-4-yl)methyl)-6-((tetrahydrofuran-3-yl)amino)pyrimidin-4(3*H*)-one (mixture of diastereomers)

Example 152: 3-((4-hydroxy-1-((*R*)-3-phenylbutanoyl)piperidin-4-yl)methyl)-6-(2-oxa-7-azaspiro[4.4]nonan-7-yl)pyrimidin-4(3*H*)-one (mixture of diastereomers)

Example 153: 3-((4-hydroxy-1-((*R*)-3-phenylbutanoyl)piperidin-4-yl)methyl)-6-(tetrahydro-2*H*-furo[2,3-*c*]pyrrol-5(3*H*)-yl)pyrimidin-4(3*H*)-one (mixture of diastereomers)

Example 154: (*R*)-3-((4-hydroxy-1-(3-phenylbutanoyl)piperidin-4-yl)methyl)-6-(((1-methyl-1*H*-pyrazol-5-yl)methyl)amino)pyrimidin-4(3*H*)-one

Example 155: (*R*)-3-((4-hydroxy-1-(3-phenylbutanoyl)piperidin-4-yl)methyl)-6-((3-methyloxetan-3-yl)amino)pyrimidin-4(3*H*)-one

Example 156: (*R*)-6-(4-(1*H*-pyrazol-5-yl)piperidin-1-yl)-3-((4-hydroxy-1-(3-phenylbutanoyl)piperidin-4-yl)methyl)pyrimidin-4(3*H*)-one

Example 157: (*R*)-6-((4-chlorobenzyl)amino)-3-((4-hydroxy-1-(3-phenylbutanoyl)piperidin-4-yl)methyl)pyrimidin-4(3*H*)-one

Example 158: (*R*)-3-((4-hydroxy-1-(3-phenylbutanoyl)piperidin-4-yl)methyl)-6-(4-(pyridin-3-ylmethyl)piperazin-1-yl)pyrimidin-4(3*H*)-one

Example 159: (*R*)-3-((4-hydroxy-1-(3-phenylbutanoyl)piperidin-4-yl)methyl)-6-(4-(pyridin-2-ylmethyl)piperazin-1-yl)pyrimidin-4(3*H*)-one

Example 160: (*R*)-6-(4,4-difluoropiperidin-1-yl)-3-((4-hydroxy-1-(3-phenylbutanoyl)piperidin-4-yl)methyl)pyrimidin-4(3*H*)-one

Example 161 : (*R*)-2-((1-((4-hydroxy-1-(3-phenylbutanoyl)piperidin-4-yl)methyl)-6-oxo-1,6-dihydropyrimidin-4-yl)amino)-*N*,*N*-dimethylacetamide

Example 162: (*R*)-6-(((2,3-dihydrobenzo[*b*][1,4]dioxin-6-yl)methyl)amino)-3-((4-hydroxy-1-(3-phenylbutanoyl)piperidin-4-yl)methyl)pyrimidin-4(3*H*)-one

Example 163: (*R*)-3-((4-hydroxy-1-(3-phenylbutanoyl)piperidin-4-yl)methyl)-6-(((tetrahydro-2*H*-pyran-4-yl)methyl)amino)pyrimidin-4(3*H*)-one

Example 164: (*R*)-*N*-(cyclopropylmethyl)-1-(1-((4-hydroxy-1-(3-phenylbutanoyl)piperidin-4-yl)methyl)-6-oxo-1,6-dihydropyrimidin-4-yl)azetidine-3-carboxamide

Example 165: (*R*)-6-(3-fluoroazetidin-1-yl)-3-((4-hydroxy-1-(3-phenylbutanoyl)piperidin-4-yl)methyl)pyrimidin-4(3*H*)-one

Example 166: (*R*)-3-((4-hydroxy-1-(3-phenylbutanoyl)piperidin-4-yl)methyl)-6-((2-hydroxyethyl)(methyl)amino)pyrimidin-4(3*H*)-one

Example 167: (*R*)-6-(cyclopentylamino)-3-((4-hydroxy-1-(3-phenylbutanoyl)piperidin-4-yl)methyl)pyrimidin-4(3*H*)-one

Example 168: (*R*)-3-((4-hydroxy-1-(3-phenylbutanoyl)piperidin-4-yl)methyl)-6-(4-methyl-3-oxopiperazin-1-yl)pyrimidin-4(3*H*)-one

Example 169: (*R*)-3-((4-hydroxy-1-(3-phenylbutanoyl)piperidin-4-yl)methyl)-6-(5-oxa-2-azaspiro[3.4]octan-2-yl)pyrimidin-4(3*H*)-one

Example 170: (*R*)-6-((1,3-dimethyl-1*H*-pyrazol-4-yl)amino)-3-((4-hydroxy-1-(3-phenylbutanoyl)piperidin-4-yl)methyl)pyrimidin-4(3*H*)-one

Example 171 : (*R*)-3-((4-hydroxy-1-(3-phenylbutanoyl)piperidin-4-yl)methyl)-6-(8-methyl-5-oxa-2,8-diazaspiro[3.5]nonan-2-yl)pyrimidin-4(3*H*)-one

Example 172: (*R*)-6-(6-acetyl-2,6-diazaspiro[3.3]heptan-2-yl)-3-((4-hydroxy-1-(3-phenylbutanoyl)piperidin-4-yl)methyl)pyrimidin-4(3*H*)-one

Example 173: (*R*)-6-(5,5-difluoro-2-azaspiro[3.3]heptan-2-yl)-3-((4-hydroxy-1-(3-phenylbutanoyl)piperidin-4-yl)methyl)pyrimidin-4(3*H*)-one

Example 174: (*R*)-3-((4-hydroxy-1-(3-phenylbutanoyl)piperidin-4-yl)methyl)-6-(7-oxa-2-azaspiro[3.5]nonan-2-yl)pyrimidin-4(3*H*)-one

Example 175: (*R*)-3-((4-hydroxy-1-(3-phenylbutanoyl)piperidin-4-yl)methyl)-6-(2-oxa-7-azaspiro[3.5]nonan-7-yl)pyrimidin-4(3*H*)-one

Example 176: (*R*)-3-((4-hydroxy-1-(3-phenylbutanoyl)piperidin-4-yl)methyl)-6-(6-oxa-2-azaspiro[3.4]octan-2-yl)pyrimidin-4(3*H*)-one

Example 177: (*R*)-3-((4-hydroxy-1-(3-phenylbutanoyl)piperidin-4-yl)methyl)-6-((2-hydroxyethyl)(pyridin-3-ylmethyl)amino)pyrimidin-4(3*H*)-one

Example 178: (*R*)-3-((4-hydroxy-1-(3-phenylbutanoyl)piperidin-4-yl)methyl)-6-(5-methyl-2,5-diazaspiro[3.4]octan-2-yl)pyrimidin-4(3*H*)-one

Example 179: (*R*)-3-((4-hydroxy-1-(3-phenylbutanoyl)piperidin-4-yl)methyl)-6-(2-oxa-6-azaspiro[3.4]octan-6-yl)pyrimidin-4(3*H*)-one

Example 180: 3-((4-hydroxy-1-((*R*)-3-phenylbutanoyl)piperidin-4-yl)methyl)-6-((1 *R*,5*S*)-3-methyl-3,6-diazabicyclo[3.2.1]octan-6-yl)pyrimidin-4(3*H*)-one

Example 181 : 3-((4-hydroxy-1-((*R*)-3-phenylbutanoyl)piperidin-4-yl)methyl)-6-((1 *S*,5*R*)-3-methyl-3,6-diazabicyclo[3.2.1]octan-6-yl)pyrimidin-4(3*H*)-one

Example 182: (*R*)-3-(4-(1-Aminocyclobutyl)phenyl)-6-((4-hydroxy-1-(3-phenylbutanoyl)piperidin-4-yl)methyl)-2-methyl-2*H*-pyrazolo[4,3-*d*]pyrimidin-7(6*H*)-one

Example 183: 3-((1-(3-Cyclobutylpropanoyl)-4-hydroxypiperidin-4-yl)methyl)-6-((2-(pyrrolidin-1-yl)ethyl)amino)pyrimidin-4(3*H*)-one

Example 184: 3-((1-(2,2-Dicyclobutylacetyl)-4-hydroxypiperidin-4-yl)methyl)-6-((2-(pyrrolidin-1-yl)ethyl)amino)pyrimidin-4(3*H*)-one

Example 191: 6-((4-Hydroxy-1-(1-methylcyclopropanecarbonyl)piperidin-4-yl)methyl)-2-methyl-3-phenyl-2*H*-pyrazolo[4,3-*d*]pyrimidin-7(6*H*)-one

Example 192: 6-((4-Hydroxy-1-(oxazole-5-carbonyl)piperidin-4-yl)methyl)-2-methyl-3-phenyl-2*H*-pyrazolo[4,3-*d*]pyrimidin-7(6*H*)-one

Example 193: 6-((1-(3,3-Dicyclopropylpropanoyl)-4-hydroxypiperidin-4-yl)methyl)-2-methyl-3-phenyl-2*H*-pyrazolo[4,3-*d*]pyrimidin-7(6*H*)-one

Example 194: (*R*)-Ethyl 4-(6-((4-hydroxy-1-(3-phenylbutanoyl)piperidin-4-yl)methyl)-2-methyl-7-oxo-6,7-dihydro-2*H*-pyrazolo[4,3-*d*]pyrimidin-3-yl)benzylcarbamate

Example 196: 6-((1-(2-Cyclopropyloxazole-5-carbonyl)-4-hydroxypiperidin-4-yl)methyl)-2-methyl-3-phenyl-2*H*-pyrazolo[4,3-*d*]pyrimidin-7(6*H*)-one

Example 199: 7-Cyclopropyl-3-((4-hydroxy-1-(3-phenylpropanoyl)piperidin-4-yl)methyl)thieno[3,2-*d*]pyrimidin-4(3*H*)-one (Intermediate F)

Example 200: 6-Chloro-3-((4-hydroxy-1-(1-methylcyclopropanecarbonyl)piperidin-4-yl)methyl)-7-phenyl-3*H*-pyrrolo[2,3-*d*]pyrimidin-4(7*H*)-one

Example 201 : 6-Chloro-3-((4-hydroxy-1-(1-methylcyclopropanecarbonyl)piperidin-4-yl)methyl)-7-(5-(hydroxymethyl)thiophen-3-yl)-3*H*-pyrrolo[2,3-*d*]pyrimidin-4(7*H*)-one

Example 202: 7-(Benzo[d][1,3]dioxol-5-yl)-6-chloro-3-((4-hydroxy-1-(1-methylcyclopropanecarbonyl)piperidin-4-yl)methyl)-3*H*-pyrrolo[2,3-*d*]pyrimidin-4(7*H*)-one

Example 203: 6-Chloro-7-(4-chlorophenyl)-3-((4-hydroxy-1-(1-methylcyclopropanecarbonyl)piperidin-4-yl)methyl)-3*H*-pyrrolo[2,3-*d*]pyrimidin-4(7*H*)-one

Example 204: 6-Chloro-7-(4-fluorophenyl)-3-((4-hydroxy-1-(1-methylcyclopropanecarbonyl)piperidin-4-yl)methyl)-3*H*-pyrrolo[2,3-*d*]pyrimidin-4(7*H*)-one

Example 205: 6-Chloro-3-((1-(2-cyclopropyloxazole-5-carbonyl)-4-hydroxypiperidin-4-yl)methyl)-7-(4-fluorophenyl)-3*H*-pyrrolo[2,3-*d*]pyrimidin-4(7*H*)-one

Example 206: 6-Chloro-3-((1-(2-cyclopropyloxazole-5-carbonyl)-4-hydroxypiperidin-4-yl)methyl)-7-(4-fluoro-3-methoxyphenyl)-3*H*-pyrrolo[2,3-*d*]pyrimidin-4(7*H*)-one

Example 207: 6-Chloro-7-(4-fluoro-3-methoxyphenyl)-3-((4-hydroxy-1-(1-methylcyclopropanecarbonyl)piperidin-4-yl)methyl)-3*H*-pyrrolo[2,3-*d*]pyrimidin-4(7*H*)-one

Example 208: 6-Chloro-3-((4-hydroxy-1-(1-methylcyclopropanecarbonyl)piperidin-4-yl)methyl)-7-(3-(1-methyl-1*H*-pyrazol-5-yl)phenyl)-3*H*-pyrrolo[2,3-*d*]pyrimidin-4(7*H*)-one

Example 209: 6-Chloro-3-((4-hydroxy-1-(1-methylcyclopropanecarbonyl)piperidin-4-yl)methyl)-7-methyl-3*H*-pyrrolo[2,3-*d*]pyrimidin-4(7*H*)-one

Example 210: 6-Chloro-3-((1-(2-cyclopropyloxazole-5-carbonyl)-4-hydroxypiperidin-4-yl)methyl)-7-(3-(1-methyl-1*H*-pyrazol-5-yl)phenyl)-3*H*-pyrrolo[2,3-*d*]pyrimidin-4(7*H*)-one

Example 211 : 6-Chloro-3-((1-(2-cyclopropyloxazole-5-carbonyl)-4-hydroxypiperidin-4-yl)methyl)-7-(3-(1-methyl-1*H*-pyrazol-4-yl)phenyl)-3*H*-pyrrolo[2,3-*d*]pyrimidin-4(7*H*)-one

Example 212: 6-Chloro-3-((4-hydroxy-1-(1-methylcyclopropanecarbonyl)piperidin-4-yl)methyl)-7-(3-(1-methyl-1*H*-pyrazol-4-yl)phenyl)-3*H*-pyrrolo[2,3-*d*]pyrimidin-4(7*H*)-one

Example 214: 7-(3-Bromophenyl)-6-chloro-3-((4-hydroxy-1-(1-methylcyclopropanecarbonyl)piperidin-4-yl)methyl)-3*H*-pyrrolo[2,3-*d*]pyrimidin-4(7*H*)-one

Example 215: 6-Chloro-3-((1-(2-cyclopropyloxazole-5-carbonyl)-4-hydroxypiperidin-4-yl)methyl)-7-phenyl-3*H*-pyrrolo[2,3-*d*]pyrimidin-4(7*H*)-one

Example 216: 6-Chloro-3-((4-hydroxy-1-(1-methyl-1*H*-pyrazole-4-carbonyl)piperidin-4-yl)methyl)-7-phenyl-3*H*-pyrrolo[2,3-*d*]pyrimidin-4(7*H*)-one

Example 217: 6-Chloro-3-((1-(2-cyclopropyloxazole-5-carbonyl)-4-hydroxypiperidin-4-yl)methyl)-7-(3-cyclopropylphenyl)-3*H*-pyrrolo[2,3-*d*]pyrimidin-4(7*H*)-one

Example 218: 6-Chloro-7-(3-cyclopropylphenyl)-3-((4-hydroxy-1-(1-methylcyclopropanecarbonyl)piperidin-4-yl) methyl)-3*H*-pyrrolo[2,3-*d*]pyrimidin-4(7*H*)-one

Example 219: 6-Chloro-7-(3-cyclopropylphenyl)-3-((4-hydroxy-1-(1-methyl-1*H*-pyrazole-4-carbonyl)piperidin-4-yl) methyl)-3*H*-pyrrolo[2,3-*d*]pyrimidin-4(7*H*)-one

Example 220: 6-Chloro-7-(4-cyclopropylphenyl)-3-((4-hydroxy-1-(1-methylcyclopropanecarbonyl)piperidin-4-yl) methyl)-3*H*-pyrrolo[2,3-*d*]pyrimidin-4(7*H*)-one

Example 221 : 6-Chloro-3-((1-(2-cyclopropyloxazole-5-carbonyl)-4-hydroxypiperidin-4-yl)methyl)-7-(4-cyclopropylphenyl)-3*H*-pyrrolo[2,3-*d*]pyrimidin-4(7*H*)-one

Example 223: 6-Bromo-3-((4-hydroxy-1-(1-methylcyclopropanecarbonyl)piperidin-4-yl)methyl)-7-phenyl-3*H*-pyrrolo[2,3-*d*]pyrimidin-4(7*H*)-one

Example 224: 3-((1-(2-Cyclopropyloxazole-5-carbonyl)-4-hydroxypiperidin-4-yl)methyl)-6-iodo-7-phenyl-3*H*-pyrrolo[2,3-*d*]pyrimidin-4(7*H*)-one

Example 225: 6-Chloro-3-((4-hydroxy-1-(1-methylcyclopropanecarbonyl)piperidin-4-yl)methyl)-7-(3-(4-(trifluoromethyl)-1*H*-pyrazol-1-yl)phenyl)-3*H*-pyrrolo[2,3-*d*]pyrimidin-4(7*H*)-one

Example 226: 6-Chloro-3-((4-hydroxy-1-(1-methylcyclopropanecarbonyl)piperidin-4-yl)methyl)-7-(3-morpholinophenyl)-3*H*-pyrrolo[2,3-*d*]pyrimidin-4(7*H*)-one

Example 227: 6-Chloro-7-(3-(4-fluoro-1*H*-pyrazol-1-yl)phenyl)-3-((4-hydroxy-1-(1-methylcyclopropanecarbonyl)piperidin-4-yl)methyl)-3*H*-pyrrolo[2,3-*d*]pyrimidin-4(7*H*)-one

Example 228: 6-Chloro-7-(4-chlorophenyl)-3-((1-(2-cyclopropyloxazole-5-carbonyl)-4-hydroxypiperidin-4-ylmethyl)-3*H*-pyrrolo[2,3-*d*]pyrimidin-4(7*H*)-one

Example 229: 6-Chloro-7-(4-chlorophenyl)-3-((4-hydroxy-1-(1-methyl-1H-pyrazole-4-carbonyl)piperidin-4-yl) methyl)-3*H*-pyrrolo[2,3-*d*]pyrimidin-4(7*H*)-one

Example 230: 6-Chloro-3-((1-(2-cyclopropyloxazole-5-carbonyl)-4-hydroxypiperidin-4-yl)methyl)-7-(4-fluoro-3-(1-methyl-1*H*-pyrazol-5-yl)phenyl)-3*H*-pyrrolo[2,3-*d*]pyrimidin-4(7*H*)-one

Example 231 : 6-Chloro-7-(4-fluoro-3-(1-methyl-1*H*-pyrazol-5-yl)phenyl)-3-((4-hydroxy-1-(1-methylcyclopropanecarbonyl)piperidin-4-yl)methyl)-3*H*-pyrrolo[2,3-*d*]pyrimidin-4(7*H*)-one

Example 232: 6-Chloro-3-((1-(2-cyclopropyloxazole-5-carbonyl)-4-hydroxypiperidin-4-yl)methyl)-7-(4-fluoro-3-(1-methyl-1*H*-pyrazol-4-yl)phenyl)-3*H*-pyrrolo[2,3-*d*]pyrimidin-4(7*H*)-one

Example 233: 6-Chloro-7-(4-fluoro-3-(1-methyl-1*H*-pyrazol-4-yl)phenyl)-3-((4-hydroxy-1-(1-methylcyclopropanecarbonyl)piperidin-4-yl)methyl)-3*H*-pyrrolo[2,3-*d*]pyrimidin-4(7*H*)-one

Example 234: 3-((4-Hydroxy-1-(1-methylcyclopropanecarbonyl)piperidin-4-yl)methyl)-4-oxo-7-phenyl-4,7-dihydro-3*H*-pyrrolo[2,3-*d*]pyrimidine-6-carbonitrile

Example 235: 7-(Benzo[*d*][1,3]dioxol-5-yl-2,2-$d_2$)-6-chloro-3-((4-hydroxy-1-(1-methylcyclopropane-1-carbonyl)piperidin-4-yl)methyl)-3,7-dihydro-4*H*-pyrrolo[2,3-*d*]pyrimidin-4-one

Example 236: 7-(Benzo[*d*][1,3]dioxol-5-yl-2,2-$d_2$)-6-chloro-3-((1-(2-cyclopropyloxazole-5-carbonyl)-4-hydroxypiperidin-4-yl)methyl)-3,7-dihydro-4*H*-pyrrolo[2,3-*d*]pyrimidin-4-one

Example 237: 3-(4-(Aminomethyl)phenyl)-6-((4-hydroxy-1-(1-methylcyclopropane-1-carbonyl)piperidin-4-yl)methyl)-2-methyl-2,6-dihydro-7*H*-pyrazolo[4,3-*d*]pyrimidin-7-one

Example 238: 3-(4-(Aminomethyl)phenyl)-6-((1-(2-cyclopropyloxazole-5-carbonyl)-4-hydroxypiperidin-4-yl)methyl)-2-methyl-2,6-dihydro-7*H*-pyrazolo[4,3-*d*]pyrimidin-7-one

Example 239: 6-Chloro-7-(3,4-dimethoxyphenyl)-3-((4-hydroxy-1-(1-methylcyclopropane-1-carbonyl)piperidin-4-yl)methyl)-3,7-dihydro-4*H*-pyrrolo[2,3-*d*]pyrimidin-4-one

Example 240: 6-Chloro-3-((1-(2-cyclopropyloxazole-5-carbonyl)-4-hydroxypiperidin-4-yl)methyl)-7-(3,4-dimethoxyphenyl)-3,7-dihydro-4*H*-pyrrolo[2,3-*d*]pyrimidin-4-one

Example 241 : 7-(4-(Aminomethyl)phenyl)-6-chloro-3-((4-hydroxy-1-(1-methylcyclopropane-1-carbonyl)piperidin-4-yl)methyl)-3,7-dihydro-4*H*-pyrrolo[2,3-*d*]pyrimidin-4-one

Example 242: 7-(4-(Aminomethyl)phenyl)-6-chloro-3-((1-(2-cyclopropyloxazole-5-carbonyl)-4-hydroxypiperidin-4-yl)methyl)-3,7-dihydro-4*H*-pyrrolo[2,3-*d*]pyrimidin-4-one

Example 243: (*R*)-4-(6-((4-Hydroxy-1-(3-phenylbutanoyl)piperidin-4-yl)methyl)-2-methyl-7-oxo-6,7-dihydro-2*H*-pyrazolo[4,3-*d*]pyrimidin-3-yl)benzoic acid

Example 244: (*R*)-3-(6-((4-Hydroxy-1-(3-phenylbutanoyl)piperidin-4-yl)methyl)-2-methyl-7-oxo-6,7-dihydro-2*H*-pyrazolo[4,3-*d*]pyrimidin-3-yl)benzoic acid

Example 245: 4-(6-((4-Hydroxy-1-(1-methylcyclopropane-1-carbonyl)piperidin-4-yl)methyl)-2-methyl-7-oxo-6,7-dihydro-2*H*-pyrazolo[4,3-*d*]pyrimidin-3-yl)benzoic acid.

In certain preferred embodiments the USP7 inhibitor is:

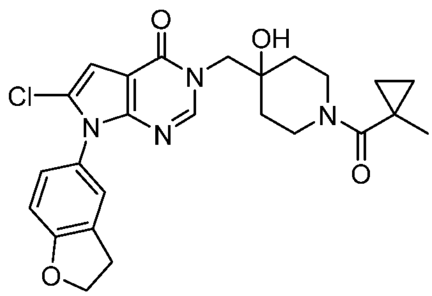

or a stereoisomer or a pharmaceutically acceptable salt thereof.

In certain preferred embodiments the USP7 inhibitor is:

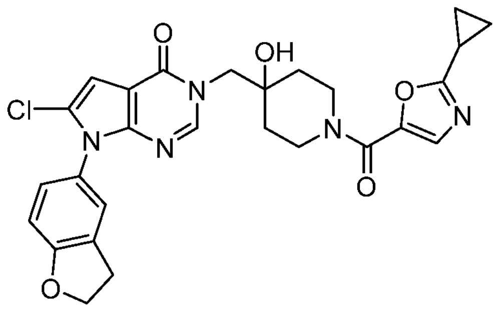

or a stereoisomer or a pharmaceutically acceptable salt thereof.

In certain preferred embodiments the USP7 inhibitor is:

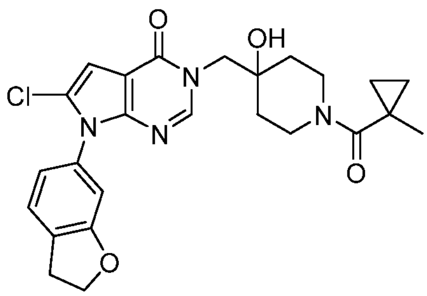

or a stereoisomer or a pharmaceutically acceptable salt thereof.

In certain preferred embodiments the USP7 inhibitor is:

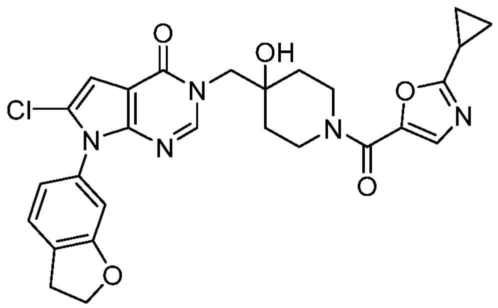

or a stereoisomer or a pharmaceutically acceptable salt thereof.

In certain preferred embodiments the USP7 inhibitor is:

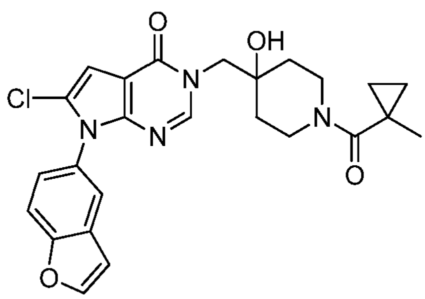

or a stereoisomer or a pharmaceutically acceptable salt thereof.

In certain preferred embodiments the USP7 inhibitor is:

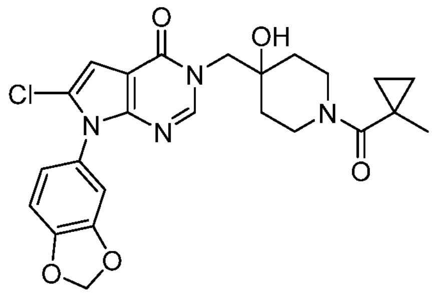

or a stereoisomer or a pharmaceutically acceptable salt thereof.

As well as suitable USP7 inhibitors to be used in accordance with the methods of the invention, all USP7 inhibitor compounds provided herein are also disclosed as compounds themselves, including their pharmaceutically acceptable salts, stereoisomers, tautomers and N-oxide derivatives thereof.

In certain alternative embodiments, the USP7 inhibitor is selected from the USP7 inhibitors provided in one or more of WO2018/073602, US 2008/0103149 A1, WO 2010/114881 A1, WO 2010/081783 A1, WO 2011/086178 A1, WO 2013/030218 A1, EP 2565186 A1, EP 1749822 A1, WO 2016/109515 A1, WO 2016/109480 A1, WO 2016/126929 A1, WO 2016/126926 A1, WO 2016/126935 A1, WO 2016/150800 A1, WO2017/158381, WO2017/158388, WO2017/212010, WO2017/212012 and US20190142834.

In regard to aspects of the invention relating to therapeutic use of compounds according to the invention, the compounds may be administered to the subject in need of treatment in an "effective amount". The term "effective amount" refers to the amount or dose of a compound which, upon single or multiple dose administration to a subject, provides therapeutic efficacy in the treatment of disease.

Therapeutically effective amounts of a compound according to the invention can comprise an amount in the range of from about 0.1 mg/kg to about 20 mg/kg per single dose. A therapeutic effective amount for any individual patient can be determined by the healthcare professional by methods understood by the skilled person. The amount of compound administered at any given time point may be varied so that optimal amounts of the compound, whether employed alone or in combination with any other therapeutic agent, are administered during the course of treatment.

When introducing elements of the present disclosure or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein or, if not defined herein, in WO2018/073602 take precedent over any dictionary or extrinsic definition.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

## EXAMPLES

### 1.1 USP7 inhibition decreases VEGF levels in primary activated fibroblasts

The role of USP7 inhibition on cell types in the tumour microenvironment other than the tumour cells themselves was investigated. AD-04 (corresponding to Example 30 in WO2018/073602) is a potent and specific USP7 inhibitor. AD-04 was screened in the cell based BioMAP assay, a clinically-relevant biomarker profiling panel which comprises various primary human cell-derived co-cultures. The screen was performed in multiple cellular disease systems: AD-04 displayed a significant impact on cytokine modulation in primary human dermal fibroblasts (HDF) co-cultured with T-cell receptor (TCR) ligands stimulated PBMCs, as well as in cancer cells (HT29 or H1299) co-cultured with HDF and stimulated PBMCs. These systems recapitulate the interactions between tumour cells, stimulated immune cells and the host stromal network. Cells were treated for 48 hours at various concentrations of AD-04 followed by cytokine measurement in co-culture supernatants by ELISA. Biomarker changes relative to the vehicle control-treated systems are presented as log-transformed ratios. Phenotypic activity profile of AD-04 showed statistically significant reduction in various biomarkers including, most strikingly, a 3-log fold decrease in secreted VEGF (sVEGF) at an inhibitor concentration of 300 nM (Figure 1 and Figure 2). The BioMap panel data findings were further confirmed in the same cancer cell line, HT29, co-cultured with activated PBMCs and multiple primary human fibroblast, including either primary lung fibroblasts WI-38 and IMR-90 or primary dermal fibroblasts HDF. *Ent*-AD-04 had no significant effect on secreted VEGF levels (Figure 3).

To confirm which cell type accounts for the decrease in VEGF observed in the co-culture system in response to

USP7 inhibition, we tested the effect of AD-04 on VEGF secretion in each cell type independently. First, four cancer cell lines were tested: LNCaP and MCF-7 are sensitive to AD-04; HT29 and H1299 are insensitive to AD-04 (data not shown). In supernatants from all tested cancer cell lines (sensitive or insensitive), sVEGF levels were not significantly affected compared to vehicle-treated cells. (Figure 4). Next, the effect of AD-04 on sVEGF levels in activated primary fibroblasts was examined. During the wound healing process or following stimulation by acute or chronic inflammatory signals, fibroblasts acquire an activated phenotype (referred to as myofibroblasts). Once activated, myofibroblasts proliferate and migrate, show contractile activity, exert physical forces to modify tissue architecture and become transcriptionally active leading to the secretion of cytokines, chemokines and extra-cellular matrix (ECM) components. In the tumour microenvironment, the majority of residual fibroblasts become activated and are known as cancer associated fibroblasts (CAF). In order to generate a myofibroblast phenotype, WI38, IMR90 and HDF were co-cultured with anti-CD3/anti-CD28 stimulated PBMCs or were stimulated by FGF-2 or TGFβ. Following activation, cells were treated with AD-04 or its enantiomer and sVEGF levels were measured by ELISA. Activated primary lung and dermal fibroblasts showed a dose-dependent decrease in sVEGF protein levels after USP7 inhibition (Figure 5). A similar decrease in sVEGF was observed in primary colorectal adenocarcinoma-associated fibroblasts upon AD-04 treatment (Figure 6). The USP7 inhibitor-mediated decrease in VEGF secretion was only observed in primary fibroblasts; immortalized lung fibroblasts (WI38-VA13) were insensitive to USP7 inhibitor treatment, probably due to the destabilized p53 in the SV-40 immortalized fibroblasts (Figure 7).

Similarly, USP7 inhibition decreased levels of intracellular VEGF protein in activated fibroblasts and CAFs, however in cancer cells and SV-40 transformed lung fibroblasts the change was not statistically significant. (Figure 8). Taken together, these results demonstrate that USP7 inhibition modulates not only secreted VEGF protein but also intra-cellular VEGF levels in activated fibroblasts, a major stromal cell population within the TME.

### 1.2 USP7 inhibition-dependent VEGF decrease is mediated *via* modulation of HIF-1α signalling

In response to wounding and during tumour growth, activated fibroblasts secrete synthesizers and modifiers of the ECM but also soluble angiogenic growth factors such as VEGF. Although *VEGF* gene expression can be controlled by multiple transcription factors, its major regulator is hypoxia-inducible factor 1 (HIF-1). HIF-1 mediates activation of *VEGF* transcription in response to hypoxia in solid tumours and in various malignant cell lines. To trigger VEGF expression by low oxygen levels, primary fibroblasts were grown in a hypoxic chamber and treated with either vehicle DMSO, AD-04 or ent-AD-04 for 48h. As expected, hypoxia dramatically induced VEGF secretion in primary human fibroblasts; VEGF secretion was decreased in a dose-dependent manner by treatment with AD-04. As in normoxia, *ent*-4 had no significant effect on secreted VEGF protein under hypoxic conditions (Figure 9).

To elucidate the role of USP7 inhibition on HIF-1α stability in activated fibroblasts, the effect of AD-04 on HIF-1α half-life was examined. HDF were pre-treated with cycloheximide followed by treatment with AD-04 or DMSO as a control and placed in a hypoxic chamber. Samples were subsequently collected at various time points for immuno-blot analysis probing for HIF-1α levels. Under these experimental conditions and following densitometry analysis, HIF-1α half-life was determined to be 6 minutes and 36 seconds in untreated cells. Treatment with AD-04 significantly reduced this value by 40% to 4 minutes (Figure 10), confirming direct modulation of HIF-1α stability by USP7 inhibition.

Given HIF-1α stability is known to be regulated at the protein level by ubiquitination, we investigated whether USP7 inhibition modulates HIF-1α polyubiquitination. Briefly, HDF were incubated in hypoxia for 3 hours followed by treatment with DMSO, AD-04 or MG132 at indicated concentrations for another 1 hour. Cells were harvested, lysed and polyubiquitinated proteins pulled down using two types of Tetra Ubiquitin Binding Entities (TUBES); with equal affinity for K48/K63 or 10 times more affinity for K63. Samples were analysed by immuno-blot using a HIF-1α antibody. As shown in Figure 11, MG132 efficiently blocked the proteasomal degradation resulting in increased polyubiquitination of HIF-1α. Treatment with USP7 inhibitor AD-04 enhanced the accumulation of K63/K48 polyubiquitinated HIF-1α (Figure 11, top panel). On the contrary, there was no difference in K63 specific polyubiquitination of HIF-1α between DMSO control and treatment with AD-04 (Figure 11, bottom panel). These findings indicate that USP7 inhibition results in K48 polyubiquitination of HIF-1α and consequently, its proteasomal-mediated degradation.

### 1.3 Mechanism of action of USP7 inhibition of VEGF secretion in primary activated fibroblasts

To understand the mechanism of action of USP7 inhibitor AD-04 and evaluate the pathways involved in the USP7 inhibition of hypoxia activated fibroblasts, RNA-seq was performed. HDF were treated with vehicle DMSO and AD-04 followed by incubation in either normoxia or hypoxia for 6 or 24 hours. Afterwards RNA was extracted and analysed by RNA-seq. Comparison of VEGF mRNA levels showed that its expression is induced under hypoxic conditions and down-regulated after the treatment with AD-04 (Figure 13). These findings suggest that USP7 inhibitor mediates VEGF modulation both at the protein and mRNA levels. To evaluate which signalling pathways are induced in hypoxia activated fibroblasts, a comparative pathway analysis was performed on of samples coming from both, non-activated (normoxia)

and hypoxia activated primary fibroblasts. The results showed that HIF1A mRNA expression network is the most significantly enriched pathway using the NCI-Nature canonical pathway database in hypoxia activated fibroblast samples (Figure 12). Furthermore, treatment with AD-04 of the same samples results in modulation of known genes in the HIF1A expression network, including the VEGF gene (Figure 14). Interestingly, hypoxia activated and USP7 inhibitor-treated primary human fibroblasts display alternative patterns of VEGFA alternative splicing. At 24 hours the mRNA expression of the anti-angiogenic short (191aa) isoform VEGF-165 is up-regulated. The pro-angiogenic long (371aa) isoform of VEGF-165 is strongly down-regulated at 24 hours (Figure 15). These data suggest compensatory feed-back loops on VEGF expression upon USP7 inhibition and further stress the importance of the USP7-mediated modulation of VEGF expression.

**1.4 The modulation of VEGF secretion in primary human fibroblasts is USP7 specific**

It is well known that the inhibition of USP7 results in degradation of the oncoprotein E3 ligase MDM2 and increase in p53 levels. To confirm that that the modulation of VEGF protein in activated fibroblasts is not mediated via MDM2-p53 axis, a benchmarking experiment was performed against clinically relevant MDM2 antagonists. Cancer cells, immortalized fibroblasts and FGF-2 activated primary fibroblasts were grown in normoxia or hypoxia, followed by treatment with vehicle, USP7 inhibitor AD-04, its inactive enantiomer (*ent*-4), Nutlin-3a, RG7112 or SAR405838 at indicated concentrations for 48 hours. Cell culture supernatants were collected and sVEGF was measure by ELISA. In parallel, cells were treated as described above in both normoxia and hypoxia followed by cell viability measurement after 72 hours. Tested MDM2 antagonists were not able to modulate the secretion of VEGF as compared to AD-04 (Figure 16 and 17). These results confirmed that modulation of VEGF secretion in primary human fibroblasts upon USP7 inhibition is not mediated *via* MDM2. Furthermore, AD-04 and its inactive enantiomer do not affect cell viability at high concentration as compared to MDM2 antagonists (Figure 18). To ensure that the effect of USP7 inhibitor is mediated *via* direct USP7 modulation, we monitored USP7 cellular target engagement using activity-based probe competition assays. Cells were treated with increasing concentrations of inhibitor, lysed, and the ubiquitin-propargylamine (Ub-PA) probe was added. AD-04 efficiently competed with the Ub-PA probe in a concentration-dependent manner ($EC_{50}$ = 11-24 nM, $\pm$5 S.D) demonstrating highly potent target engagement in primary fibroblasts, immortalized fibroblasts and cancer cells (Figures 19 and 20). The specificity of our selective USP7 inhibitor AD-04 on VEGF secretion in primary human fibroblasts was confirmed by USP7 CRISPR knock-out. CRISPR/Cas9 induced knockdown of USP7 in primary HDF was verified by immuno-blot analysis using anti-USP7 antibody and resulted in almost complete abrogation of sVEGF protein detected in cell culture supernatants after 48h culture in hypoxia (Figure 21).

**1.5 AD-04 inhibits fibroblast invasion and downregulates MMP-7 secretion in invading fibroblasts**

To examine the effect of USP7 inhibition on proliferation of primary fibroblast, immortalised fibroblast, endothelial cells and cancer cells, live cell time-lapse imaging was used. The results showed no effect of AD-04 on cancer cell, fibroblasts or endothelial cells proliferation (Figure 22). Next, the effect of AD-04 on cell migration was investigated using the scratch wound cell migration assay. Treatment with AD-04 did not modulate migration (scratch wound healing process) in any of tested cells indicating that USP7 inhibition does not have an effect on cell migration in either cancer cells, fibroblasts or endothelial cells (Figure 23). Finally, the function of AD-04 in the invasive capability of the cells was examined in scratch wound cell invasion assay. The number of invading cancer cells and immortalized fibroblasts did not change after the treatment with AD-04. However, invasion of primary fibroblast through Matrigel® basement membrane was greatly reduced upon USP7 inhibition as compared to vehicle-treated cells (Figure 24). These findings demonstrated the ability of AD-04 to inhibit basement membrane remodelling by primary activated fibroblasts.

It has been demonstrated that in addition to VEGF, matrix metalloproteinases have been implicated in tumour invasion, and metastasis through degradation of ECM components. To further explore whether USP7 inhibition modulates MMP levels in invading fibroblasts, cell culture supernatants from HDF invasion assay were collected and presence of various MMP was detected using Luminex® Multiplex assay. The results showed significant reduction in MMP-7 levels in samples treated with USP7 inhibitor as compared to vehicle-treated sample. *Ent* AD-04 and Avastin did not have significant effect on MMP-7 levels (Figure 25).

**1.6 USP7 inhibition reduces tube formation *in vitro* and decreases MMP-2 levels in endothelial-fibroblast cell co-culture**

In addition to their primary role in synthesis and maintenance of the extracellular matrix, fibroblasts have the capacity to alter the mechanical extracellular microenvironment and therefore regulate vascularization processes. Fibroblast-derived proteins, including growth factors and matrix proteins, have been shown to induce, support and modulate endothelial cell sprouting and the expansion of capillary-like networks (tubes) *in vitro.* The effect of USP7

inhibition on HDF capacity to support the formation of capillary-like structures was assessed in a co-culture system with primary human endothelial cells (HUVEC). To examine the effect of AD-04 on *de-novo* tube formation, co-cultures were treated immediately post-seeding. The effect of AD-04 on pre-formed tubes was investigated by treating co-cultures several days after seeding when tubes already formed. After 14 days of incubation, co-culture of HDF and HUVEC resulted in the formation of vascular tubes. However, USP7 inhibition led to significant reduction in tube length in a dose-dependent manner (Figure 26, top panel). Interestingly, when pre-formed capillary-like networks were treated with AD-04, the inhibition of tube formation was significantly less pronounced (Figure 26, bottom panel). The average length of capillary-like structure was determined (Figure 26, charts). Taken together, these results indicate that USP7 inhibition results in prevention of *de novo* tube formation from HDF and HUVEC co-culture. In addition to fibroblast and endothelial cell co-culture, tubes can also be formed within 6 hours when HUVEC are grown on Matrigel®. Accordingly, direct effect of USP7 inhibition was examined on HUVEC tube formation. AD-04 treatment did not disrupt HUVEC tube formation on Matrigel® (Figure 27) supporting the previous findings where USP7 inhibition impacts only activated fibroblasts and that the effect observed on tube formation is mediated by USP7 inhibitors in primary fibroblasts.

Next, the underlying mechanism of tube formation inhibition was investigated. The induction of MMP-2 was reported to increases tube formation by endothelial cells *in vitro* while MMP-2-deficient mice showed suppression of tumour-induced angiogenesis. To determine whether secretion of MMP-2 is modulated by USP7 inhibition, supernatants from HUVEC and HDF co-cultures were collected after 14 days. As shown in Figure 28, a dose dependent decrease in MMP-2 levels upon USP7 inhibition was observed. *Ent*-AD-04 and Avastin did not have significant impact on MMP-2 levels.

**1.7 US7 inhibition suppresses *in vivo* growth of tumour xenografts, decreases serum VEGF levels and facilitates the recruitment of immune cells into the TME**

To examine the *in vivo* antitumor activity of USP7 inhibition and its impact on the tumour stroma, a syngeneic mouse xenograft model of CT-26 cells in BALB/C mice was established. As a positive control, Sorafenib, a multikinase inhibitor which exhibits both antitumor and antiangiogenic properties was used. The CT-26 mouse cancer cell line is not sensitive to AD-04 inhibition *in vitro,* making it a good model to study the impact of USP7 inhibition on the TME (Figure 29). As shown in Figure 30, AD-04 inhibited the growth of CT26 xenografts in a dose-dependent manner as evidence by decrease in tumour volume. The highest AD-04 dose had similar tumour growth inhibition (53%) as treatment with the positive control Sorafenib (58%). Both AD-04 and Sorafenib treatments exhibited low toxicity, as no significant bodyweight loss was induced in tumour-bearing mice by either treatment (Figure 29, left panel). As observed in primary fibroblasts *in vitro,* measurement of circulating VEGF in mouse serum showed a significant dose-dependent decrease in animals treated with AD-04 (Figure 31).

TME has been shown to play a key role in tumour progression and prognosis. Efficacy of anti-cancer therapies depends on TME profile. For instance, tumour immune cell infiltration is considered to be an important factor determining successful immune checkpoint inhibition and is associated with improved survival of patients. To understand whether AD-04 modulates the recruitment of immune cells into tumours, flow cytometry was used to profile several immune populations. We first assessed the frequency of various T cell subpopulations among tumour-infiltrating lymphocytes (TILs). The results showed a significant increase in cytotoxic T-lymphocytes after AD-04 treatment while no significant change was observed in CD4 lymphocytes. In contrast, a trend in Treg/CD8 ratio decrease upon USP7 inhibition was observed. Analysis of myeloid cell infiltrate showed decrease in macrophages after the treatment with USP7 inhibitor (Figure 32).

Finally, mode of action of AD-04 *in vivo* was analysed by RNA-seq. Analysis of the syngeneic CT-26 tumour samples showed different splicing of tumour VEGFA mRNA; a decrease in the VEGFA short isoform and increase in the long VEGFA isoform was observed after the treatment with the USP7 inhibitor AD-04. Pathway analysis demonstrated that the HIF1A mRNA expression network was in the top 10 most significantly enriched pathways (NCI-Nature definition) in tumour samples treated with USP7 inhibitor at 100mg/kg for 10 days. Moreover, individual genes within HIF1A pathway were differentially modulated upon USP7 inhibition. These results support previous findings and confirm the concept of USP7 modulation of TME *via* HIF-1$\alpha$ pathway both *in vivo* and *in vitro.*

**1.7 Fibroblast-mediated anti-cancer effects of US7 inhibition are observed for multiple USP7 inhibitors**

To demonstrate that the effects of USP7 inhibition are not restricted to AD-04, an alternative USP7 inhibitor was assessed. ADC-159 is a USP7 inhibitor with potency and selectivity equivalent to AD-04. ADC-159 has the structure:

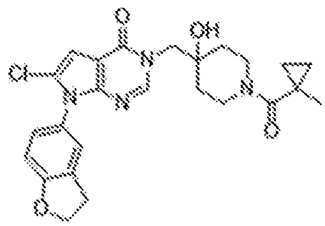

To assess its potency in a functional assay, HDF were treated with vehicle or various concentrations of USP7 inhibitor ADC-159 and incubated for 48 hours in a hypoxic chamber. Cell culture supernatants were collected, and secreted VEGF was measured by ELISA. As shown in Figure 33, ADC-159 decreased VEGF levels in a dose-dependent manner showing comparable potency to AD-04. Comparable results were observed for other USP7 inhibitors ADC-160, ADC-198, ADC-199 and ADC-556 (Figure 34). Furthermore, in HDF and HUVEC tube formation assay, ADC-159 showed inhibitory effect on newly formed tubes in a dose-dependent manner while it did not have significant effect on already established tubes (Figure 35). The inhibitory effect of ADC-159 on tube formation is comparable to AD-04.

To examine the *in vivo* antitumor activity of USP7 inhibition by ADC-159 and its impact on the tumour stroma, ADC-159 was tested in a syngeneic mouse xenograft model of CT-26 cells in BALB/C mice. Similarly to AD-04, the CT-26 mouse cancer cell line is not sensitive to ADC-159 *in vitro,* making it a good model to study the impact of USP7 inhibition on the TME.

Investigation in the CT-26 syngeneic mouse model showed that ADC-159 prevents tumour vessel formation *in vivo.* Control animals treated with vehicle only exhibited tumour vessel formation at the tumour site, characterised by fine branching of mature blood vessels, the fine branches detecting by positive staining for endothelial marker CD31 and pericyte marker NG2 (Figure 36A). In contrast, ADC-159 treatment prevented this fine branching of mature blood vessels at the tumour site (Figure 36B).

ADC-159 treatment resulted in a significant reduction in tumour area in the CT-26 *in vivo* model compared to control animals treated with vehicle alone (Figure 37A) despite the resistance of CT-26 cells to direct USP7 inhibition by ADC-159. ADC-159 therapy also resulted in a significant increase compared to vehicle controls in the necrosis area at the tumour site (Figure 37B), providing further evidence of the effects of USP7 inhibition on the TME resulting in reduced tumour growth.

**1.8 USP inhibitors combine synergistically with immune checkpoint inhibitors (ICIs)**

As a consequence of their TME reprograming activities, USP7 inhibitors have the potential to deliver combination efficacy with other agents known to modulate the TME such as immune checkpoint inhibitors. In the syngeneic CT-26 mouse model, USP7 inhibition (ADC-159) alone results in reduced tumour volume versus vehicle controls (Figure 38A). Mice treated with a combination of USP7 inhibitor (ADC-159) and an immune checkpoint inhibitor (anti-PD-L1 or anti-CTLA4 antibodies) resulted in a significant further reduction in tumour volume. In particular, ADC-159 produced a tumour growth inhibition (TGI) of 23%, very similar to the level achieved by anti-PD-L1 (22%). The combination of ADC-159 and anti-PD-L1 exhibited synergistic efficacy leading to further reduced tumour growth (TGI=43%). Treatment with anti-CTLA-4 resulted in 43% of TGI which was similar to TGI produced by ADC-159 and anti-CTLA-4 combo (Figure 38A). No significant changes in body weight were observed, indicating the treatments were well tolerated (Figure 38B).

The synergy between USP7 inhibitor and ICI results in significantly improved survival in an *in vivo* tumour model. Figure 39 shows the survival plot from the syngeneic CT-26 mouse model described herein, where mice were treated with USP7 inhibitor ADC-159 alone, anti-PD-L1 or anti-CTLA4 alone, or a combination of USP7 inhibitor ADC-159 and anti-PD-L1 or anti-CTLA4. Vehicle treatment only served as control.

Figure 39 shows that mice treated with the combination of a USP7 inhibitor and an ICI had significantly increased survival compared to either alone. The effect was greater than an additive effect of two therapies, evidenced by anti-PD-L1 treatment alone resulting in no improvement in survival compared to vehicle controls. Treatment with ADC-159 resulted in a modest but significant increase in survival. In contrast, the combination of ADC-159 with anti-PD-L1 resulted in a much greater increase in survival. The same synergistic combination effect was also observed with ADC-159 and anti-CTLA4 where survival at 50 days was as high as 80% (Figure 39A). The effect on tumour growth of each treatment is shown in Figure 39B.

Combination with a USP7 inhibitor resulted in mice treated with an immune checkpoint inhibitor exhibiting increased infiltration of cytotoxic T lymphocytes into the tumour (Figure 40). Without being bound by theory, it is hypothesised that the synergistic effect of the combination arises from the increased recruitment of TILs as a result of USP7 inhibition provides a larger population of effector cells at the tumour site able to be activated by the ICIs.

### 1.9 Conclusions

USP7 inhibitors have previously been shown to modulate the ubiquitylation of the oncoprotein MDM2 and inhibit the cancer cell proliferation. To the best of our knowledge, the data reported herein represents the first published role of USP7 in the reprogramming of TME, targeting major stromal cell population-fibroblasts.

We performed phenotypic screen of a USP7 inhibitor in a disease relevant co-culture system and identified VEGF as a main biomarker modulated by USP7 inhibition. We have found that this effect is specific only to activated fibroblasts and not cancer cells or immortalized fibroblasts. To become activated and acquire myofibroblast phenotype, normal fibroblasts can be stimulated with different molecules including FGF-2, TGFβ or immune cells. Reduction in secreted VEGF upon USP7 inhibition was observed in patient cancer-associated fibroblasts, demonstrating robust effect on myofibroblasts, regardless of the way of their activation (Figure 1-7). Moreover, VEGF decrease is not limited only to the secreted protein but is also observed at intracellular level (Figure 8).

The specificity of USP7 inhibition on VEGF modulation was confirmed by CRISPR/Cas9 USP7 knockout where decrease in secreted VEGF levels from activated fibroblasts was comparable to the one observed with small molecule inhibitor (Figure 21). In addition, high potency against USP7 in a cellular target engagement assay in primary fibroblasts ($EC_{50}$ = 11-24 nM, Figure 20) demonstrates that the effect of USP7 inhibitors is mediated *via* direct USP7 modulation.

The major activator of *VEGF* gene expression is HIF-1α transcriptional factor. Multiple stimuli, such as growth factors can induce VEGF expression in a HIF-1-dependent manner in normoxic cells. However, HIF-1α protein is undetectable in most cell types due to rapid degradation by the ubiquitin-proteasome system. On the contrary, hypoxia induces the accumulation of HIF-1α protein and consequently, VEGF protein. Indeed, low oxygen levels induce VEGF secretion in fibroblasts as presented in Figure 9. We identified that the mechanism of action of USP7 inhibition in activated fibroblasts is *via* modulation of HIF-1α protein. We showed that USP7 inhibition results in reduction of HIF-1α half-life and K48 specific polyubiquitination, leading to its proteasomal-mediated degradation (Figure 11 and 13). RNA-seq of hypoxia-activated fibroblasts revealed that upon USP7 inhibition, in addition to VEGF protein level, VEGF mRNA is also decreased (Figure 12). This may be indicative of feed-back regulation of the HIF1 pathway, supporting the critical USP7 function in modulating an essential stress response pathway in the TME. Furthermore, results from pathway analysis showed that HIF-1α signalling pathway is highly ranked in response to USP7 inhibition with known genes involved in HIF-1α signalling being modulated (Figure 14 and 15). These findings support a role for USP7 in direct modulation of HIF-1α protein and HIF1 pathway in the TME.

We benchmarked the observed effect of a USP7 inhibitor (AD-04) against established MDM2 antagonists and showed that all three tested MDM2 antagonists do not modulate secreted VEGF protein levels in activated fibroblasts as compared to AD-04 (Figures 17 and 18). These results demonstrate a strong differentiation from MDM2 compounds. We also demonstrate a differentiation from the anti-angiogenic compound bevacizumab, anti-VEGF monoclonal antibody (Figure 26).

A crosstalk between fibroblasts, immune cells, endothelial and cancer cells results in induction of growth factors, cytokines, extra cellular matrix proteins and the matrix-degrading enzymes metalloproteinases, required for the tumour cell proliferation and metastasis. We sought to explore whether USP7 inhibition has an impact on cell function within TME such as proliferation, migration and invasion. AD-04 did not have any significant effect on proliferation and migration of any cell type within the TME (Figure 22 and 23), it showed however a strong inhibition of fibroblast invasion through basement membrane (Figure 24). These findings are important given the fibroblasts ability to degrade ECM and promote invasion of cancer cells. A tumour invasion mechanism has been reported in which cancer cells invade the collagen matrix by binding to fibroblasts through the integrin/fibronectin interaction. A possible mechanism of fibroblast invasion inhibition upon the treatment with a USP7 inhibitor could be through observed decrease in MMP7 levels in invading fibroblasts (Figure 25) since MMP-7 degrades the major components of the tubular basement membrane such as elastin, collagen type IV and laminin to induce epithelial-mesenchymal transition (EMT). Another role of fibroblast in reprogramming of TME is regulation of vascularization process. We showed that USP7 inhibition prevents *de novo* tube formation in fibroblast and endothelial cell co-culture *in vitro* (Figure 26). USP7 inhibition, however, does not directly affect endothelial cells tube formation as AD-04 is not able to modulate *de novo* endothelial cells formation in absence of fibroblasts (Figure 27) demonstrating that USP7 inhibitor AD-04 targets only fibroblast cell population within the TME. MMP-2 is known to be important in tube formation *in vitro.* Decrease in MMP-2 levels upon USP7 inhibition observed from fibroblast and endothelial cell co-culture points towards the possible mechanism of action of tube formation inhibition. The mRNA expression level of MMP-2 as well as VEGF showed a positive correlation with HIF-1α in tissue samples from colon adenoma and colon cancer supporting our new findings related to USP7 inhibition. Taken together, these results clearly demonstrate that upon USP7 inhibition fibroblasts undergo ECM remodelling and thus reprogram the TME.

While USP7 inhibition did not affect CT-26 cell line proliferation *in vitro* nor have a significant effect on secreted VEGF levels (Figure 29), *in vivo* experiments showed USP-7 inhibitor-mediated tumour growth inhibition in CT-26 syngeneic xenografts (Figures 30 and 37-39). The effect of USP7 inhibition on tumour growth inhibition *in vivo* was similar to the Sorafenib positive control treated group (Figure 30). In a syngeneic tumour model, USP7 inhibition synergistically

combined with immune checkpoint inhibitor treatment to result in significant improvements in survival (Figure 39).

We observed a significant decrease in circulating VEGF protein levels from the mouse serum samples (Figure 31), suggesting a strong general effect of USP7 inhibition on the overall whole-body levels of the VEGF growth factor. The increase in number of cytotoxic lymphocytes and reduction of macrophages within the tumours after the treatment with the USP7 inhibitor further confirm the reprogramming of TME by infiltrating the immune cells (Figures 32 and 40). Moreover, RNA-seq analysis of tumour samples confirmed that the USP7 inhibitor modulates pathways known to strongly impact the TME *in vivo,* including the HIF-1$\alpha$ pathway.

Altogether, demonstrated herein are completely novel modes of action of USP7 in modulating and reprograming the TME by directly impacting the levels of the VEGF growth factor in fibroblasts and modulating the tumour immune microenvironment. The role of USP7 in reprogramming the TME is not linked to the previously characterised role of USP7 in modulating the oncoprotein MDM2. Described herein is a unique role for USP7 in primary or cancer-associated fibroblasts and not a mechanism of action observed in cancer cells or other primary cells present in the TME. The data provided herein supports novel therapeutic strategies for USP7 inhibitors by inhibiting USP7 function in fibroblasts, especially CAFs.

**Materials and methods**

Cells and culture conditions

All primary cells and cell lines were obtained from the American Type Culture Collection (ATCC), authenticated by STR profiling (Promega) and shown to be mycoplasma-free using the MycoAlert mycoplasma detection (Lonza; LT07-318). For growth, cells were maintained at 37 °C in a humidified atmosphere with 5% $CO_2$. HT29 (colorectal) cells were cultured in McCoy's medium 5A supplemented with 10% (v/v) FBS, 1% (v/v) penicillin-streptomycin, 1% (v/v) L-glutamine. LNCaP (prostate) cells were cultured in RPMI supplemented with 10% (v/v) FBS, 1% (v/v) penicillin-streptomycin, 1% (v/v) L-glutamine. H1299 (lung) and CT26 (mouse colon carcinoma) cells were cultured in RPMI supplemented with 10% (v/v) FBS and 1% (v/v) penicillin-streptomycin. MCF7 (breast) cells were cultured in Eagle's Minimum Essential Medium supplemented with 10% (v/v) FBS, 0.01 mg/mL human recombinant insulin and 1% (v/v) penicillin-streptomycin. HDF were cultured in fibroblasts basal media supplemented with Fibroblast Growth Kit-Low Serum (final concentration for each component is as follows: L-glutamine 7.5 mM; rh FGF basic 5 ng/mL; rh Insulin 5 $\mu$g/mL; Hydrocortisone 1 $\mu$g/mL; Ascorbic acid 50 $\mu$g/mL; Fetal bovine serum 2%). WI38, WI38-VA13 and IMR-90 were cultured in Eagle's Minimum Essential Medium supplemented with 10% (v/v) FBS. HUVEC were cultured on flasks coated with 0.2% gelatin in Vascular cell basal media supplemented with Endothelial Cell Growth Kit-BBE (final concentration for each component is as follows: Bovine brain extract (BBE) 0.2%; rh EGF 5 ng/mL; L-glutamine 10 mM; Heparin sulfate 0.75 Units/mL; Hydrocortisone 1 $\mu$g/mL; Ascorbic acid 50 $\mu$g/mL and Fetal bovine serum: 2%). Medium and supplements were purchased from Life Technologies and ATCC except where indicated.

Target engagement assay

HT-29, CT-26, IMR-90 and HDF cells were treated with vehicle (DMSO) or USP7 inhibitors for 2. HDF were placed in hypoxic chamber while other cells remained in normoxia. Following incubation, cells were washed extensively thrice with 1 $\times$ PBS and harvested in TE lysis buffer containing 50 mM Tris-HCl (pH7.4), 150 mM NaCl, 5 mM $MgCl_2$, 0.5 mM EDTA, 0.5% NP40, 10% glycerol, 2 mM DTT and clarified cell lysates (30 $\mu$g) incubated with Ub-PA (8 $\mu$g/mL final concentration) in assay buffer containing 50 mM Tris-HCl (pH7.6), 5 mM $MgCl_2$, 250 mM sucrose, 0.5 mM EDTA, and 2 mM DTT for 30 min. The reaction was terminated by the addition of LDS sample buffer (Life Technologies) and heated to 70 °C. Samples were then analyzed by western blotting using the Cell Signaling anti-USP7 Ab (#4833; 1 :1,000 dilution). $EC_{50}$ values were determined upon densitometry analysis. Band intensities were quantified using ImageJ software where the upper bands (USP7-Ub) and lower bands (USP7) were calculated as a percentage of the corresponding DMSO controls (-/+ Ub-PA) and values were then normalized to the sum of the lower and upper bands for each concentration.

Cell proliferation assay

Cells were seeded in 96-well plate format (typically 4000 cells/well and treated after 24 h with increasing concentrations of compound (ranging from 100 $\mu$M to 1 nM) in normoxic or hypoxic conditions as indicated. After 72h cell viability was assessed by CellTiter-Glo using a Synergy 4 plate reader (BioTek). Analysis and $EC_{50}$ values were derived using GraphPad Prism (GraphPad Software, Inc, La Jolla, CA; four-parameter logistic function). Data are presented as mean $\pm$ s.d. ($n \geq 3$).

*In vitro* co-culture tube formation assay and immunostaining

HDF were seeded in 96-well plate (2500 cells/well). Once HDF formed a monolayer, media was removed and HUVECs (2500 cells/well) were seeded on top of HDF. To assay the effect on new vessel formation, cultures were treated with AD-04 (10nM-1μM), its inactive enantiomer, DMSO and Avastin 24h post-seeding. The effect on existing vessels was assayed by allowing tubes to form prior to treatment. Cells were treated every 3-days during 14 days of incubation, followed by washing with PBS and fixation with 4% formaldehyde for 15 min at room temperature (RT). Cells were permeabilized with 1 × PBS containing 0.1% Triton X-100 for 5 min at RT and blocked 30 min in 1% BSA/PBS. Afterwards, cells were incubated with CD31 antibody (Thermo Fisher, MA5-13188) at 1/50 in 1%BSA/0.1%Tween-20/PBS overnight at 4°C 1/50 followed by incubation with secondary Alexa fluor 488F goat-anti-mouse (Thermo Fisher, A28175) at 1/2000 dilution for 3h at RT. Immunolabeled samples were counterstained with Hoechst 33342 nuclear dye (Thermo Fisher, 62249) at 1/1000 dilution for 15 min. Tubes were visualized with IN Cell Analyzer 2000 using a 2X objective. Tube length was measured and quantified using the AngioTool software for each imaging session (available in the public domain at https://ccrod,cancer, gov/confluence/display/ROB2/Downloads

Matrigel® Tube Formation Assay

Each well of a pre-chilled 96-well plate was coated with 50μL of unpolymerized growth factor reduced Matrigel® (9.2 mg/mL) and incubated at 37°C in 5% $CO_2$ for 2h. HUVECs were harvested with trypsin, and $1.5\times10^4$ cells were resuspended in 100 μL complete endothelial cell growth medium. Cells were treated with the vehicle (DMSO), AD-04, ent-AD-04 and Avastin at different concentration before plating onto the Matrigel®-coated plates. After approximately 6 hours of incubation at 37°C in 5% $CO_2$, images of the center of each were taken at 4X magnification.

Ubiquitination assays

Cells were incubated under hypoxic condition for 3h followed by 1h treatment with a proteasome inhibitor, MG132 (Sigma) at 10 μM, AD-04 at 1 μM or DMSO. Cells were lysed in a buffer containing 50mM Tris-HCl, pH 7.5, 150mM NaCl, 1mM EDTA, 1% NP-40, 10% Glycerol, 50mM NaF, 5mM sodium pyrophosphate, 10mM glycerol phosphate, 1mM sodium orthovanadate, protease and phosphatase inhibitor tablet and 50uM PR-619. Lysates were pre-cleared by centrifugation at 4 ºC for 15 mins at 16,000 x g and protein concentration determined using BCA method. 20 ul of samples was taken as input. Pre-cleared supernatant containing 0.5mg of total protein was added to 20 ul equilibrated Agarose-TUBE 1 or TUBE 2 beads (Life Sensors) and incubated for 2 hours at 4°C on a rocker platform. Beads were collected by low speed centrifugation (1000-5000xg, 4ºC) for 5 minutes and supernatant taken as FT. Beads were washed twice with 1ml TBS-T (20mM Tris-HCl, pH 8.0, 0.15M NaCl, 0.1% Tween-20) and collected by low speed centrifugation. Finally, beads were resuspended in 30ul of SDS reducing sample buffer and boiled for 10min at 95°C. Samples were subjected to Western blot analysis by using anti- HIF-1a antibody (D2U3T, Rabbit mAb #14179 Cell Signalling).

Cyclohexamide chase assay

In order to determine the half-life of HIF1α in cells, HDF cells were incubated for 3h in hypoxic conditions followed by treatment with DMSO or AD-04 at 1μM in the presence of cycloheximide (100μg/mL) to block nascent protein synthesis. Cells were harvested and lysed at different time points and subjected to Western blot analysis using anti- HIF-1a antibody (D2U3T, Rabbit mAb #14179 Cell Signalling) and β-actin (Santa Cruz: A5316; 1:5000) as a loading control.

MMP detection

Concentrations of MMP2 and MMP7 were determined in cell culture supernatants in triplicates using Luminex human cytokine/chemokine multiplex kits (Millipore, St. Charles, MO, USA) according to the manufacturer's protocol.

Detection of VEGF using enzyme-linked immunosorbent assay (ELISA)

For the co-culture experiments, $5\times10^4$ cancer cells were seeded in transwell in 0.5mL complete growth medium and placed on the top of $0.1\times10^5$ fibroblasts plated in 12-well in 1 mL low serum growth media overnight. Following day cancer cells were washed with PBS and media replaced with reduced (1%) serum growth media. $0.5\times10^6$ PBMC were plated on top of fibroblasts, incubated for 45 min and activated with CD3 (1μg/ml final concentration) and CD28 (5μg/ml final concentration). Afterwards co-cultures were treated with vehicle (DMSO), AD-04 and ent-AD-04 for 48h at 37°C in 5% $CO_2$. Monoculture experiments, $0.1\times10^5$ cells were seeded in 12-well in 1mL of complete growth media (cancer cells) or low serum growth media (fibroblasts) and incubated overnight. Next day, cancer cells were washed with PBS and media

replaced with reduced (1%) serum growth media followed by treatment with vehicle (DMSO), AD-04, ent-AD-04, MDM-2 antagonists nutlin-3a (Tocris; #3984), SAR405838 (MedchemExpress; MI-773, #HY-17493) and RG7112 (MedchemExpress; #HY-10959) for 48h at 37°C in 5% $CO_2$ in normoxia or hypoxia. Afterwards, cell culture media was collected, cell debris were removed by centrifugation the concentration of VEGF in the cell culture supernatants was measured using the human/mouse VEGF immunoassay Quantikine ELISA kit (R&D systems, Minneapolis,MN, USA) according to the manufacturer's instructions. For the intracellular VEGF detection, total protein concentration was determined by BCA (Pierce Chemical, USA) and VEGF levels determined using the same kit described above.

BioMap phenotypic screen

BioMAP systems were employed using primary human cells. These studies follow the guidelines for human subjects research under HHS human subjects regulations (45 CFR Part 46) for the United States. Human neonatal foreskin fibroblasts (HDFn) from 3 donors were pooled and cultured according to the supplier's (Lonza, Inc., Allendale, NJ) recommendation. Peripheral blood mononuclear cells (PBMC) were prepared from buffy coats from normal human donors according to standard methods. Autoimmune HDFSAg system consisted of primary human dermal fibroblasts (HDF) co-cultured with PBMCs stimulated with T-cell receptor (SAg) to model chronic T cell activation and inflammation. Stromal Oncology Colorectal Cancer and Non-Small Cell Lung Cancer (NSCLC) panels were composed of cancer cells (HT29 or H1299), HDF and PBMCs stimulated with SAg. This model captures the interactions between tumour cells, stimulated immune cells and the host stromal network. Co-cultures were activated with SAg, (20 ng/ml) and treated with vehicle and AD-04 at the concentration of 10, 3.3, 1.1 and 0.37μM for 48h. Afterwards, biomarkers were measured in supernatants from co-cultures using ELISA as following: MCP-1, VCAM-1, Collagen I, IP-10, MMP-1, sIL-10, sIL-17A, sIL-17F, sIL-2, sIL-6, SRB, sTGFb, sTNFa, sVEGF, IL-8, MIG, MCSF, uPAR, Col-III, IP-10, EGFR, HGF, Pal-1, PBMC Cytotoxicity, tPA, uPA, sGranzyme, sPGE2, slFg, Sil-13, sMDC, Collagen III, MMP-9, TIMP-2, CEACAM5, Keratin 20. Biomarker levels are presented as log-transformed ratios ( $\log 10 \frac{AD\ 04}{Vehicle\ control}$ ).

CRISPR/Cas9 RNPs knock-out

USP7 specific crRNA and the non-specific tracrRNA were mixed in equimolar concentrations in a microcentrifuge tube to form tracrRNA:crRNA duplex (guide RNA). Samples were heated at 95°C for 5min and allowed to cool down at RT. To form ribonucleoprotein (RNP) complex of recombinant Cas9 coupled to the guide RNA, Cas9 enzyme (21uM final concentration) was added to the tracRNA:crRNA duplex. RNP complex was incubated at RT for 20 min. Prior to electroporation, HDF were harvested by trypsinisation and washed with PBS. Pellets containing $5x10^5$ cells were mixed with 94 μl of Nucleofector solution (Amaxa Human Dermal Fibroblasts Nucleofector, Lonza), 5μl of the correct RNP or 2μg total pmaxGFP and 1μl of Alt-R Cas9 Electroporation Enhancer (final concentration 1μM) were added to each tube, mixed and transferred to electroporation cuvette. Subsequently, the cells were nucleofected by using the U-020 program from the nucleofection device (Nucleofector IIb Device) and 500 μl of pre-warmed culture medium was immediately added to the cells. Cells were grown for 9 days allowing the phenotype to develop.

Migration and invasion assays

96-well ImageLock plates were coated with 0.1 mg/ml growth factor reduced Matrigel® and incubated for 1h at 37°C. Cells were seeded a density of 10,000-40,000 cells/well in 100 μL/well and incubated overnight. Next day wounds were simultaneously created in all wells using the WoundMaker. After wounding, media was aspirated from each well and cells were washed twice with PBS. For invasion cells were overlayed with 50μL of the Matrigel® top layer at 3mg/ml and incubated for 30 min at 37°C. Afterwards, 100 μl of culture media containing vehicle (DMSO), AD-04, ent-AD-04 and Avastin was added to each well. Cell plates were placed into the IncuCyte live-cell analysis system and each well imaged using 10x objective every 2 hours for the total of 5-days. Images were analysed using IncuCyte scratch wound protocol and results presented as percentage of wound confluence.

Tumour model

For subcutaneous tumour implantation, CT26 cells were injected subcutaneously ($1x10^6$ cells in 200 μL of RPMI) in the right flank of 8- to 10-week-old BALB/c female mice. Mice received Subcutaneous infusion of vehicle, Subcutaneous infusion of AD-04 with ALZET® pumps at a dose of 30 mg/kg/day and 100 mg/kg/day or oral administration of sorafenib at 50mg/kg/administration once every day for total of ten days. Tumour volume (in $mm^3$) was measured twice a week with a digital caliper and calculated by the following formula: volume = $(width)^2 \times length/2$. Body weight was measured twice a week. On day 10 post-implantation mice were sacrificed, and tumours were harvested for downstream experiments.

For the immune checkpoint inhibitor *in vivo* experiments, BALB/c mouse was injected subcutaneously in the right rear flank with 5x $10^5$ viable CT-26 tumour cells in 0.1 mL of PBS. ADC-159 (75 mg/kg and 100 mg/kg) was dosed daily while anti-PD-L1 or anti-CTLA4 (10 mg/kg) were dosed every 3 days. Tumour volume ($mm^3$) was measured with callipers three times a week using the equation: Tumour Volume (TV) = $0.5((W)^2 \times L)$, where W is the shortest tumour diameter (width) and L is the longest perpendicular diameter (length), in millimetres. Body weights were measured on the same days during the dosing phase. Tumour growth inhibition (TGI) at Day 13 was calculated using the following formula: TGI (%) = 1-($TV_t/TV_v$) *100%, where $TV_t$ is the tumour volume of the treated group on Day 13 and $TV_v$ is the tumour volume of the vehicle control group on Day 13. Graphing and statistical analysis of tumour volume using one-way ANOVA followed by Dunnett's multiple comparisons test. Except for the five animals assigned for sample collection at Day 13, the remaining animals were terminated when their individual tumour volume exceeded 1800 $mm^3$. Animals with the TV not exceeding 1800 mm3 were terminated for sample collection at the end of the study, on Day 57 and were considered as alive in the survival analysis. Mice were euthanised using approved humane methods. All procedures involving the care and use of animals were approved by a local IACUC group and conducted by trained personnel in accordance with AAALAC regulations and good veterinary practice.

Flow cytometry experiment

Harvested tumours were collected in HBSS medium, minced and incubated 30 minutes at 37°C in non-enzymatic cell dissociation buffer followed by mechanical dissociation through a 70 μm filter. Viable cells were then enriched using Ficoll® gradient. All cell suspensions were counted and one million viable cells were seeded in 96-well plates in 100 μL of Staining Buffer for acquisition. Non-specific binding was performed using mouse FcR blocking reagent. Viobility 405/452 Fixable Dye (Miltenyi Biotec) was used to assess cell viability. The antibodies directed against the CD45, CD335 (Nkp46), FoxP3 CD8a, CD4, CD3e, CD19, F4/80, CD11c, CD11b (Miltenyi Biotec), Ly6G and Ly6C (Biolegend) were added. The stained cells were analyzed with a Fortessa X20 cytometer (BD Biosciences).

**Histology and Immunofluorescence**

Freshly collected tumour tissues (5 animals/group) were placed in 10% NBF and fixed for 24 hours at RT followed by trimming to the thickness which did not exceed 3-5mm. After rinsing with running water, the specimens were transferred to the Vacuum Tissue Processor (HistoCore PEARL, Leica) for dehydration, then embedded into FFPE blocks using Tissue embedding center (EG1150, Leica). FFPE blocks were sectioned with a manual rotary microtome (RM2235, Leica), 4 μm thickness/section. Sections were processed for staining with hematoxylin and eosin (H&E) or for immunofluorescent (IF) analysis. For IF sections were stained with primary antibodies specific for CD31 (abcam), NG2 (sigma) and cell nuclei were counterstained with DAPI. All stained sections were scanned with Pannoramic Digital Slide Scanners for 40x magnification (3DHISTECH, Pannoram ic SCAN). All the images were analyzed with HALOTM platform were tumour area and large areas of necrosis were quantified. Non-tumour tissue on the periphery was excluded.

**Compound synthesis**

Abbreviations and Acronyms

aq: aqueous; Boc: *tert*-butyloxycarbonyl; br: broad; DCM: dichloromethane; d: doublet (spectral); DIPEA: diisopropylethylamine; DMF: *N*,*N*-dimethylformamide; DMSO: dimethylsulfoxide; EtOAc: ethyl acetate; ESI: electrospray ionisation; h: hour(s); HATU: *N*-[(dimethylamino)-1*H*-1,2,3-triazolo-[4,5-*b*]pyridin-1-ylmethylene]-*N*-methylmethanaminium hexafluorophosphate *N*-oxide; HPLC: high pressure liquid chromatography; LC: liquid chromatography; LCMS: liquid chromatography mass spectrometry; M: molar; *m*/*z*: mass-to-charge ratio; MeOH: methanol; min: minute(s); MS: mass spectrometry; m: multiplet (spectral); NMR: nuclear magnetic resonance; Ph: phenyl; ppm: parts per million; q: quartet (spectral); $R_T$: retention time; rt: room temperature; s: singlet; TFA: trifluoroacetic acid; t: triplet; UV: ultraviolet; v/v: volume per unit volume.

General Experimental Conditions

*Solvents and reagents*

Common organic solvents that were used in reactions (e.g. DMF, DCM, and MeOH) were purchased anhydrous from Sigma-Aldrich® in Sure/Seal™ bottles and were handled appropriately under nitrogen. Water was deionised using an Elga PURELAB Option-Q. All other solvents used (i.e. for work-up procedures and purification) were generally HPLC grade and were used as supplied from various commercial sources. Unless otherwise stated, all starting materials used

were purchased from commercial suppliers and used as supplied.

*Microwave synthesis*

Microwave experiments were carried out using a Biotage Initiator™ Eight instrument. The system gives good reproducibility and control at temperature ranges from 60-250°C and pressures of up to a maximum of 20 bar.

*Flash chromatography*

Purification of compounds by flash chromatography was achieved using a Biotage Isolera Four system. Unless otherwise stated, Biotage KP-Sil SNAP cartridge columns (10-340 g) or Grace GraceResolv cartridge columns (4-330 g) were used along with the stated solvent system and an appropriate solvent gradient depending on compound polarity. In the case of more polar and basic compounds, Biotage KP-NH SNAP cartridge columns (11 g) were used.

*NMR spectroscopy*

$^{1}H$ NMR spectra were recorded at ambient temperature using a Bruker Ascend (500 MHz) spectrometer. All chemical shifts ($\delta$) are expressed in ppm. Residual solvent signals were used as an internal standard and the characteristic solvent peaks were corrected to the reference data outlined in J. Org. Chem., 1997, 62, p7512-7515; in other cases, NMR solvents contained tetramethylsilane, which was used as an internal standard.

*Liquid Chromatography Mass Spectrometry (LCMS)*

Liquid Chromatography Mass Spectrometry (LCMS) experiments to determine retention times ($R_T$) and associated mass ions were performed using the following methods:
Method A: The system consisted of an Agilent Technologies 6130 quadrupole mass spectrometer linked to an Agilent Technologies 1290 Infinity LC system with UV diode array detector and autosampler. The spectrometer consisted of an electrospray ionization source operating in positive and negative ion mode. LCMS experiments were performed on each sample submitted using the following conditions: LC Column: Agilent Eclipse Plus C18 RRHD, 1.8 μm, 50 x 2.1 mm maintained at 40 °C. Mobile phases: A) 0.1% (v/v) formic acid in water; B) 0.1% (v/v) formic acid in acetonitrile.

| Gradient Time (min) | Flow (mL/min) | %A | %B |
|---|---|---|---|
| 0.00 | 0.5 | 80 | 20 |
| 1.80 | 0.5 | 0 | 100 |
| 2.20 | 0.5 | 0 | 100 |
| 2.50 | 0.5 | 80 | 20 |
| 3.00 | 0.5 | 80 | 20 |

Method B: The system consisted of an Agilent Technologies 6140 single quadrupole mass spectrometer linked to an Agilent Technologies 1290 Infinity LC system with UV diode array detector and autosampler. The spectrometer consisted of a multimode ionization source (electrospray and atmospheric pressure chemical ionizations) operating in positive and negative ion mode. LCMS experiments were performed on each sample submitted using the following conditions: LC Column: Zorbax Eclipse Plus C18 RRHD, 1.8 μm, 50 x 2.1 mm maintained at 40 °C. Mobile phases: A) 0.1% (v/v) formic acid in water; B) 0.1% (v/v) formic acid in acetonitrile.

| Gradient Time (min) | Flow (mL/min) | %A | %B |
|---|---|---|---|
| 0.00 | 1.0 | 95 | 5 |
| 1.80 | 1.0 | 0 | 100 |
| 2.20 | 1.0 | 0 | 100 |
| 2.21 | 1.0 | 95 | 5 |
| 2.50 | 1.0 | 95 | 5 |

Example 1 (ADC-159): 6-Chloro-7-(2,3-dihydrobenzofuran-5-yl)-3-((4-hydroxy-1-(1-methylcyclopropane-1-carbonyl) piperidin-4-yl)methyl)-3,7-dihydro-4*H*-pyrrolo[2,3-*d*]pyrimidin-4-one

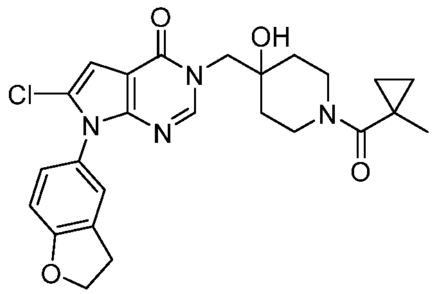

*Step 1: 4,6-Dichloro-7-(2,3-dihydrobenzofuran-5-yl)-7H-pyrrolo[2,3-d]pyrimidine:* A suspension of 4,6-dichloro-7*H*-pyrrolo[2,3-*d*]pyrimidine (500 mg, 2.66 mmol) [commercially available], 2,3-dihydrobenzofuran-5-boronic acid (1.31 g, 7.98 mmol), 1,10-phenanthroline (958 mg, 5.32 mmol) and copper(II) acetate (966 mg, 5.32 mmol) in DMF (30 mL) was stirred at rt under air overnight. The resulting mixture was diluted with EtOAc (100 mL) and washed with 1:1 saturated brine/water solution (3 x 100 mL). The aqueous phase was extracted with EtOAc (50 mL). The combined organic phases were dried (phase separator) and concentrated *in vacuo.* The residue was purified by flash chromatography (0-40% EtOAc in cyclohexane) to give the title compound (392 mg, 46%) as a white solid. LCMS (Method A): $R_T$ = 1.43 min, *m/z* = 306 $[M+H]^+$.

*Step 2: 6-Chloro-7-(2,3-dihydrobenzofuran-5-yl)-3,7-dihydro-4H-pyrrolo[2,3-d]pyrimidin-4-one:* A suspension of 4,6-dichloro-7-(2,3-dihydrobenzofuran-5-yl)-7*H*-pyrrolo[2,3-*d*]pyrimidine (392 mg, 1.24 mmol) in 2M HCl (aq) solution (2.5 mL, 4.96 mmol) and 1,4-dioxane (5 mL) was heated under microwave irradiation at 120 °C for 2 h before the mixture was concentrated *in vacuo* and then dried in a vacuum oven overnight to give crude title compound (358 mg, 84%) as a pink/brown solid that was used without further purification. LCMS (Method A): $R_T$ = 0.94 min, *m/z* = 288 $[M+H]^+$.

*Step 3: tert-Butyl 4-((6-chloro-7-(2,3-dihydrobenzofuran-5-yl)-4-oxo-4,7-dihydro-3H-pyrrolo[2,3-d]pyrimidin-3-yl)methyl)-4-hydroxypiperidine-1-carboxylate:* A suspension of 6-chloro-7-(2,3-dihydrobenzofuran-5-yl)-3,7-dihydro-4*H*-pyrrolo[2,3-*d*]pyrimidin-4-one (357 mg, 1.04 mmol), tert-butyl 1-oxa-6-azaspiro[2.5]octane-6-carboxylate (442 mg, 2.08 mmol) [commercially available] and cesium carbonate (372 mg, 1.14 mmol) in DMF (8 mL) was heated at 80 °C for 4 h. After cooling, the reaction mixture was diluted with EtOAc and washed twice with brine solution. The aqueous phase was twice extracted with EtOAc and the combined organics were dried (phase separator) and concentrated *in vacuo.* The residue was purified by flash chromatography (0-80% EtOAc in cyclohexane) to give the title compound (169 mg, 33%). LCMS (Method A): $R_T$ = 1.45 min, *m/z* = 501 $[M+H]^+$.

*Step 4: 6-Chloro-7-(2,3-dihydrobenzofuran-5-yl)-3-((4-hydroxypiperidin-4-yl)methyl)-3,7-dihydro-4H-pyrrolo [2,3-d]pyrimidin-4-one:* A solution of tert-butyl 4-((6-chloro-7-(2,3-dihydrobenzofuran-5-yl)-4-oxo-4,7-dihydro-3*H*-pyrrolo[2,3-*d*]pyrimidin-3-yl)methyl)-4-hydroxypiperidine-1-carboxylate (169 mg, 0.337 mmol) in DCM (3 mL) and TFA (1.5 mL, 19.5 mmol) was stirred at rt for 1 h. The reaction mixture was added to a pre-conditioned (using 1:4 MeOH/DCM) 5 g SCX-2 column. The bound product was washed with 20 mL of 1:4 MeOH/DCM and then eluted with 20 mL of 1:4 7N $NH_3$ in MeOH/DCM. The fractions containing product were evaporated under reduced pressure. The residue was freeze-dried from acetonitrile/water to give the title compound (118 mg, 86%) as an off-white solid. LCMS (Method A): $R_T$ = 0.76 min, *m/z* = 401 $[M+H]^+$.

*Step 5: 6-Chloro-7-(2,3-dihydrobenzofuran-5-yl)-3-((4-hydroxy-1-(1-methylcyclopropane-1-carbonyl)piperidin-4-yl)methyl)-3,7-dihydro-4H-pyrrolo[2,3-d]pyrimidin-4-one:* To a stirred solution of 6-chloro-7-(2,3-dihydrobenzofuran-5-yl)-3-((4-hydroxypiperidin-4-yl)methyl)-3,7-dihydro-4H-pyrrolo[2,3-*d*]pyrimidin-4-one (59.6 mg, 0.149 mmol), 1-methylcyclopropane-1-carboxylic acid (14.9 mg, 0.149 mmol) and HATU (67.8 mg, 0.178 mmol) in anhydrous DCM (1 mL) was added DIPEA (78 uL, 0.446 mmol) and the solution stirred for 30 min. The mixture was washed with saturated sodium hydrogen carbonate (aq) solution (4 mL). The aqueous layer was separated and extracted with DCM (2 x 2 mL). The combined organic phase was dried (phase separator) and concentrated *in vacuo.* The residue was purified by flash chromatography (50-100% EtOAc in cyclohexane; then 0-15% MeOH in EtOAc) and freeze-dried to give the title compound (53.9 mg, 74%). LCMS (Method B): $R_T$ = 1.08 min, *m/z* = 483 $[M+H]^+$. $^1H$ NMR (500 MHz, DMSO-$d_6$): $\delta$ 8.07 (s, 1H), 7.28 (s, 1H), 7.12 (d, 1H), 6.91 (d, 1H), 6.76 (s, 1H), 4.90 (s, 1H), 4.64 (t, 2H), 4.01 (s, 2H), 3.98-3.90 (m, 2H), 3.26-3.02 (m, 4H), 1.58-1.45 (m, 2H), 1.45-1.35 (m, 2H), 1.21 (s, 3H), 0.80-0.73 (m, 2H), 0.55-0.49 (m, 2H).

ADC-159 is particularly advantageous as it exhibits potent and selective USP7 inhibition equivalent to AD-04 (see Figures 33 and 35) but also exhibit improved oral availability compared to AD-04.

The properties of ADC-159 are further characterised in Table 1:

| 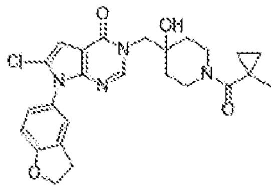 | ADC-159 |
|---|---|
| USP7 FP $IC_{50}$ (μM) | 0.386 (0.159*) |
| RS4;11 / LNCaP $EC_{50}$ (μM) | 0.165 / 0.076 |
| Ub-MDM2 MSD / TE (HCT116) - $EC_{50}$ (μM) | 0.093 / 0.126 |
| MW/$LogD_{7.4}$/tPSA | 483/2.5/87 |
| LM $CL_{hep}$ (h/r/m/d/c) | 9/27/55/22/- |
| Hep $CL_{hep}$ (h/r/m/d/c) | 9/43/78/-/- |
| MDCK (A:B, B:A, $P_{app}$ ratio) | 0.2, 50.1, 276 |
| Caco-2 (A:B, B:A, $P_{app}$ ratio) | 8.5, 34.7, 4.1 |
| PPB Fu (h/r/m) | 0.15 / 0.12 / 0.10 |
| KSol (μM) / TSol (uM) | 199 / 500 |
| hERG (μM) | 15.6 |
| Cyps (uM): 1A2, 2C9, 2C19, 2D6, 3A4 | >20 / 11 / >20 / >20 / >20 |
| TDI shift: CYP3A4 ($IC_{50}$/μM) | N/A (>20) |

Table 1

Example 2 (ADC-160): 6-Chloro-3-((1-(2-cyclopropyloxazole-5-carbonyl)-4-hydroxypiperidin-4-yl)methyl)-7-(2,3-dihydrobenzofuran-5-yl)-3,7-dihydro-4*H*-pyrrolo[2,3-*d*]pyrimidin-4-one

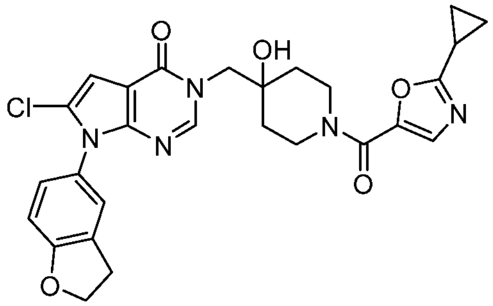

To a stirred solution of 6-chloro-7-(2,3-dihydrobenzofuran-5-yl)-3-((4-hydroxypiperidin-4-yl)methyl)-3,7-dihydro-4*H*-pyrrolo[2,3-*d*]pyrimidin-4-one (26.0 mg, 0.065 mmol), 2-cyclopropyloxazole-5-carboxylic acid (10.9 mg, 0.071 mmol) and HATU (29.6 mg, 0.078 mmol) in anhydrous DCM (1 mL) was added DIPEA (34 μL, 0.195 mmol) and the solution was stirred for 30 min. The mixture was washed with saturated sodium hydrogen carbonate (aq) solution (1 mL). The aqueous layer was separated and extracted with DCM (2 x 1 mL). The combined organic phase was dried (phase separator) and concentrated *in vacuo.* The residue was purified by flash chromatography (0-100% EtOAc in cyclohexane; then 0-5% MeOH in EtOAc) and freeze-dried to give the title compound (27.0 mg, 76%). LCMS (Method B): $R_T$ = 1.09 min, *m/z* = 536 $[M+H]^+$. $^1H$ NMR (500 MHz, DMSO-$d_6$): δ 8.07 (s, 1H), 7.49 (s, 1H), 7.28 (d, 1H), 7.12 (dd, 1H), 6.91 (d, 1H), 6.75 (s, 1H), 4.98 (s, 1H), 4.64 (t, 2H), 4.17-3.88 (m, 4H), 3.54-3.30 (m, 1H), 3.28-3.04 (m, 3H), 2.20-2.13 (m, 1H), 1.66-1.54 (m, 2H), 1.50-1.41 (m, 2H), 1.12-1.06 (m, 2H), 1.01-0.96 (m, 2H).

Example 3 (ADC-198): 6-Chloro-7-(2,3-dihydrobenzofuran-6-yl)-3-[[4-hydroxy-1-(1-methylcyclopropanecarbonyl)-4-piperidyl]methyl]pyrrolo[2,3-*d*]pyrimidin-4-one

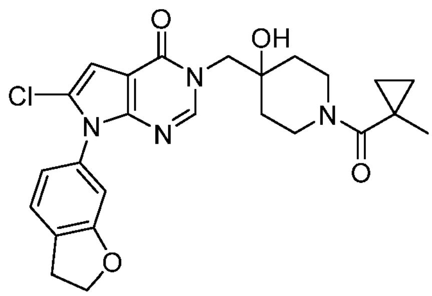

*Step 1: 4,6-Dichloro-7-(2,3-dihydrobenzofuran-6-yl)-7H-pyrrolo[2,3-d]pyrimidine:* A suspension of 4,6-dichloro-7*H*-pyrrolo[2,3-*d*]pyrimidine (100 mg, 0.532 mmol), 2,3-dihydrobenzofuran-6-boronic acid (262 mg, 1.60 mmol), 1,10-phenanthroline (192 mg, 1.06 mmol) and copper(II) acetate (193 mg, 1.06 mmol) in DMF (10 mL) was stirred at rt under air over 64 h. The resulting mixture was diluted with water (30 mL) and extracted with EtOAc (3 x 50 mL). The combined organic phases were dried (anhydrous $MgSO_4$) and concentrated *in vacuo.* The residue was purified by flash chromatography (2-50% EtOAc in cyclohexane) to give the title compound (160 mg, 88%) as a viscous clear oil. LCMS (Method A): $R_T$ = 1.44 min, *m/z* = 306 $[M+H]^+$.

*Step 2: 6-Chloro-7-(2,3-dihydrobenzofuran-6-yl)-3,7-dihydro-4H-pyrrolo[2,3-d]pyrimidin-4-one:A* suspension of 4,6-dichloro-7-(2,3-dihydrobenzofuran-6-yl)-7*H*-pyrrolo[2,3-*d*]pyrimidine (160 mg, 0.471 mmol) in 2M HCl (aq) solution (1.3 mL, 2.59 mmol) and 1,4-dioxane (3 mL) was heated under microwave irradiation at 120 °C for 2 h before the mixture was concentrated *in vacuo* and then dried in a vacuum oven overnight to give the crude title compound (150 mg, 92%) as a dark yellow solid that was used without further purification. LCMS (Method A): $R_T$ = 0.96 min, *m/z* = 288 $[M+H]^+$.

*Step 3: tert-Butyl4-((6-chloro-7-(2,3-dihydrobenzofuran-6-yl)-4-oxo-4,7-dihydro-3H-pyrrolo[2,3-d]pyrimidin-3-yl)methyl)-4-hydroxypiperidine-1-carboxylate:* A suspension of 6-chloro-7-(2,3-dihydrobenzofuran-6-yl)-3,7-dihydro-4*H*-pyrrolo[2,3-*d*]pyrimidin-4-one (150 mg, 0.434 mmol), tert-butyl 1-oxa-6-azaspiro[2.5]octane-6-carboxylate (201 mg, 0.943 mmol) and cesium carbonate (169 mg, 0.519 mmol) in DMF (4 mL) was heated at 80 °C for 4 h. After cooling, the reaction mixture was diluted with EtOAc and washed twice with brine solution. The aqueous phase was twice extracted with EtOAc and the combined organic phase was dried (phase separator) and concentrated *in vacuo.* The residue was purified by flash chromatography (0-100% EtOAc in cyclohexane) to give the title compound (80.4 mg, 37%). LCMS (Method A): $R_T$ = 1.45 min, *m/z* = 501 $[M+H]^+$.

*Step 4: 6-Chloro-7-(2,3-dihydrobenzofuran-6-yl)-3-((4-hydroxypiperidin-4-yl)methyl)-3,7-dihydro-4H-pyrrolo*

*[2,3-d]pyrimidin-4-one:* A solution of tert-butyl 4-((6-chloro-7-(2,3-dihydrobenzofuran-6-yl)-4-oxo-4,7-dihydro-3*H*-pyrrolo [2,3-*d*]pyrimidin-3-yl)methyl)-4-hydroxypiperidine-1-carboxylate (80 mg, 0.160 mmol) in DCM (1.5 mL) and TFA (0.75 mL, 9.73 mmol) was stirred at rt for 1 h. The reaction mixture was added to a pre-conditioned (using 1:4 MeOH/DCM) 5 g SCX-2 column. The bound product was washed with 20 mL of 1:4 MeOH/DCM and then eluted with 20 mL of 1:4 7N $NH_3$ in MeOH/DCM. The fractions containing product were evaporated under reduced pressure to give the title compound (66.1 mg, quantitative) as an off-white solid. LCMS (Method A): $R_T$ = 0.63 min, *m/z* = 401 $[M+H]^+$.

*Step 5: 6-Chloro-7-(2,3-dihydrobenzofuran-6-yl)-3-((4-hydroxy-1-(1-methylcyclopropane-1-carbonyl)piperidin-4-yl)methyl)-3,7-dihydro-4H-pyrrolo[2,3-d]pyrimidin-4-one:* To a stirred suspension of 6-chloro-7-(2,3-dihydrobenzofuran-6-yl)-3-((4-hydroxypiperidin-4-yl)methyl)-3,7-dihydro-4H-pyrrolo[2,3-*d*]pyrimidin-4-one (66.1 mg, 0.165 mmol), 1-methylcyclopropane-1-carboxylic acid (17.5 mg, 0.175 mmol) and HATU (66.4 mg, 0.175 mmol) in anhydrous DCM (2 mL) was added DIPEA (122 μL, 0.699 mmol) and the solution was stirred for 1 h. The reaction mixture was diluted with DCM and washed twice with saturated sodium hydrogen carbonate (aq) solution. The aqueous phase was separated and extracted with DCM. The combined organic phase was dried (phase separator) and concentrated *in vacuo.* The residue was purified by flash chromatography (0-100% EtOAc in cyclohexane; then 0-5% MeOH in EtOAc) and freeze-dried and dried in a vacuum oven to give the title compound (62.4 mg, 78%). LCMS (Method A): $R_T$ = 1.10 min, *m/z* = 483 $[M+H]^+$. $^1H$ NMR (500 MHz, DMSO-$d_6$): δ 8.07 (s, 1H), 7.42-7.37 (m, 1H), 6.88-6.82 (m, 2H), 6.77 (s, 1H), 4.90 (s, 1H), 4.68-4.61 (m, 2H), 4.01 (s, 2H), 3.98-3.90 (m, 2H), 3.29-3.25 (m, 2H), 3.24-3.05 (br s, 2H), 1.56-1.46 (m, 2H), 1.43-1.36 (m, 2H), 1.21 (s, 3H), 0.79-0.75 (m, 2H), 0.54-0.49 (m, 2H).

<u>Example 4 (ADC-199): 6-Chloro-3-((1-(2-cyclopropyloxazole-5-carbonyl)-4-hydroxypiperidin-4-yl)methyl)-7-(2,3-dihydrobenzofuran-6-yl)-3,7-dihydro-4*H*-pyrrolo[2,3-*d*]pyrimidin-4-one</u>

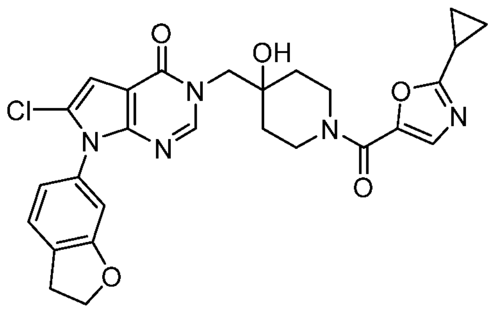

A solution of 2-cyclopropyloxazole-5-carboxylic acid (15.2 mg, 0.100 mmol), HATU (45.5 mg, 0.120 mmol) and DIPEA (52 μL, 0.299 mmol) in anhydrous DCM (2.5 mL) was stirred for 5 min followed by addition of 6-chloro-7-(2,3-dihydrobenzofuran-6-yl)-3-((4-hydroxypiperidin-4-yl)methyl)-3,7-dihydro-4*H*-pyrrolo[2,3-*d*]pyrimidin-4-one (40.0 mg, 0.100 mmol) and the solution was stirred at rt for 3 h. The mixture was diluted with saturated sodium hydrogen carbonate (aq) solution and extracted three times with DCM. The combined organic phase was dried (phase separator) and concentrated *in vacuo.* The residue was purified by flash chromatography (0-100% EtOAc in cyclohexane; then 0-10% MeOH in EtOAc, KP-NH) and freeze-dried to afford the title compound (28.5 mg, 53%). LCMS (Method B): $R_T$ = 1.10 min, *m/z* = 536 $[M+H]^+$. $^1H$ NMR (500 MHz, DMSO-$d_6$): δ 8.08 (s, 1H), 7.50 (s, 1H), 7.42-7.39 (m, 1H), 6.88-6.83 (m, 2H), 6.77 (s, 1H), 5.00 (s, 1H), 4.65 (t, 2H), 4.03 (br s, 4H), 3.41 (br s, 1H), 3.28 (d, 2H), 3.18 (br s, 1H), 2.17 (tt, 1H), 1.60 (t, 2H), 1.46 (d, 2H), 1.12-1.07 (m, 2H), 1.01-0.97 (m, 2H).

<u>Example 5 (ADX-556): 7-(Benzo[*d*][1,3]dioxol-5-yl)-6-chloro-3-((4-hydroxy-1-(1-methylcyclopropane-1-carbonyl)piperidin-4-yl)methyl)-3,7-dihydro-4*H*-pyrrolo[2,3-*d*]pyrimidin-4-one</u>

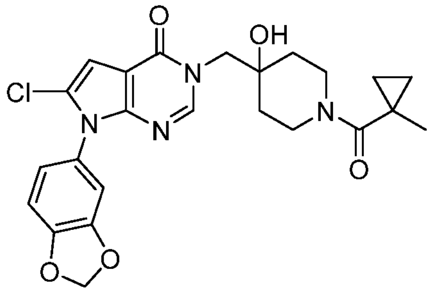

This compound was prepared by the methods which are described in WO 2018/073602 (Example 202).

Example 6: 7-(1-Benzofuran-5-yl)-6-chloro-3-((4-hydroxy-1-(1-methylcyclopropane-1-carbonyl)piperidin-4-yl) methyl)-3,7-dihydro-4*H*-pyrrolo[2,3-*d*]pyrimidin-4-one

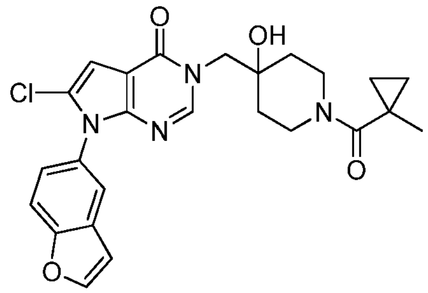

*Step 1: 7-(1-Benzofuran-5-yl)-4,6-dichloro-7H-pyrrolo[2,3-d]pyrimidine:* A suspension of 4,6-dichloro-7*H*-pyrrolo[2,3-*d*]pyrimidine (385 mg, 2.05 mmol), 1-benzofuran-5-ylboronic acid (1 g, 6.17 mmol), boric acid (506 mg, 8.19 mmol), copper(II) acetate (744 mg, 4.10 mmol) and 1,10-phenanthroline (738 mg, 4.10 mmol) in DMF (20.5 mL) was stirred at 50 °C for 5 days. Upon cooling to rt the reaction mixture was diluted with 10% aq. ammonium hydroxide (40 mL) and extracted with DCM (3 x 40 mL) using a Biotage phase separator. The combined organic phases were concentrated under reduced pressure and the resulting residue was purified by flash chromatography (0%, 2%, 4% then 6% EtOAc in cyclohexane (isocratic)) to give the title compound (31.3 mg, 5%) as a white solid. LCMS (method B): $R_T$ = 1.45 min, *m/z* = 304, 306 $[M+H]^+$.

*Step 2: 7-(1-Benzofuran-5-yl)-6-chloro-3,7-dihydro-4H-pyrrolo[2,3-d]pyrimidin-4-one hydrochloride: A* suspension of 7-(1-benzofuran-5-yl)-4,6-dichloro-7*H*-pyrrolo[2,3-*d*]pyrimidine (31.3 mg, 0.103 mmol) in 2 M $HCl_{(aq)}$ (0.21 mL, 0.420 mmol) and 1,4-dioxane (1.1 mL) was heated under microwave irradiation 120 °C for 2 h. The reaction mixture was concentrated under reduced pressure and the residue dried in a vacuum oven at 50 °C to give the title compound (34 mg, 102%) as a red/brown solid. This material was used without further purification. LCMS (method B): $R_T$ = 0.99 min, *m/z* = 286, 288 $[M+H]^+$.

*Step 3: 7-(1-Benzofuran-5-yl)-6-chloro-3-((4-hydroxy-1-(1-methylcyclopropane-1-carbonyl)piperidin-4-yl) methyl)-3,7-dihydro-4H-pyrrolo[2,3-d]pyrimidin-4-one:* A suspension of 7-(1-benzofuran-5-yl)-6-chloro-3,7-dihydro-4*H*-pyrrolo[2,3-*d*]pyrimidin-4-one hydrochloride (34 mg, 0.106 mmol), (1-methylcyclopropyl)(1-oxa-6-azaspiro[2.5]octan-6-yl)methanone (WO 2018/073602 A1, incorporated herein by reference) (30.9 mg, 0.1583mmol) and cesium carbonate (75.7 mg, 0.232 mmol) in DMF (1.1 mL) was stirred at 80 °C for 18 h. Upon cooling to rt the reaction mixture was diluted with saturated aq. ammonium chloride (15 mL) and the resulting suspension was extracted with DCM ( 3 x 10 mL) using a Biotage phase separator. The combined organic phases were concentrated under reduced pressure and the residue was purified by flash chromatography (0-100% EtOAc in cyclohexane, then 0-10% MeOH in EtOAc) and prep-HPLC to give the title compound (1.9 mg, 3.7%) as white solid after lyophilisation. LCMS (method B): $R_T$ = 1.13 min, *m/z* = 481, 483 $[M+H]^+$. $^1$H NMR (500 MHz, DMSO-d6): 8.16 (d, J = 2.2 Hz, 8.07 (s, 7.80 (d, J = 8.8 Hz, 7.77 (d, J = 2.1 Hz, 7.36 (dd, J = 8.7, 2.1 Hz, 7.08 (dd, J = 2.2, 0.9 Hz, 6.82 (s, 4.92 (s, 4.02 (s, 2H), 3.98 - 3.92 (m, 2H), 3.17 (br s, 2H), 1.58 - 1.46 (m, 2H), 1.44 - 1.36 (m, 2H), 1.21 (s, 3H), 0.79 - 0.75 (m, 2H), 0.54 - 0.50 (m, 2H).

## Claims

1. A USP7 inhibitor for use in a method of treating cancer by inhibiting USP7 activity in fibroblasts, the method comprising administering to a subject in need thereof a composition comprising the USP7 inhibitor.

2. The USP7 inhibitor for use according to claim 1, wherein administration of the composition comprising the USP7 inhibitor treats the cancer:

   by inhibiting USP7 activity in cancer associated fibroblasts (CAFs);
   and/or by reducing the level of VEGF in the serum of the subject;
   and/or by reducing the level of VEGF in the tumour microenvironment;
   and/or by inhibiting production of VEGF by cancer-associated fibroblasts (CAFs).

3. The USP7 inhibitor for use according to any preceding claim, wherein the USP7 inhibitor destabilizes hypoxia-inducible transcription factor (HIF1$\alpha$), thereby inhibiting VEGF production by cancer-associated fibroblasts.

4. The USP7 inhibitor for use according to any preceding claim, wherein administration of the USP7 inhibitor modulates the tumour immune environment;

   and/or wherein administration of the USP7 inhibitor increases tumour-infiltrating lymphocytes (TILs), preferably CD8+ TILs;
   and/or wherein administration of the composition comprising the USP7 inhibitor decreases the proportion of Treg cells relative to CD8+ T cells in the tumour microenvironment (TME) and/or decreases the number of macrophages in the TME.

5. The USP7 inhibitor for use of any preceding claim, wherein administration of the composition comprising the USP7 inhibitor treats the cancer by inhibiting extra-cellular matrix (ECM) remodelling by cancer-associated fibroblasts,

   wherein the ECM remodelling inhibited by the USP7 inhibitor optionally comprises fibroblast-epithelial cell tube formation, optionally *de novo* tube formation,
   and/or wherein the ECM remodelling inhibited by the USP7 inhibitor optionally comprises degradation of the basement membrane, optionally degradation of the tubular basement membrane.

6. The USP7 inhibitor for use according to any preceding claim, wherein administration of the composition comprising the USP7 inhibitor treats the cancer by inhibiting EMT transition;,

   and/or wherein administration of the composition comprising the USP7 inhibitor inhibits fibroblast secretion of MMP7 and/or MMP2;,
   and/or wherein administration of the composition comprising the USP7 inhibitor inhibits fibroblast invasion;,
   and/or wherein administration of the composition comprising the USP7 inhibitor treats the cancer by inhibiting angiogenesis, optionally neo-angiogenesis.

7. The USP7 inhibitor for use according to any preceding claim, wherein the cancer is formed of cancer cells, and the cancer cells are resistant to the USP7 inhibitor;

   and/or wherein the cancer is formed of cancer cells and the cancer cells are resistant to inhibitors of the MDM2 pathway, optionally resistant to MDM2 inhibitors;
   and/or wherein the cancer is metastatic cancer.

8. The USP7 inhibitor for use according to any preceding claim, wherein the cancer is a solid tumour and is optionally selected from: renal cancer (e.g., renal cell carcinoma), breast cancer, brain tumours, lymphomas (e.g., Hodgkin's and non-Hodgkin's lymphoma, lymphocytic lymphoma, primary CNS lymphoma, B-cell lymphoma (e.g. CLL), T-cell lymphoma (e.g. Sezary Syndrome)), nasopharyngeal carcinomas, melanoma (e.g., metastatic malignant melanoma), prostate cancer, colon cancer, lung cancer, bone cancer, pancreatic cancer, skin cancer, cancer of the head or neck (e.g. head and neck squamous cell carcinoma (HNSCC)), cutaneous carcinoma, cutaneous or intraocular malignant melanoma, uterine cancer, ovarian cancer, rectal cancer, cancer of the anal region, stomach cancer, testicular cancer, carcinoma of the fallopian tubes, carcinoma of the endometrium, carcinoma of the cervix, carcinoma of the vagina, carcinoma of the vulva, cancer of the oesophagus, cancer of the small intestine, cancer of the endocrine

system, cancer of the thyroid gland, cancer of the parathyroid gland, cancer of the adrenal gland, sarcoma of soft tissue, cancer of the urethra, cancer of the penis, cancer of the bladder, neoplasm of the central nervous system (CNS), spinal axis tumour, brain stem glioma, pituitary adenoma, Kaposi's sarcoma, epidermoid cancer, squamous cell cancer, mesothelioma,
preferably breast cancer or carcinoma, preferably adenocarcinoma, preferably colorectal carcinoma or prostate carcinoma.

**9.** The USP7 inhibitor for use according to any preceding claim wherein the subject has previously been administered an initial therapeutic agent and did not exhibit a response, or has relapsed, wherein the initial therapeutic agent was optionally an inhibitor of the MDM2 pathway, for example an inhibitor of MDM2.

**10.** The USP7 inhibitor for use according to any preceding claim wherein the method further comprises administration of an additional therapeutic agent, wherein the additional therapeutic agent is preferably administered in combination with the composition comprising the USP7 inhibitor and wherein the additional therapeutic agent is optionally selected from a checkpoint inhibitor and an anti-angiogenic agent.

**11.** A USP7 inhibitor for use according to any preceding claim wherein the USP7 inhibitor is a compound of formula (I):

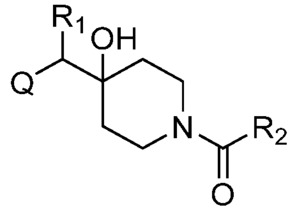

(I)

including a pharmaceutically acceptable salt, tautomer, stereoisomer or N-oxide derivative thereof, wherein:

$R_1$ is H, OH or an optionally substituted alkyl group;
$R_2$ is an optionally substituted C1-C6 alkyl, optionally substituted C2-C6 alkenyl,
optionally substituted C2-C6 alkynyl, optionally substituted C3-C6 cycloalkyl, optionally substituted C4-C6 alkylcycloalkyl, optionally substituted C4-C6 aryl, optionally substituted C3-C6 heteroaryl, optionally substituted C4-C8 aryloxy, optionally substituted C7-C10 arylalkyl or optionally substituted C5-C10 heteroarylalkyl group; and
Q is an optionally substituted nitrogen containing heterocyclyl group, preferably selected from:

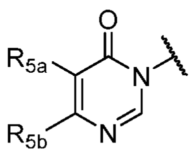
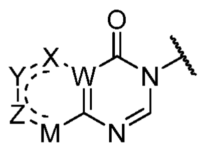
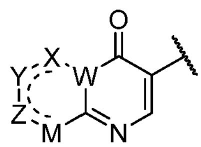

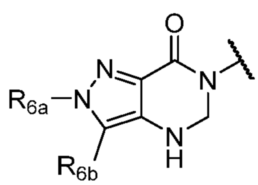
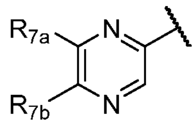
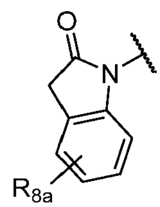

wherein

Wis N orC
X is S, O, N, or CH
Y is $CR_{6a}$, $CR_{9a}$, N, or $NR_{6a}$,
Z is $CR_{6b}$, N, $NR_{6b}$, $NR_{9b}$, or O

M is absent or $CR_{8a}$
wherein if X is S, Z is N and M is absent; and wherein if M is $CR_{8a}$ Y is not N;
$R_{5a}$ is H, halo, optionally substituted C1-C6 alkyl, or optionally substituted amino;
$R_{5b}$ is H, halo, optionally substituted C1-C6 alkyl, optionally substituted C2-C6 alkynyl, benzyl, optionally monosubstituted C3-C6 heteroaryl, optionally substituted C3-C6 heterocycloalkyl, optionally substituted C1-C6 alkoxy, NR'R", or $R^a$NR'R",

wherein $R^a$ is C1-C6 alkyl or C2-C6 alkenyl; and
wherein R' and R" are each independently selected from H, oxo-substituted C1-C6 alkyl, hydroxy-substituted C1-C6 alkyl, optionally substituted C1-C6 alkoxy, optionally substituted C3-C6 cycloalkyl, optionally substituted C1-C7 alkylamine, optionally substituted C2-C7 alkenylamine, optionally substituted C3-C10 heterocycloalkyl, optionally substituted C4-C10 aryl, optionally substituted C3-C10 heteroaryl, optionally substituted C5-C10 alkylaryl, optionally substituted C4-C10 alkylheterocycloalkyl, and C4-C6 alkylheteroaryl, or wherein R' and R" together form an optionally substituted C3-C8 heterocycloalkyl including the N to which they are attached;

$R_{6a}$ is H, optionally substituted C1-C6 alkyl, optionally substituted amino, optionally substituted C4-C6 aryl, optionally substituted C1-C6 sulfide, optionally substituted C1-C6 sulfonyl, or optionally substituted amino;
$R_{6b}$ is H, cyano, halo, optionally substituted C1-C6 alkyl, optionally substituted C2-C6 alkenyl, optionally substituted C2-C6 alkynyl, optionally substituted C3-C6 cycloalkyl, optionally substituted C4-C6 cycloalkenyl, optionally substituted C2-C6 ynol, optionally substituted C4-C6 aryl, optionally substituted C3-C6 heteroaryl, optionally substituted amino;
$R_{7a}$ is H;
$R_{7b}$ is H or optionally substituted C4-C6 aryl
or wherein $R_{7a}$ and $R_{7b}$ together form an optionally substituted C1-C6 aryl group together with the carbons to which they are attached;
$R_{8a}$ is H or is optionally substituted C4-C6 aryl;
$R_{9a}$ is Cl, F, Br, I, or cyano;
$R_{9b}$ is H, optionally substituted C1-C6 alkyl, optionally substituted C4-C6 aryl, optionally substituted C3-C8 heteroaryl, C1-C6 alkoxy,
for example wherein the USP7 inhibitor is

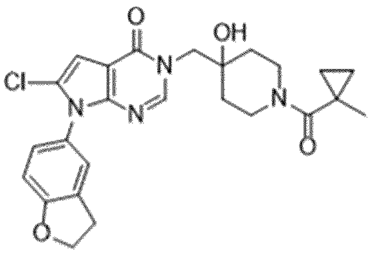

.

**12.** A USP7 inhibitor for use in a method of treating cancer by inhibiting USP7 activity in fibroblasts, the method comprising administering to a subject in need thereof a combination therapy, the combination therapy comprising a composition comprising the USP7 inhibitor and a composition comprising an immune checkpoint inhibitor.

**13.** An immune checkpoint inhibitor for use in a method of treating cancer, the method comprising administering to a subject in need thereof a combination therapy, the combination therapy comprising a composition comprising a USP7 inhibitor and a composition comprising the immune checkpoint inhibitor; wherein, in said method, the USP7 inhibitor inhibits USP7 activity in fibroblasts.

**14.** A combination therapy for use in a method of treating cancer by inhibiting USP7 activity in fibroblasts, the method comprising administering to a subject in need thereof the combination therapy, the combination therapy comprising a composition comprising a USP7 inhibitor and a composition comprising an immune checkpoint inhibitor.

**15.** The USP7 inhibitor, immune checkpoint inhibitor or combination therapy for use according to any one of claims 12 to 14, wherein the immune checkpoint inhibitor is selected from an anti-PD1 antibody, an anti-PD-L1 antibody and an anti-CTLA4 antibody.

## Patentansprüche

1. USP7-Inhibitor zur Verwendung in einem Verfahren zur Behandlung von Krebs durch die Hemmung der USP7-Aktivität in Fibroblasten, wobei das Verfahren die Verabreichung einer Zusammensetzung, die den USP7-Inhbitor umfasst, an ein bedürftiges Individuum umfasst.

2. USP7-Inhibitor zur Verwendung nach Anspruch 1, wobei die Verabreichung der Zusammensetzung, die den USP7-Inhibitor umfasst, den Krebs wie folgt behandelt:

   durch Hemmen der USP7-Aktivität in mit dem Krebs assoziierten Fibroblasten (CAFs);
   und/oder durch Senken des VEGF-Spiegels im Serum des Individuums;
   und/oder durch Senken des VEGF-Spiegels in der Tumor-Mikroumgebung;
   und/oder durch Hemmen der Produktion von VEGF durch mit dem Krebs assoziierte Fibroblasten (CAFs).

3. USP7-Inhibitor zur Verwendung nach einem der vorangegangenen Ansprüche, wobei der USP7-Inhibitor den Hypoxie-induzierbaren Transkriptionsfaktor (HIF1$\alpha$) destabilisiert, wodurch die VEGF-Produktion durch mit dem Krebs assoziierte Fibroblasten gehemmt wird.

4. USP7-Inhibitor zur Verwendung nach einem der vorangegangenen Ansprüche, wobei die Verabreichung des USP7-Inhibitors die Tumor-Immunumgebung moduliert;

   und/oder wobei die Verabreichung des USP7-Inhibitors die tumorinfiltrierenden Lymphozyten (TILs), vorzugsweise CD8+ TILs, erhöht;
   und/oder wobei die Verabreichung der Zusammensetzung, die den USP7-Inhibitor umfasst, den Anteil an Treg-Zellen bezogen auf CD8+ T-Zellen in der Tumor-Mikroumgebung (TME) verringert und/oder die Anzahl an Makrophagen in der TME verringert.

5. USP7-Inhibitor zur Verwendung nach einem der vorangegangenen Ansprüche, wobei die Verabreichung der Zusammensetzung, die den USP7-Inhibitor umfasst, den Krebs durch Hemmen des Umbaus der extrazellulären Matrix (EZM) durch mit dem Krebs assoziierte Fibroblasten behandelt,
   wobei der durch den USP7-Inhibitor gehemmte EZM-Umbau gegebenenfalls die Bildung von Fibroblasten-Epithelzellen-Tubuli, gegebenenfalls eine De-novo-Tubuli-Bildung, umfasst, und/oder wobei der durch den USP7-Inhibitor gehemmte EZM-Umbau gegebenenfalls den Abbau der Basalmembran, gegebenenfalls den Abbau der tubulären Basalmembran, umfasst.

6. USP7-Inhibitor zur Verwendung nach einem der vorangegangenen Ansprüche, wobei die Verabreichung der Zusammensetzung, die den USP7-Inhibitor umfasst, den Krebs durch Hemmung der EMT-Transition behandelt;

   und/oder wobei die Verabreichung der Zusammensetzung, die den USP7-Inhibitor umfasst, die Fibroblastensekretion von MMP7 und/oder MMP2 hemmt;
   und/oder wobei die Verabreichung der Zusammensetzung, die den USP7-Inhibitor umfasst, das Eindringen von Fibroblasten hemmt;
   und/oder wobei die Verabreichung der Zusammensetzung, die den USP7-Inhibitor umfasst, den Krebs durch Hemmung von Angiogenese, gegebenenfalls Neoangiogenese, hemmt.

7. USP7-Inhibitor zur Verwendung nach einem der vorangegangenen Ansprüche, wobei der Krebs aus Krebszellen besteht und die Krebszellen resistent gegen den USP7-Inhibitor sind;

   und/oder wobei der Krebs aus Krebszellen besteht und die Krebszellen resistent gegen Inhibitoren des MDM2-Wegs, gegebenenfalls resistent gegen MDM2-Inhibitoren, sind;
   und/oder wobei der Krebs ein metastatischer Krebs ist.

8. USP7-Inhibitor zur Verwendung nach einem der vorangegangenen Ansprüche, wobei der Krebs ein solider Tumor ist und gegebenenfalls aus Folgenden ausgewählt ist: Nierenkrebs (z. B. Nierenzellkarzinomen), Brustkrebs, Hirntumoren, Lymphomen (z. B. Hodgkin- und Non-Hodgkin-Lymphomen, lymphozytischen Lymphomen, primären ZNS-Lymphomen, B-Zell-Lymphomen (z. B. CLL), T-Zell-Lymphomen (z. B. Sézary-Syndrom)), Nasopharynxkarzinomen, Melanomen (z. B. metastatischen malignen Melanomen), Prostatakrebs, Kolonkrebs, Lungenkrebs, Knochenkrebs, Pankreaskrebs, Hautkrebs, Kopf-Hals-Krebs (z. B. Plattenepithelkarzinomen des Kopf-Hals-Bereichs (HNSCC)),

kutanen Karzinomen, kutanen oder intraokularen malignen Melanomen, Uteruskrebs, Ovarialkrebs, Rektalkrebs, Krebs des Analbereichs, Magenkrebs, Hodenkrebs, Karzinomen der Eileiter, Karzinomen des Endometriums, Karzinomen des Zervix, Karzinomen der Vagina, Karzinomen der Vulva, Ösophaguskrebs, Dünndarmkrebs, Krebs des endokrinen Systems, Schilddrüsenkrebs, Nebenschilddrüsenkrebs, Nebennierenkrebs, Weichgewebesarkomen, Harnröhrenkrebs, Peniskrebs, Blasenkrebs, Neoplasmen des Zentralnervensystems (ZNS), Spinalachsentumoren, Hirnstammgliomen, Hypophysenadenomen, Kaposi-Sarkomen, Epidermoidkrebs, Plattenepithelkrebs, Mesotheliomen,
vorzugsweise Brustkrebs oder -karzinomen, vorzugsweise Adenokarzinomen, vorzugsweise Kolorektalkarzinomen oder Prostatakarzinomen.

**9.** USP7-Inhibitor zur Verwendung nach einem der vorangegangenen Ansprüche, wobei dem Individuum zuvor ein anfängliches Therapeutikum verabreicht wurde, das darauf aber nicht ansprach oder einen Rückfall erlitten hat, wobei das anfängliche Therapeutikum gegebenenfalls ein Inhibitor des MDM2-Wegs, beispielsweise ein Inhibitor von MDM2, war.

**10.** USP7-Inhibitor zur Verwendung nach einem der vorangegangenen Ansprüche, wobei das Verfahren weiters die Verabreichung eines weiteren Therapeutikums umfasst, wobei das weitere Therapeutikum vorzugsweise in Kombination mit der Zusammensetzung, die den USP7-Inhibitor umfasst, verabreicht wird und wobei das weitere Therapeutikum gegebenenfalls aus einem Checkpoint-Inhibitor und einem antiangiogenen Mittel ausgewählt ist.

**11.** USP7-Inhibitor zur Verwendung nach einem der vorangegangenen Ansprüche, wobei der USP7-Inhibitor eine Verbindung der Formel (I) ist:

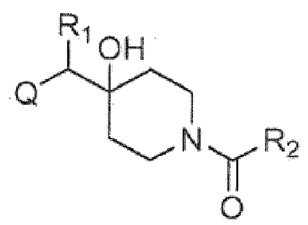

(I)

einschließlich eines pharmazeutisch annehmbaren Salzes, Tautomers, Stereoisomers oder N-Oxid-Derivats davon, worin:

**$R_1$** H**,** OH oder eine gegebenenfalls substituierte Alkylgruppe ist;
$R_2$ eine gegebenenfalls substituierte C1-C6-Alkyl-, gegebenenfalls substituierte C2-C6-Alkenyl-, gegebenenfalls substituierte C2-C6-Alkinyl-, gegebenenfalls substituierte C3-C6-Cycloalkyl-, gegebenenfalls substituierte C4-C6-Alkylcycloalkyl-, gegebenenfalls substituierte C4-C6-Aryl-, gegebenenfalls substituierte C3-C6-Heteroaryl-, gegebenenfalls substituierte C4-C8-Aryloxy-, gegebenenfalls substituierte C7-C10-Arylalkyl- oder gegebenenfalls substituierte C5-C10-Heteroarylalkylgruppe ist; und
Q ein gegebenenfalls substituierter Stickstoff ist, der eine Heterocyclylgruppe enthält, die vorzugsweise aus folgenden ausgewählt ist:

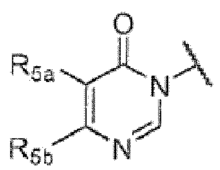

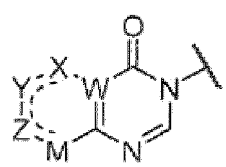

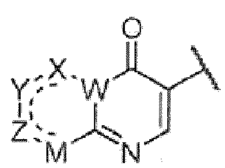

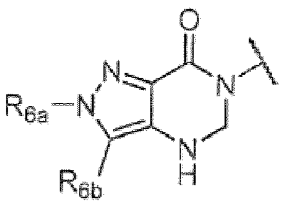 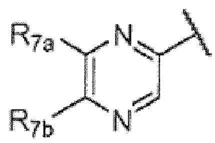 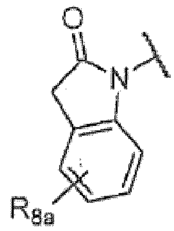

worin:

W N oder C ist,
X S, O, N oder CH ist,
Y $CR_{6a}$, $CR_{9a}$, N oder $NR_{6a}$ ist,
Z $CR_{6b}$, N, $NR_{6b}$, $NR_{9b}$ oder O ist,
m fehlt oder $CR_{8a}$ ist,
worin, wenn X S ist, Z N ist und M fehlt; und worin, wenn M $CR_{8a}$ ist, Y nicht N ist;
$R_{5a}$ H, Halogen, gegebenenfalls substituiertes C1-C6-Alkyl oder gegebenenfalls substituiertes Amino ist;
$R_{5b}$ H, Halogen, gegebenenfalls substituiertes C1-C6-Alkyl, gegebenenfalls substituiertes C2-C6-Alkinyl, Benzyl, gegebenenfalls monosubstituiertes C3-C6-Heteroaryl, gegebenenfalls substituiertes C3-C6-Heterocycloalkyl, gegebenenfalls substituiertes C1-C6-Alkoxy, NR'R" oder $R^a$NR'R" ist,
worin $R^a$ C1-C6-Alkyl oder C2-C6-Alkenyl ist; und
worin R' und R" jeweils unabhängig voneinander aus H, Oxo-substituiertem C1-C6-Alkyl, Hydroxy-substituiertem C1-C6-Alkyl, gegebenenfalls substituiertem C1-C6-Alkoxy, gegebenenfalls substituiertem C3-C6-Cycloalkyl, gegebenenfalls substituiertem C1-C7-Alkylamin, gegebenenfalls substituiertem C2-C7-Alkenylamin, gegebenenfalls substituiertem C3-C10-Heterocycloalkyl, gegebenenfalls substituiertem C4-C10-Aryl, gegebenenfalls substituiertem C3-C10-Heteroaryl, gegebenenfalls substituiertem C5-C10-Alkylaryl, gegebenenfalls substituiertem C4-C10-Alkylheterocycloalkyl und C4-C6-Alkylheteroaryl ausgewählt sind oder worin R' und R" zusammen ein gegebenenfalls substituiertes C3-C8-Heterocycloalkyl bilden, das den N einschließt, an den sie gebunden sind;
$R_{6a}$ H, Cyano, Halogen, gegebenenfalls substituiertes C1-C6-Alkyl, gegebenenfalls substituiertes C2-C6-Alkenyl, gegebenenfalls substituiertes C2-C6-Alkinyl, gegebenenfalls substituiertes C3-C6-Cycloalkyl, gegebenenfalls substituiertes C4-C6-Cycloalkenyl, gegebenenfalls substituiertes C2-C6-Inol, gegebenenfalls substituiertes C4-C6-Aryl, gegebenenfalls substituiertes $C_3$-$C_6$-Heteroaryl, gegebenenfalls substituiertes Amino ist;
$R_{7a}$ H ist;
$R_{7b}$ H oder gegebenenfalls substituiertes C4-C6-Aryl ist,
oder worin $R_{7a}$ und $R_{7b}$ zusammen mit den Kohlenstoffatomen, an die sie gebunden sind, eine gegebenenfalls substituierte C1-C6-Arylgruppe bilden;
$R_{8a}$ H oder gegebenenfalls substituiertes C4-C6-Aryl ist;
$R_{9a}$ Cl, F, Br, I oder Cyano ist;
$R_{9b}$ H, gegebenenfalls substituiertes C1-C6-Alkyl, gegebenenfalls substituiertes C4-C6-Aryl, gegebenenfalls substituiertes C3-C8-Heteroaryl, C1-C6-Alkoxy ist,
wobei der USP7-Inhibitor beispielsweise

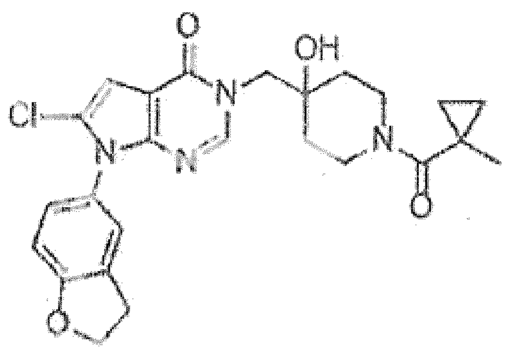

ist.

12. USP7-Inhibitor zur Verwendung in einem Verfahren zur Behandlung von Krebs durch Hemmung der USP7-Aktivität in Fibroblasten, wobei das Verfahren die Verabreichung einer Kombinationstherapie an ein bedürftiges Individuum umfasst, wobei die Kombinationstherapie eine den USP7-Inhibitor umfassende Zusammensetzung und eine einen Immuncheckpoint-Inhibitor umfassende Zusammensetzung umfasst.

13. Immuncheckpoint-Inhibitor zur Verwendung in einem Verfahren zur Behandlung von Krebs, wobei das Verfahren die Verabreichung einer Kombinationstherapie an ein bedürftiges Individuum umfasst, wobei die Kombinationstherapie eine einen USP7-Inhibitor umfassende Zusammensetzung und eine den Immuncheckpoint-Inhibitor umfassende Zusammensetzung umfasst; wobei der USP7-Inhibitor in diesem Verfahren USP7-Aktivität in Fibroblasten hemmt.

14. Kombinationstherapie zur Verwendung in einem Verfahren zur Behandlung von Krebs durch Hemmung von USP7-Aktivität in Fibroblasten, wobei das Verfahren die Verabreichung der Kombinationstherapie an ein bedürftiges Individuum umfasst, wobei die Kombinationstherapie eine einen USP7-Inhibitor umfassende Zusammensetzung und eine einen Immuncheckpoint-Inhibitor umfassende Zusammensetzung umfasst.

15. USP7-Inhibitor, Immuncheckpoint-Inhibitor oder Kombinationstherapie zur Verwendung nach einem der Ansprüche 12 bis 14, wobei der Immuncheckpoint-Inhibitor aus einem Anti-PD1-Antikörper, einem Anti-PD-L1-Antikörper und einem Anti-CTLA4-Antikörper ausgewählt ist.

**Revendications**

1. Inhibiteur d'USP7 pour une utilisation dans un procédé de traitement d'un cancer par inhibition de l'activité de l'USP7 dans des fibroblastes, le procédé comprenant l'administration à un sujet qui le nécessite d'une composition comprenant l'inhibiteur d'USP7.

2. Inhibiteur d'USP7 pour une utilisation selon la revendication 1, dans lequel l'administration de la composition comprenant l'inhibiteur d'USP7 traite le cancer :

   par inhibition de l'activité de l'USP7 dans des fibroblastes associés au cancer (CAF) ;
   et/ou par réduction du niveau de VEGF dans le sérum du sujet ;
   et/ou par réduction du niveau de VEGF dans le microenvironnement tumoral ;
   et/ou par inhibition de la production de VEGF par des fibroblastes associés au cancer (CAF).

3. Inhibiteur d'USP7 pour une utilisation selon une quelconque revendication précédente, dans lequel l'inhibiteur d'USP7 déstabilise le facteur de transcription inductible par l'hypoxie (HIF1$\alpha$), inhibant ainsi la production de VEGF par des fibroblastes associés au cancer.

4. Inhibiteur d'USP7 pour une utilisation selon une quelconque revendication précédente, dans lequel l'administration de l'inhibiteur d'USP7 module l'environnement immunitaire tumoral ;

   et/ou dans lequel l'administration de l'inhibiteur d'USP7 augmente les lymphocytes infiltrant la tumeur (TIL), de préférence les TIL CD8+ ;
   et/ou dans lequel l'administration de la composition comprenant l'inhibiteur d'USP7 diminue la proportion de cellules Treg par rapport aux lymphocytes T CD8+ dans le microenvironnement tumoral (TME) et/ou diminue le nombre de macrophages dans le TME.

5. Inhibiteur d'USP7 pour une utilisation selon une quelconque revendication précédente, dans lequel l'administration de la composition comprenant l'inhibiteur d'USP7 traite le cancer par inhibition du remodelage de la matrice extracellulaire (ECM) par des fibroblastes associés au cancer,

   dans lequel le remodelage de l'ECM inhibé par l'inhibiteur d'USP7 comprend facultativement la formation de tubes de cellules épithéliales de fibroblastes, facultativement la formation de tubes de *novo,*
   et/ou dans lequel le remodelage de l'ECM inhibé par l'inhibiteur d'USP7 comprend facultativement la dégradation de la membrane basale, facultativement la dégradation de la membrane basale tubulaire.

6. Inhibiteur d'USP7 pour une utilisation selon une quelconque revendication précédente, dans lequel l'administration de la composition comprenant l'inhibiteur d'USP7 traite le cancer par inhibition de la transition EMT ;

et/ou dans lequel l'administration de la composition comprenant l'inhibiteur d'USP7 inhibe la sécrétion par les fibroblastes de MMP7 et/ou de MMP2 ;
et/ou dans lequel l'administration de la composition comprenant l'inhibiteur d'USP7 inhibe invasion des fibroblastes ;
et/ou dans lequel l'administration de la composition comprenant l'inhibiteur d'USP7 traite le cancer par inhibition de l'angiogenèse, facultativement de la néo-angiogenèse.

7. Inhibiteur d'USP7 pour une utilisation selon une quelconque revendication précédente, dans lequel le cancer est formé de cellules cancéreuses, et les cellules cancéreuses sont résistantes à l'inhibiteur d'USP7 ; et/ou dans lequel le cancer est formé de cellules cancéreuses et les cellules cancéreuses sont résistantes aux inhibiteurs de la voie MDM2, facultativement résistantes aux inhibiteurs de MDM2 ;
et/ou dans lequel le cancer est un cancer métastatique.

8. Inhibiteur d'USP7 pour une utilisation selon une quelconque revendication précédente, dans lequel le cancer est une tumeur solide et est facultativement choisi parmi : un cancer rénal (par exemple, carcinome à cellules rénales), un cancer du sein, des tumeurs du cerveau, des lymphomes (par exemple, lymphome hodgkinien et non hodgkinien, lymphome lymphocytaire, lymphome du SNC primaire, lymphome à cellules B (par exemple CLL), lymphome à cellules T (par exemple syndrome de Sézary)), des carcinomes nasopharyngés, un mélanome (par exemple, mélanome malin métastatique), un cancer de la prostate, un cancer du côlon, un cancer du poumon, un cancer des os, un cancer du pancréas, un cancer de la peau, un cancer de la tête ou du cou (par exemple carcinome épidermoïde de la tête et du cou (HNSCC)), un carcinome cutané, un mélanome malin cutané ou intraoculaire, un cancer de l'utérus, un cancer de l'ovaire, un cancer rectal, un cancer de la région anale, un cancer de l'estomac, un cancer des testicules, un carcinome des trompes de Fallope, un carcinome de l'endomètre, un carcinome du col de l'utérus, un carcinome du vagin, un carcinome de la vulve, un cancer de l'œsophage, un cancer de l'intestin grêle, un cancer du système endocrinien, un cancer de la glande thyroïde, un cancer de la glande parathyroïde, un cancer des glandes surrénales, un sarcome des tissus mous, un cancer de l'urètre, un cancer du pénis, un cancer de la vessie, un néoplasme du système nerveux central (SNC), une tumeur de l'axe vertébral, un gliome du tronc cérébral, un adénome pituitaire, un sarcome de Kaposi, un cancer épidermoïde, un cancer des cellules squameuses, un mésothéliome,
de préférence un cancer du sein ou un carcinome, de préférence un adénocarcinome, de préférence un carcinome colorectal ou un carcinome de la prostate.

9. Inhibiteur d'USP7 pour une utilisation selon une quelconque revendication précédente dans lequel le sujet a préalablement reçu une administration d'un agent thérapeutique initial et n'a pas présenté de réponse, ou a rechuté, dans lequel l'agent thérapeutique initial était facultativement un inhibiteur de la voie MDM2, par exemple un inhibiteur de MDM2.

10. Inhibiteur d'USP7 pour une utilisation selon une quelconque revendication précédente dans lequel le procédé comprend en outre l'administration d'un agent thérapeutique additionnel, dans lequel l'agent thérapeutique additionnel est de préférence administré en combinaison avec la composition comprenant l'inhibiteur d'USP7 et dans lequel l'agent thérapeutique additionnel est facultativement choisi parmi un inhibiteur de point de contrôle et un agent anti-angiogénique.

11. Inhibiteur d'USP7 pour une utilisation selon une quelconque revendication précédente dans lequel l'inhibiteur d'USP7 est un composé de formule (I) :

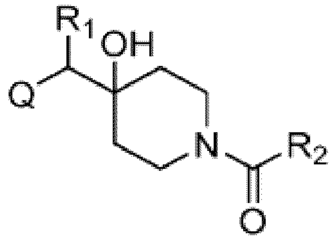

(I)

incluant un sel, un tautomère, un stéréoisomère ou un dérivé *N*-oxyde pharmaceutiquement acceptable de celui-ci,

dans lequel :

$R_1$ est H, OH ou un groupe alkyle facultativement substitué ;

$R_2$ est un groupe alkyle en C1-C6 facultativement substitué, alcényle en C2-C6 facultativement substitué, alcynyle en C2-C6 facultativement substitué, cycloalkyle en C3-C6 facultativement substitué, alkylcycloalkyle en C4-C6 facultativement substitué, aryle en C4-C6 facultativement substitué, hétéroaryle en C3-C6 facultativement substitué, aryloxy en C4-C8 facultativement substitué, arylalkyle en C7-C10 facultativement substitué ou hétéroarylalkyle en C5-C10 facultativement substitué ; et

Q est un groupe hétérocyclyle contenant de l'azote facultativement substitué, de préférence choisi parmi :

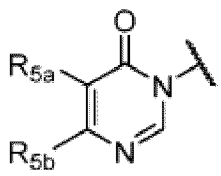
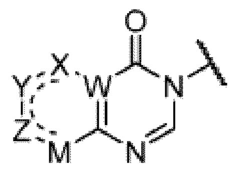
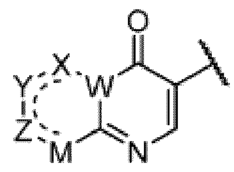
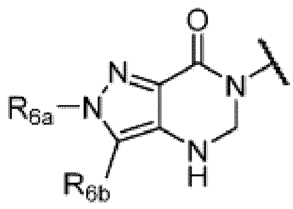
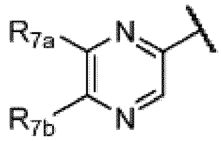
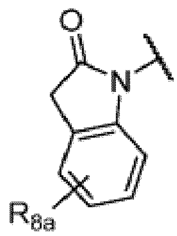

dans lequel

W est N ou C

X est S, O, N ou CH

Y est $CR_{6a}$, $CR_{9a}$, N ou $NR_{6a}$,

Z est $CR_{6b}$, N, $NR_{6b}$, $NR_{9b}$ ou O

M est absent ou est $CR_{8a}$

dans lequel si X est S, Z est N et M est absent ; et dans lequel si M est $CR_{8a}$, Y n'est pas N ; $R_{5a}$ est H, un halogéno, un alkyle en C1-C6 facultativement substitué, ou un amino facultativement substitué ;

$R_{5b}$ est H, un halogéno, un alkyle en C1-C6 facultativement substitué, un alcynyle en C2-C6 facultativement substitué, un benzyle, un hétéroaryle en C3-C6 facultativement monosubstitué, un hétérocycloalkyle en C3-C6 facultativement substitué, un alcoxy en C1-C6 facultativement substitué, NR'R", ou $R^aNR'R''$,

dans lequel $R^a$ est un alkyle en C1-C6 ou un alcényle en C2-C6 ; et

dans lequel R' et R" sont chacun indépendamment choisis parmi H, un alkyle en C1-C6 oxo-substitué, un alkyle en C1-C6 hydroxy-substitué, un alcoxy en C1-C6 facultativement substitué, un cycloalkyle en C3-C6 facultativement substitué, une alkylamine en C1-C7 facultativement substituée, une alcénylamine en C2-C7 facultativement substituée, un hétérocycloalkyle en C3-C10 facultativement substitué, un aryle en C4-C10 facultativement substitué, un hétéroaryle en C3-C10 facultativement substitué, un alkylaryle en C5-C10 facultativement substitué, un alkylhétérocycloalkyle en C4-C10 facultativement substitué, et un alkylhétéroaryle en C4-C6, ou dans lequel R' et R" forment ensemble un hétérocycloalkyle en C3-C8 facultativement substitué incluant le N auquel ils sont fixés ;

$R_{6a}$ est H, un alkyle en C1-C6 facultativement substitué, un amino facultativement substitué, un aryle en C4-C6 facultativement substitué, un sulfure en C1-C6 facultativement substitué, un sulfonyle en C1-C6 facultativement substitué, ou un amino facultativement substitué ;

$R_{6b}$ est H, un cyano, un halogéno, un alkyle en C1-C6 facultativement substitué, un alcényle en C2-C6 facultativement substitué, un alcynyle en C2-C6 facultativement substitué, un cycloalkyle en C3-C6 facultativement substitué, un cycloalcényle en C4-C6 facultativement substitué, un ynol en C2-C6 facultativement substitué, un aryle en C4-C6 facultativement substitué, un hétéroaryle en C3-C6 facultativement substitué, un amino facultativement substitué ;

$R_{7a}$ est H ;

$R_{7b}$ est H ou un aryle en C4-C6 facultativement substitué ou dans lequel $R_{7a}$ et $R_{7b}$ forment ensemble un groupe aryle en C1-C6 facultativement substitué conjointement avec les carbones auxquels ils sont fixés ;

$R_{8a}$ est H ou est un aryle en C4-C6 facultativement substitué ;

$R_{9a}$ est Cl, F, Br, I, ou un cyano ;
$R_{9b}$ est H, un alkyle en C1-C6 facultativement substitué, un aryle en C4-C6 facultativement substitué, un hétéroaryle en C3-C8 facultativement substitué, un alcoxy en C1-C6,
par exemple dans lequel l'inhibiteur d'USP7 est

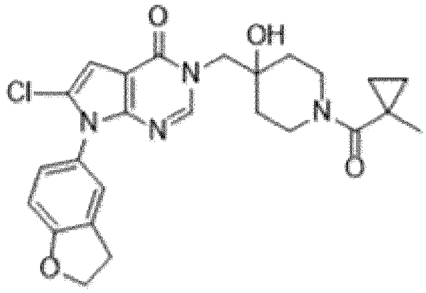

**12.** Inhibiteur d'USP7 pour une utilisation dans un procédé de traitement d'un cancer par inhibition de l'activité de l'USP7 dans des fibroblastes, le procédé comprenant l'administration à un sujet qui le nécessite d'une combinaison thérapeutique, la combinaison thérapeutique comprenant une composition comprenant l'inhibiteur d'USP7 et une composition comprenant un inhibiteur de point de contrôle immunitaire.

**13.** Inhibiteur de point de contrôle immunitaire pour une utilisation dans un procédé de traitement d'un cancer, le procédé comprenant l'administration à un sujet qui le nécessite d'une combinaison thérapeutique, la combinaison thérapeutique comprenant une composition comprenant un inhibiteur d'USP7 et une composition comprenant l'inhibiteur de point de contrôle immunitaire ; dans lequel, dans ledit procédé, l'inhibiteur d'USP7 inhibe l'activité de l'USP7 dans des fibroblastes.

**14.** Combinaison thérapeutique pour une utilisation dans un procédé de traitement d'un cancer par inhibition de l'activité de l'USP7 dans des fibroblastes, le procédé comprenant l'administration à un sujet qui le nécessite de la combinaison thérapeutique, la combinaison thérapeutique comprenant une composition comprenant un inhibiteur d'USP7 et une composition comprenant un inhibiteur de point de contrôle immunitaire.

**15.** Inhibiteur d'USP7, inhibiteur de point de contrôle immunitaire ou combinaison thérapeutique pour une utilisation selon l'une quelconque des revendications 12 à 14, dans lesquels l'inhibiteur de point de contrôle immunitaire est choisi parmi un anticorps anti-PD1, un anticorps anti-PD-L1 et un anticorps anti-CTLA4.

Figure 1

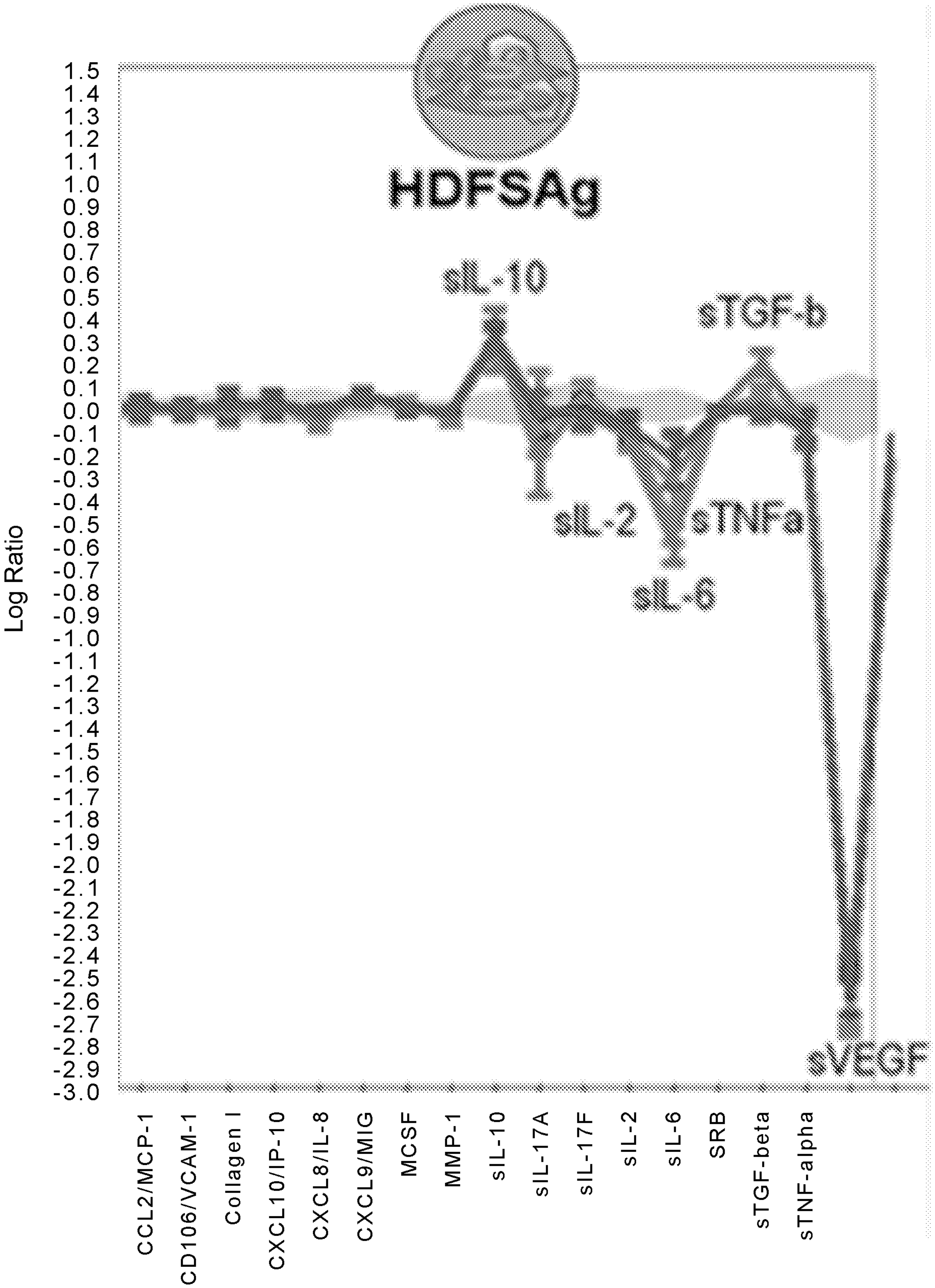
HDFSAg
Log Ratio
1.5
1.4
1.3
1.2
1.1
1.0
0.9
0.8
0.7
0.6
0.5
0.4
0.3
0.2
0.1
0.0
-0.1
-0.2
-0.3
-0.4
-0.5
-0.6
-0.7
-0.8
-0.9
-1.0
-1.1
-1.2
-1.3
-1.4
-1.5
-1.6
-1.7
-1.8
-1.9
-2.0
-2.1
-2.2
-2.3
-2.4
-2.5
-2.6
-2.7
-2.8
-2.9
-3.0
sIL-10
sTGF-b
sIL-2
sTNFa
sIL-6
sVEGF
CCL2/MCP-1
CD106/VCAM-1
Collagen I
CXCL10/IP-10
CXCL8/IL-8
CXCL9/MIG
MCSF
MMP-1
sIL-10
sIL-17A
sIL-17F
sIL-2
sIL-6
SRB
sTGF-beta
sTNF-alpha

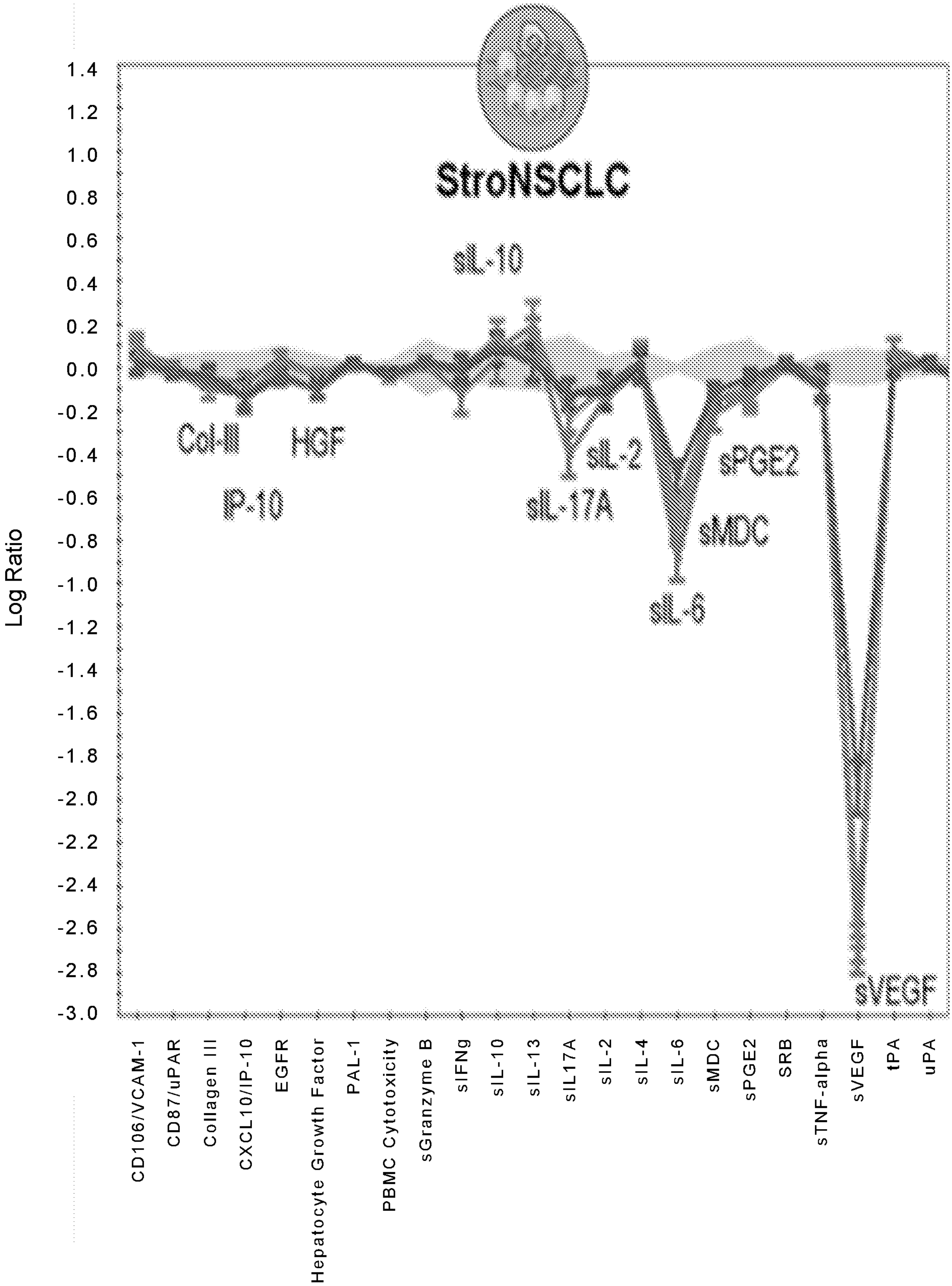
StroNSCLC
Log Ratio
1.4
1.2
1.0
0.8
0.6
0.4
0.2
0.0
-0.2
-0.4
-0.6
-0.8
-1.0
-1.2
-1.4
-1.6
-1.8
-2.0
-2.2
-2.4
-2.6
-2.8
-3.0
sIL-10
Col-III
HGF
IP-10
sIL-2
sIL-17A
sPGE2
sMDC
sIL-6
sVEGF
CD106/VCAM-1
CD87/uPAR
Collagen III
CXCL10/IP-10
EGFR
Hepatocyte Growth Factor
PAL-1
PBMC Cytotoxicity
sGranzyme B
sIFNg
sIL-10
sIL-13
sIL17A
sIL-2
sIL-4
sIL-6
sMDC
sPGE2
SRB
sTNF-alpha
sVEGF
tPA
uPA

Figure 2

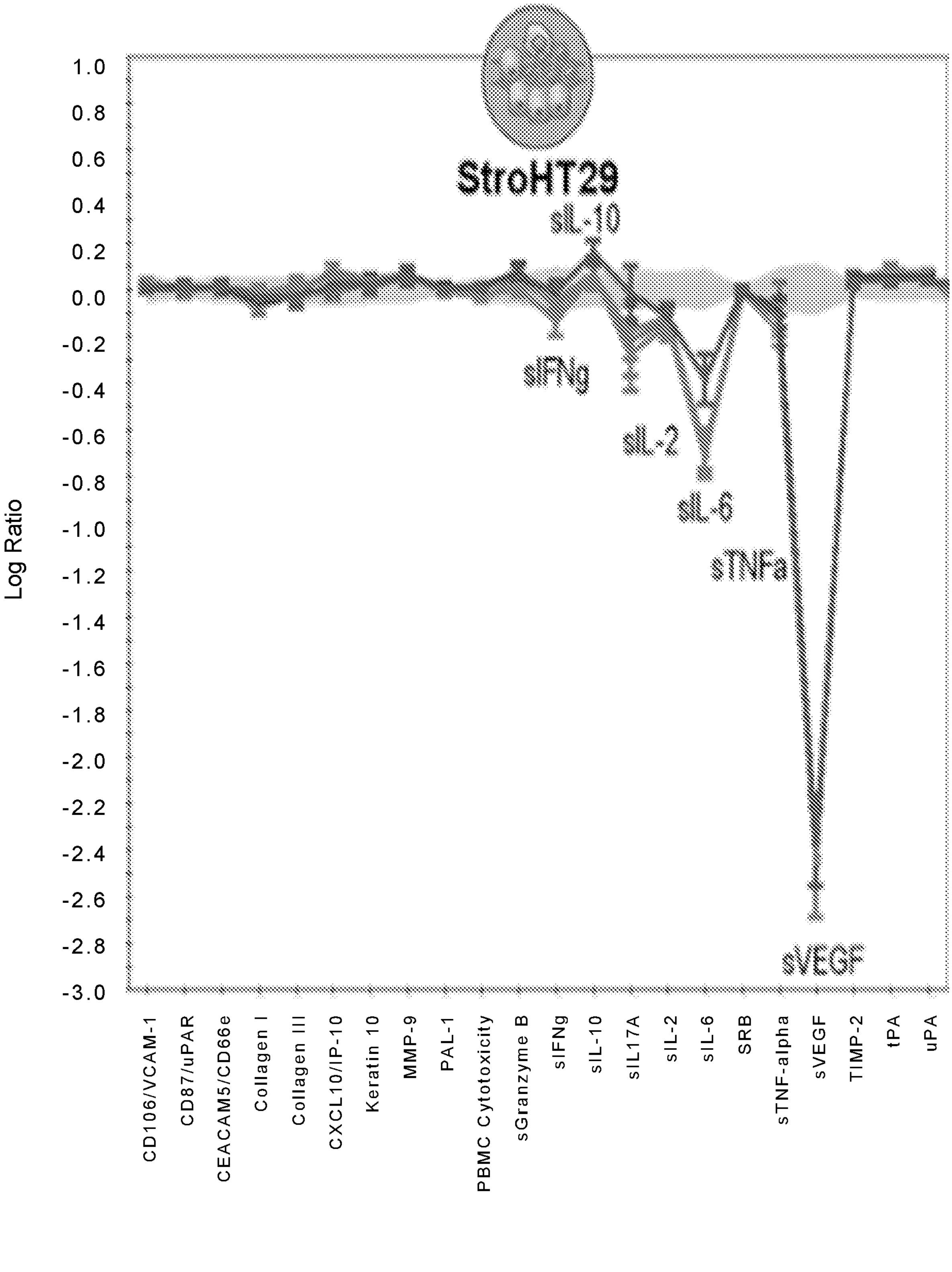


Figure 2 (continued)

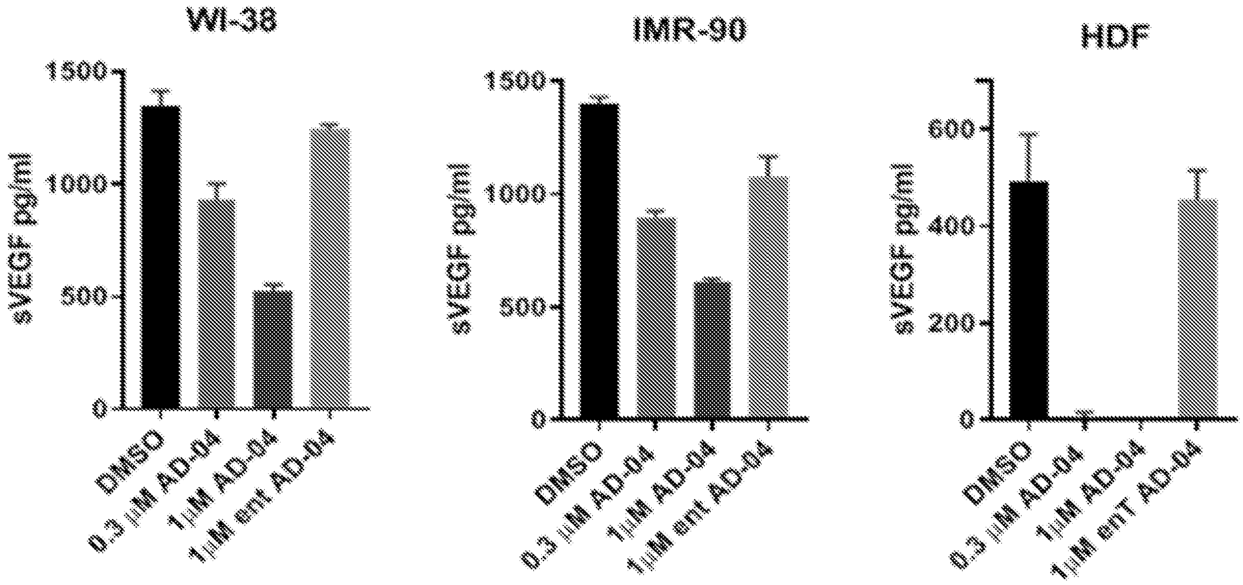
WI-38
sVEGF pg/ml
1500
1000
500
0
DMSO
0.3 μM AD-04
1μM AD-04
1μM ent AD-04
IMR-90
sVEGF pg/ml
1500
1000
500
0
DMSO
0.3 μM AD-04
1μM AD-04
1μM ent AD-04
HDF
sVEGF pg/ml
600
400
200
0
DMSO
0.3 μM AD-04
1μM AD-04
1μM enT AD-04

Figure 3

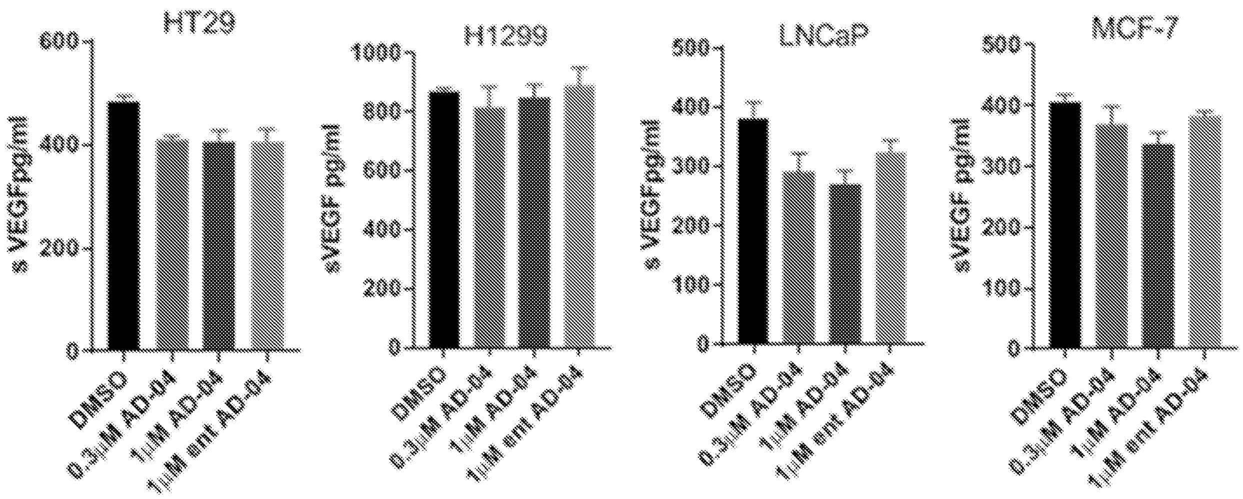
HT29
s VEGFpg/ml
600
400
200
0
DMSO
0.3μM AD-04
1μM AD-04
1μM ent AD-04
H1299
sVEGF pg/ml
1000
800
600
400
200
0
DMSO
0.3μM AD-04
1μM AD-04
1μM ent AD-04
LNCaP
s VEGFpg/ml
500
400
300
200
100
0
DMSO
0.3μM AD-04
1μM AD-04
1μM ent AD-04
MCF-7
sVEGF pg/ml
500
400
300
200
100
0
DMSO
0.3μM AD-04
1μM AD-04
1μM ent AD-04

Figure 4

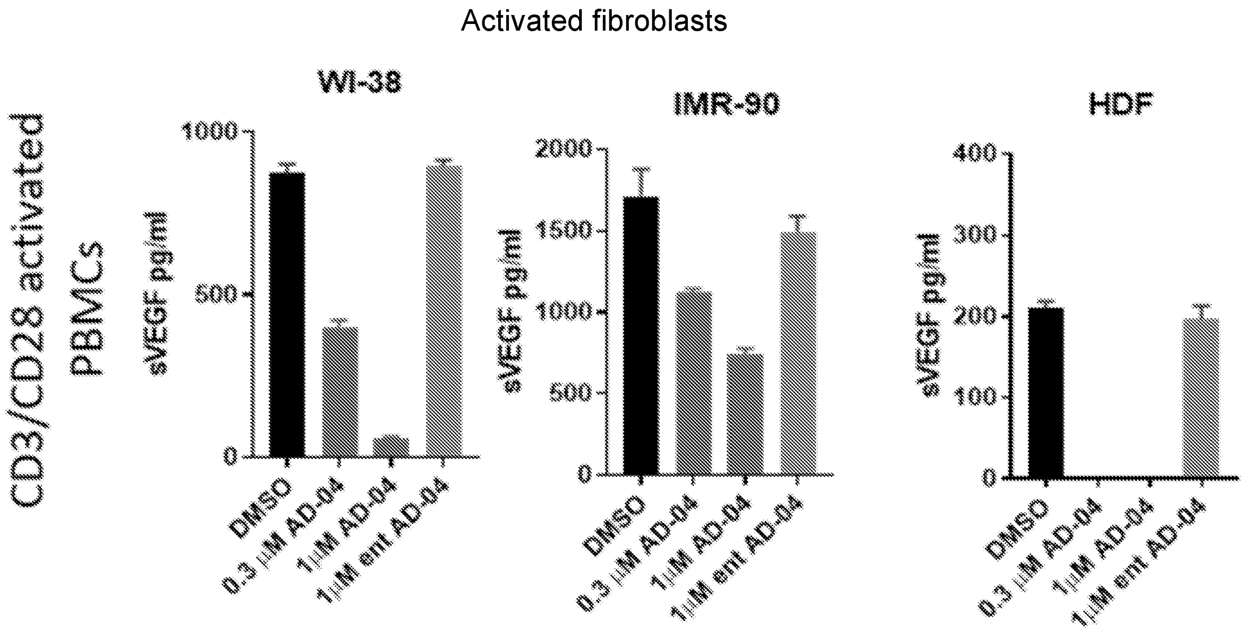

Activated fibroblasts
CD3/CD28 activated PBMCs
WI-38
IMR-90
HDF
sVEGF pg/ml
1000
500
0
2000
1500
1000
500
0
400
300
200
100
0
DMSO
0.3 μM AD-04
1μM AD-04
1μM ent AD-04


Figure 5A

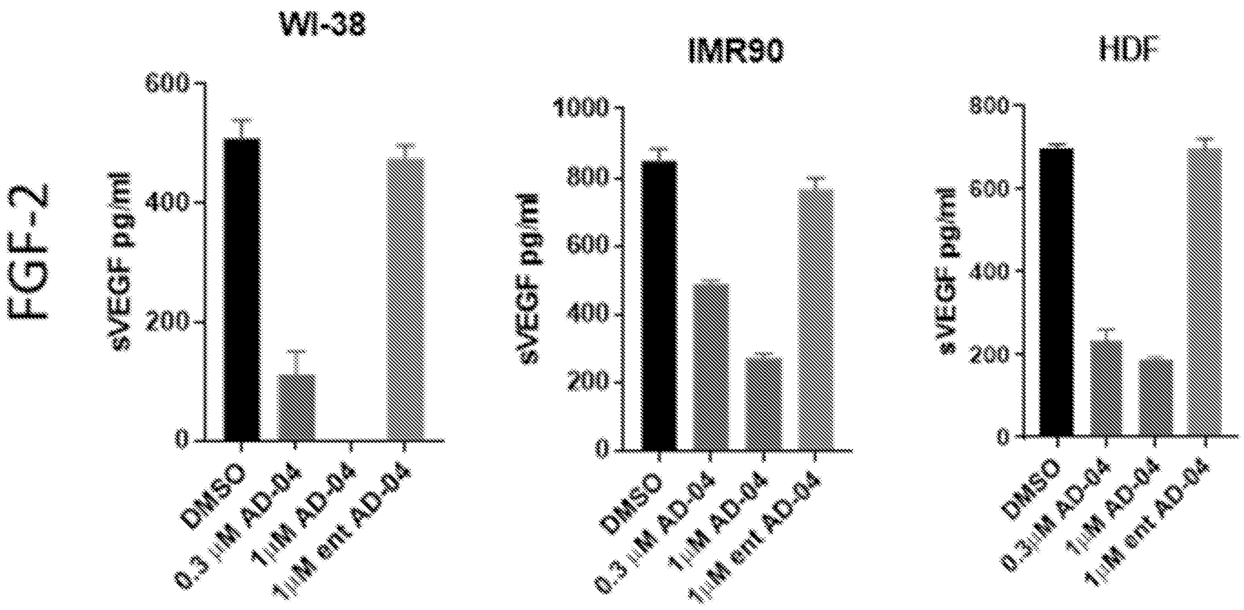
FGF-2
WI-38
IMR90
HDF
sVEGF pg/ml
600
400
200
0
1000
800
600
400
200
0
800
600
400
200
0
DMSO
0.3 μM AD-04
1μM AD-04
1μM ent AD-04
DMSO
0.3 μM AD-04
1μM AD-04
1μM ent AD-04
DMSO
0.3μM AD-04
1μM AD-04
1μM ent AD-04

Figure 5B

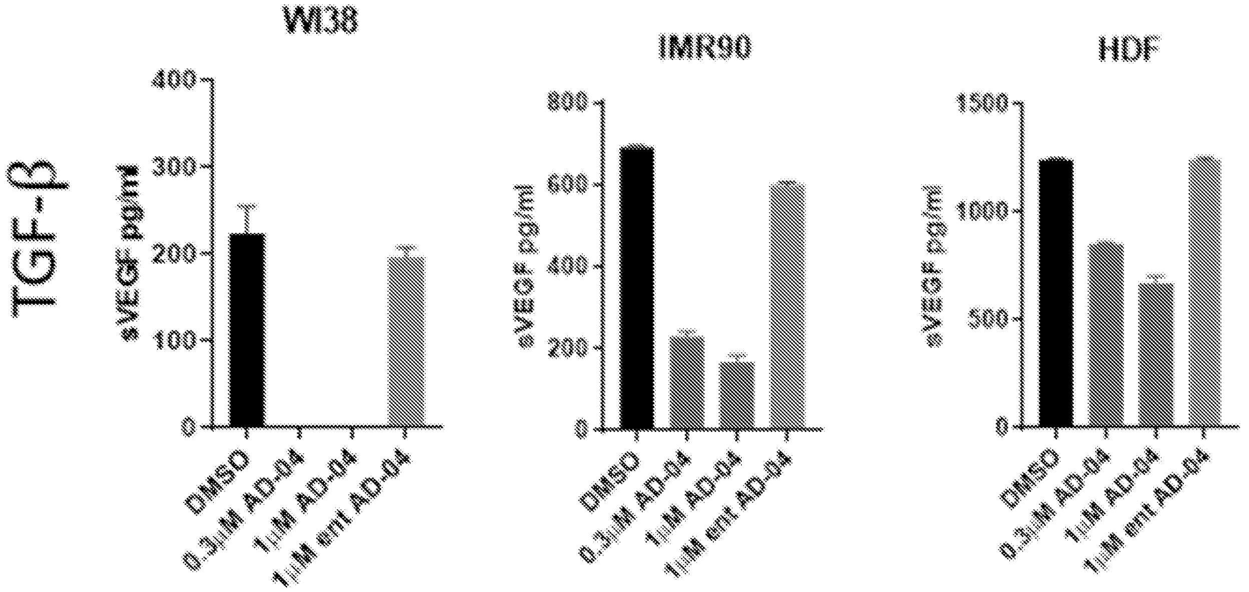
TGF-β
WI38
IMR90
HDF
sVEGF pg/ml
400
300
200
100
0
800
600
400
200
0
1500
1000
500
0
DMSO
0.3μM AD-04
1μM AD-04
1μM ent AD-04

Figure 5C

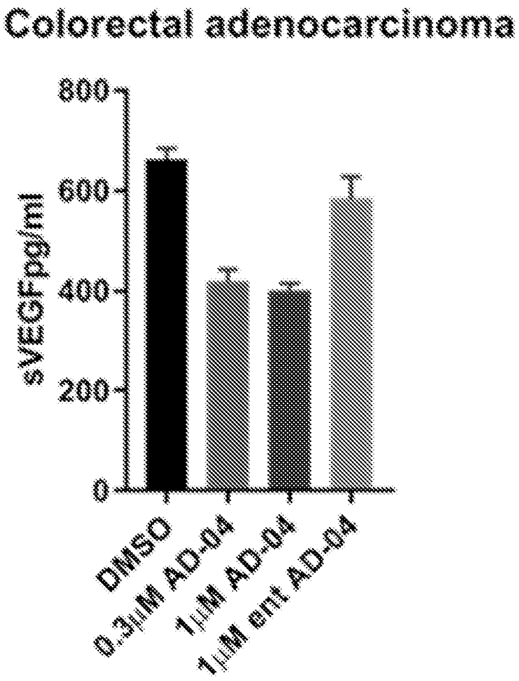


Figure 6

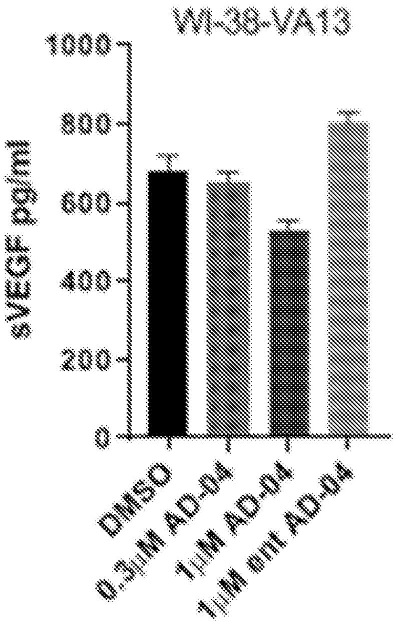


Figure 7

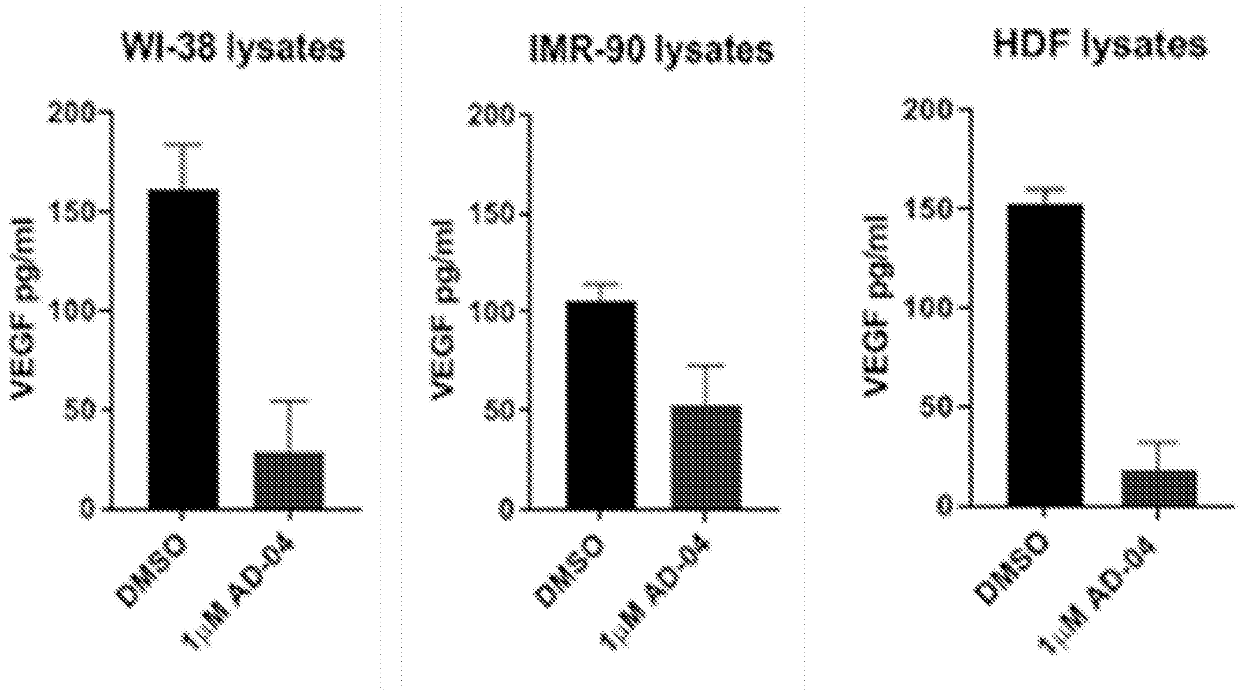
WI-38 lysates
IMR-90 lysates
HDF lysates
VEGF pg/ml
200
150
100
50
0
DMSO
1μM AD-04

Figure 8

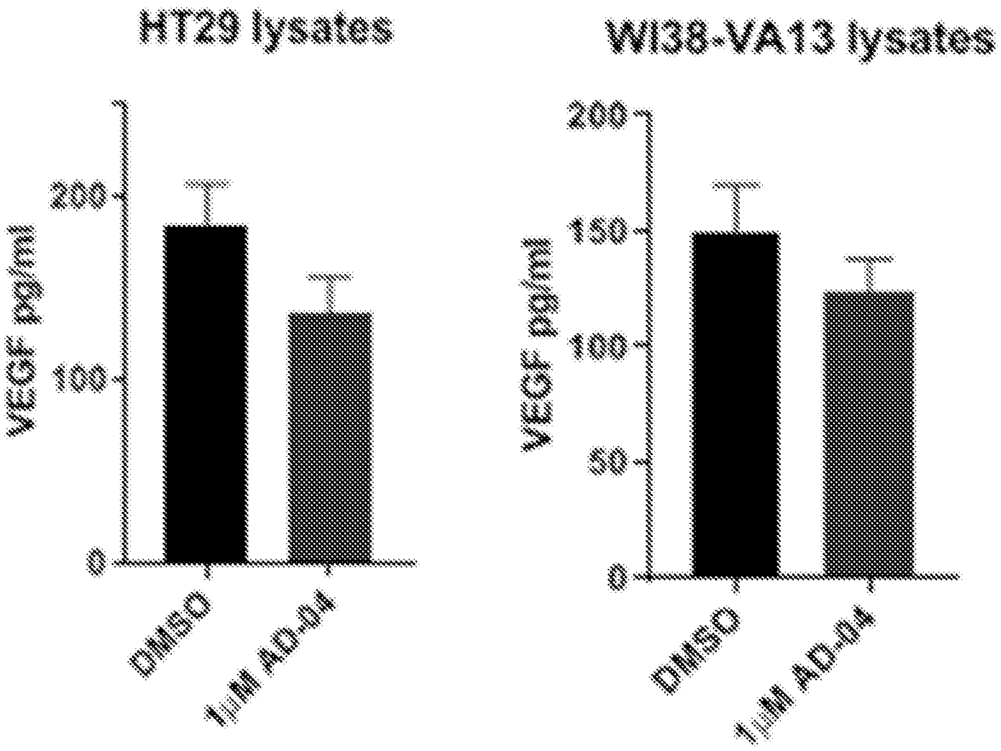


Figure 8 (continued)

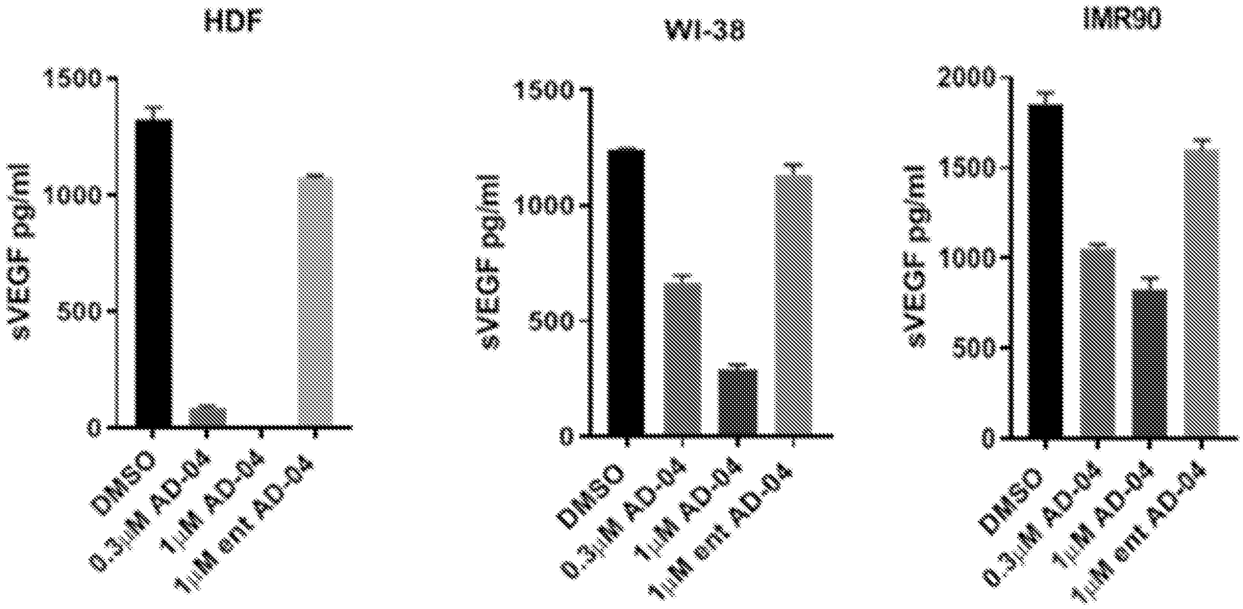
HDF
sVEGF pg/ml
1500
1000
500
0
DMSO
0.3μM AD-04
1μM AD-04
1μM ent AD-04
WI-38
sVEGF pg/ml
1500
1000
500
0
DMSO
0.3μM AD-04
1μM AD-04
1μM ent AD-04
IMR90
sVEGF pg/ml
2000
1500
1000
500
0
DMSO
0.3μM AD-04
1μM AD-04
1μM ent AD-04

Figure 9

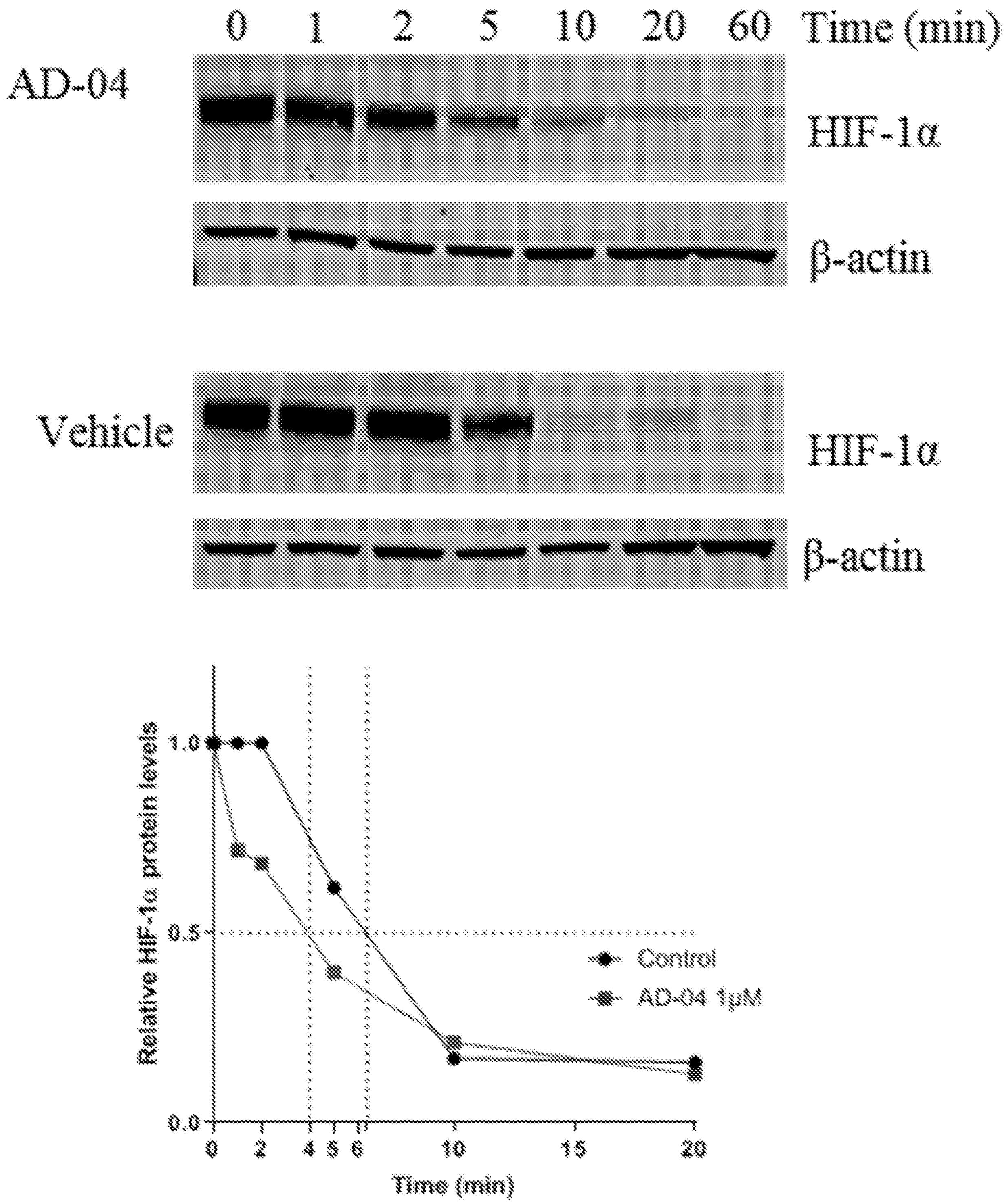
0
1
2
5
10
20
60
Time (min)
AD-04
HIF-1α
β-actin
Vehicle
HIF-1α
β-actin
Relative HIF-1α protein levels
1.0
0.5
0.0
0
2
4
5
6
10
15
20
Time (min)
Control
AD-04 1μM

Figure 10

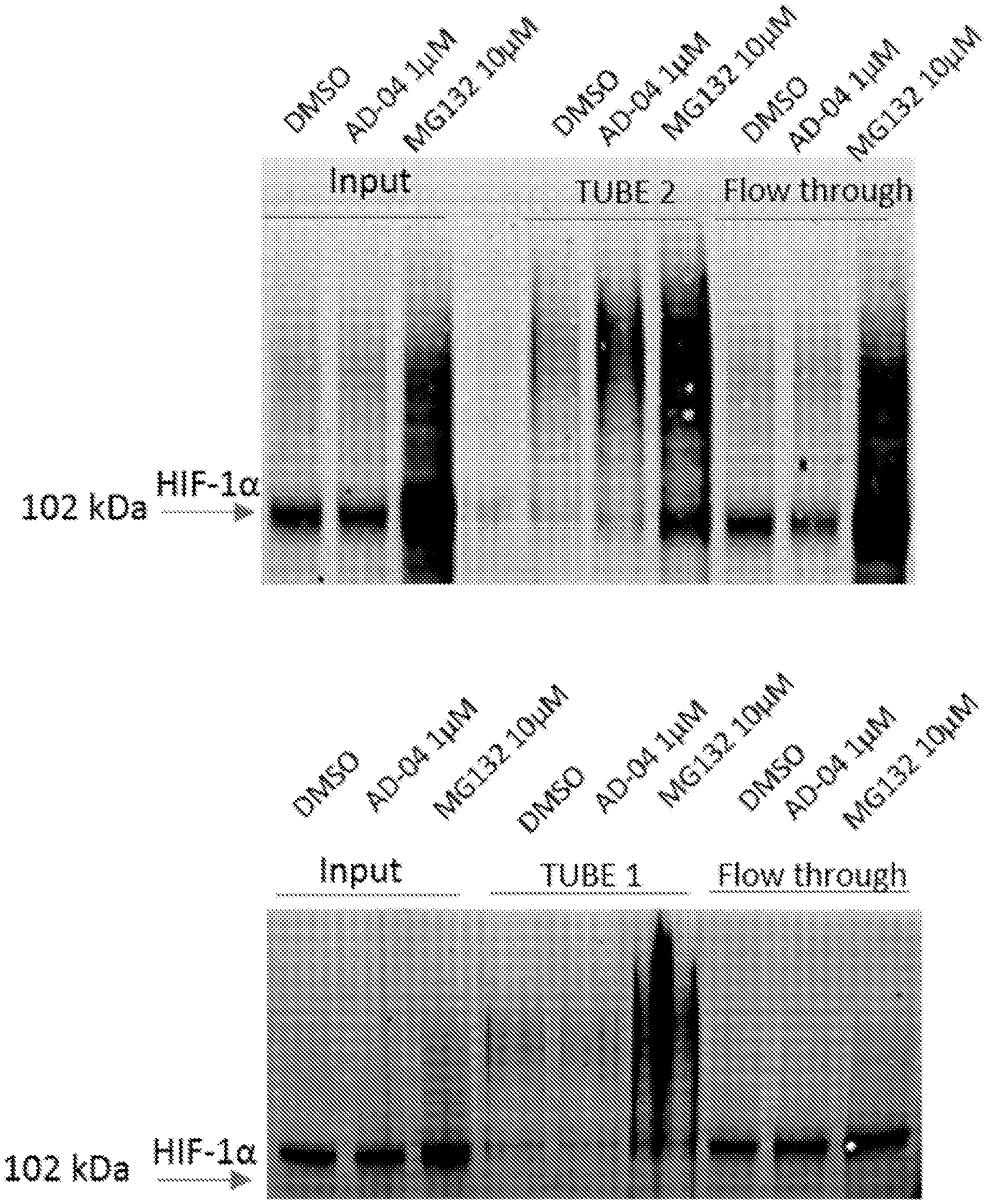
DMSO
AD-04 1μM
MG132 10μM
DMSO
AD-04 1μM
MG132 10μM
DMSO
AD-04 1μM
MG132 10μM
Input
TUBE 2
Flow through
102 kDa
HIF-1α
DMSO
AD-04 1μM
MG132 10μM
DMSO
AD-04 1μM
MG132 10μM
DMSO
AD-04 1μM
MG132 10μM
Input
TUBE 1
Flow through
102 kDa
HIF-1α

Figure 11

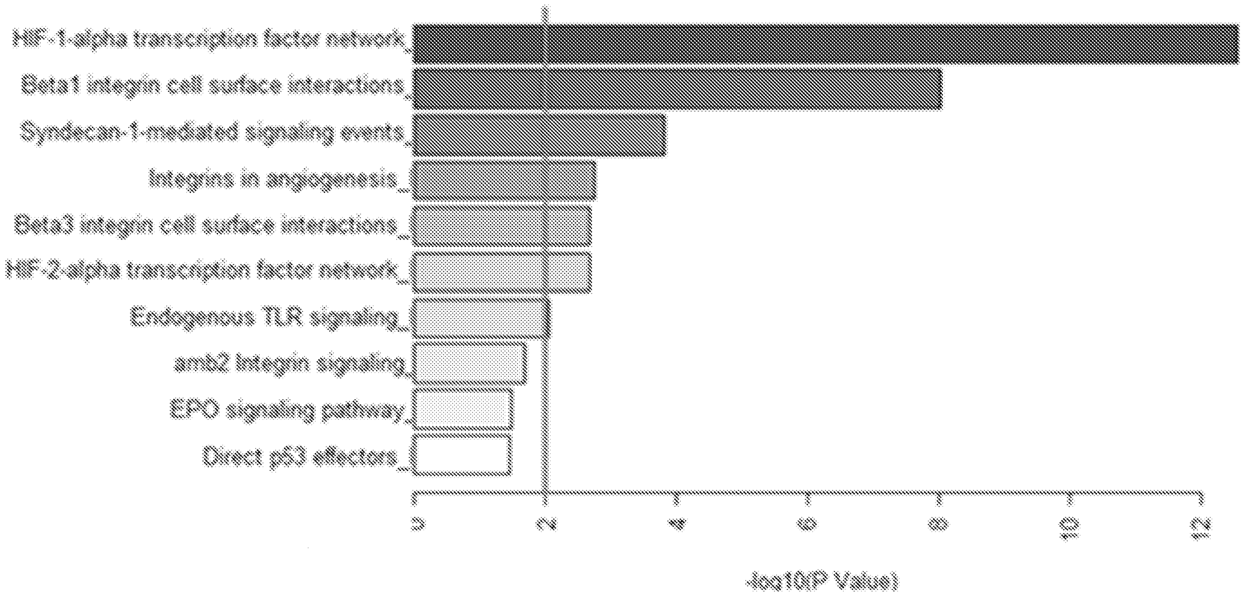
HIF-1-alpha transcription factor network
Beta1 integrin cell surface interactions
Syndecan-1-mediated signaling events
Integrins in angiogenesis
Beta3 integrin cell surface interactions
HIF-2-alpha transcription factor network
Endogenous TLR signaling
amb2 Integrin signaling
EPO signaling pathway
Direct p53 effectors
0
2
4
6
8
10
12
-log10(P Value)

Figure 12

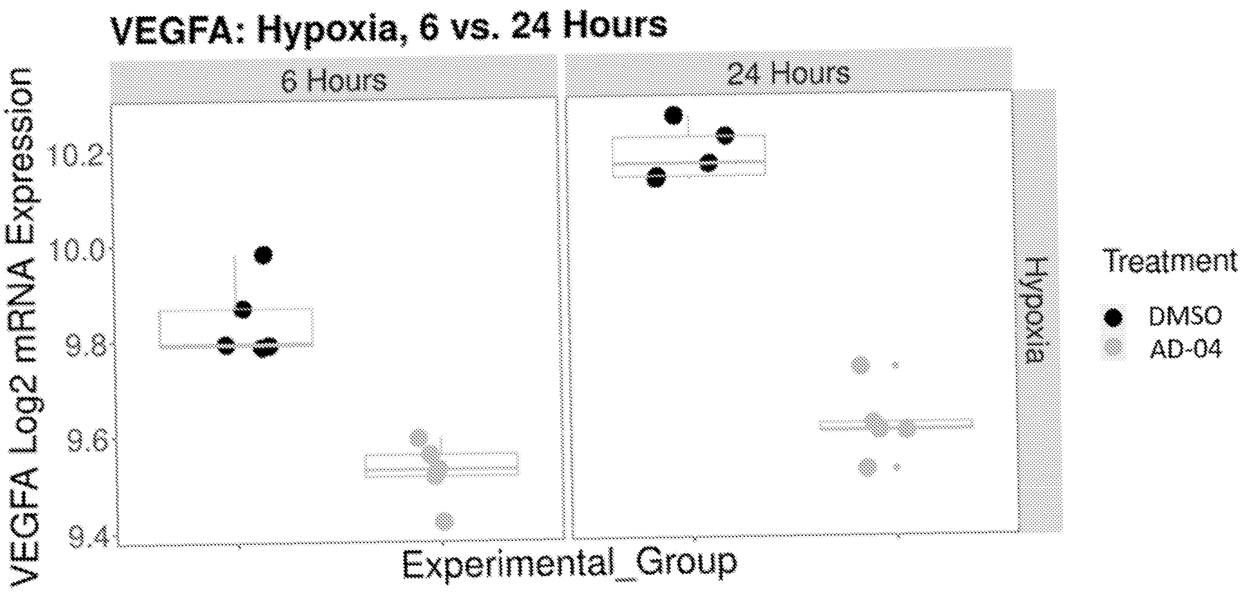
VEGFA: Hypoxia, 6 vs. 24 Hours
6 Hours
24 Hours
VEGFA Log2 mRNA Expression
10.2
10.0
9.8
9.6
9.4
Hypoxia
Experimental_Group
Treatment
DMSO
AD-04

Figure 13

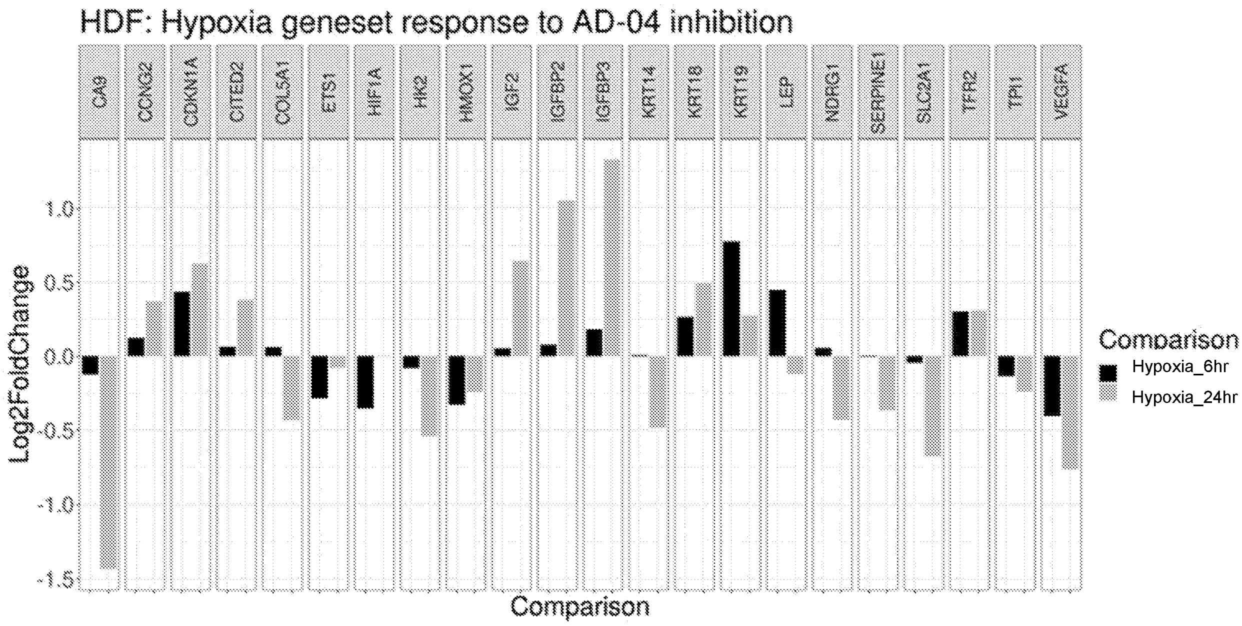
HDF: Hypoxia geneset response to AD-04 inhibition
CA9
CCNG2
CDKN1A
CITED2
COL5A1
ETS1
HIF1A
HK2
HMOX1
IGF2
IGFBP2
IGFBP3
KRT14
KRT18
KRT19
LEP
NDRG1
SERPINE1
SLC2A1
TFR2
TPI1
VEGFA
Log2FoldChange
1.0
0.5
0.0
-0.5
-1.0
-1.5
Comparison
Comparison
Hypoxia_6hr
Hypoxia_24hr

Figure 14

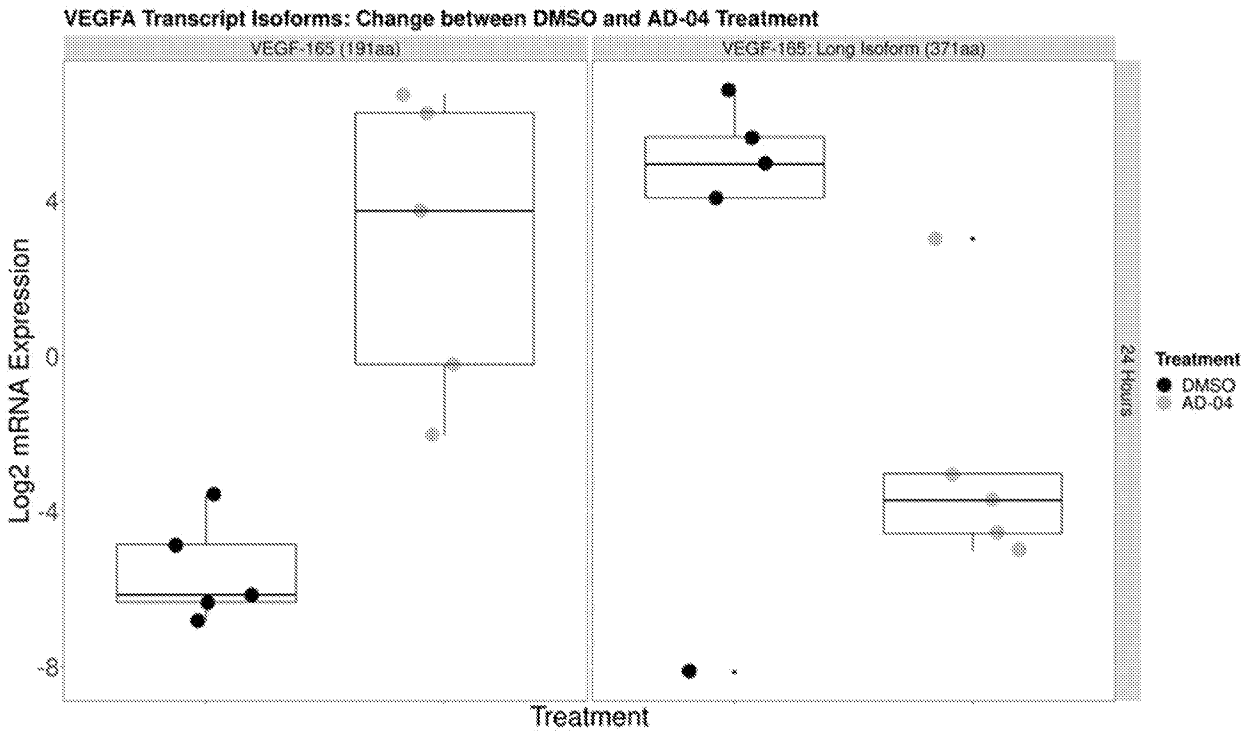
VEGFA Transcript Isoforms: Change between DMSO and AD-04 Treatment
VEGF-165 (191aa)
VEGF-165: Long Isoform (371aa)
24 Hours
Log2 mRNA Expression
4
0
-4
-8
Treatment
Treatment
DMSO
AD-04

Figure 15

AD-04

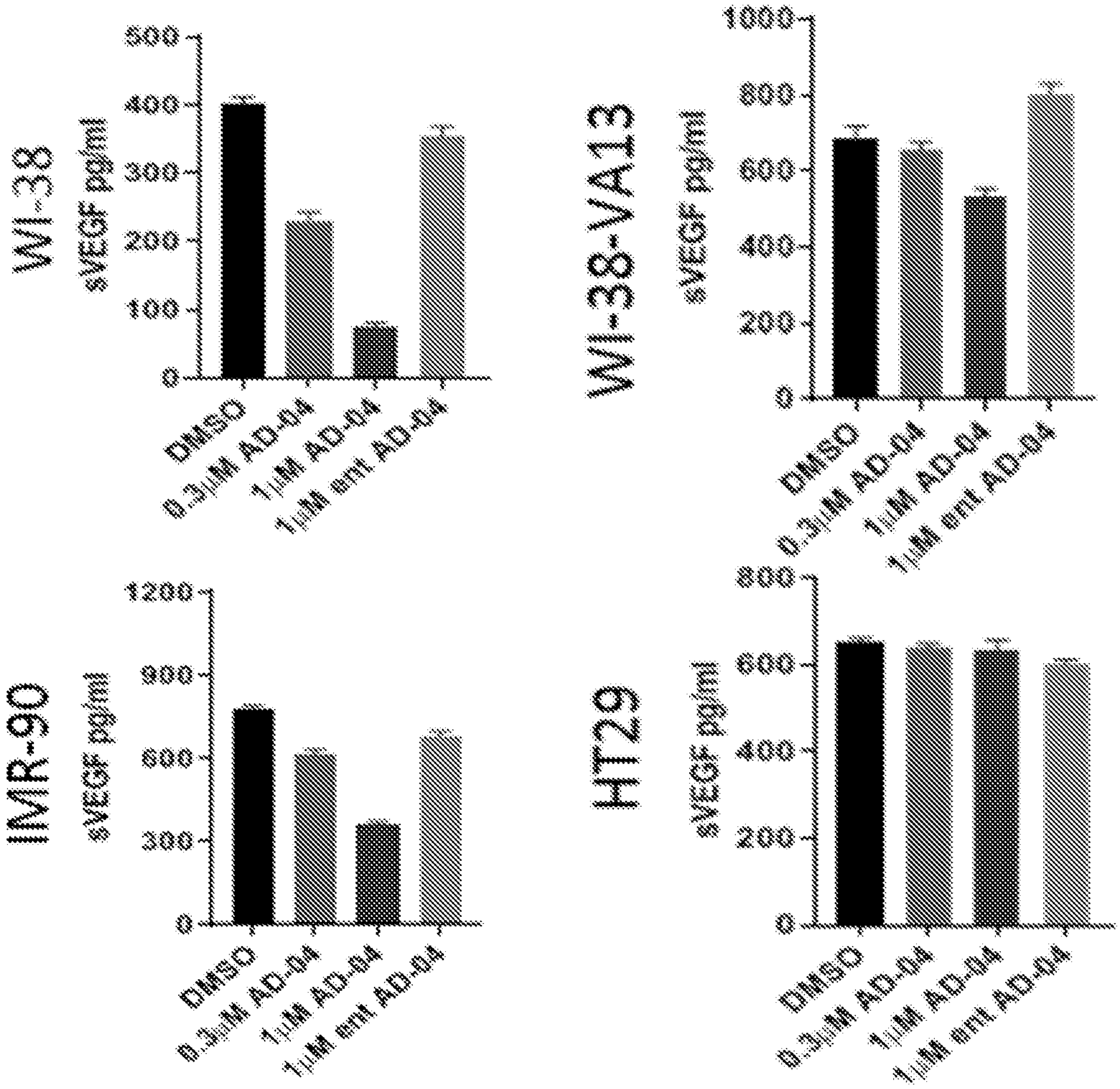


Figure 16

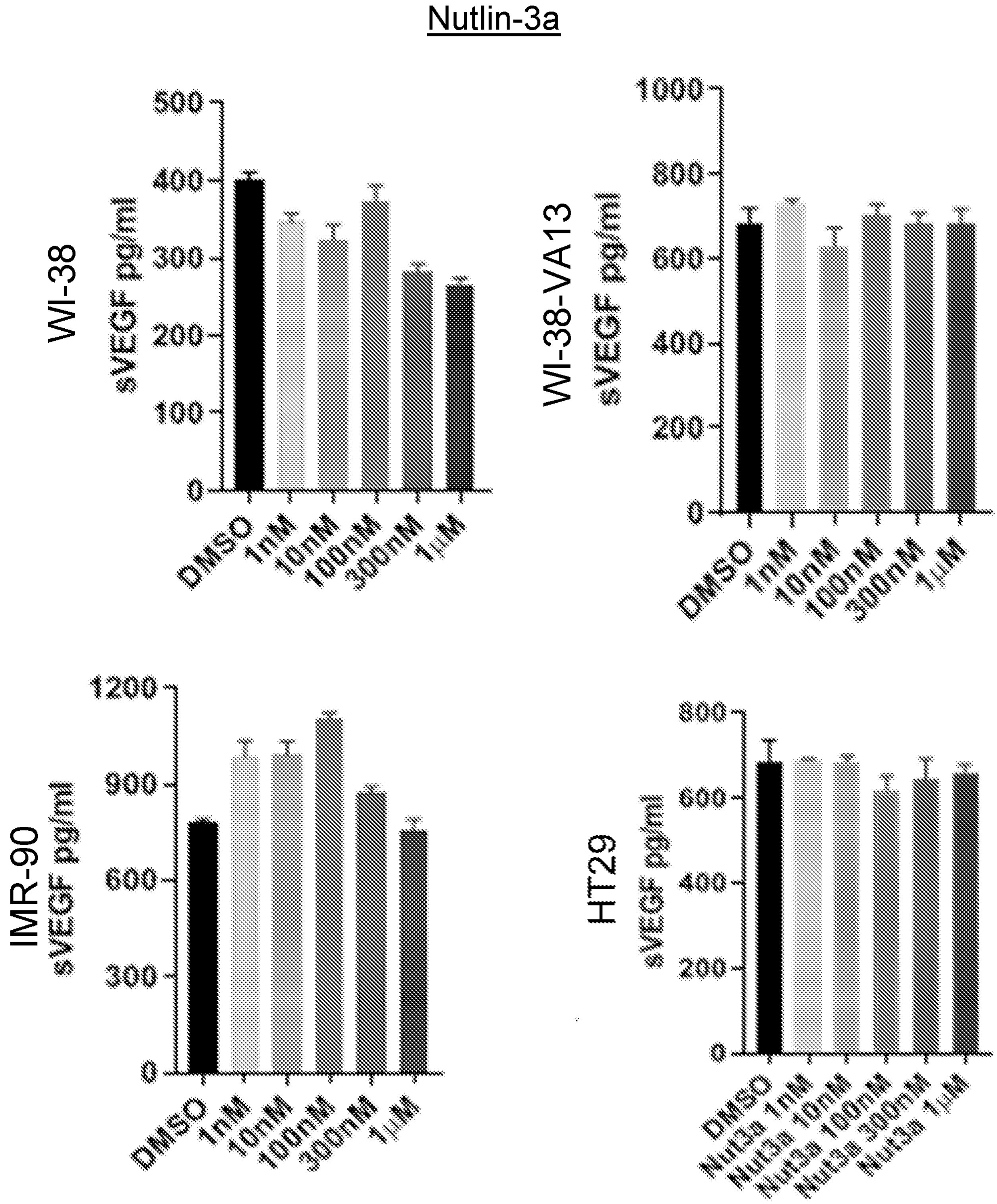


Figure 16 (continued)

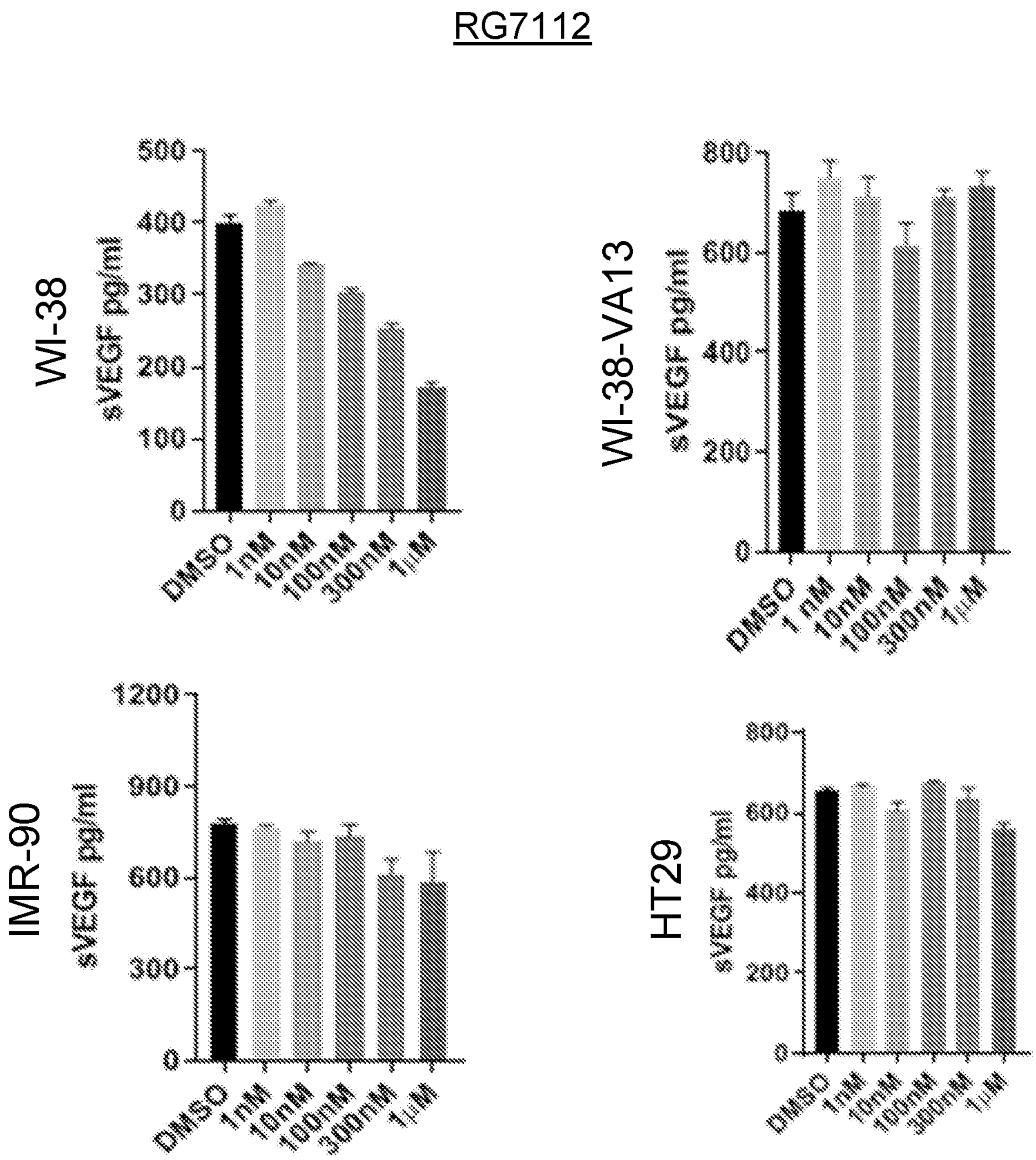


Figure 16 (continued)

SAR405838

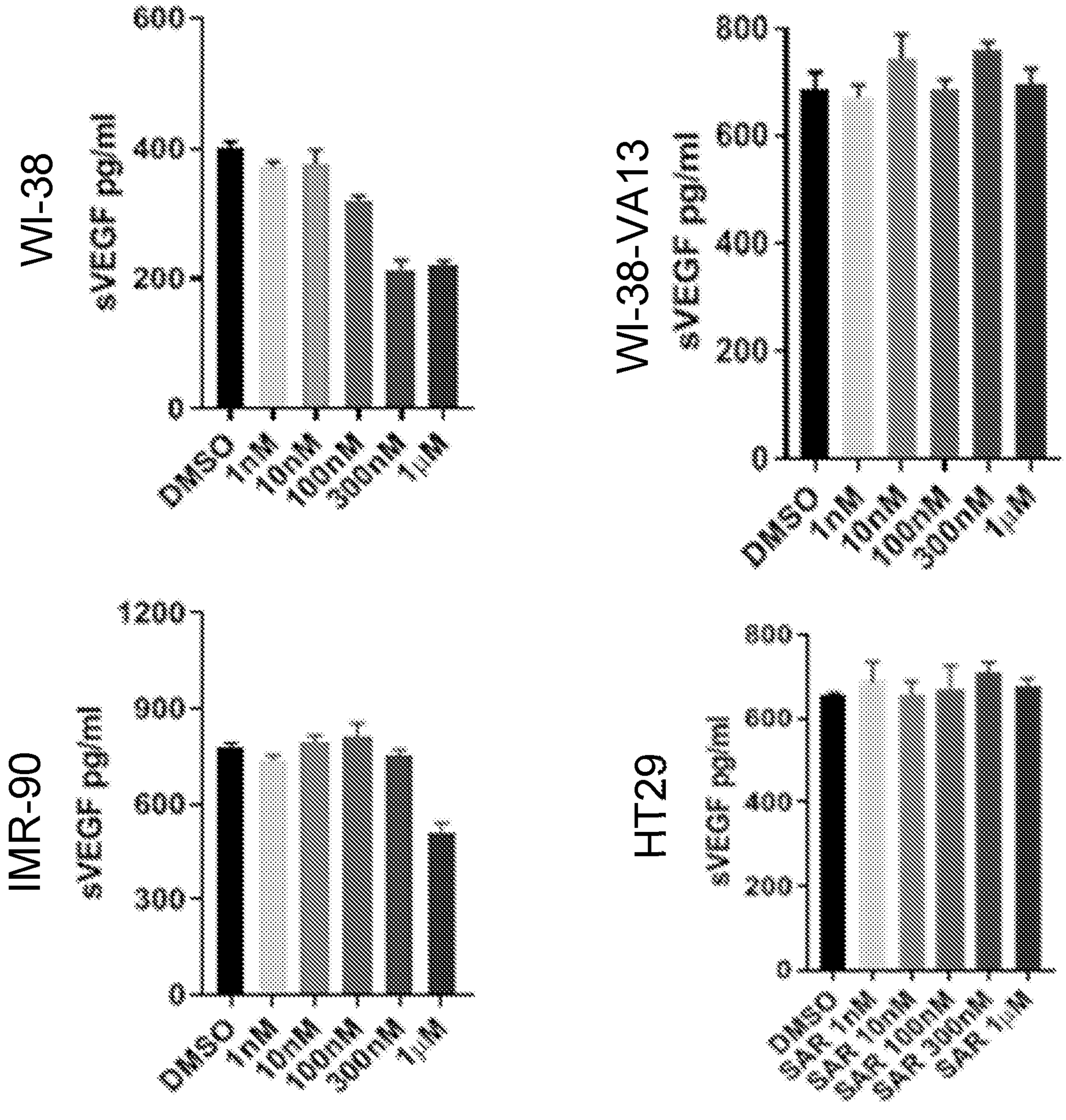


Figure 16 (continued)

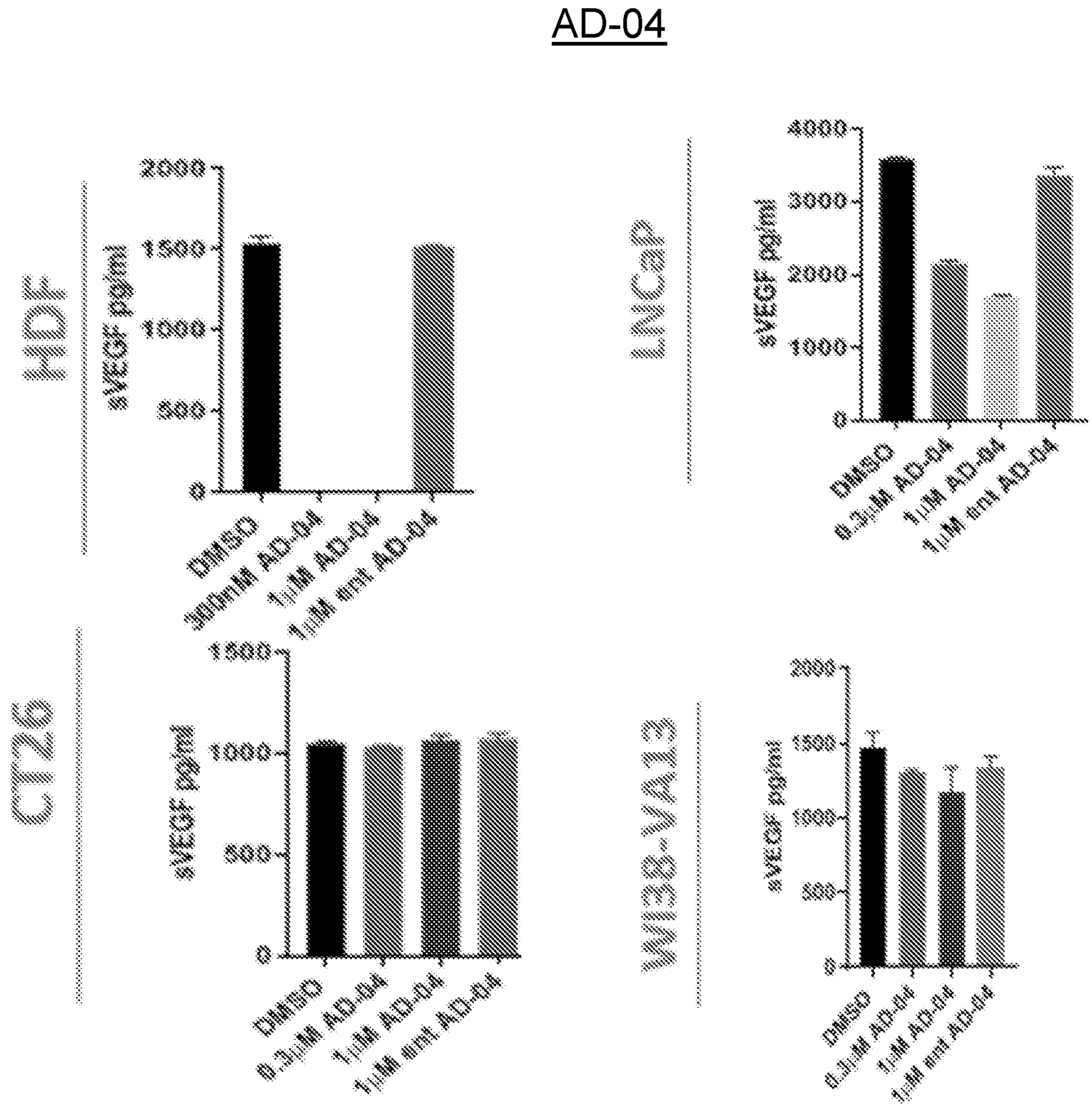
AD-04
HDF
sVEGF pg/ml
2000
1500
1000
500
0
DMSO
300nM AD-04
1μM AD-04
1μM ent AD-04
LNCaP
sVEGF pg/ml
4000
3000
2000
1000
0
DMSO
0.3μM AD-04
1μM AD-04
1μM ent AD-04
CT26
sVEGF pg/ml
1500
1000
500
0
DMSO
0.3μM AD-04
1μM AD-04
1μM ent AD-04
WI38-VA13
sVEGF pg/ml
2000
1500
1000
500
0
DMSO
0.3μM AD-04
1μM AD-04
1μM ent AD-04

Figure 17

Nutlin-3a

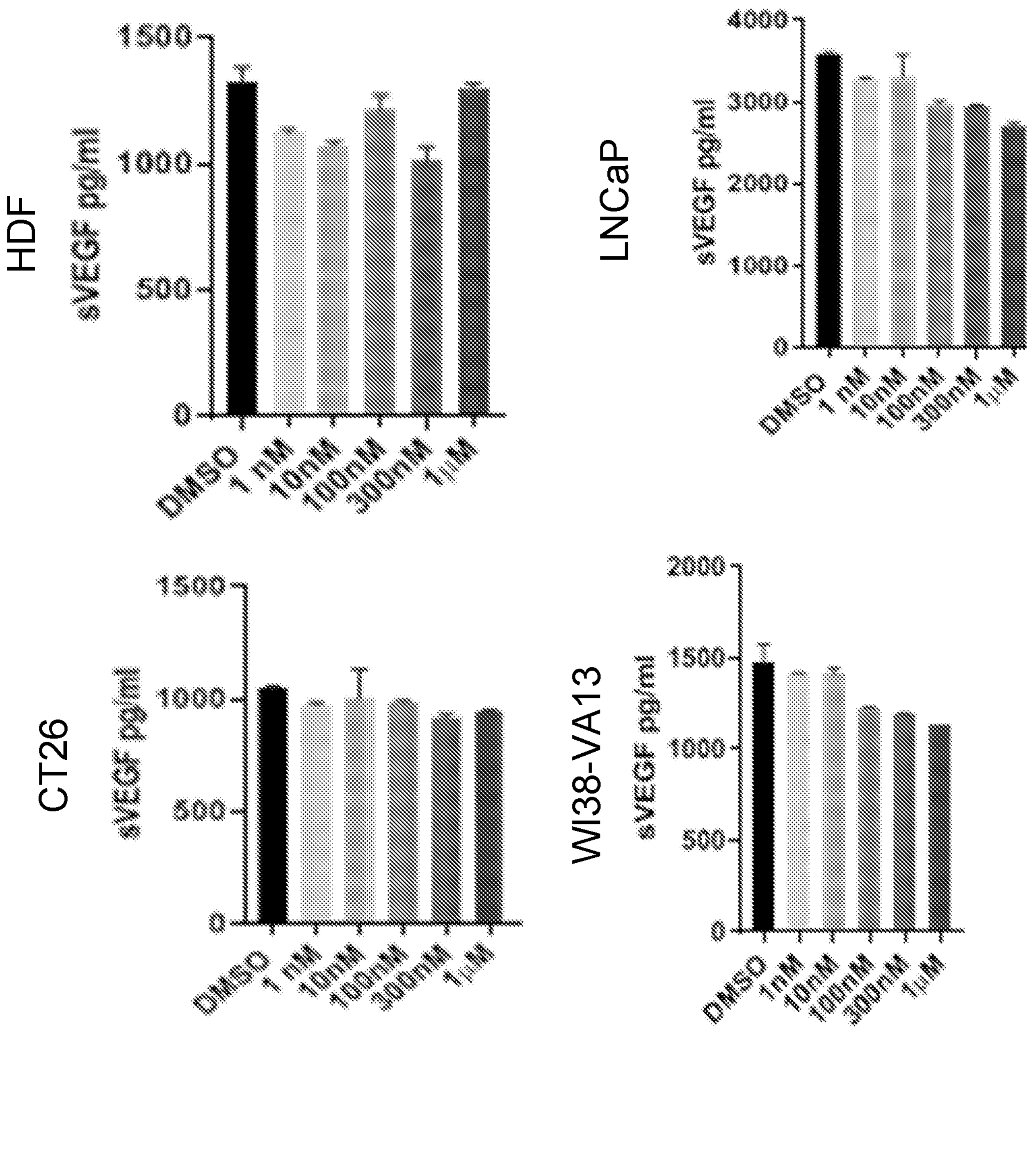


Figure 17 (continued)

RG7112

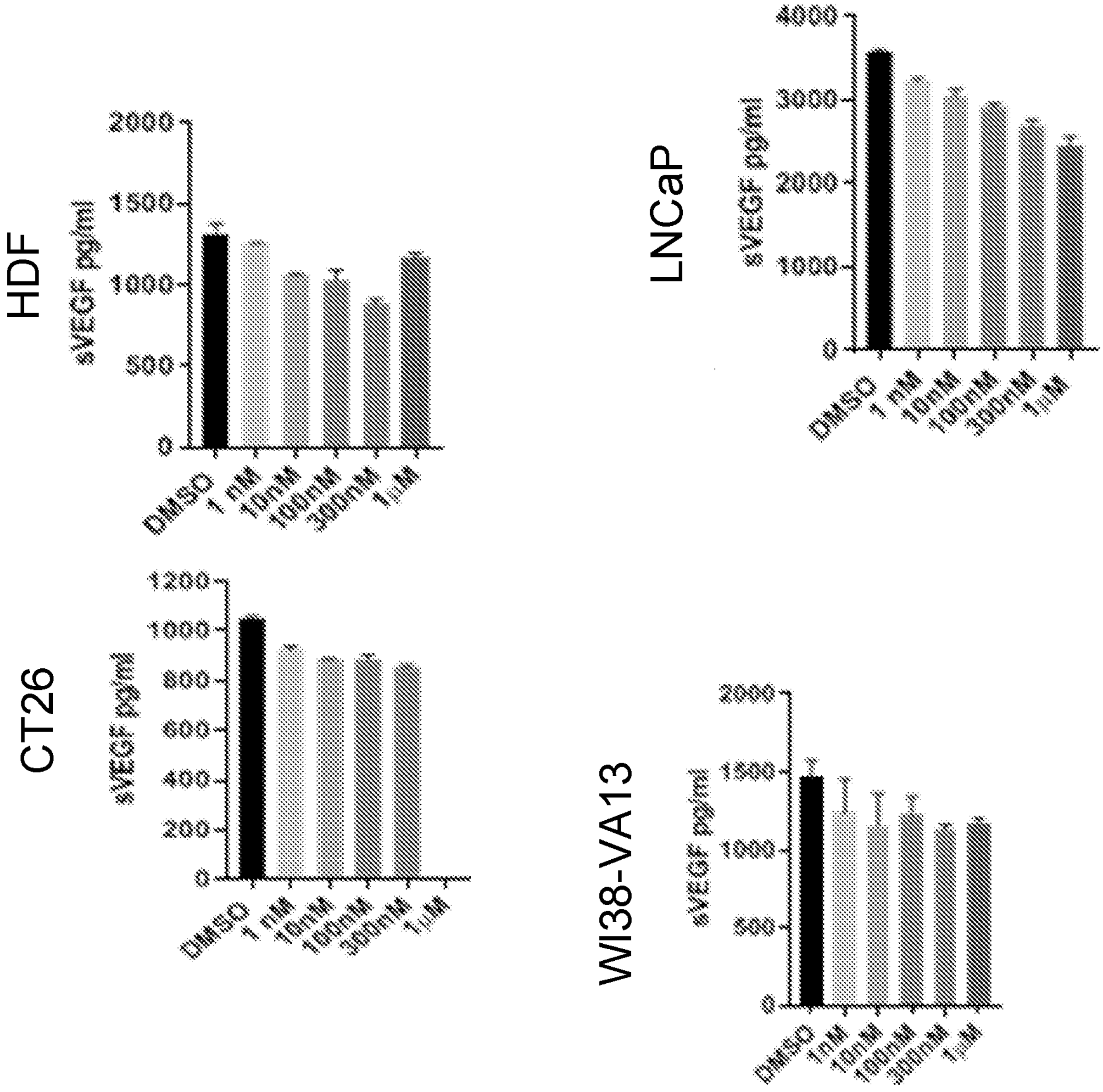


Figure 17 (continued)

SAR405838

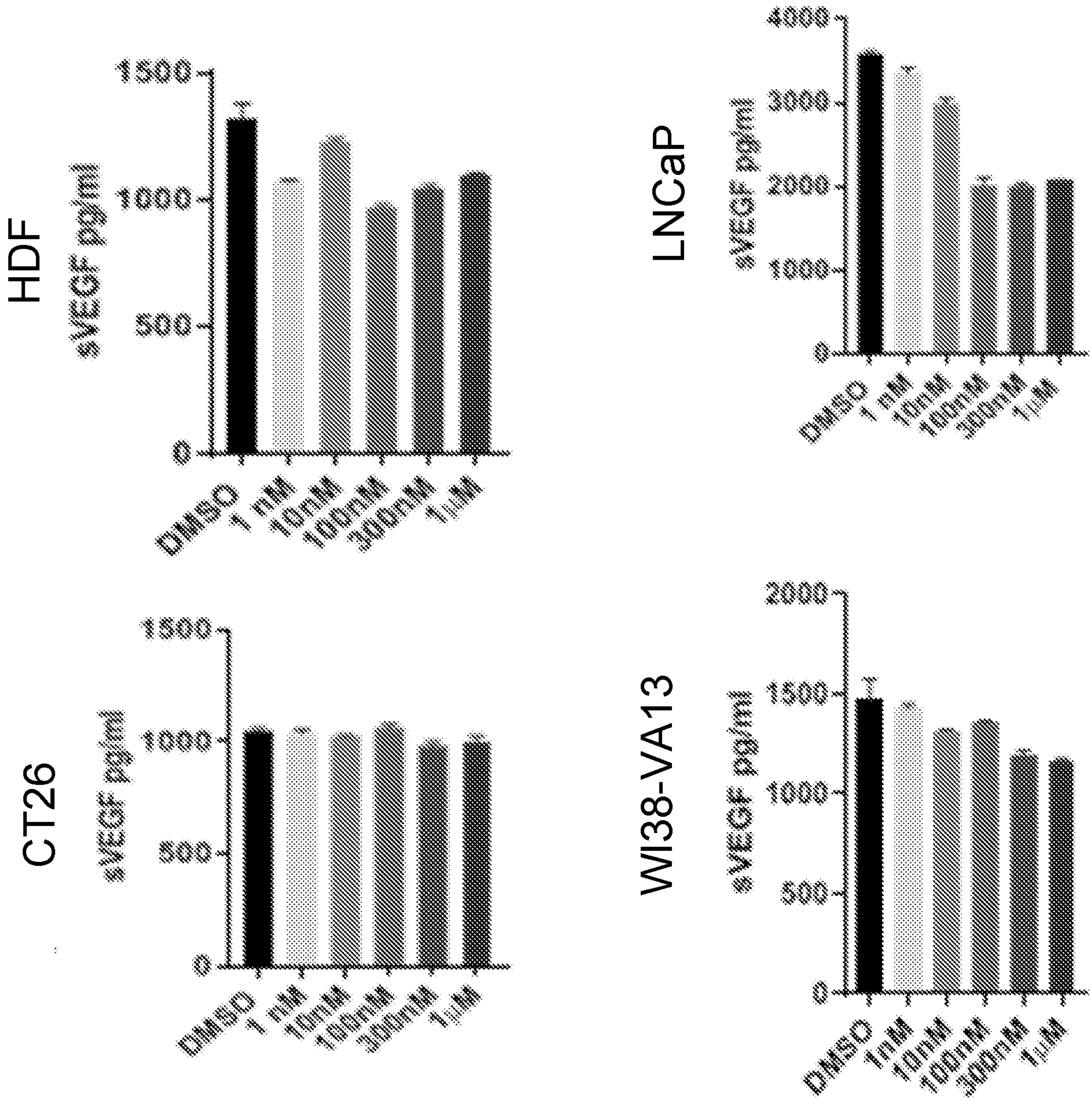


Figure 17 (continued)

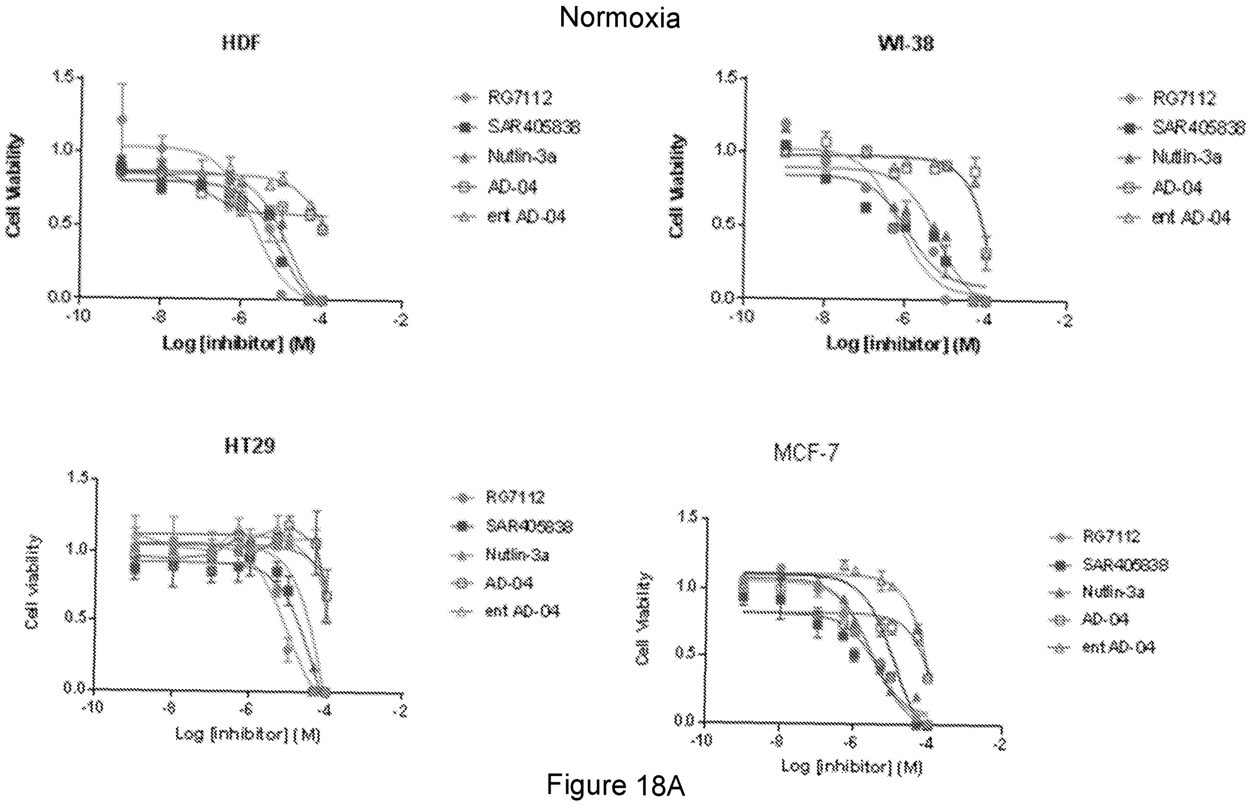
Normoxia
HDF
WI-38
HT29
MCF-7
Cell Viability
Cell viability
Log [inhibitor] (M)
RG7112
SAR405838
Nutlin-3a
AD-04
ent AD-04

Figure 18A

Normoxia

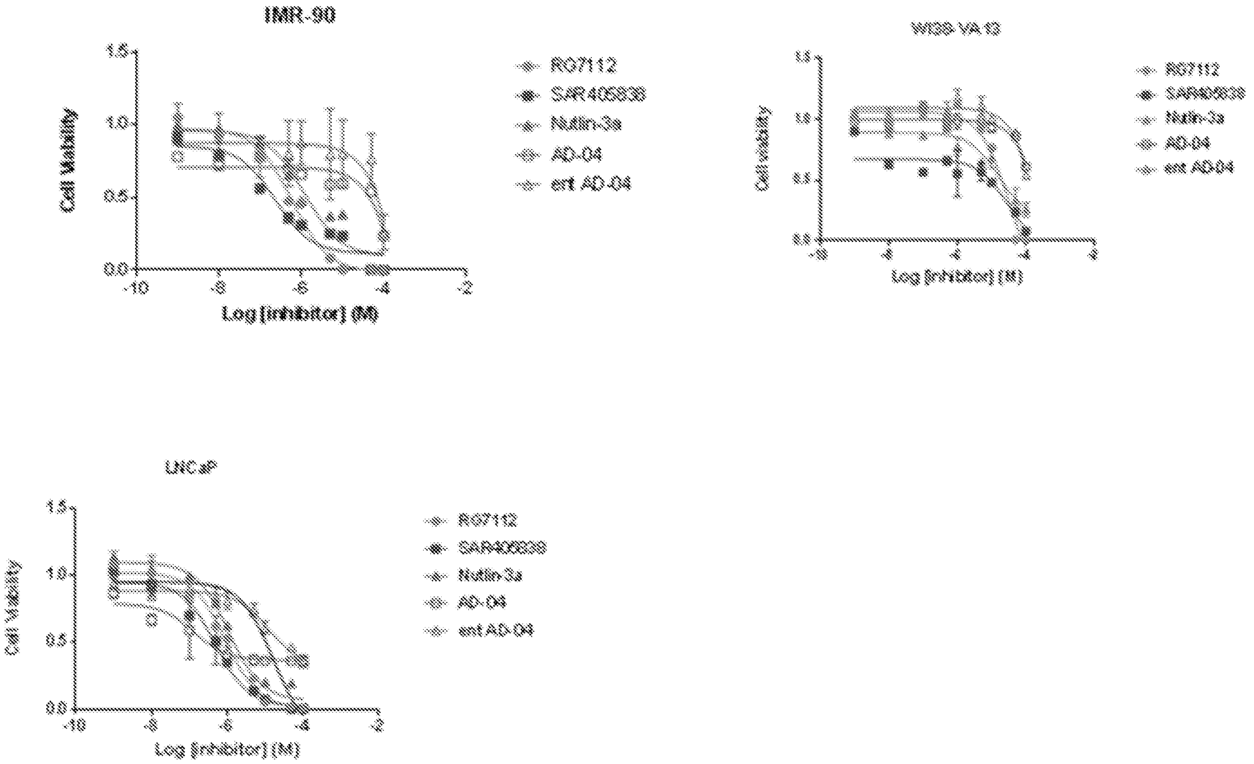


Figure 18B

Hypoxia

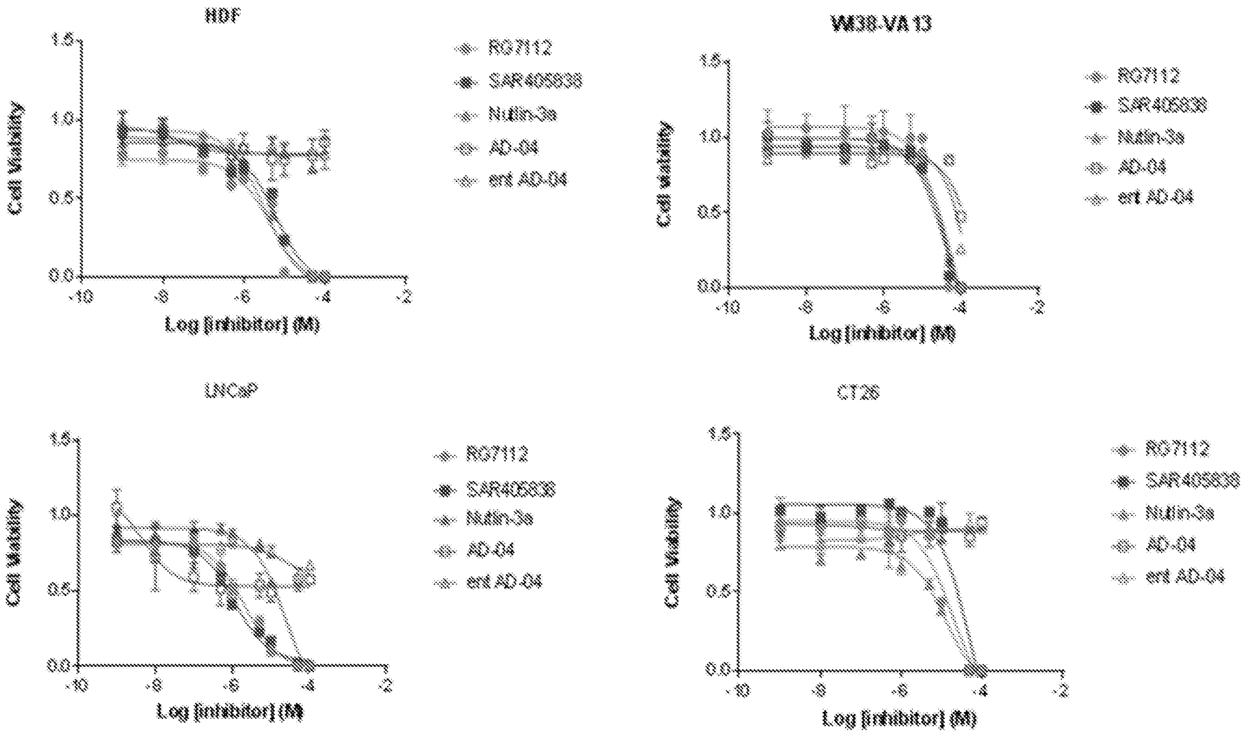


Figure 18C

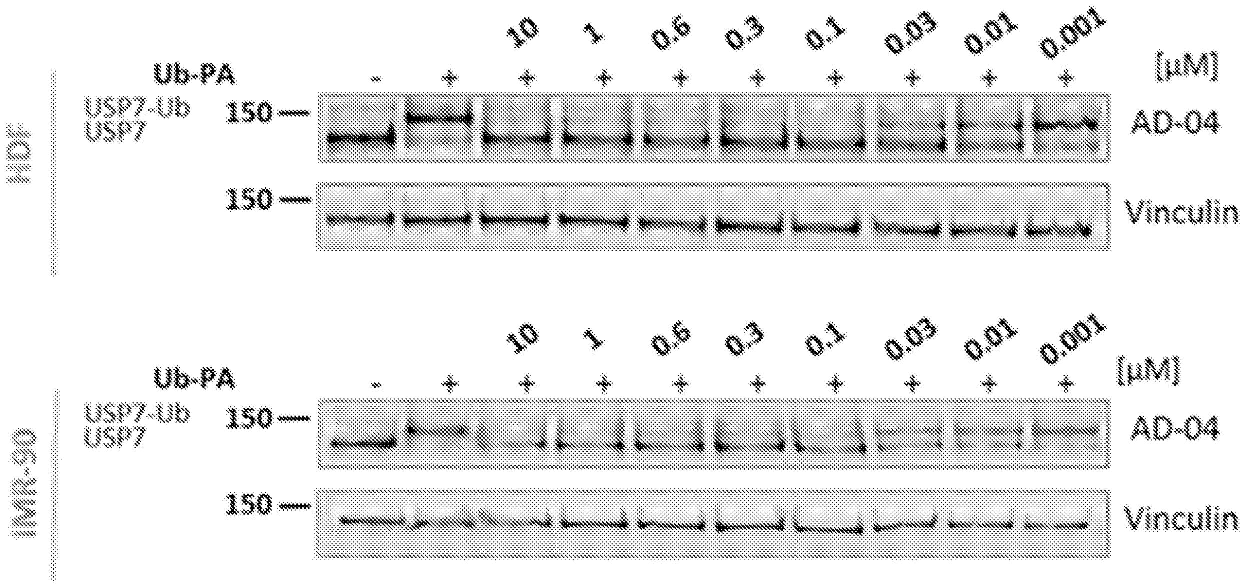
10
1
0.6
0.3
0.1
0.03
0.01
0.001
Ub-PA
-
+
[µM]
HDF
USP7-Ub
USP7
150
AD-04
Vinculin
IMR-90

Figure 19A

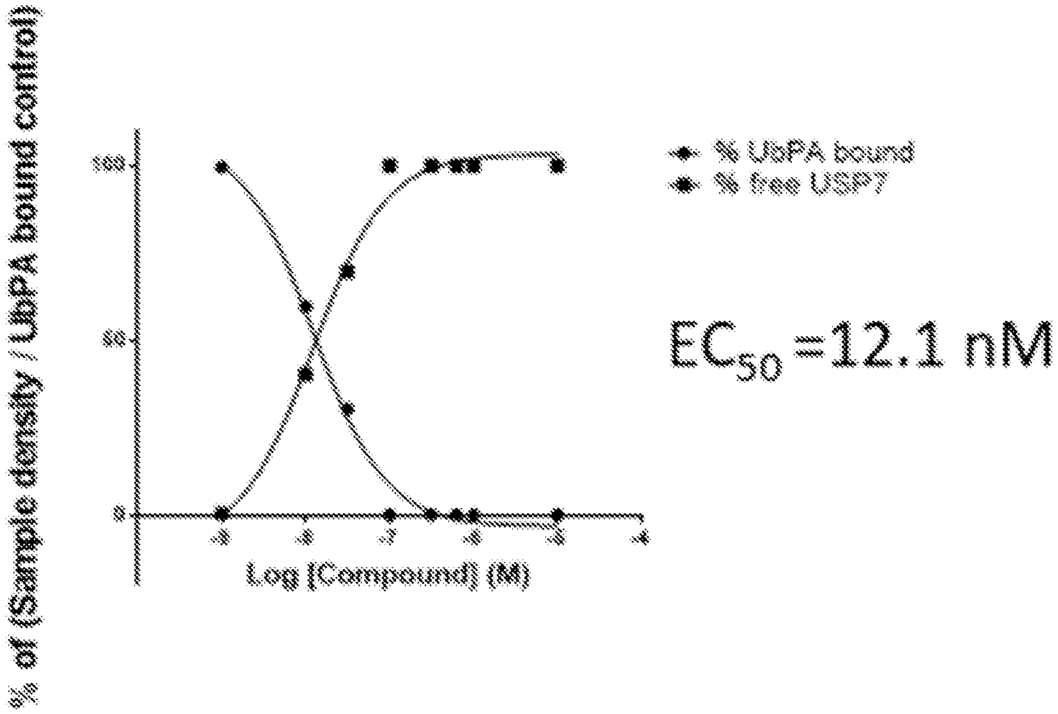
% of (Sample density / UbPA bound control)
100
50
0
Log [Compound] (M)
% UbPA bound
% free USP7
EC50 =12.1 nM

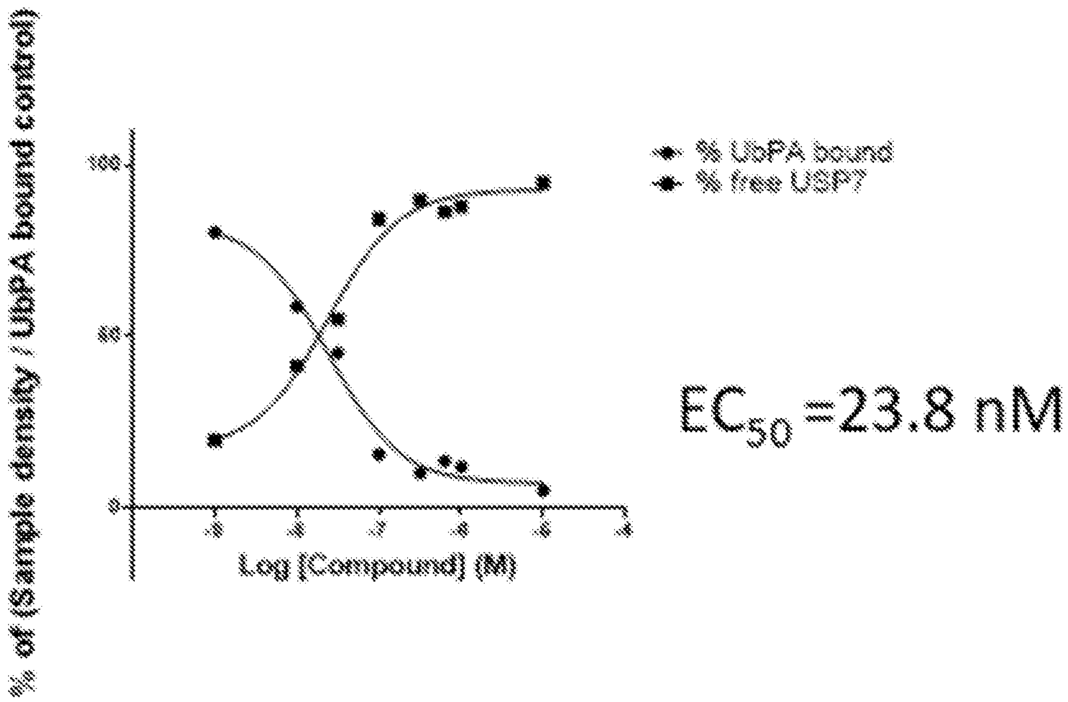
% of (Sample density / UbPA bound control)
100
50
0
Log [Compound] (M)
% UbPA bound
% free USP7
EC50 =23.8 nM

Figure 19B

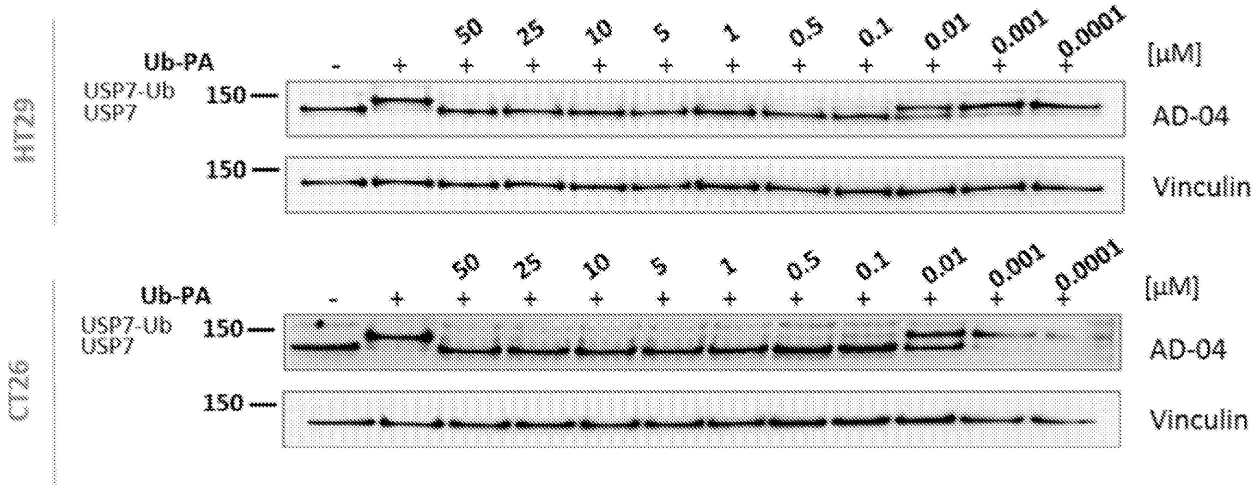
HT29
Ub-PA
- + + + + + + + + + + +
50 25 10 5 1 0.5 0.1 0.01 0.001 0.0001 [µM]
USP7-Ub
USP7
150
AD-04
150
Vinculin
CT26
Ub-PA
- + + + + + + + + + + +
50 25 10 5 1 0.5 0.1 0.01 0.001 0.0001 [µM]
USP7-Ub
USP7
150
AD-04
150
Vinculin

Figure 20A

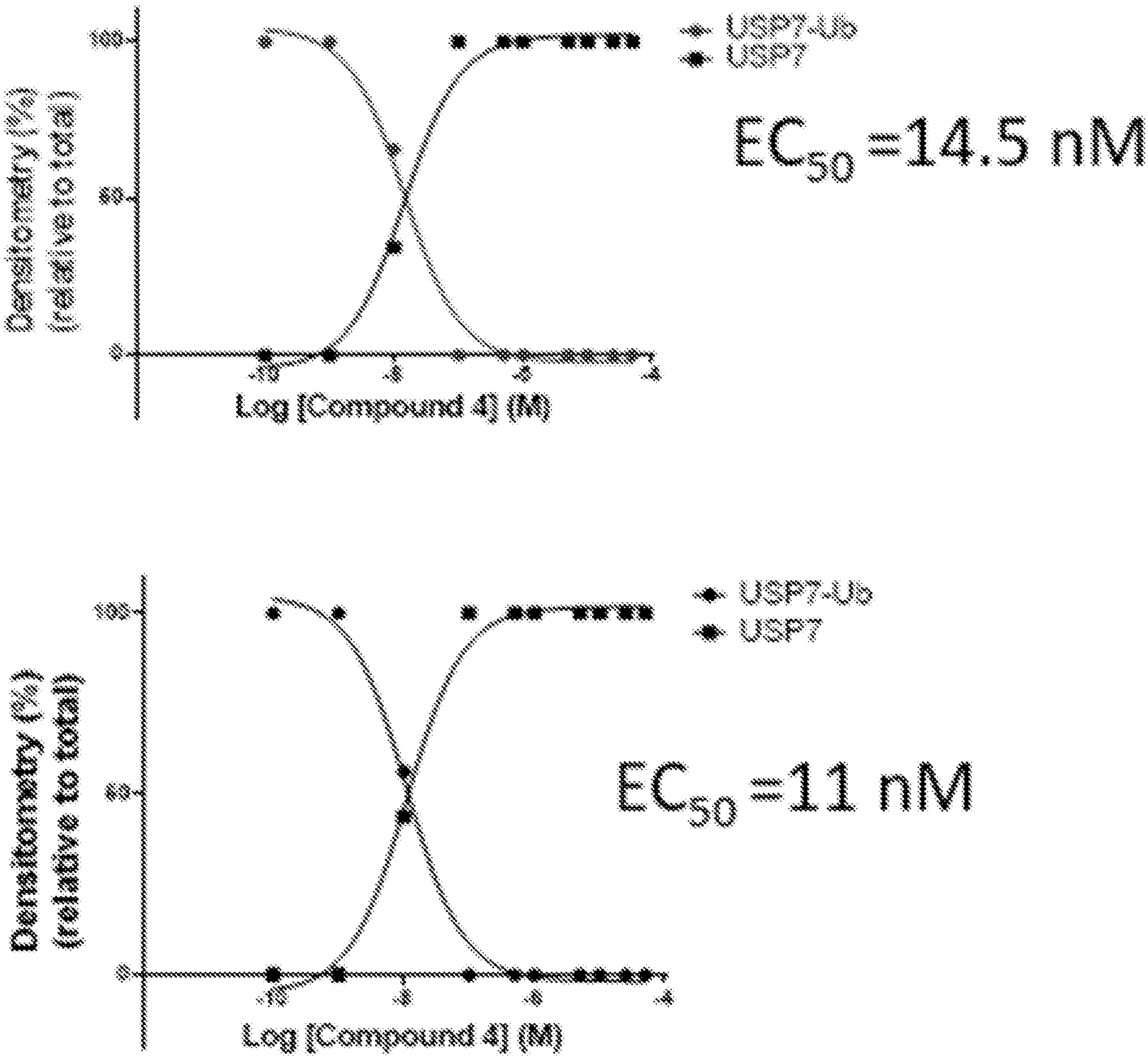
USP7-Ub
USP7
Densitometry (%)
(relative to total)
Log [Compound 4] (M)
EC50 =14.5 nM
USP7-Ub
USP7
Densitometry (%)
(relative to total)
Log [Compound 4] (M)
EC50 =11 nM

Figure 20B

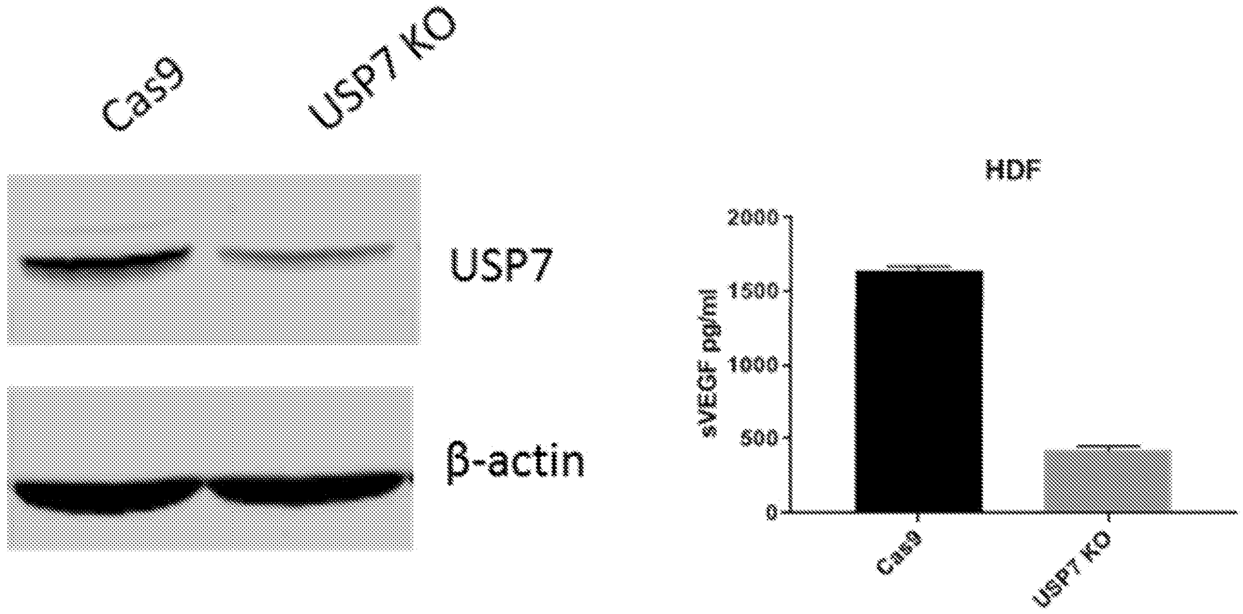
Cas9
USP7 KO
USP7
β-actin
HDF
2000
1500
1000
500
0
sVEGF pg/ml
Cas9
USP7 KO

Figure 21

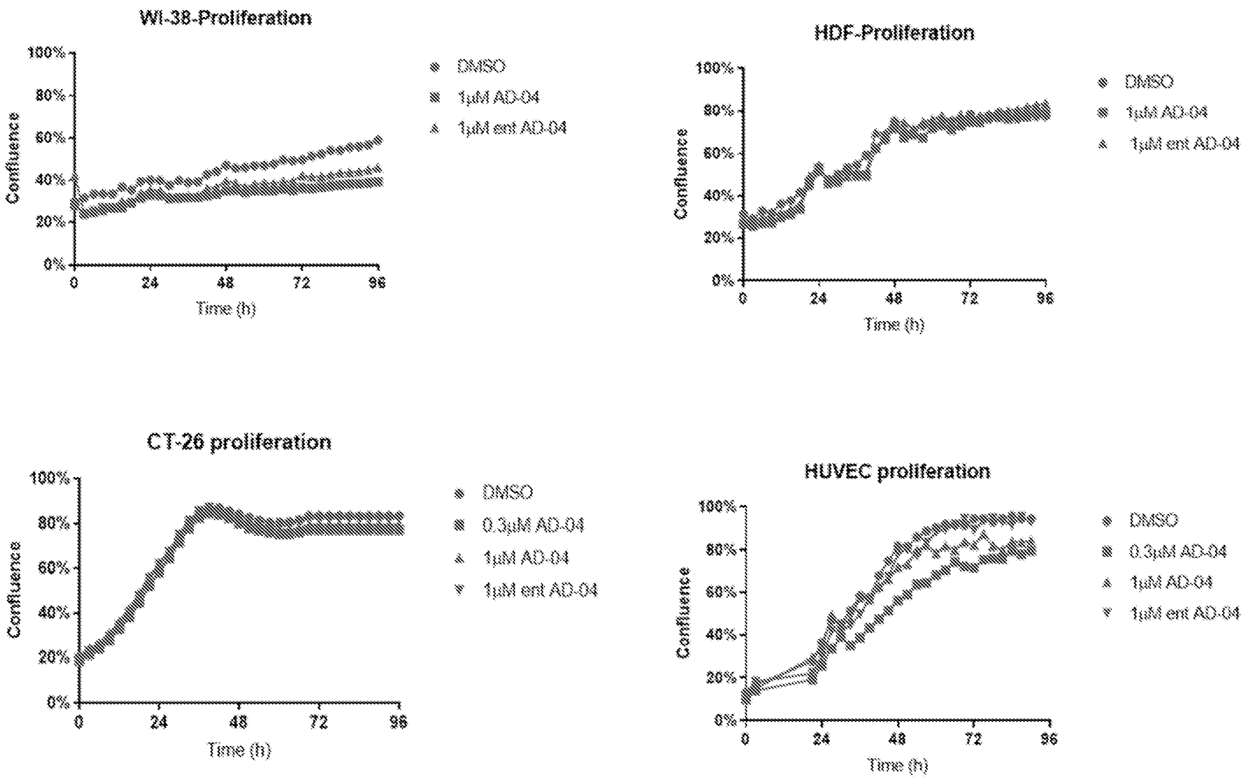
WI-38-Proliferation
100%
80%
60%
40%
20%
0%
Confluence
0
24
48
72
96
Time (h)
DMSO
1μM AD-04
1μM ent AD-04
HDF-Proliferation
100%
80%
60%
40%
20%
0%
Confluence
0
24
48
72
96
Time (h)
DMSO
1μM AD-04
1μM ent AD-04
CT-26 proliferation
100%
80%
60%
40%
20%
0%
Confluence
0
24
48
72
96
Time (h)
DMSO
0.3μM AD-04
1μM AD-04
1μM ent AD-04
HUVEC proliferation
100%
80%
60%
40%
20%
0%
Confluence
0
24
48
72
96
Time (h)
DMSO
0.3μM AD-04
1μM AD-04
1μM ent AD-04

Figure 22

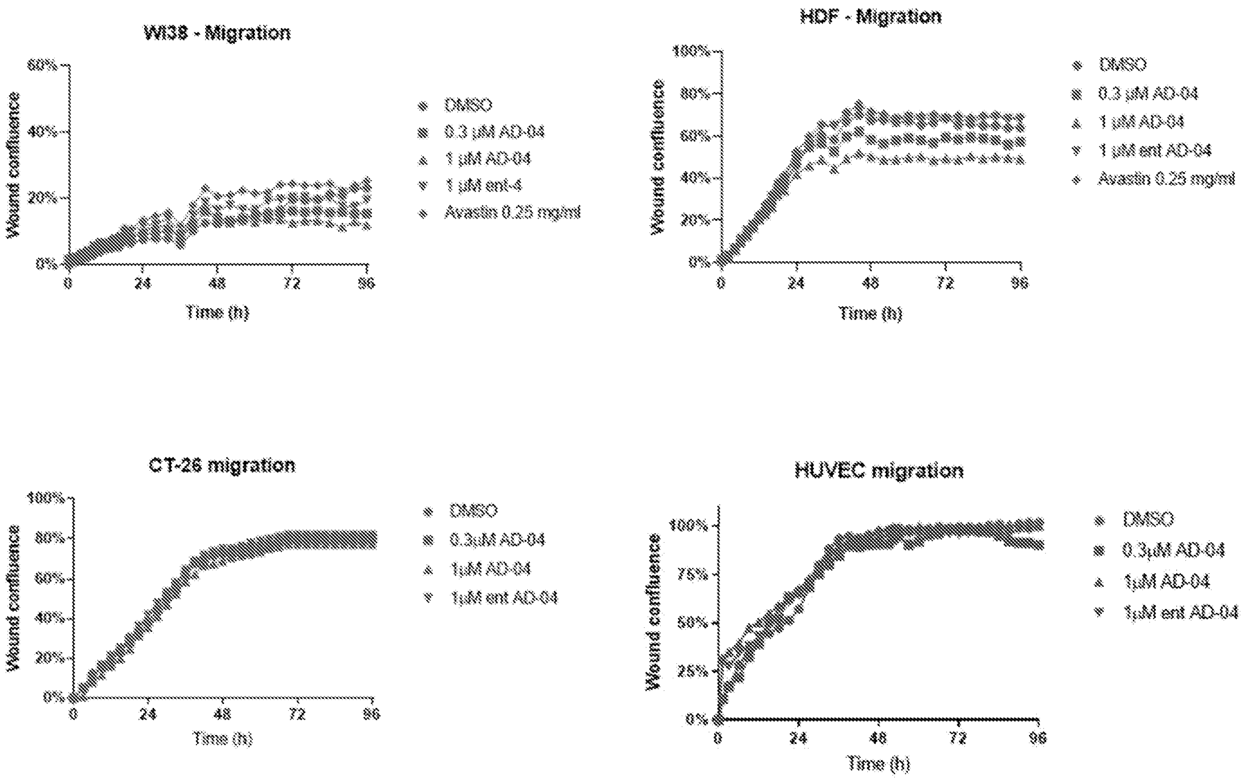
WI38 - Migration
Wound confluence
0%
20%
40%
60%
0
24
48
72
96
Time (h)
DMSO
0.3 µM AD-04
1 µM AD-04
1 µM ent-4
Avastin 0.25 mg/ml
HDF - Migration
Wound confluence
0%
20%
40%
60%
80%
100%
0
24
48
72
96
Time (h)
DMSO
0.3 µM AD-04
1 µM AD-04
1 µM ent AD-04
Avastin 0.25 mg/ml
CT-26 migration
Wound confluence
0%
20%
40%
60%
80%
100%
0
24
48
72
96
Time (h)
DMSO
0.3µM AD-04
1µM AD-04
1µM ent AD-04
HUVEC migration
Wound confluence
0%
25%
50%
75%
100%
0
24
48
72
96
Time (h)
DMSO
0.3µM AD-04
1µM AD-04
1µM ent AD-04

Figure 23

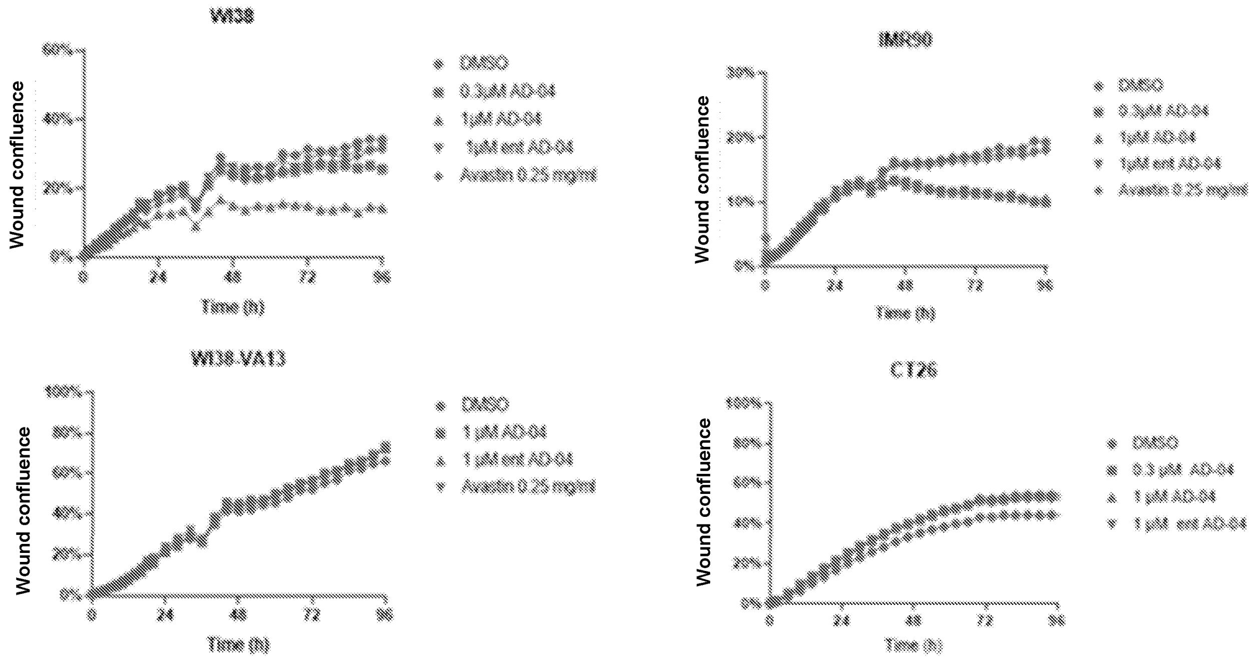
WI38
Wound confluence
Time (h)
DMSO
0.3µM AD-04
1µM AD-04
1µM ent AD-04
Avastin 0.25 mg/ml
IMR90
Wound confluence
Time (h)
DMSO
0.3µM AD-04
1µM AD-04
1µM ent AD-04
Avastin 0.25 mg/ml
WI38-VA13
Wound confluence
Time (h)
DMSO
1 µM AD-04
1 µM ent AD-04
Avastin 0.25 mg/ml
CT26
Wound confluence
Time (h)
DMSO
0.3 µM AD-04
1 µM AD-04
1 µM ent AD-04

Figure 24

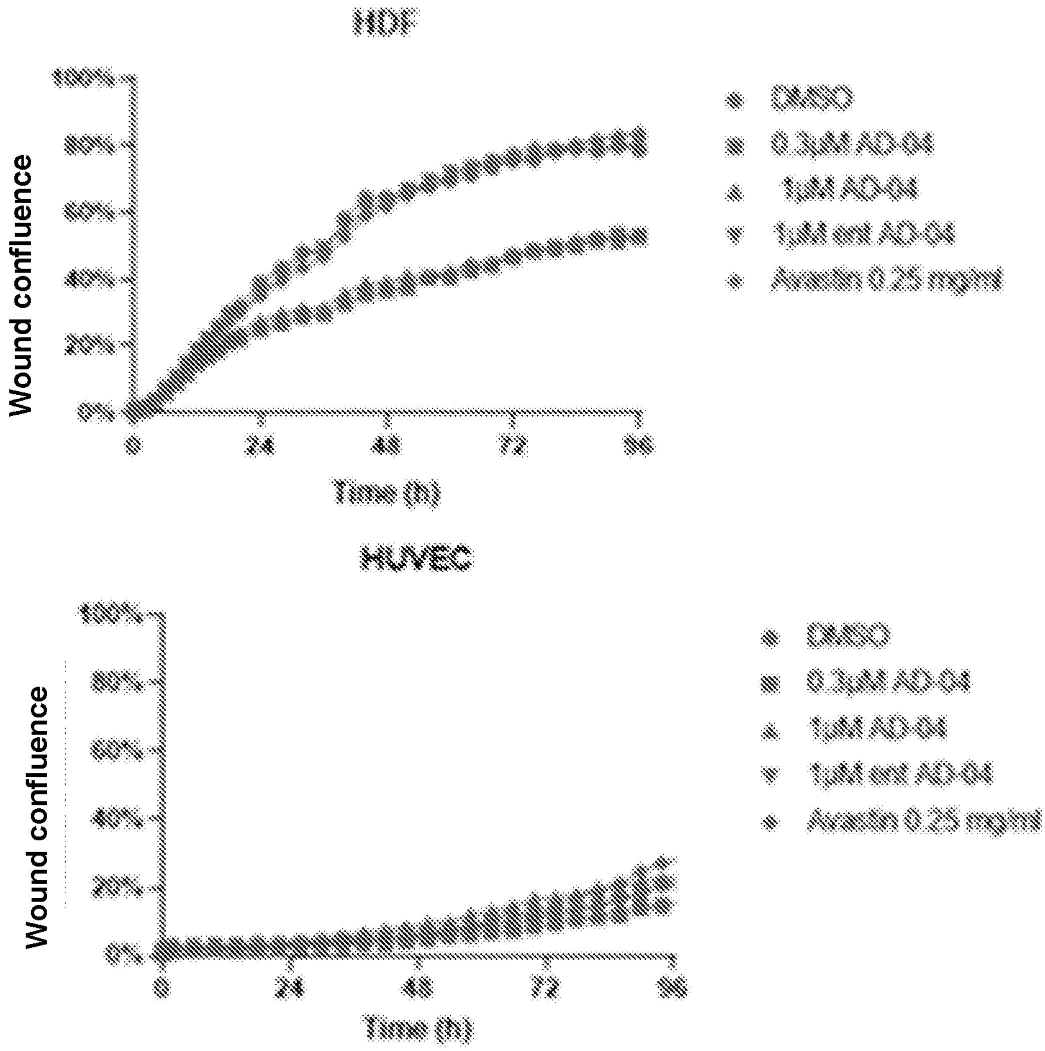


Figure 24 (continued)

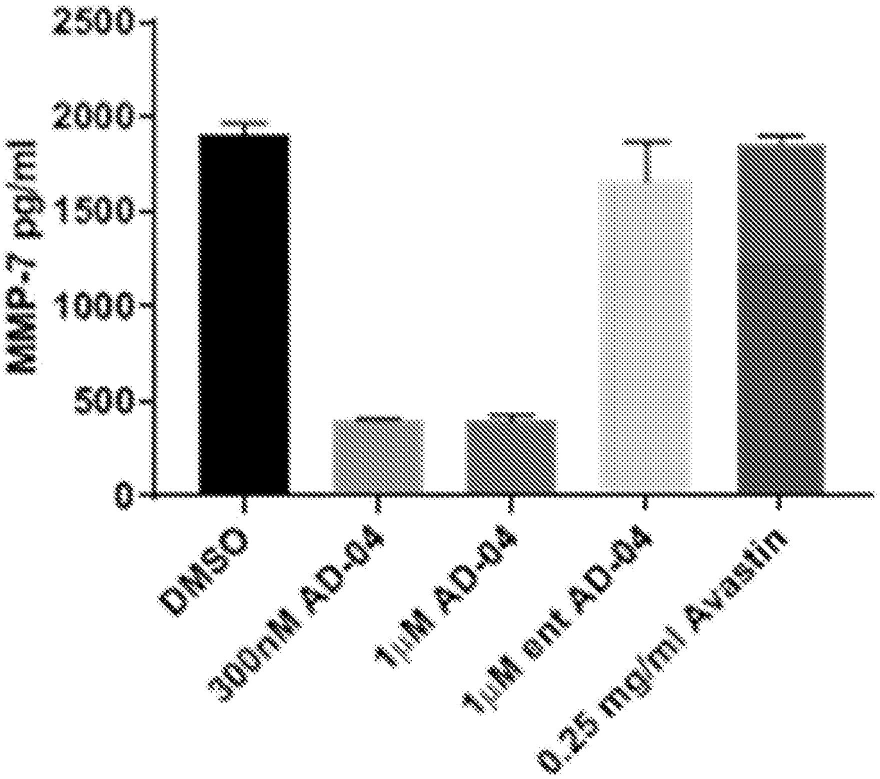
MMP-7 pg/ml
2500
2000
1500
1000
500
0
DMSO
300nM AD-04
1μM AD-04
1μM ent AD-04
0.25 mg/ml Avastin

Figure 25

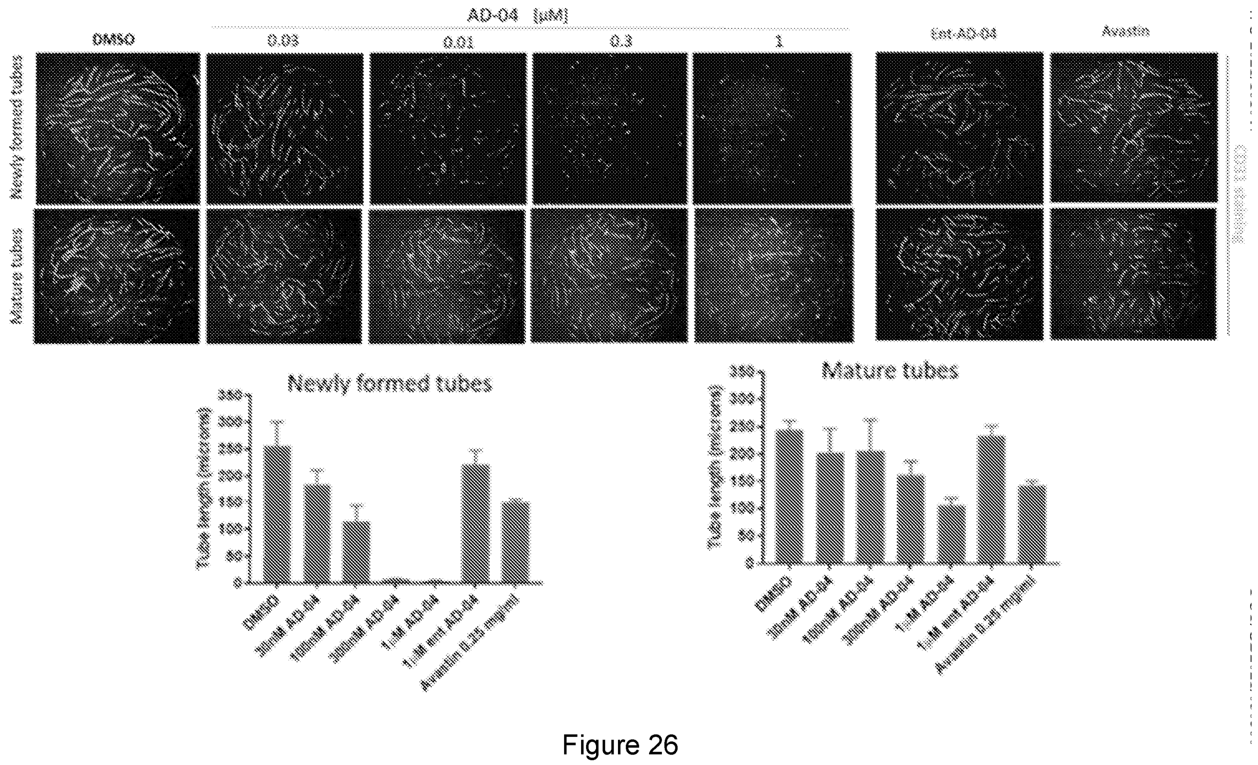
AD-04 [µM]
DMSO
0.03
0.01
0.3
1
Ent-AD-04
Avastin
Newly formed tubes
Mature tubes
CD31 staining
Newly formed tubes
Mature tubes
Tube length (microns)
DMSO
30nM AD-04
100nM AD-04
300nM AD-04
1µM AD-04
1µM ent AD-04
Avastin 0.25 mg/ml

Figure 26

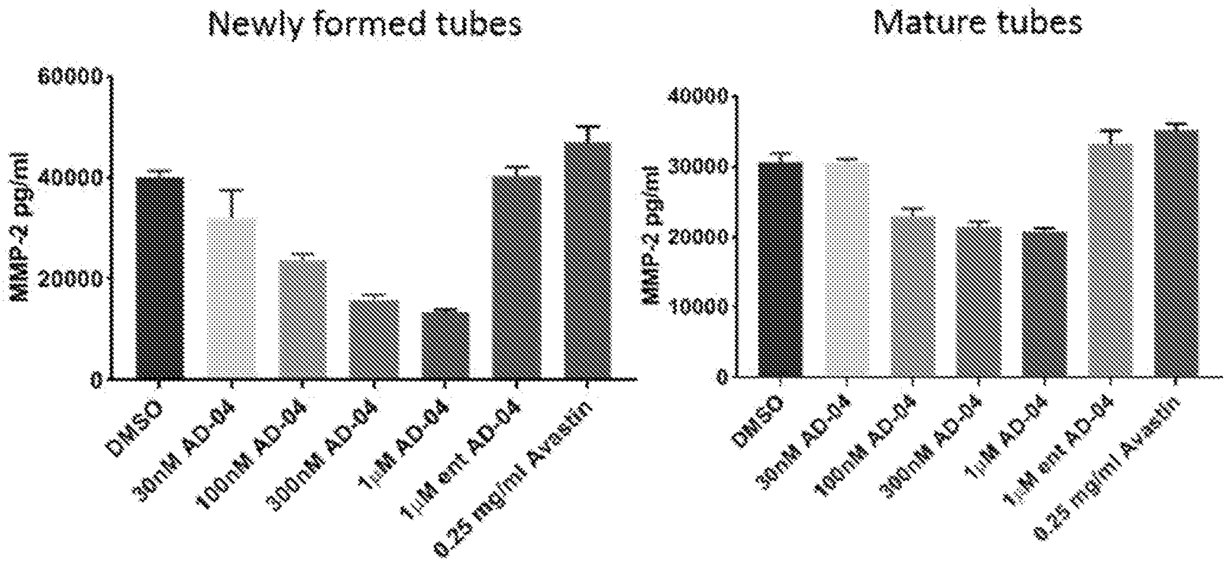


Figure 27

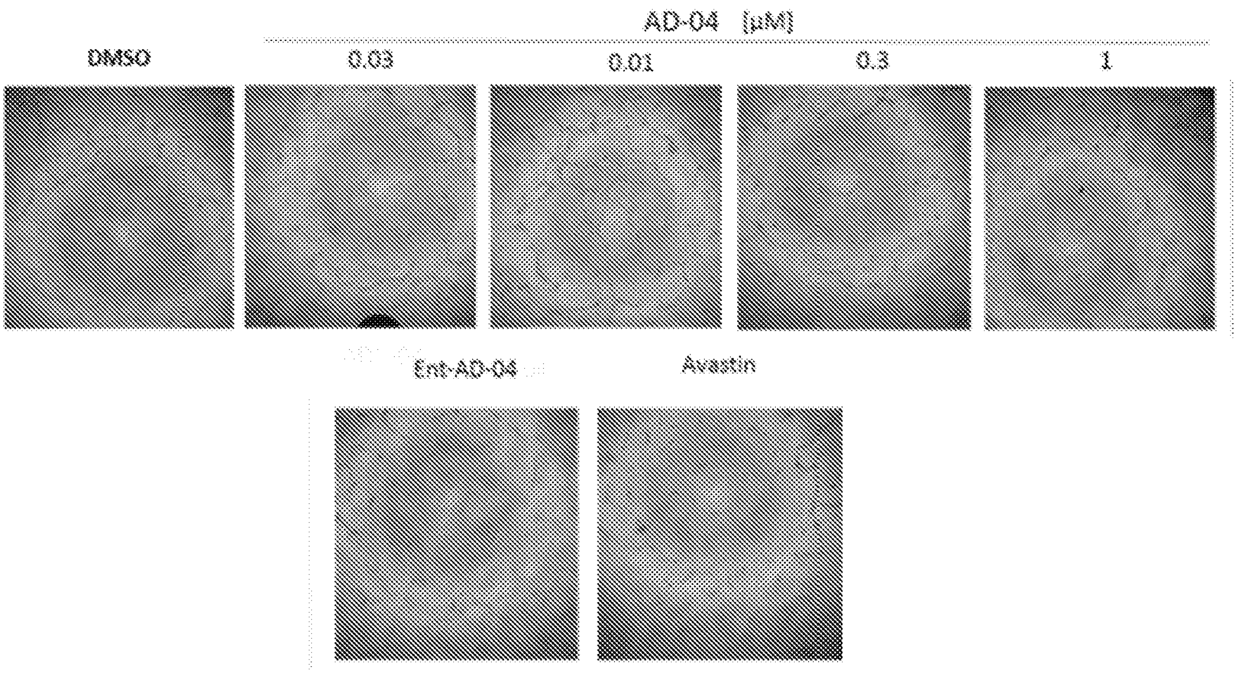


Figure 28

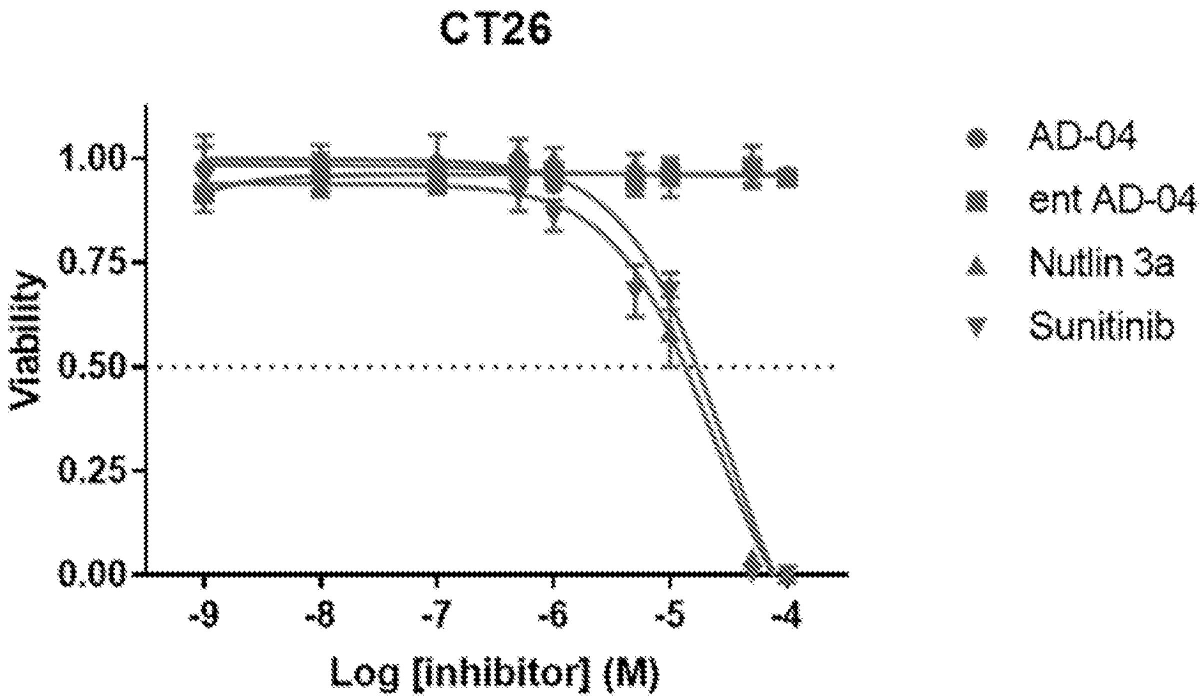
CT26
1.00
0.75
0.50
0.25
0.00
Viability
-9
-8
-7
-6
-5
-4
Log [inhibitor] (M)
AD-04
ent AD-04
Nutlin 3a
Sunitinib

Figure 29

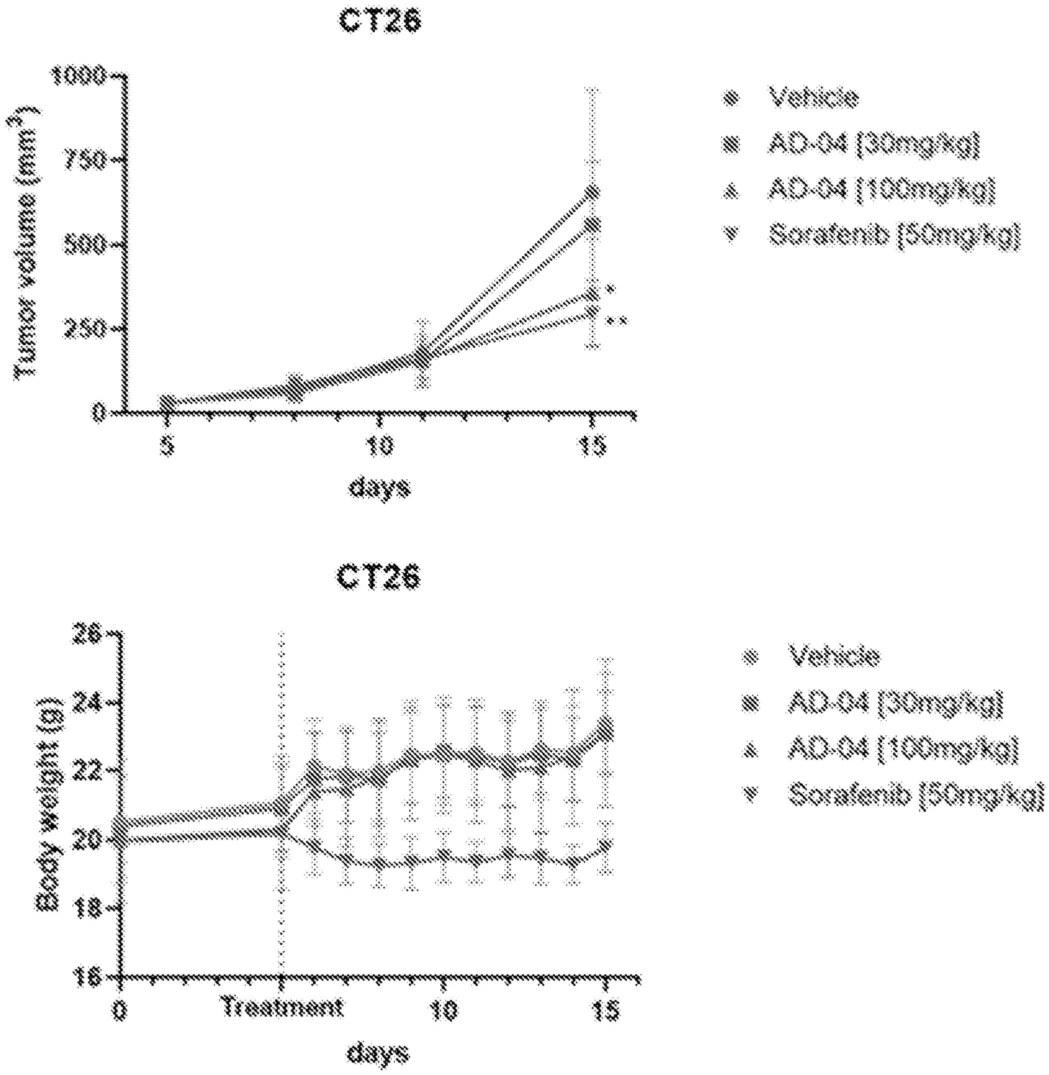
CT26
1000
750
500
250
0
Tumor volume (mm3)
5
10
15
days
*
**
Vehicle
AD-04 [30mg/kg]
AD-04 [100mg/kg]
Sorafenib [50mg/kg]
CT26
26
24
22
20
18
16
Body weight (g)
0
Treatment
10
15
days
Vehicle
AD-04 [30mg/kg]
AD-04 [100mg/kg]
Sorafenib [50mg/kg]

Figure 30

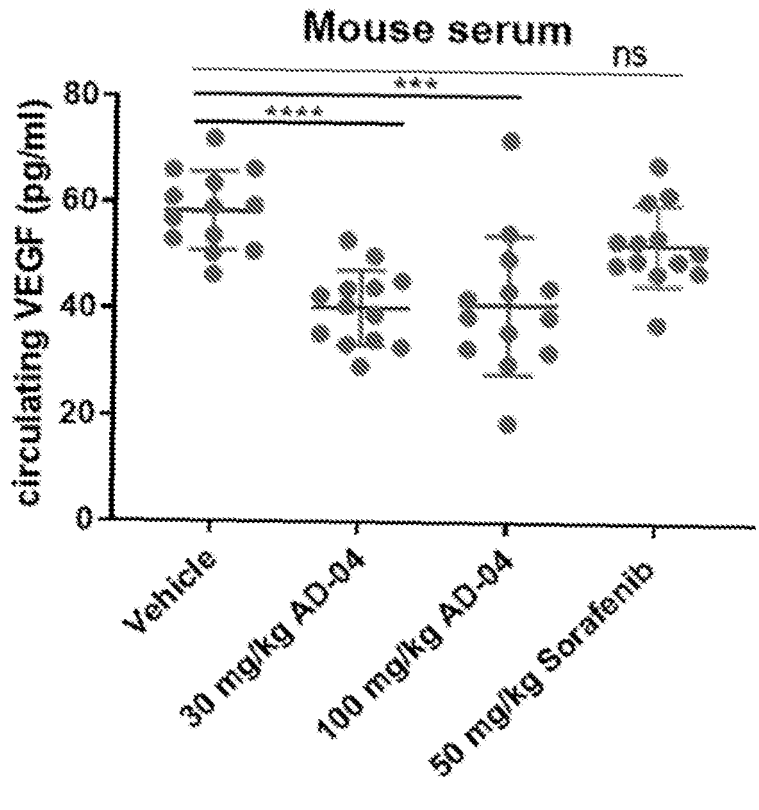
Mouse serum
ns
***
****
circulating VEGF (pg/ml)
80
60
40
20
0
Vehicle
30 mg/kg AD-04
100 mg/kg AD-04
50 mg/kg Sorafenib

Figure 31

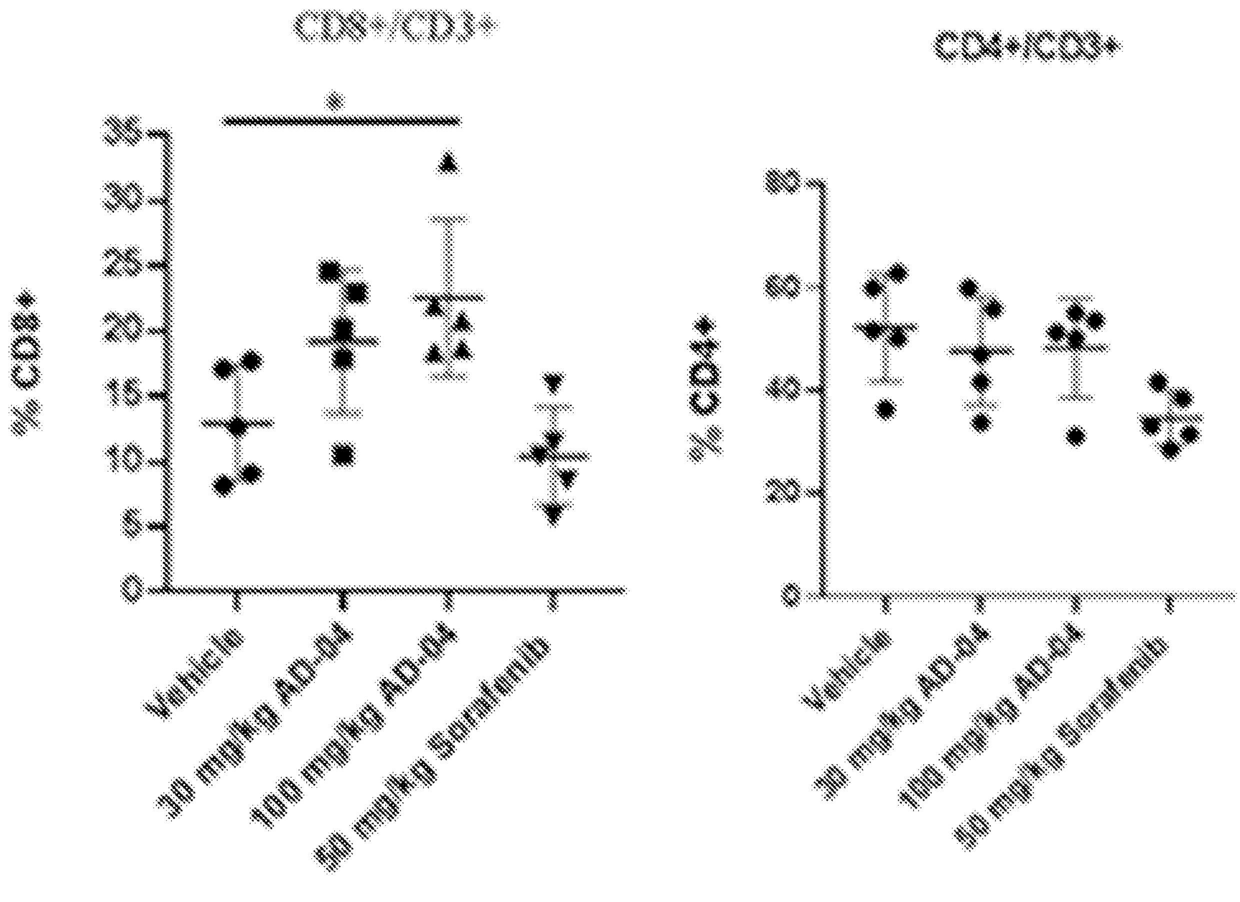
CD8+/CD3+
*
% CD8+
35
30
25
20
15
10
5
0
Vehicle
30 mg/kg AD-04
100 mg/kg AD-04
50 mg/kg Sorafenib
CD4+/CD3+
% CD4+
80
60
40
20
0
Vehicle
30 mg/kg AD-04
100 mg/kg AD-04
50 mg/kg Sorafenib

Figure 32

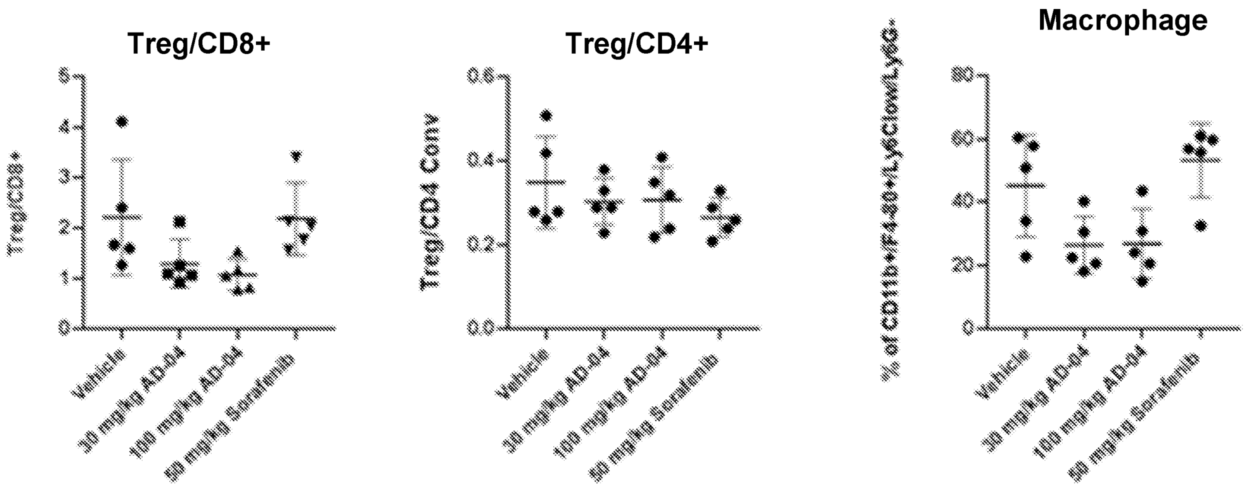


Figure 32 (continued)

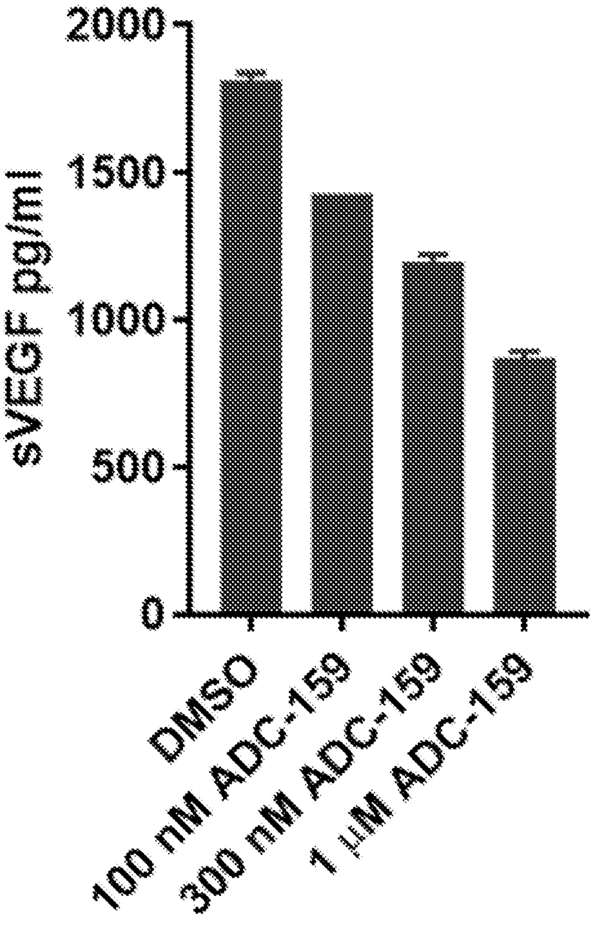
sVEGF pg/ml
2000
1500
1000
500
0
DMSO
100 nM ADC-159
300 nM ADC-159
1 μM ADC-159

Figure 33

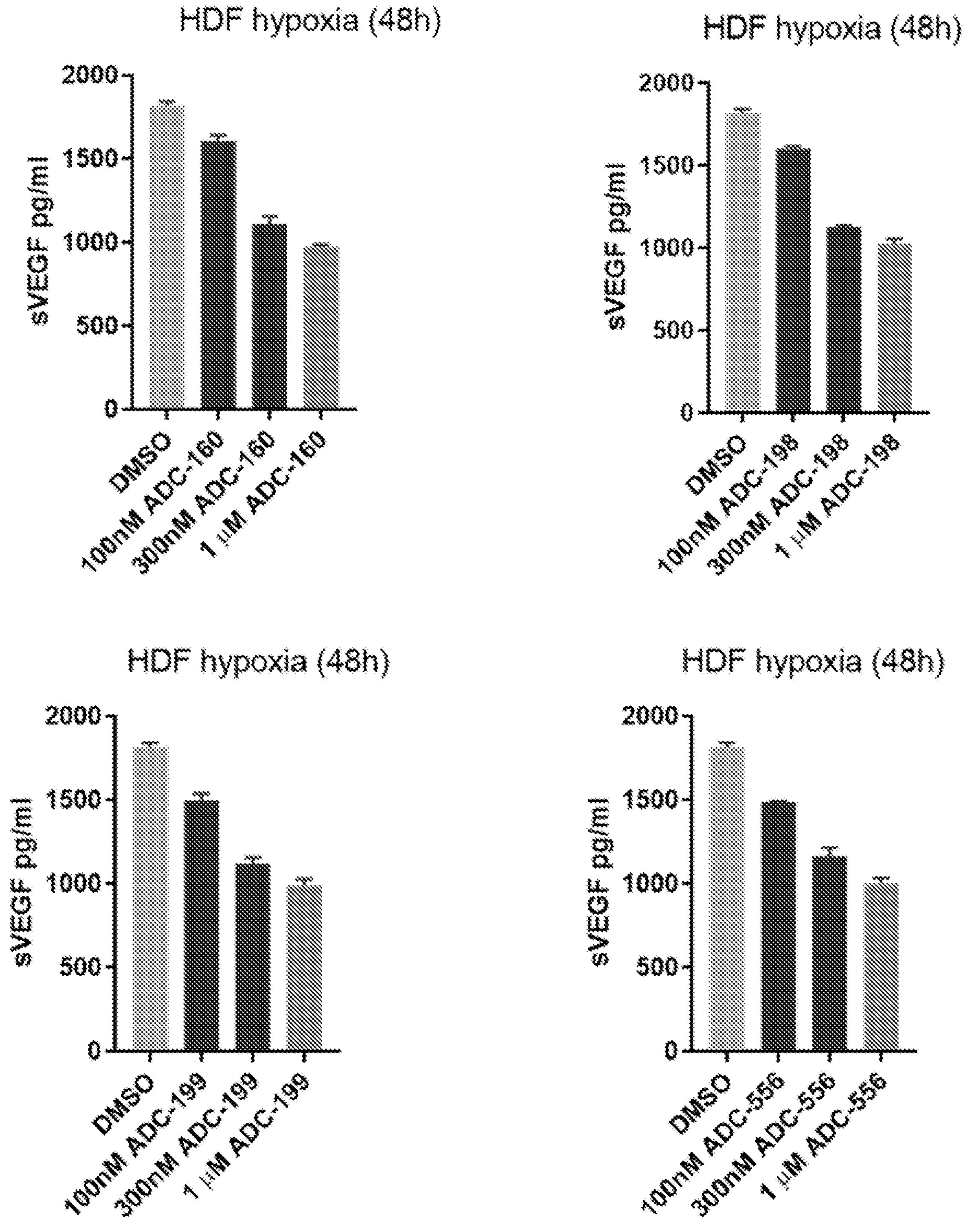
HDF hypoxia (48h)
sVEGF pg/ml
2000
1500
1000
500
0
DMSO
100nM ADC-160
300nM ADC-160
1 μM ADC-160
HDF hypoxia (48h)
sVEGF pg/ml
2000
1500
1000
500
0
DMSO
100nM ADC-198
300nM ADC-198
1 μM ADC-198
HDF hypoxia (48h)
sVEGF pg/ml
2000
1500
1000
500
0
DMSO
100nM ADC-199
300nM ADC-199
1 μM ADC-199
HDF hypoxia (48h)
sVEGF pg/ml
2000
1500
1000
500
0
DMSO
100nM ADC-556
300nM ADC-556
1 μM ADC-556

Figure 34

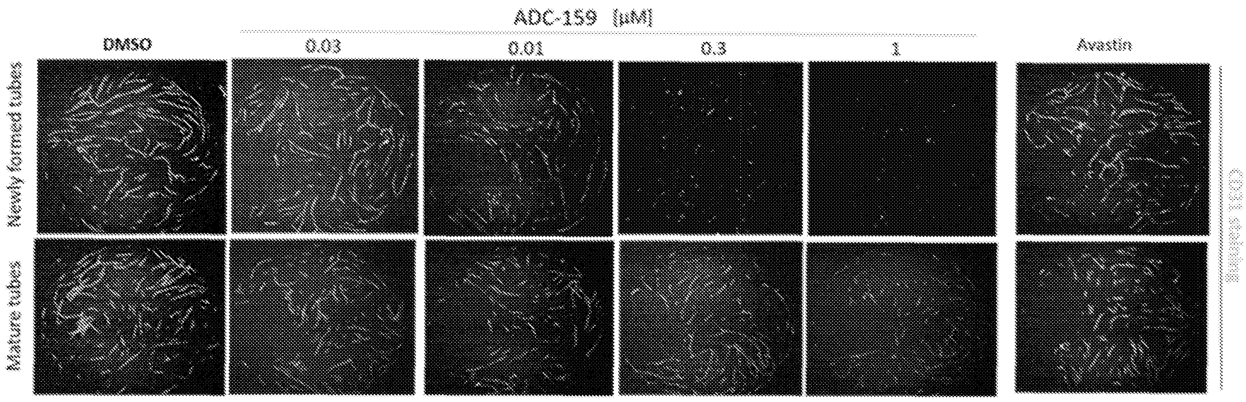
ADC-159 [µM]
DMSO
0.03
0.01
0.3
1
Avastin
Newly formed tubes
Mature tubes
CD31 staining

Figure 35A

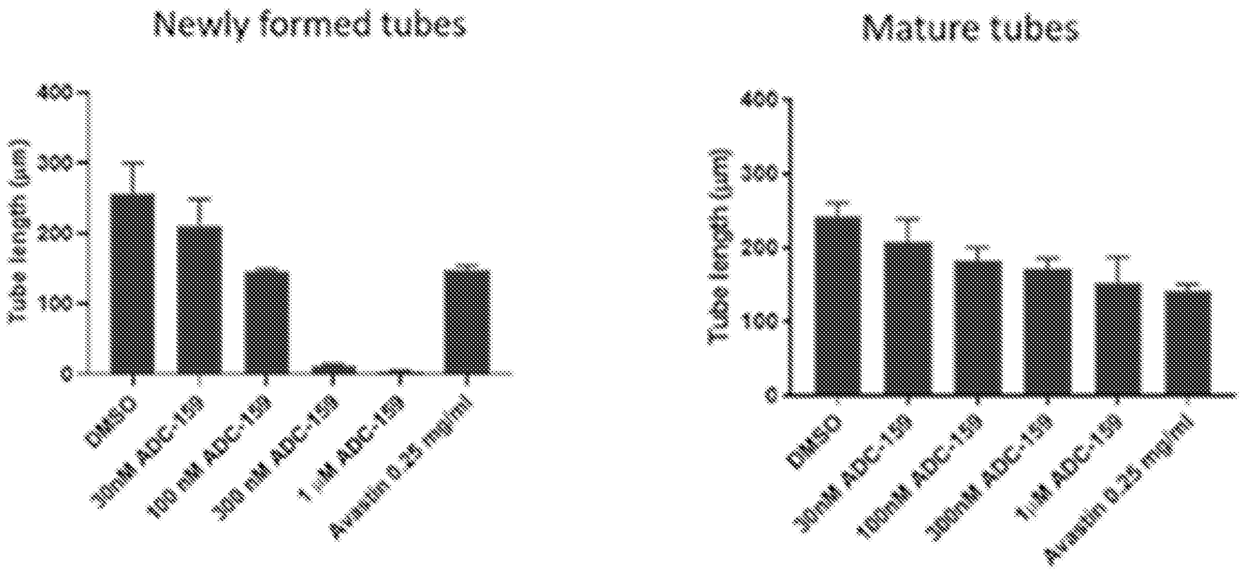
Newly formed tubes
Tube length (µm)
400
300
200
100
0
DMSO
30nM ADC-159
100 nM ADC-159
300 nM ADC-159
1 µM ADC-159
Avastin 0.25 mg/ml
Mature tubes
Tube length (µm)
400
300
200
100
0
DMSO
30nM ADC-159
100nM ADC-159
300nM ADC-159
1 µM ADC-159
Avastin 0.25 mg/ml

Figure 35B

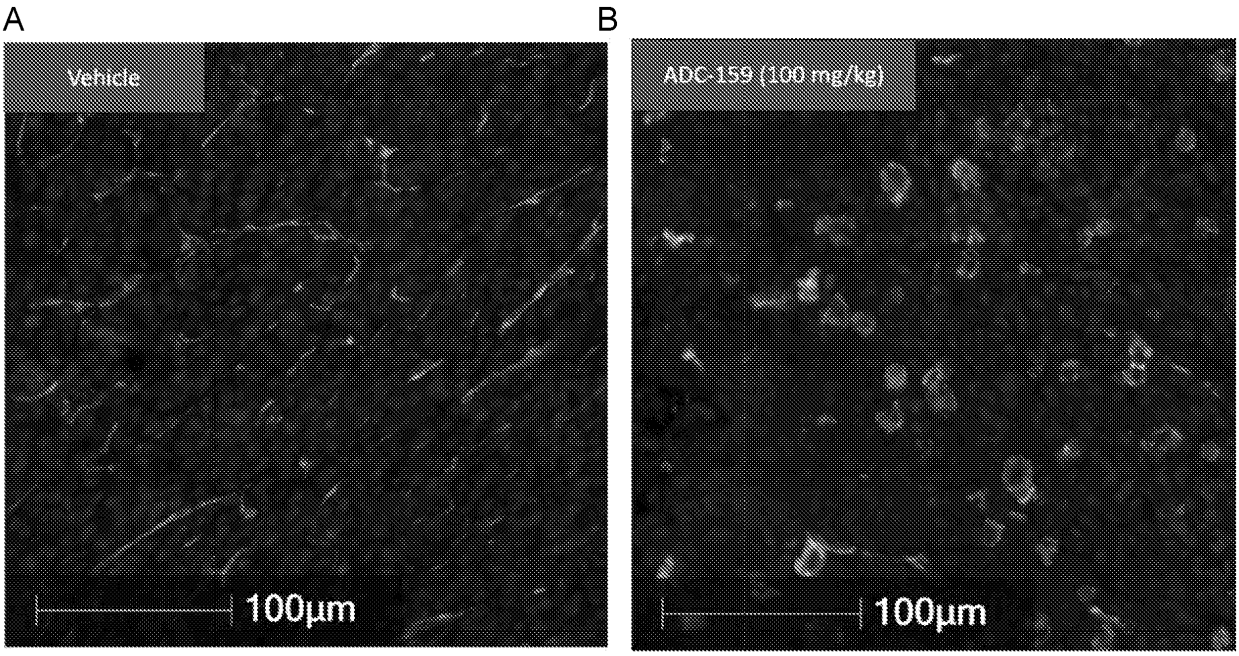


Figure 36

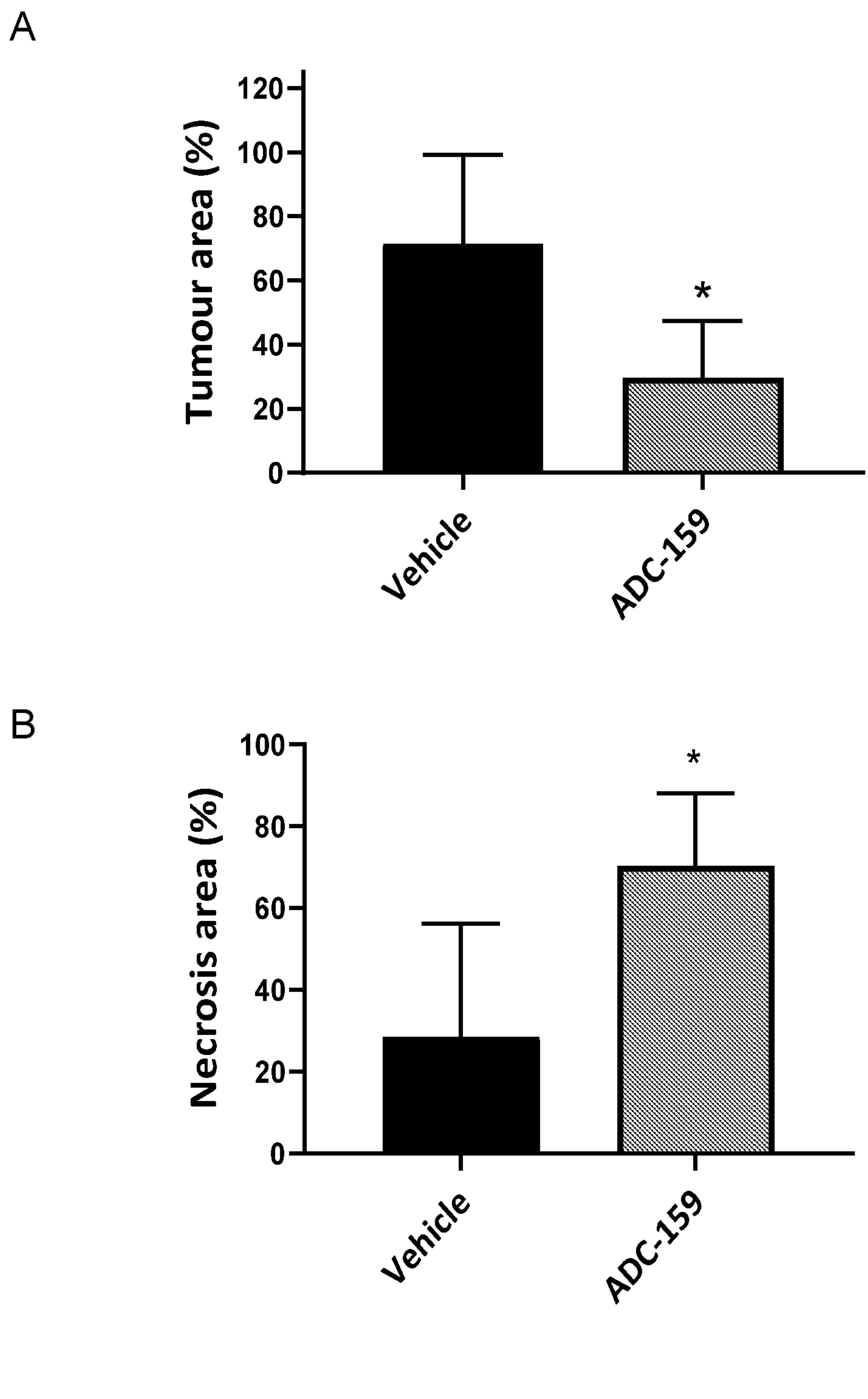
A
120
100
80
60
40
20
0
Tumour area (%)
*
Vehicle
ADC-159
B
100
80
60
40
20
0
Necrosis area (%)
*
Vehicle
ADC-159

Figure 37

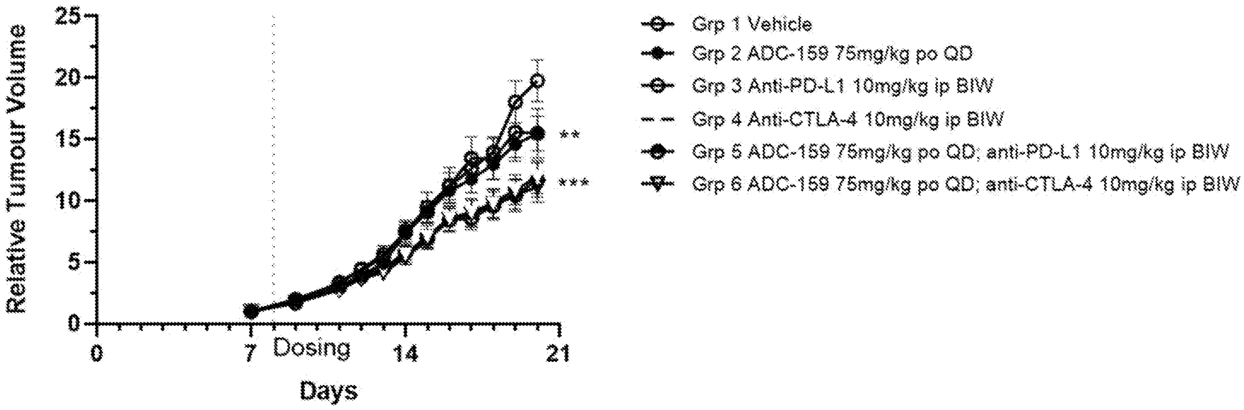
Relative Tumour Volume
25
20
15
10
5
0
0
7
Dosing
14
21
Days
**
***
Grp 1 Vehicle
Grp 2 ADC-159 75mg/kg po QD
Grp 3 Anti-PD-L1 10mg/kg ip BIW
Grp 4 Anti-CTLA-4 10mg/kg ip BIW
Grp 5 ADC-159 75mg/kg po QD; anti-PD-L1 10mg/kg ip BIW
Grp 6 ADC-159 75mg/kg po QD; anti-CTLA-4 10mg/kg ip BIW

Figure 38A

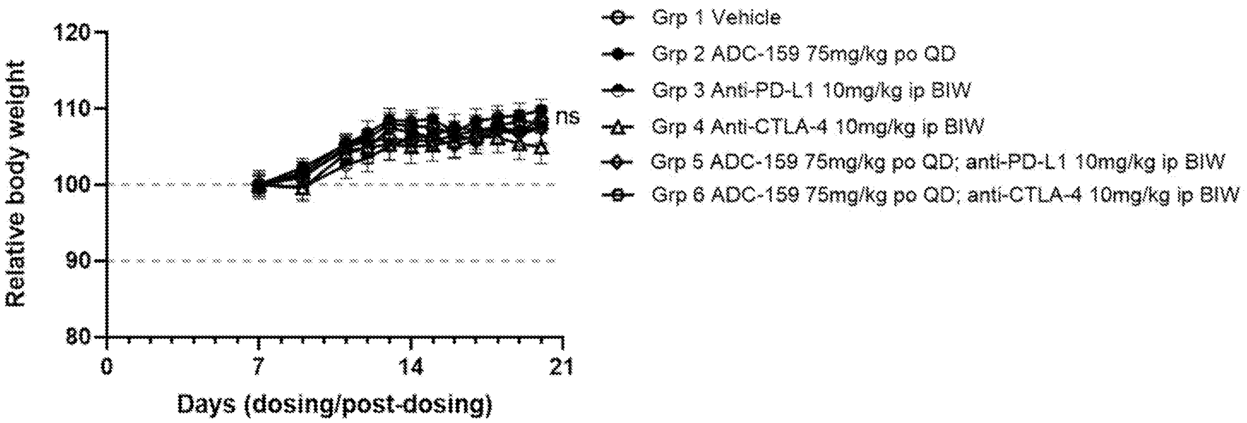
Relative body weight
120
110
100
90
80
0
7
14
21
Days (dosing/post-dosing)
ns
Grp 1 Vehicle
Grp 2 ADC-159 75mg/kg po QD
Grp 3 Anti-PD-L1 10mg/kg ip BIW
Grp 4 Anti-CTLA-4 10mg/kg ip BIW
Grp 5 ADC-159 75mg/kg po QD; anti-PD-L1 10mg/kg ip BIW
Grp 6 ADC-159 75mg/kg po QD; anti-CTLA-4 10mg/kg ip BIW

Figure 38B

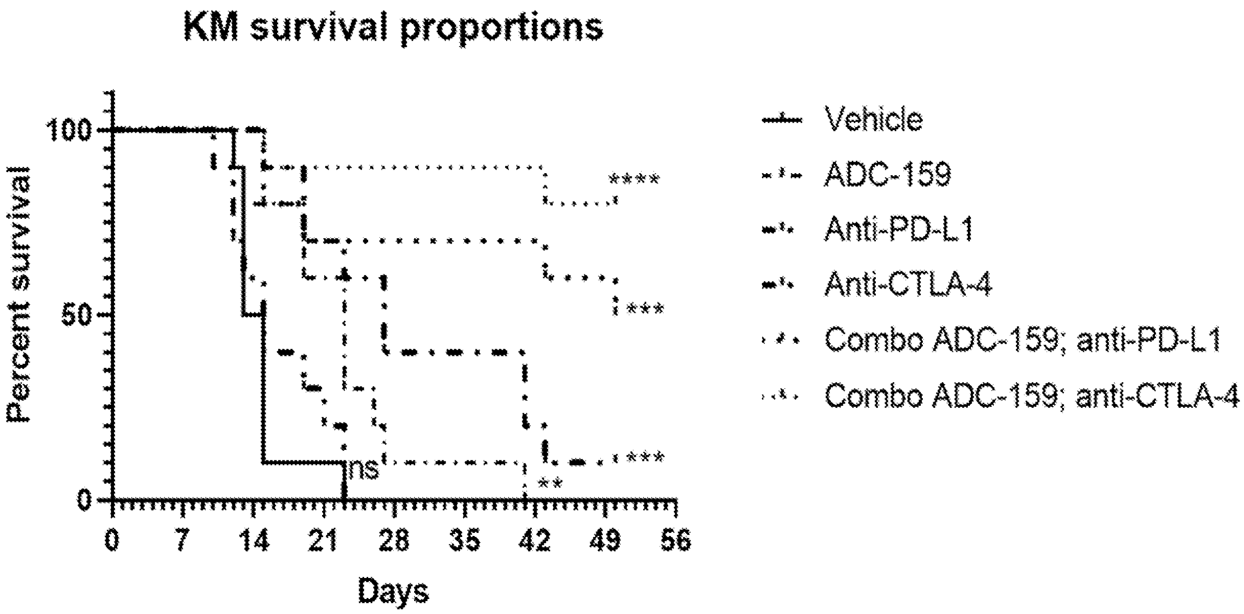
KM survival proportions
Percent survival
100
50
0
0
7
14
21
28
35
42
49
56
Days
****
***
***
**
ns
Vehicle
ADC-159
Anti-PD-L1
Anti-CTLA-4
Combo ADC-159; anti-PD-L1
Combo ADC-159; anti-CTLA-4

Figure 39A

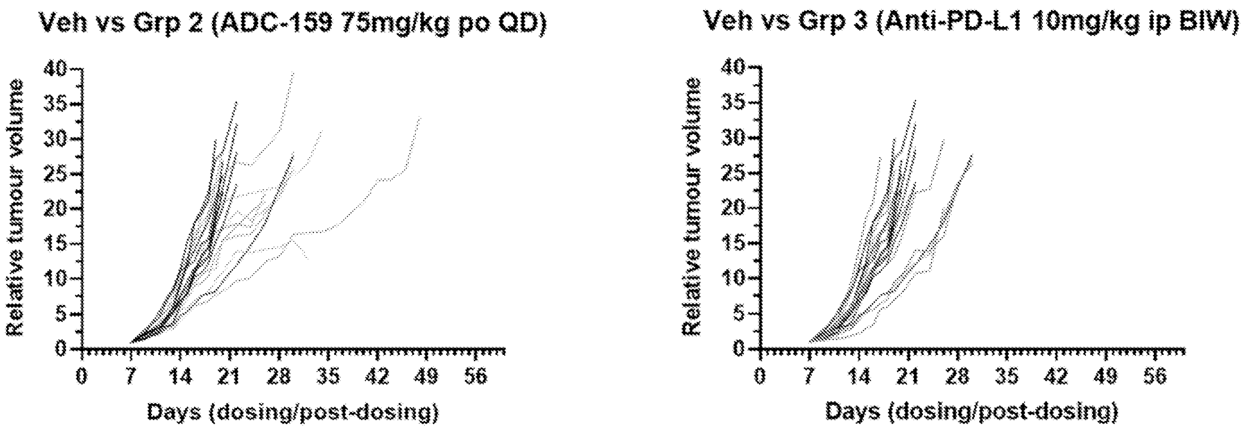
Veh vs Grp 2 (ADC-159 75mg/kg po QD)
Relative tumour volume
40
35
30
25
20
15
10
5
0
0 7 14 21 28 35 42 49 56
Days (dosing/post-dosing)
Veh vs Grp 3 (Anti-PD-L1 10mg/kg ip BIW)
Relative tumour volume
40
35
30
25
20
15
10
5
0
0 7 14 21 28 35 42 49 56
Days (dosing/post-dosing)

Figure 39B

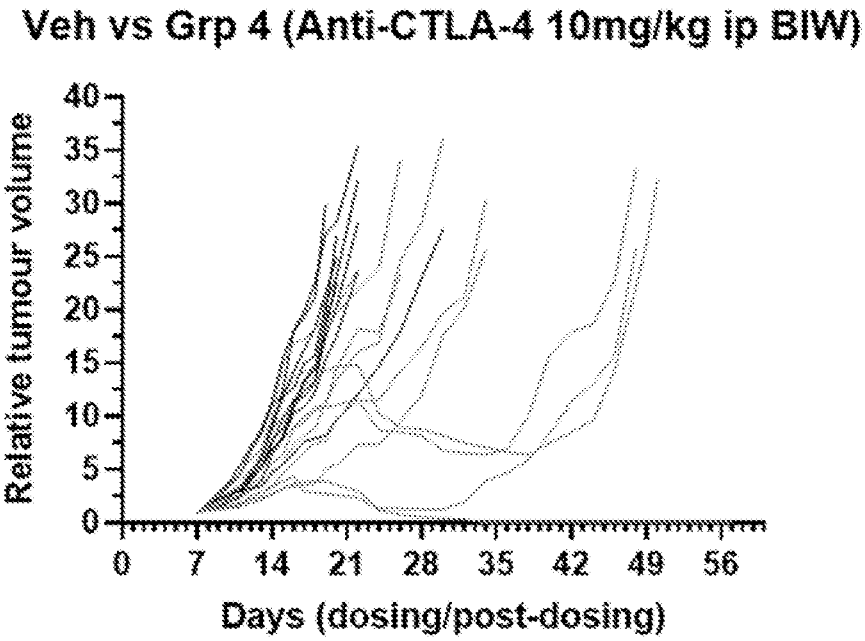


Figure 39B (continued)

Veh vs Grp 5 (ADC-159 75mg/kg po QD; anti-PD-L1 10mg/kg ip BIW)

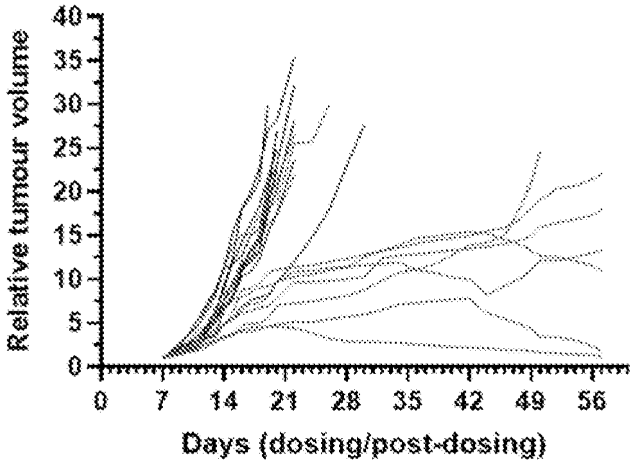


Veh vs Grp 6 (ADC-159 75mg/kg po QD; anti-CTLA-4 10mg/kg ip BIW)

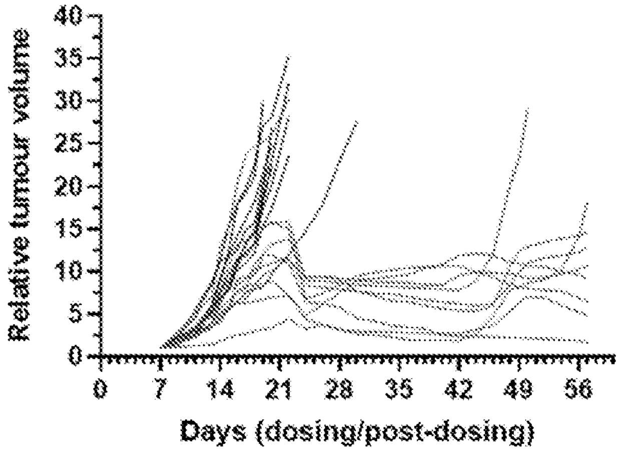


Figure 39B (continued)

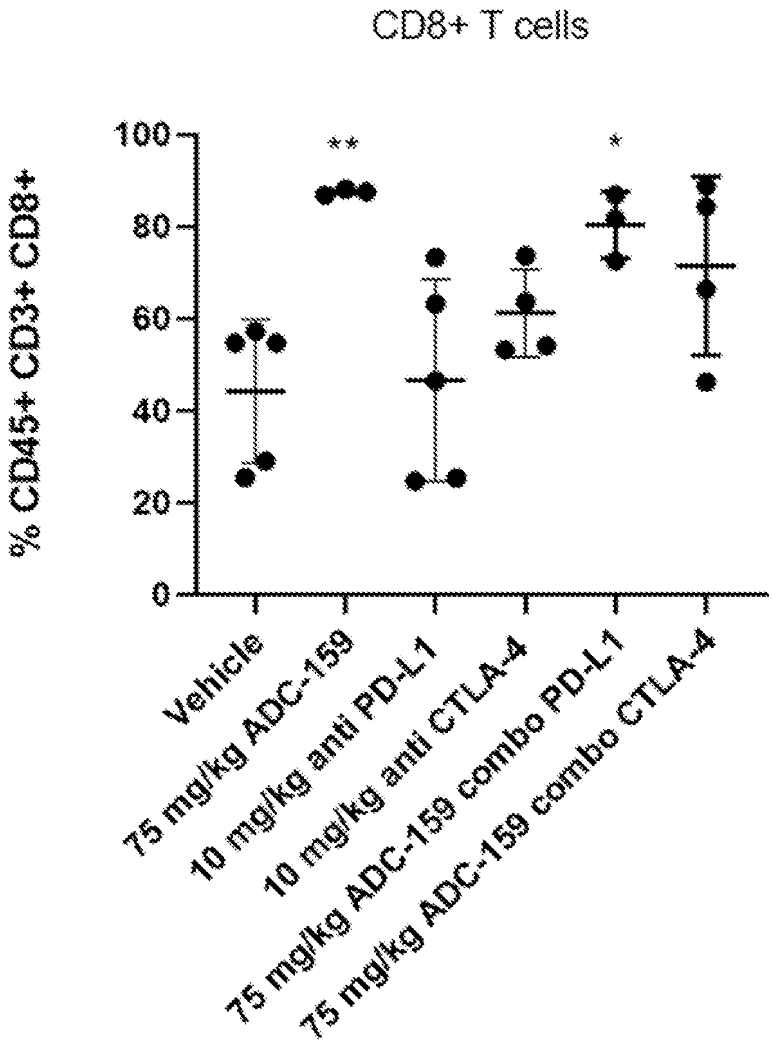
CD8+ T cells
% CD45+ CD3+ CD8+
100
80
60
40
20
0
**
*
Vehicle
75 mg/kg ADC-159
10 mg/kg anti PD-L1
10 mg/kg anti CTLA-4
75 mg/kg ADC-159 combo PD-L1
75 mg/kg ADC-159 combo CTLA-4

Figure 40

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018073602 A
- US 20080103149 A1
- WO 2010114881 A1
- WO 2010081783 A1
- WO 2011086178 A1
- WO 2013030218 A1
- EP 2565186 A1
- EP 1749822 A1
- WO 2016109515 A1
- WO 2016109480 A1
- WO 2016126929 A1
- WO 2016126926 A1
- WO 2016126935 A1
- WO 2016150800 A1
- WO 2017158381 A
- WO 2017158388 A
- WO 2017212010 A
- WO 2017212012 A
- US 20190142834 A
- WO 2018073602 A1


### Non-patent literature cited in the description


- *J. Org. Chem.*, 1997, vol. 62, 7512-7515